(19) Europäisches Patentamt
European Patent Office
Office européen des brevets

(11) EP 4 722 211 A1

(12) **EUROPEAN PATENT APPLICATION**
published in accordance with Art. 153(4) EPC

(43) Date of publication:
**08.04.2026 Bulletin 2026/15**

(21) Application number: **24810468.9**

(22) Date of filing: **23.05.2024**

(51) International Patent Classification (IPC):
***C07D 471/04** (2006.01)* ***A61K 31/437** (2006.01)*
***A61P 35/00** (2006.01)*

(52) Cooperative Patent Classification (CPC):
**A61K 31/437; A61P 35/00; C07D 471/04**

(86) International application number:
**PCT/CN2024/094997**

(87) International publication number:
**WO 2024/240231 (28.11.2024 Gazette 2024/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **24.05.2023 CN 202310593058**
**14.08.2023 CN 202311019921**
**28.09.2023 CN 202311275317**
**20.12.2023 CN 202311767590**

(71) Applicant: **Changchun Genescience Pharmaceutical Co., Ltd.**
**Changchun, Jilin 130012 (CN)**

(72) Inventors:
- **FU, Donglin**
  **Changchun, Jilin 130012 (CN)**
- **HU, Zhilong**
  **Changchun, Jilin 130012 (CN)**
- **HAN, Tian**
  **Changchun, Jilin 130012 (CN)**
- **SHEN, Guangyuan**
  **Changchun, Jilin 130012 (CN)**
- **ZHANG, Chen**
  **Changchun, Jilin 130012 (CN)**
- **FAN, Jingrong**
  **Changchun, Jilin 130012 (CN)**
- **HUANG, Yue**
  **Changchun, Jilin 130012 (CN)**
- **LU, Biao**
  **Changchun, Jilin 130012 (CN)**
- **YANG, Fanglong**
  **Changchun, Jilin 130012 (CN)**
- **WANG, Siqin**
  **Changchun, Jilin 130012 (CN)**
- **JIN, Lei**
  **Changchun, Jilin 130012 (CN)**

(74) Representative: **Gille Hrabal Partnerschaftsgesellschaft mbB**
**Patentanwälte**
**Brucknerstraße 20**
**40593 Düsseldorf (DE)**

# (54) FGFR2/3 SELECTIVE INHIBITOR, PHARMACEUTICAL COMPOSITION AND USE THEREOF

(57) Provided are a compound represented by formula (I) and a racemate, a stereoisomer, a tautomer, an isotope label, an oxynitride, a solvate, a polymorph, a metabolite, an ester, a prodrug, or a pharmaceutically acceptable salt thereof. The compound has a good FGFR2/3 inhibitory effect, and can be used for treating or preventing FGFR2/3-mediated 5 disorders and diseases, and preparing drugs for treating or preventing such disorders and diseases.

EP 4 722 211 A1

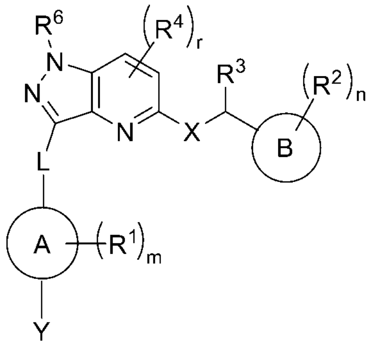

(I)

## Description

The present application claims:

priority to a prior application of Patent Application No. 202310593058.0 filed with the China National Intellectual Property Administration on May 24, 2023 and entitled "SELECTIVE FGFR3 INHIBITOR, PHARMACEUTICAL COMPOSITION, AND USE THEREOF";
priority to a prior application of Patent Application No. 202311019921.8 filed with the China National Intellectual Property Administration on August 14, 2023 and entitled "SELECTIVE FGFR3 INHIBITOR, PHARMACEUTICAL COMPOSITION, AND USE THEREOF";
priority to a prior application of Patent Application No. 202311275317.1 filed with the China National Intellectual Property Administration on September 28, 2023 and entitled "SELECTIVE FGFR3 INHIBITOR, PHARMACEUTICAL COMPOSITION, AND USE THEREOF"; and
priority to a prior application of Patent Application No. 202311767590.6 filed with the China National Intellectual Property Administration on December 20, 2023 and entitled "SELECTIVE FGFR3 INHIBITOR, PHARMACEUTICAL COMPOSITION, AND USE THEREOF";
and the above prior applications are incorporated herein by reference in their entireties.

## TECHNICAL FIELD

The present invention relates to the field of medicine technology, and specifically to a selective FGFR2/3 inhibitor, a pharmaceutical composition, and use thereof.

## BACKGROUND

Fibroblast growth factor receptor (FGFR), an important member of tyrosine kinase receptor family, is a tyrosine kinase receptor having about 800 amino acids and comprising three extracellular immunoglobulin-like domains (I/II/III), a single transmembrane domain and a cytoplasmic tyrosine kinase domain. In humans, FGFRs comprise four typical tyrosine kinase receptors (FGFR 1-4) and one FGFR5 lacking the intracellular tyrosine kinase domain. The ligands of FGFRs, fibroblast growth factor (FGF), comprise 18 members. Under normal physiological conditions, FGFR binds to its ligand, fibroblast growth factor, and FGFR is dimerized and phosphorylated. When FGF binds to FGFR, the signaling pathway is activated and amplified, to activate the downstream signaling pathway, such as JAK/STAT pathway, phospholipase C pathway, phosphoinositide-3- kinase PI3K and MAPK signaling pathway (Turner, N., Grose, R., Nat. Ref. Cancer 2010; 10: 116-129; Brooks, N.S. et al., Clin Cancer Res. 2012; 18: 18551862; and Dienstmann, R. et al., Ann. Oncol. 2014; 25:552-563).

In 2015, the journal of Clinical Cancer Research published the mutations of FGFRs in samples from 4853 patients with various cancers described by a new generation of sequencing technology, comprising mutations, amplifications and rearrangements. Among 4853 cancers sequenced, 360 FGFR mutations were observed in 343 cases (17 cancers had multiple FGFR variations) in the research, with a total incidence of 7.1% (Helsten T, Elkin S, Arthur E, Tomson BN, Carter J, Kurzrock R. The FGFR Landscape in Cancer: Analysis of 4,853 Tumors by Next-Generation Sequencing. Clin Cancer Res. 2016;22(1):259-267. doi:10.1158/1078-0432.CCR-14-3212). The components of FGFR signal transduction often change in human cancers, and some pre-clinical models provide striking evidence about the carcinogenic potential of abnormal FGFR signal transduction in carcinogenesis, thus confirming that FGFR signal transduction is an attractive target for cancer treatment.

Fibroblast growth factor receptor 3 (FGFR3) is a transmembrane tyrosine kinase receptor protein that plays an important role in cartilage development and cartilage homeostasis maintenance. FGFR3 is considered as a negative regulatory molecule in the development of endochondral bone. It is initially expressed in chondrocytes at the mesenchymal aggregation center in the early stage of bone development, and then expressed in chondrocytes of proliferative zone and prehypertrophic zone in growth plate cartilage and articular cartilage. Mutation of human FGFR3 gene will lead to a series of skeletal deformities.

The enhanced point mutation of FGFR3 leads to skeletal dysplasia with short stature as a clinical manifestation, comprising thanatophoric dysplasia (TD I/II) and achondroplasia (ACH). However, the loss-of-function point mutation of human FGFR3 will cause CATSHL syndrome, that is, hearing loss, hypomegasoma and camptodactilia.

Tyrosine kinase inhibitors can be divided into non-covalent inhibitors and covalent inhibitors. The non-covalent inhibitors further comprise multi-target or selective inhibitors. The non-covalent multi-target FGFR inhibitors comprise, for example, Dovitinib, Nintedanib, Lenvatinib, Ponatinib, derazantinib and e -7090, which have activities on FGFR, VEGFR, PDGFR (platelet-derived growth factor receptor) and other kinase proteins. The multi-target TKIs (tyrosine kinase inhibitors) have shown clinical benefits. Ponatinib and Nintedanib were approved for treatment of myeloid leukemia

and non-small cell lung cancer in 2012 and 2014 respectively. The toxicity of multi-target FGFR inhibitors is related to the inhibition on various kinases, especially the inhibition on VEGFRs, which limits the therapeutic dose. Therefore, more selective non-covalent FGFR inhibitors are developed. These drugs comprise AZD4547, Infigatinib, PD173074, LY2874455, Debio1347, ASP5878, and Rogaratinib. Their selectivity for FGFR1-3 is higher than those for VEGFR and other kinases (Marseglia G, Lodola A, Mor M, Castelli R. Expert Opin Ther Pat. 2019 Dec;29(12):965-977). These compounds have been proved to be effective in FGFR-dependent cancers in clinical trials, but there are toxic and side effects such as hyperphosphatemia caused by FGFR1. Besides hyperphosphatemia and diarrhea, the FGFR inhibitors also have common clinical side effects such as fatigue, skin toxicity such as hand-foot syndrome, hair loss, nail bed infection, onychomycosis, dry skin and dry mouth, and usually cause dysgeusia (Kommalapati A, Tella SH, Borad M, Javle M, Mahipal A. Cancers (Basel). 2021 Jun 13;13(12):2968).

To sum up, the FGFR signaling pathway plays an important role in human cancers and bone development and is an attractive therapeutic target. However, pan-FGFR inhibitors have major toxic and side effects such as hyperphosphatemia and diarrhea. There are an unmet clinical need and an urgent need to develop a highly selective FGFR inhibitor.

## SUMMARY

To solve the above technical problems, the present invention provides a compound of Formula (I), and a racemate, a stereoisomer, a tautomer, an isotopically labeled compound, an N-oxide, a solvate, a polymorph, a metabolite, an ester, a prodrug or a pharmaceutically acceptable salt thereof:

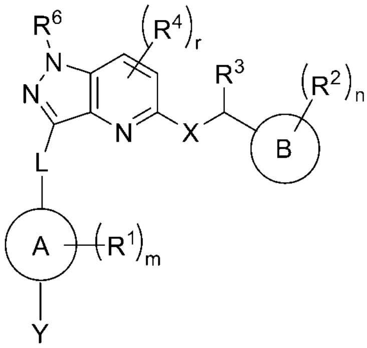

Formula (I)

in which:

Ring A and Ring B are the same or different and are each independently selected from a $C_{6-14}$ aromatic ring, a 5-to 14-membered heteroaromatic ring or a 5- to 14-membered heterocyclic ring;

L is absent or selected from -N($R^a$)-C(=O)- or -$CR^b$=$CR^c$-; and when L is absent, Ring A is directly attached to the pyrazole ring via a chemical bond;

$R^a$ is selected from hydrogen, or the following groups which are unsubstituted or optionally substituted with one, two or more $R^{a1}$: a $C_{1-6}$ alkyl and a $C_{3-6}$ cycloalkyl, where each $R^{a1}$ is the same or different, and independently selected from hydroxyl, cyano, halogen, a $C_{1-6}$ alkyl, a $C_{1-6}$ alkoxy, and a $C_{3-6}$ cycloalkyl;

$R^b$ and $R^c$ are the same or different, and each independently selected from hydrogen, halogen, or the following groups which are unsubstituted or optionally substituted with one, two or more $R^{b1}$: a $C_{1-6}$ alkyl, a $C_{1-6}$ alkoxy, and a $C_{3-6}$ cycloalkyl, where each $R^{b1}$ is the same or different, and independently selected from hydroxyl, cyano, halogen, a $C_{1-6}$ alkyl, a $C_{1-6}$ alkoxy, and a $C_{3-6}$ cycloalkyl;

X is selected from O, S or NH;

Y is absent, or selected from the following groups which are unsubstituted or optionally substituted with one, two or more $R^y$: -S(=O)-$R^{y1}$, -S(=O)$_2$-$R^{y2}$, -S(=O)(=N$R^{y3}$)-$R^{y4}$, a $C_{3-12}$ cycloalkyl, a 3- to 14-membered heterocyclyl, a $C_{6-14}$ aryl, and a 5- to 14-membered heteroaryl, where $R^{y1}$, $R^{y2}$, $R^{y3}$, and $R^{y4}$ are the same or different, and are absent or each independently selected from hydrogen, a $C_{1-6}$ alkyl, or a $C_{3-6}$ cycloalkyl; each $R^y$ is the same or different, and independently selected from hydrogen, hydroxyl, cyano, halogen, oxo (=O), or the following groups which are unsubstituted or optionally substituted with one, two or more $R^{y'}$: a $C_{1-6}$ alkyl, a $C_{1-6}$ alkoxy, a $C_{3-6}$ cycloalkyl, a 3- to 8-membered heterocyclyl, a $C_{6-14}$ aryl, a 5- to 14-membered heteroaryl, =N-$R^{y5}$, -C(=O)-$R^{y6}$, -q=O)O-$R^{y7}$, -S(=O)$_2$-$R^{y8}$, -S(=O)(=N$R^{y9}$)-$R^{y10}$, and -P(=O)($R^{y11}$)($R^{y12}$), in which $R^{y5}$, $R^{y6}$, $R^{y7}$, $R^{y8}$, $R^{y9}$, $R^{y10}$, $R^{y11}$, and $R^{y12}$ are the same or different, and each independently selected from hydrogen, a $C_{1-6}$ alkyl, and a $C_{3-6}$ cycloalkyl; each $R^{y'}$ is the same or different, and independently selected from hydrogen, hydroxyl, cyano, halogen, oxo (=O), or the following groups which are unsubstituted or optionally substituted with one, two or more $R^{y''}$: a $C_{1-6}$ alkyl, a $C_{1-6}$ alkoxy, a $C_{3-6}$ cycloalkyl,

a 3- to 8-membered heterocyclyl, -C(=O)-$NR^{y13}R^{y14}$, -C(=O)-$R^{y15}$, -C(=O)O-$R^{y16}$, -$OR^{y17}$, -$S(=O)_2$-$R^{y18}$, -$S(=O)_2$-$NH_2$, -S(=O)(=$NR^{y19}$)-$R^{y20}$, -P(=O)($R^{y21}$)($R^{y22}$), and amino, where each $R^{y''}$ is the same or different, and independently selected from hydrogen, hydroxyl, cyano, halogen, oxo (=O), amino, a $C_{1-6}$ alkyl, a $C_{1-6}$ alkoxy, a $C_{3-6}$ cycloalkyl, a $C_{3-6}$ cycloalkyl, a 3- to 8-membered heterocyclyl, -C(=O)-$NR^{y23}R^{y24}$, -C(=O)-$R^{y25}$, -C(=O)O-$R^{y26}$, -$OR^{y27}$, -$S(=O)_2$-$R^{y28}$, -S(=O)(=$NR^{y29}$)-$R^{y30}$, and -P(=O)($R^{y31}$)($R^{y32}$), where $R^{y13}$, $R^{y14}$, $R^{y15}$, $R^{y16}$, $R^{y17}$, $R^{y18}$, $R^{y19}$, $R^{y20}$, $R^{y21}$, $R^{y22}$, $R^{y23}$, $R^{y24}$, $R^{y25}$, $R^{y26}$, $R^{y27}$, $R^{y28}$, $R^{y29}$, $R^{y30}$, $R^{y31}$, and $R^{y32}$ are the same or different, and each independently selected from hydrogen, a $C_{1-6}$ alkyl, or a $C_{3-6}$ cycloalkyl;

each $R^1$ is the same or different, and independently selected from hydrogen, halogen, cyano, hydroxyl, oxo (=O), or the following groups which are unsubstituted or optionally substituted with one, two or more $R^{11}$: amino, a $C_{1-6}$ alkyl, a $C_{1-6}$ alkoxy, a $C_{3-8}$ cycloalkyl, a 3- to 8-membered heterocyclyl, -C(=O)-$NH_2$, -$S(=O)_2$-$C_{1-6}$ alkyl, and - S(=O)(=NH)-$C_{1-6}$ alkyl; or two $R^1$ attached to the same atom, together with the atom to which they are attached, form a 3- to 12-membered heterocyclic ring or a $C_{3-12}$ alkyl ring that is unsubstituted or optionally substituted with one, two or more $R^{11}$; or two $R^1$ attached to different atoms, together with the atoms to which they are respectively attached, form a 3- to 12-membered heterocyclic ring or a $C_{3-12}$ alkyl ring that is unsubstituted or optionally substituted with one, two or more $R^{11}$, where each $R^{11}$ is the same or different, and independently selected from H, cyano, oxo (=O), halogen, or the following groups which are unsubstituted or optionally substituted with one, two or more $R^{12}$: a $C_{1-6}$ alkyl, a $C_{1-6}$ alkoxy, -$S(=O)_2$-$R^{y8}$, -S(=O)(=$NR^{y9}$)-$R^{y10}$, a $C_{3-12}$ cycloalkyl, a 3- to 12-membered heterocyclyl, and a 5-to 14-membered heteroaryl, in which each $R^{12}$ is the same or different, and independently selected from hydrogen, hydroxyl, cyano, halogen, a $C_{1-6}$ alkyl, a $C_{1-6}$ alkoxy, a $C_{3-6}$ cycloalkyl, a $C_{6-14}$ aryl, or a 5- to 14-membered heteroaryl;

each $R^2$ and $R^4$ are the same or different, and independently selected from hydrogen, halogen, cyano, hydroxyl, oxo (=O), or the following groups which are unsubstituted or optionally substituted with one, two or more $R^{21}$: amino, a $C_{1-6}$ alkyl, a $C_{1-6}$ alkoxy, a $C_{3-8}$ cycloalkyl, a 3- to 8-membered heterocyclyl, -C(=O)-$NH_2$, -$S(=O)_2$-$C_{1-6}$ alkyl, a $C_{6-14}$ aryl, and a 5- to 14-membered heteroaryl, where each $R^{21}$ is the same or different, and independently selected from halogen, CN, amino, hydroxyl, oxo (=O), a $C_{1-6}$ alkyl, or a $C_{1-6}$ alkoxy;

$R^3$ is selected from hydrogen, a $C_{1-6}$ alkyl, a $C_{1-6}$ haloalkyl, or a cyano-$C_{1-6}$ alkyl;

$R^6$ is selected from hydrogen, or a $R^{6a}$-$C_{1-4}$ alkyl, where $R^{6a}$ is selected from

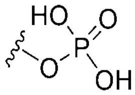 ;

m and n are the same or different, and each independently selected from 0, 1, 2, 3, 4, 5 or 6; and

r is selected from 0, 1 or 2.

According to some embodiments, Ring A is selected from a $C_{6-10}$ aromatic ring, a 5- to 10-membered heteroaromatic ring, or a 5- to 10-membered heterocyclic ring.

According to some embodiments, Ring A is selected from a benzene ring, a pyridine ring, a pyrimidine ring, a naphthalene ring, a quinoline ring, a 1,8-naphthyridine ring, a piperidine ring, a piperazine ring,

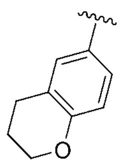 ,

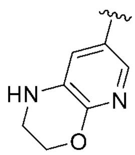 , 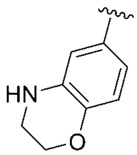 , 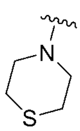 , 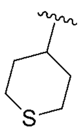 , or 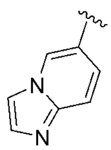 .

According to some embodiments, Ring B is selected from a benzene ring or a 5- to 6-membered heteroaromatic ring.

According to some embodiments, Ring B is selected from a pyridine ring or a pyridazine ring.

According to some embodiments, L is absent or selected from -NH-C(=O)- or -CH=CH-.

According to some embodiments, X is selected from O.

According to some embodiments, Y is absent.

According to some embodiments, Y is selected from the following groups which are unsubstituted or optionally substituted with one, two or more $R^y$: -S(=O)-$R^{y1}$, -S(=O)$_2$-$R^{y2}$, -S(=O)(=N$R^{y3}$)-$R^{y4}$, a $C_{3-10}$ cycloalkyl, a 3- to 10-membered heterocyclyl, and a 5- to 8-membered heteroaryl, where $R^{y1}$, $R^{y2}$, $R^{y3}$, and $R^{y4}$ are the same or different, and are absent or each independently selected from hydrogen, a $C_{1-6}$ alkyl, or a $C_{3-6}$ cycloalkyl.

According to some embodiments, Y is selected from the following groups which are unsubstituted or optionally substituted with one, two or more $R^y$: phenyl, piperazinyl, piperidyl, pyrazolyl,

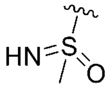, 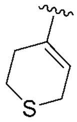, 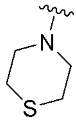, 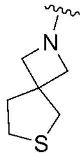,

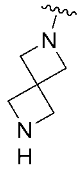, 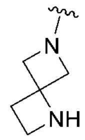, 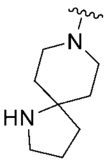, 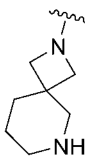, 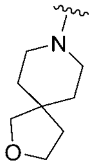, 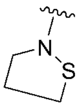, 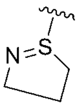, 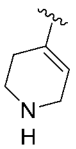, 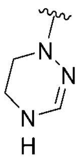, 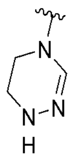, 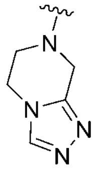 or 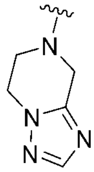.

According to some embodiments, each $R^y$ is the same or different, and independently selected from hydrogen, hydroxyl, cyano, halogen, oxo (=O), a $C_{1-6}$ alkyl, a $C_{1-6}$ haloalkyl, a hydroxy-$C_{1-6}$ alkyl, an amino-$C_{1-6}$ alkyl, a cyano-$C_{1-6}$ alkyl, a carboxy-$C_{1-6}$ alkyl, a $C_{1-6}$ alkyl-NH-$C_{1-6}$ alkyl, a ($C_{1-6}$ alkyl)$_2$N-$C_{1-6}$ alkyl, a $C_{1-6}$ alkoxy, a $C_{3-6}$ cycloalkyl, a -$C_{1-6}$ alkyl-(hydroxy substituted $C_{3-8}$ cycloalkyl), a -$C_{1-6}$ alkyl-S(=O)$_2$-$C_{1-6}$ alkyl, a -$C_{1-6}$ alkyl-S(=O)$_2$-NH$_2$, a $C_{6-10}$ aryl, a 5- to 10-membered heteroaryl, -N-$R^{y5}$, -C(=O)-$R^{y6}$, -C(=O)O-$R^{y7}$, -S(=O)$_2$-$R^{y8}$, -S(-O)(-N$R^{y9}$)-$R^{y10}$, - P(=O)($R^{y11}$)($R^{y12}$), or a 3- to 8-membered heterocyclyl that is optionally substituted with one, two or more $R^{y'}$, where each $R^{y'}$ is the same or different, and independently selected from cyano, halogen, oxo (=O) or a -S(=O)$_2$-$C_{1-6}$ alkyl; and $R^{y5}$, $R^{y6}$, $R^{y7}$, $R^{y8}$, $R^{y9}$, $R^{y10}$, $R^{y11}$, and $R^{y12}$ are the same or different and each independently selected from hydrogen, a $C_{1-6}$ alkyl, or a $C_{3-6}$ cycloalkyl.

According to some embodiments, each $R^y$ is the same or different and independently selected from hydroxyl, cyano, halogen, oxo (=O), a $C_{1-3}$ alkyl, a $C_{1-3}$ haloalkyl, a hydroxyl-$C_{1-4}$ alkyl, an amino-$C_{1-4}$ alkyl, a cyano-$C_{1-3}$ alkyl, a carboxy-$C_{1-4}$ alkyl, a $C_{1-4}$ alkyl-NH-$C_{1-4}$ alkyl, a ($C_{1-4}$ alkyl)$_2$N-$C_{1-4}$ alkyl, a $C_{1-3}$ alkoxy, a $C_{3-6}$ cycloalkyl, a -$C_{1-3}$ alkyl-(hydroxy substituted $C_{3-6}$ cycloalkyl), a -$C_{1-3}$ alkyl-S(=O)$_2$-$C_{1-3}$ alkyl, a -$C_{1-3}$ alkyl-S(=O)$_2$-NH$_2$, phenyl, a 5- to 6-membered heteroaryl, =N-$R^{y5}$, -C(=O)-$R^{y6}$, -C(=O)O-$R^{y7}$, -S(=O)$_2$-$R^{y8}$, -S(-O)(-N$R^{y9}$)-$R^{y10}$, -P(=O)($R^{y11}$)($R^{y12}$), or a 3- to 6-membered heterocyclyl that is optionally substituted with one, two or more $R^{y1}$, where each $R^{y'}$ is the same or different, and independently selected from oxo (=O) or a -S(=O)$_2$-$C_{1-3}$ alkyl; and $R^{y5}$, $R^{y6}$, $R^{y7}$, $R^{y8}$, $R^{y9}$, $R^{y10}$, $R^{y11}$, and $R^{y12}$ are the same or different, and each independently selected from hydrogen, a $C_{1-6}$ alkyl, or a $C_{3-6}$ cycloalkyl.

According to some embodiments, each $R^y$ is the same or different, and independently selected from methyl, ethyl, n-propyl, iso-propyl, cyclopropyl, cyclobutyl, cyclopentyl, trifluoromethyl, 2-hydroxylethyl, oxo (=O), =N-$CH_3$, -S(=O)$_2$-$CH_3$, -C(=O)O-$CH_3$, -C(-O)O-$C_2H_5$, pyrimidinyl, -C($CH_3$)$_2$OH, -C($CH_3$)$_2$CN, -S(=O)$_2$-$C_2H_5$, -S(=O)$_2$-CH($CH_3$)$_2$, -$CH_2$COOH,

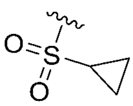, 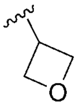, 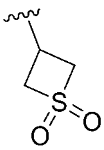, 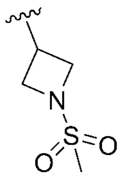, 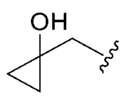, 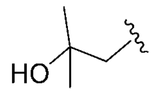, 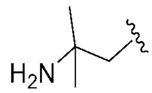,

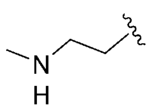, 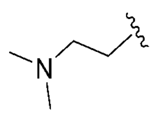, 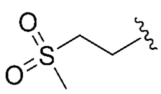 or 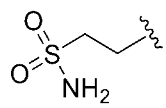.

According to some embodiments, Y is selected from

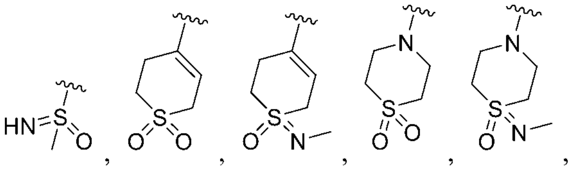
, , , , ,

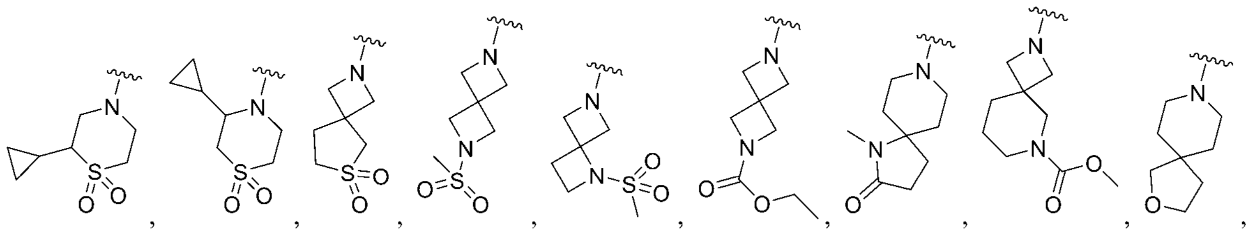
, , , , , , , , ,

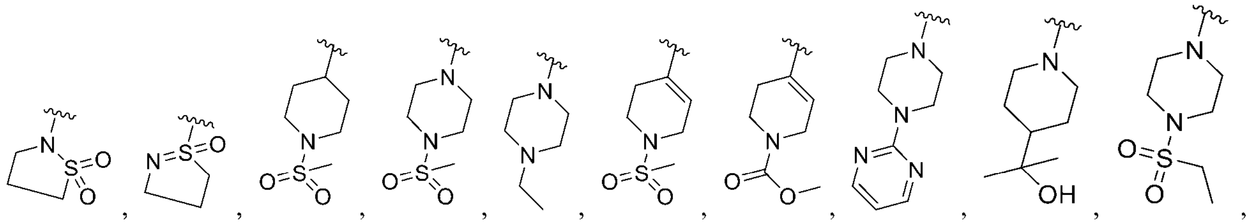
, , , , , , , , , ,

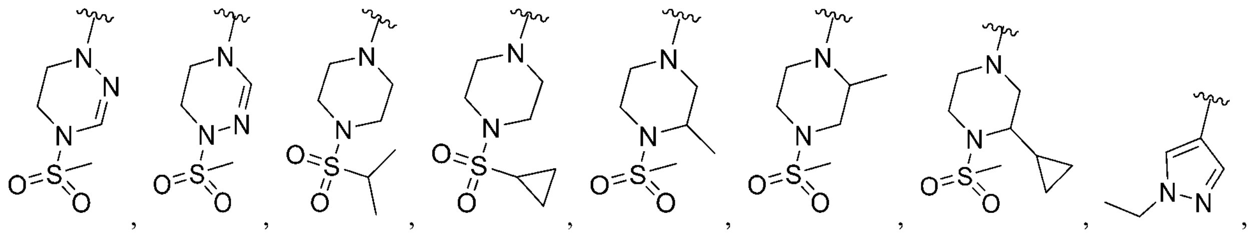
, , , , , , , ,

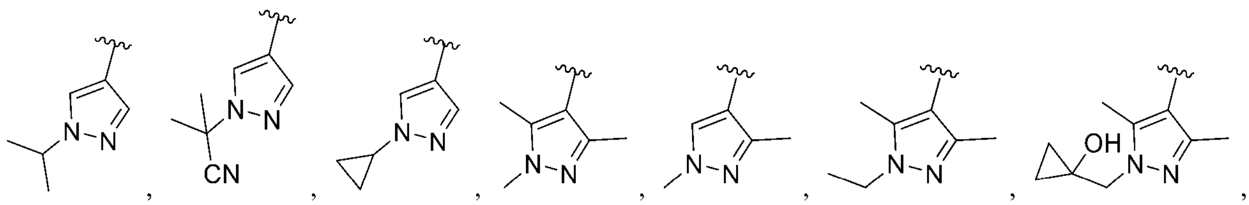
, , , , , , ,

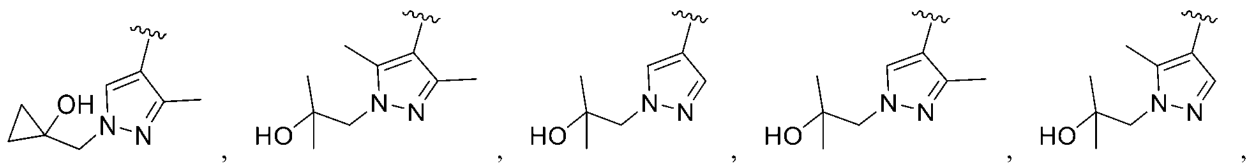
, , , , ,

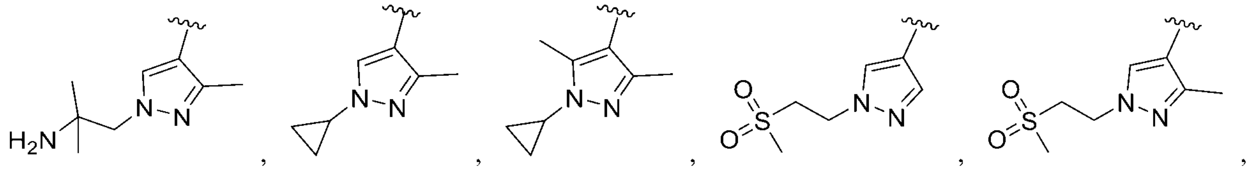
, , , , ,

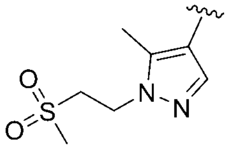, 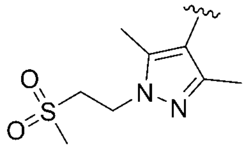, 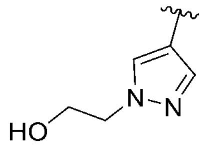, 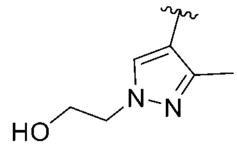, 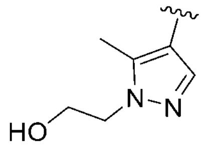,

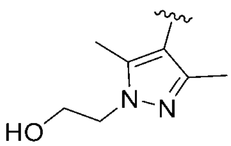, 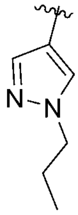, 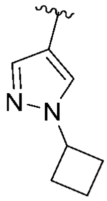, 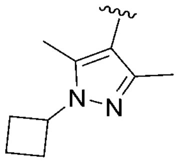, 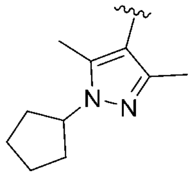, 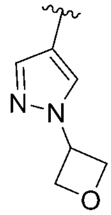, 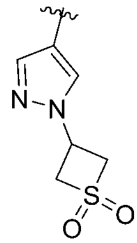,

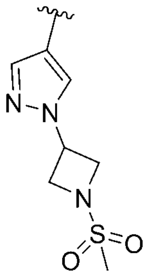, 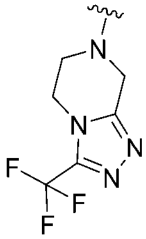, 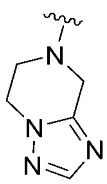, 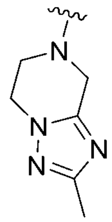, 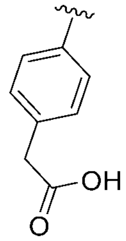, 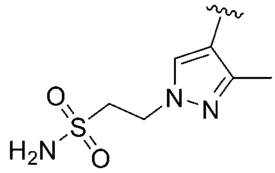, 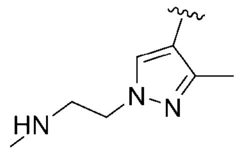

or

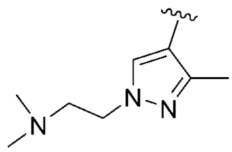.

According to some embodiments, each $R^1$ is the same or different, and independently selected from halogen, cyano, hydroxyl, oxo (=O), or the following groups which are unsubstituted or optionally substituted with one, two or more $R^{11}$: amino, a $C_{1-6}$ alkyl, a $C_{1-6}$ alkoxy, a $C_{3-6}$ cycloalkyl, a 3- to 6-membered heterocyclyl, $-C(=O)-NH_2$, a $-S(=O)_2-C_{1-3}$ alkyl, and a $-S(=O)(=NH)-C_{1-3}$ alkyl; or two $R^1$ attached to the same atom, together with the atom to which they are attached, form a 3- to 8-membered heterocyclic ring that is unsubstituted or optionally substituted with one, two or more $R^{11}$; or two $R^1$ attached to different atoms, together with the atoms to which they are respectively attached, form a 3- to 8-membered heterocyclic ring that is unsubstituted or optionally substituted with one, two or more $R^{11}$, where each $R^{11}$ is the same or different, and independently selected from H, cyano, oxo (=O), halogen, or the following groups which are unsubstituted or optionally substituted with one, two or more $R^{12}$: a $C_{1-6}$ alkyl, a $C_{1-6}$ alkoxy, $-S(=O)_2-CH_3$, $-S(=O)(=NH)-CH_3$, a $C_{3-6}$ cycloalkyl, a 3- to 8-membered heterocyclyl, and a 5- to 6-membered heteroaryl, where each $R^{12}$ is the same or different, and independently selected from hydrogen, hydroxyl, cyano, halogen, a $C_{1-6}$ alkyl, a $C_{1-6}$ alkoxy, a $C_{3-6}$ cycloalkyl, phenyl, or a 5- to 6-membered heteroaryl.

According to some embodiments, each $R^1$ is the same or different, and independently selected from F, Cl, Br, cyano, oxo (=O), methoxy, $-S(=O)_2-CH_3$, $-S(=O)(=NH)-CH_3$, $-S(=O)(=N-CH_3)-CH_3$,

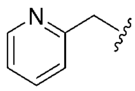

or $-C(=O)-N(CH_3)_2$;or two $R^1$ attached to the same atom, together with the atom to which they are attached, form a piperidyl that is unsubstituted or optionally substituted with one, two or more $R^{11}$.

According to some embodiments, each $R^{11}$ is the same or different, and independently selected from hydrogen, 2,2,2-trifluoroethyl, $-S(=O)_2-CH_3$, or cyclobutyl.

According to some embodiments,

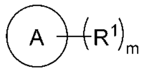

is selected from phenyl,

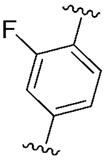, 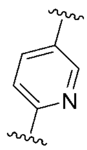, 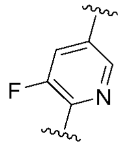,

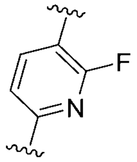, 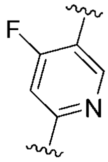, 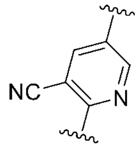, 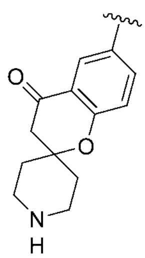, 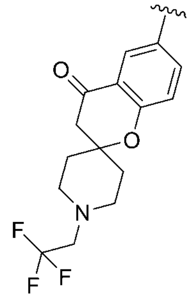, 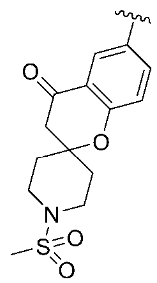, 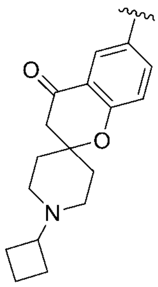,

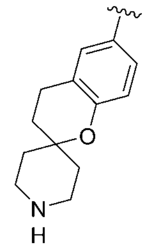, 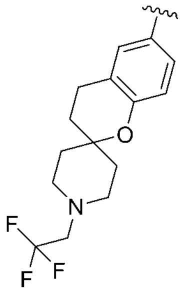, 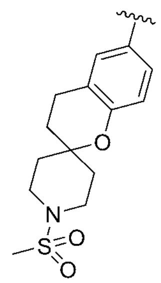, 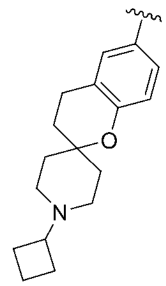, 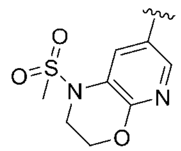, 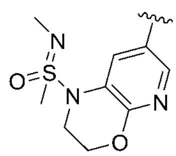,

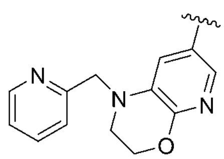, 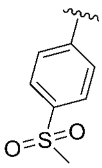, 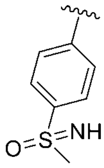, 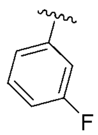, 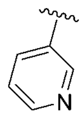, 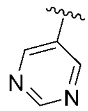, 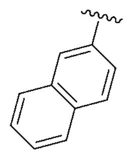, 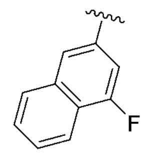, 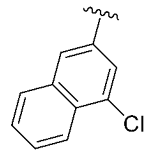,

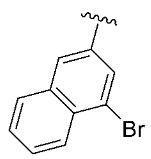, 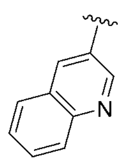, 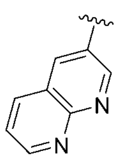, 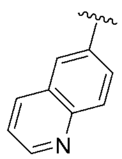, 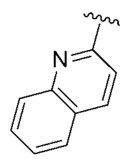, 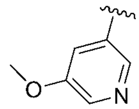, 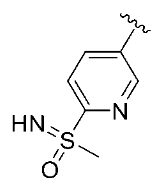, 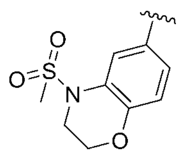,

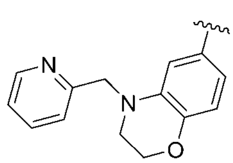, 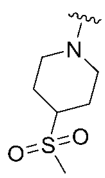, 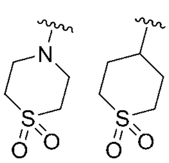, 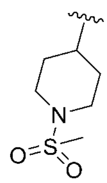, 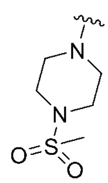 or 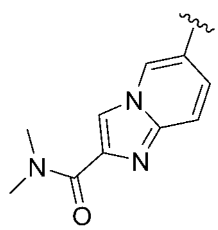.

According to some embodiments, each $R^2$ is the same or different, and independently selected from hydrogen, F, Cl, Br, cyano, or the following groups that are unsubstituted or optionally substituted with one, two or more $R^{21}$: a $C_{1-3}$ alkyl, a $C_{1-3}$ alkoxy, pyrazolyl, and pyrimidinyl, where $R^{21}$ is the same or different, and independently selected from halogen, CN, or methyl.

According to some embodiments, each $R^2$ is the same or different, and independently selected from Cl, methyl,

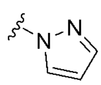, 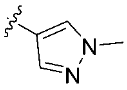, or 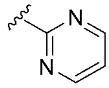.

According to some embodiments,

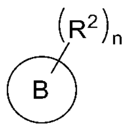

is selected from

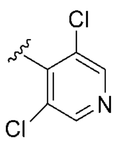, 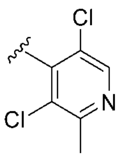, 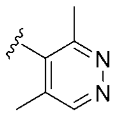, 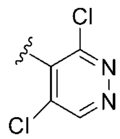,

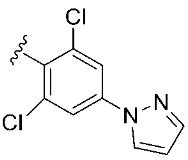, 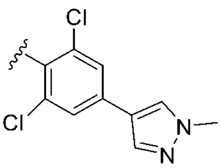, or 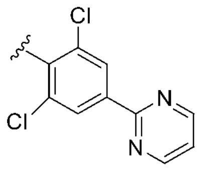.

According to some embodiments, each $R^4$ is the same or different, and independently selected from hydrogen, halogen, cyano, hydroxy, amino, a $C_{1-6}$ alkyl, or a $C_{1-6}$ alkoxy.

According to some embodiments, each $R^4$ is the same or different, and independently selected from halogen, cyano, methyl or methoxy.

According to some embodiments, $R^3$ is selected from hydrogen, a $C_{1-3}$ alkyl, or a $C_{1-3}$ haloalkyl. According to some embodiments, $R^3$ is selected from methyl.

According to some embodiments, $R^6$ is selected from hydrogen.

According to some embodiments, $R^6$ is selected from

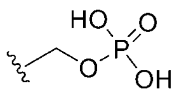.

According to some embodiments, the compound of Formula (I) has a structure shown below:

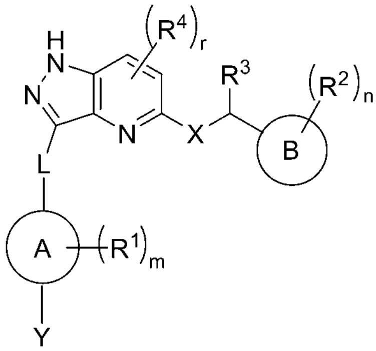

Formula II

where Ring A, Ring B, $R^1$, $R^2$, $R^3$, $R^4$, L, X, Y, m, n, and r are as defined herein.

According to some embodiments, the compound of Formula (I) has a structure shown below:

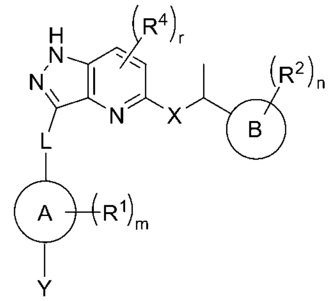

,

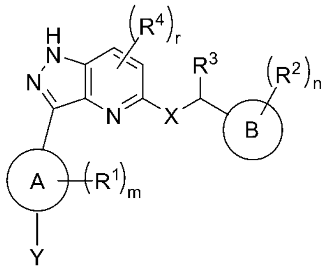

,

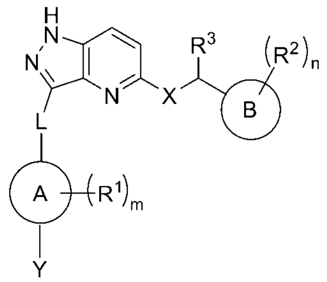

,

Formula II-1 Formula II-2 Formula II-3

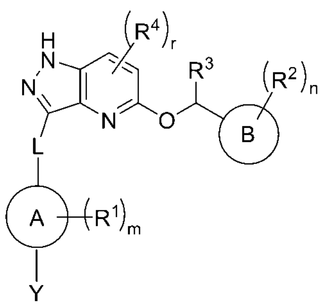

,

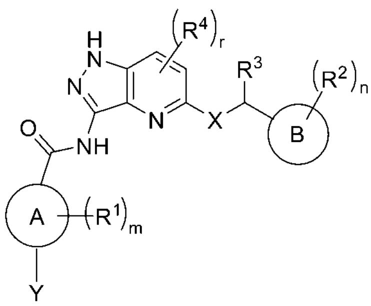

Formula II-4 Formula II-5

where Ring A, Ring B, $R^1$, $R^2$, $R^3$, $R^4$, L, X, Y, m, n, and r are as defined herein.

According to some embodiments, the compound of Formula (I) has a structure shown below:

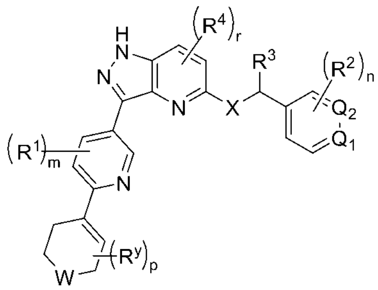

,

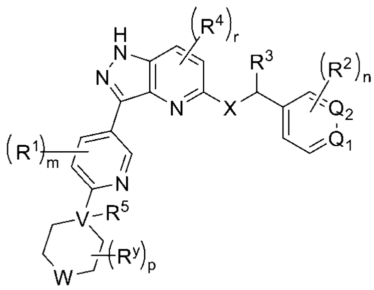

,

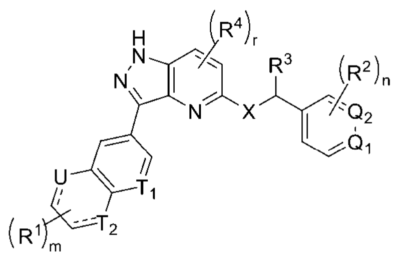

,

Formula III-1 Formula III-2 Formula III-3

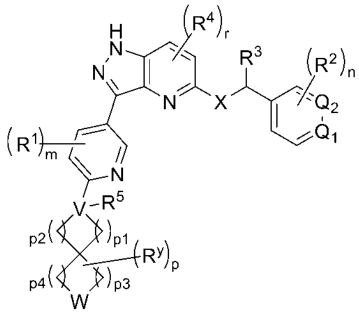, 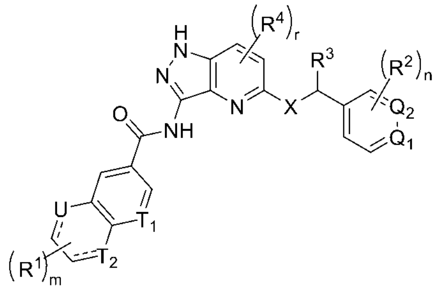, 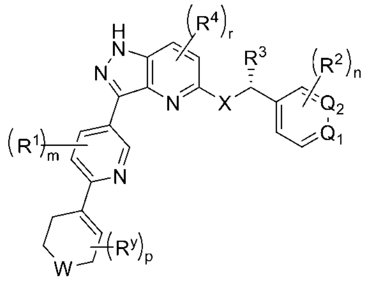,

Formula III-4 Formula III-5 Formula III-6

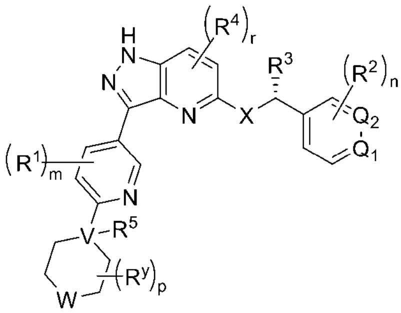,

Formula III-7

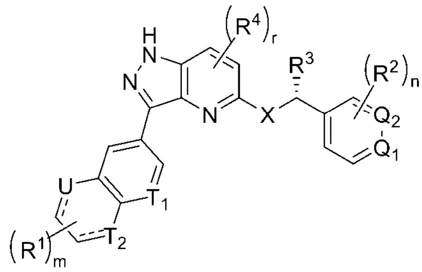,

Formula III-8

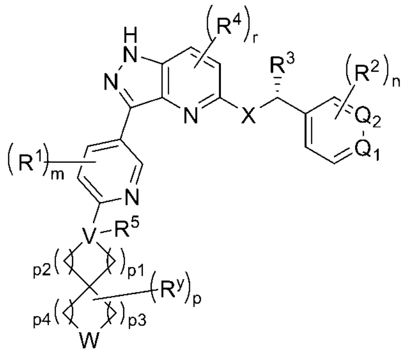,

Formula III-9

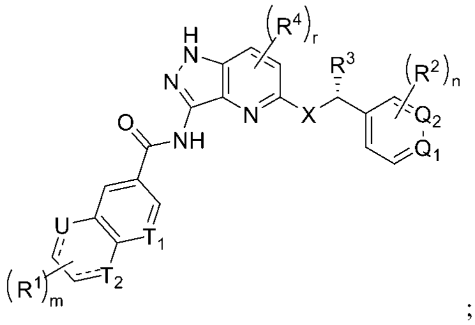;

Formula III-10

where $R^1$, $R^2$, $R^3$, $R^4$, $R^y$, X, m, n, and r are as defined herein, W is selected from O, S, $CH_2$, NH,

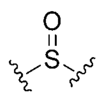,

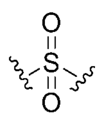, or 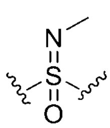;

$Q_1$ and $Q_2$ are the same or different, and each independently selected from CH or N; $T_1$ is selected from CH or N; $T_2$ and U are the same or different, and each independently selected from O, S, N, CH, NH or $CH_2$; V is selected from N or C, and when V is N, $R^5$ is absent; $R^5$ is absent or selected from hydrogen, hydroxyl, cyano, a $C_{1-6}$ alkyl, a $C_{1-6}$ alkoxy, a $C_{1-6}$ haloalkyl, a $C_{1-6}$ haloalkoxy, or a $C_{3-6}$ cycloalkyl; p, p1, p2, p3, and p4 are the same or different, and each independently selected from 0, 1, 2, 3, 4, or 5; and

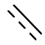

represents a single bond or a double bond.

According to some embodiments, the compound of Formula (I) has a structure shown below:

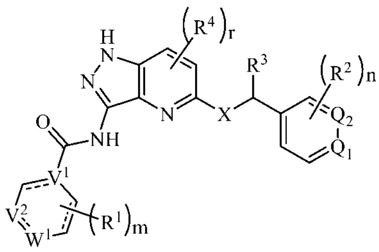

,

Formula IV-1

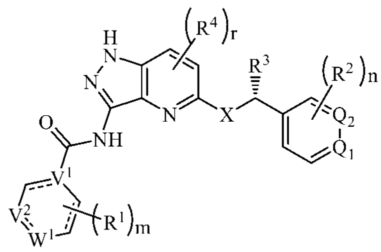

;

Formula IV-2

where $R^1$, $R^2$, $R^3$, $R^4$, X, m, n, and r are as defined herein, $W^1$ is selected from O, S, $CH_2$, NH, CH, N,

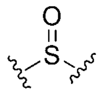

,

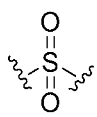

, or

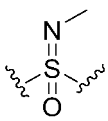

;

$Q_1$ and $Q_2$ are the same or different, and each independently selected from CH or N; $V^1$ is selected from N, C or CH; $V^2$ is selected from N, NH, C, CH or $CH_2$; and 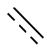 represents a single bond or a double bond.

According to some embodiments, the compound of Formula (I) has a structure shown below:

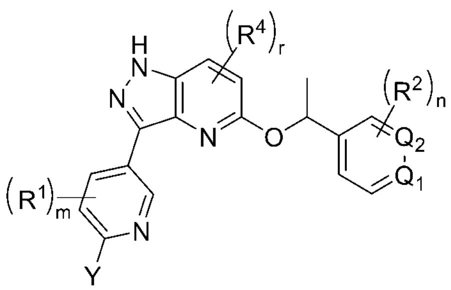

,

Formula V-1

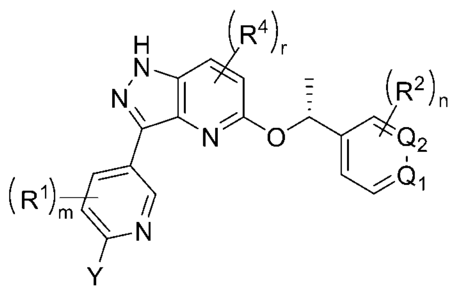

;

Formula V-2

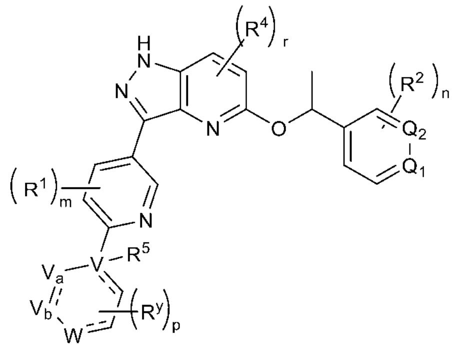

,

Formula V-3

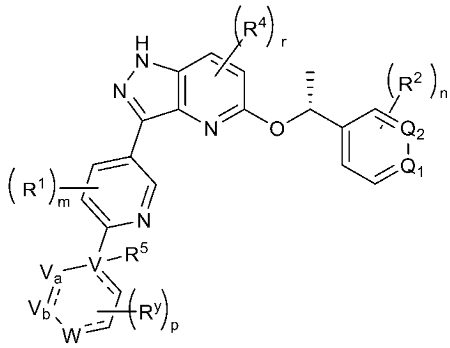

;

Formula V-4

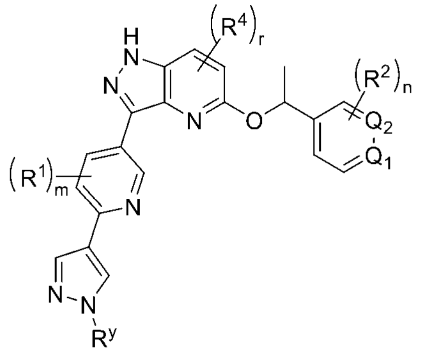

,

Formula V-5

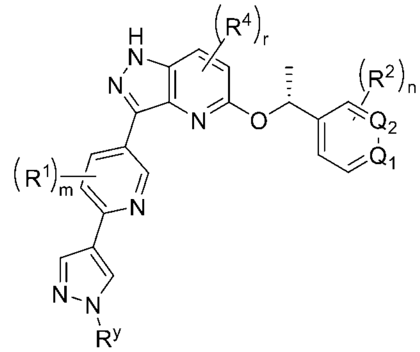

;

Formula V-6

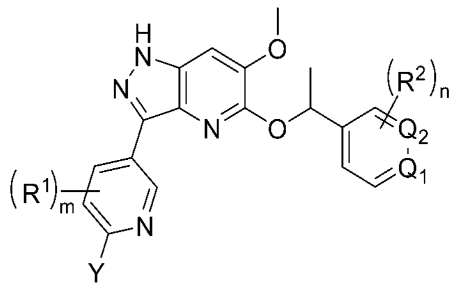

,

Formula V-7

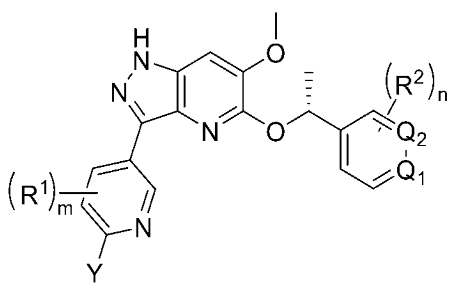

;

Formula V-8

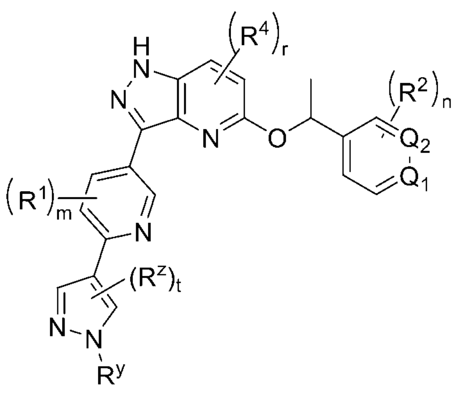

,

Formula V-9

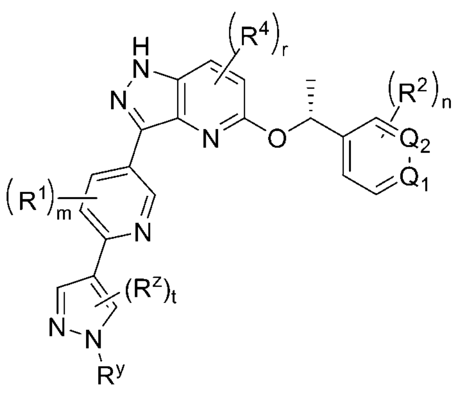

;

Formula V-10

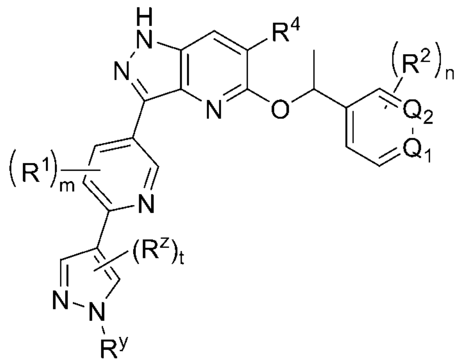

,

Formula V-11

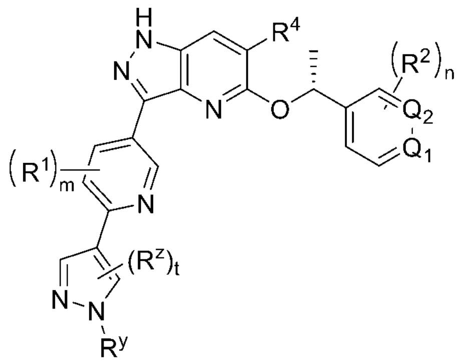

;

Formula V-12

where $R^z$ is as defined for $R^y$ herein, and preferably, $R^z$ is selected from H, halogen, cyano, hydroxyl, or the following groups which are unsubstituted or optionally substituted with one, two or more $R^{z1}$: amino, a $C_{1-6}$ alkyl, a $C_{1-6}$ alkoxy, and a $C_{3-6}$ cycloalkyl, where each $R^{z1}$ is the same or different, and independently selected from hydrogen, hydroxyl, cyano, halogen, oxo (=O), amino, a $C_{1-6}$ alkyl, or a $C_{3-6}$ cycloalkyl; further preferably, $R^z$ is selected from H, halogen, cyano, methyl, ethyl, propyl, or cyclopropyl; $R^1$, $R^2$, $R^4$, $R^y$, Y, m, n, and r are as defined herein, and W is selected from O, S, $CH_2$, NH,

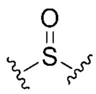 , 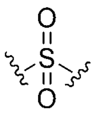 , or 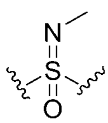 ;

$Q_1$ and $Q_2$ are the same or different, and each independently selected from CH or N; V is selected from N or C, and when V is selected from N, $R^5$ is absent; $V_a$ and $V_b$ are the same or different, and each independently selected from CH, $CH_2$, N, or NH;$R^5$ is absent or selected from hydrogen, hydroxyl, cyano, a $C_{1-6}$ alkyl, a $C_{1-6}$ alkoxy, a $C_{1-6}$ haloalkyl, a $C_{1-6}$ haloalkoxy or a $C_{3-6}$ cycloalkyl; p is selected from 0, 1, 2, 3, 4, or 5; t is selected from 0, 1, or 2; and

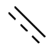

represents a single bond or a double bond.

According to some embodiments, the compound of Formula (I) has a structure shown below:

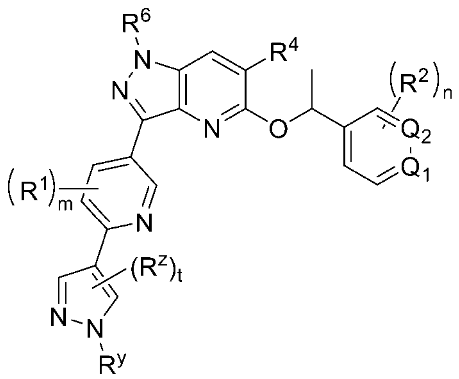

,

Formula V-13

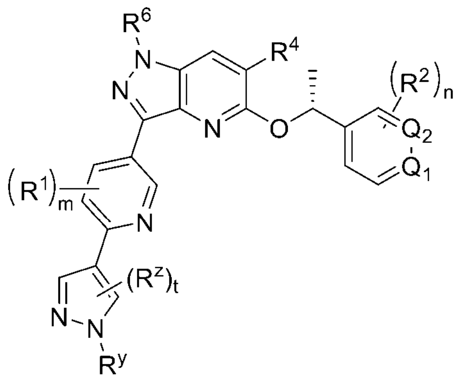

;

Formula V-14

where $R^1$, $R^2$, $R^4$, $R^6$, $R^y$, $R^z$, $Q_1$, $Q_2$, m, n, and t are as defined herein.

According to some embodiments, among the compound of Formula (I) and the racemate, the stereoisomer, the tautomer, the isotopically labeled compound, the N-oxide, the solvate, the polymorph, the metabolite, the ester, the prodrug or the pharmaceutically acceptable salt thereof, the exemplary and non-limiting examples of the compound of

Formula (I) comprise:

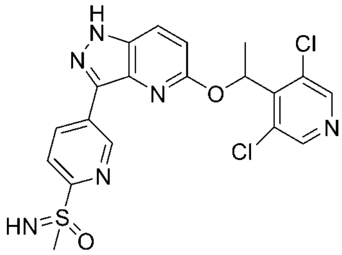
Cpd-01

,

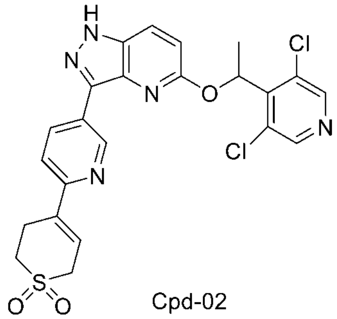
Cpd-02

,

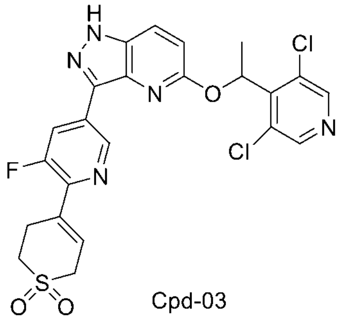
Cpd-03

,

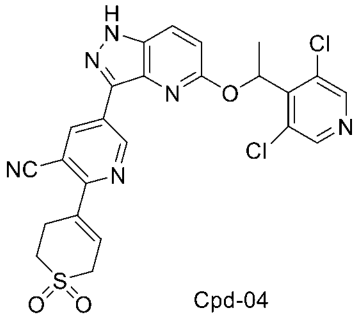
Cpd-04

,

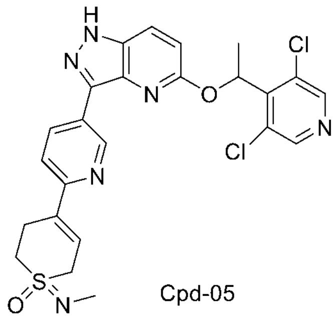
Cpd-05

,

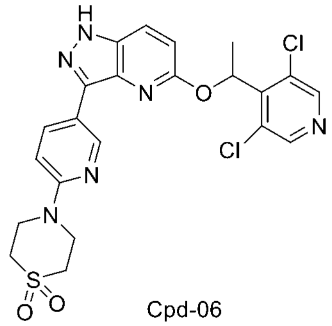
Cpd-06

,

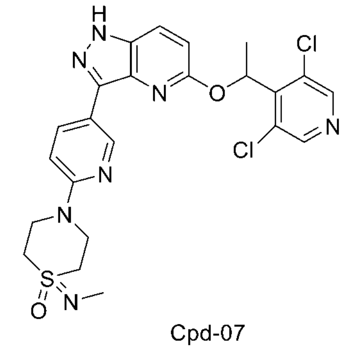
Cpd-07

,

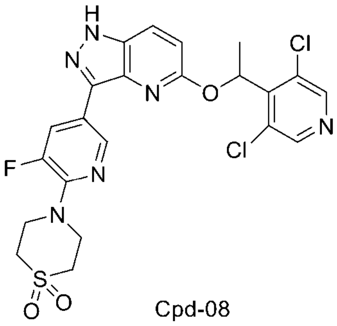
Cpd-08

,

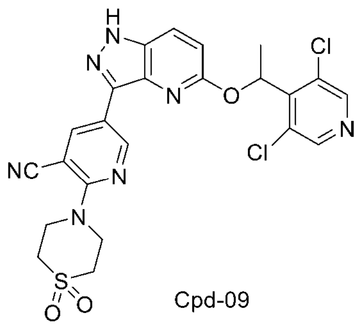
Cpd-09

,

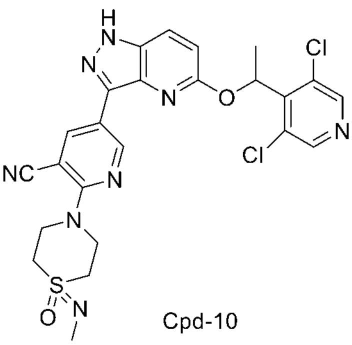
Cpd-10

,

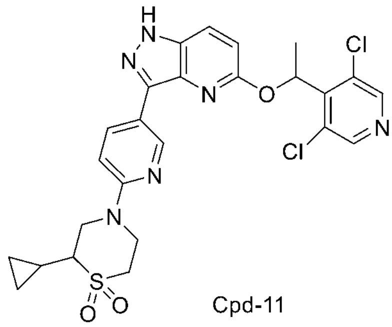
Cpd-11

,

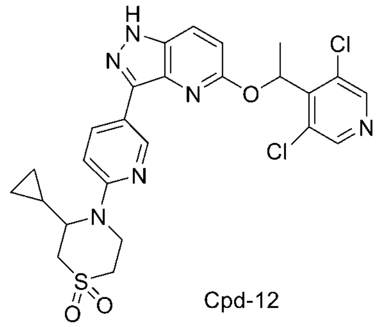
Cpd-12

,

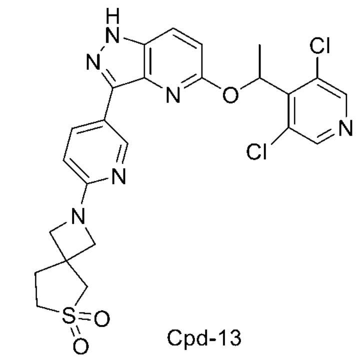
Cpd-13

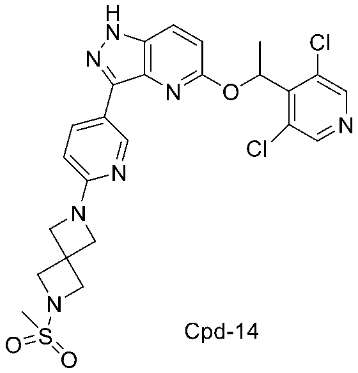
Cpd-14
,

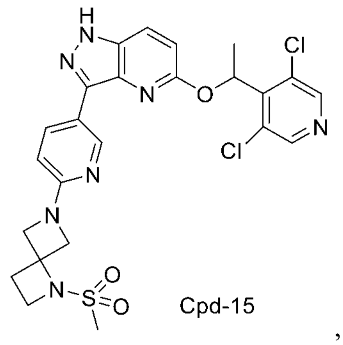
Cpd-15
,

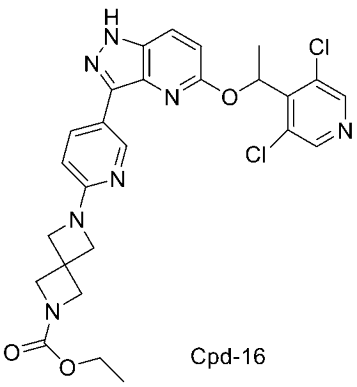
Cpd-16
,

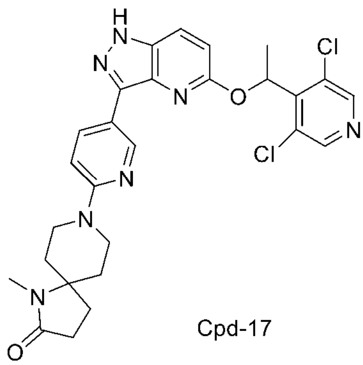
Cpd-17
,

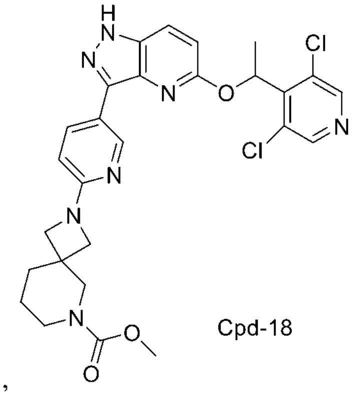
Cpd-18
,

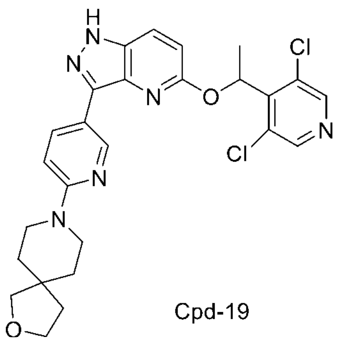
Cpd-19
,

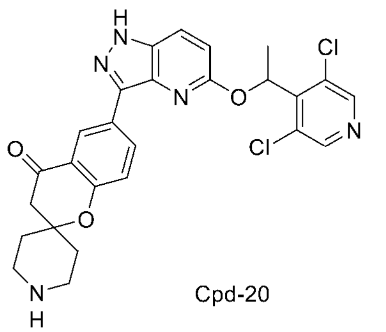
Cpd-20
,

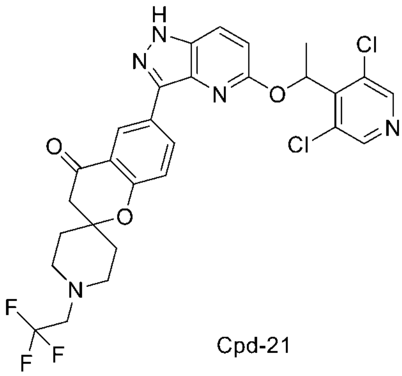
Cpd-21
,

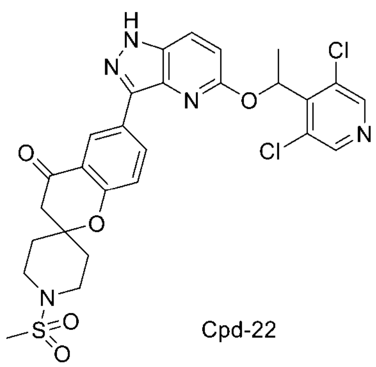
Cpd-22
,

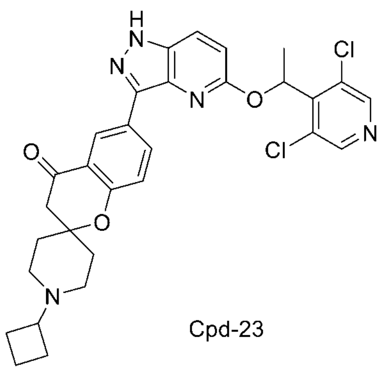
Cpd-23
,

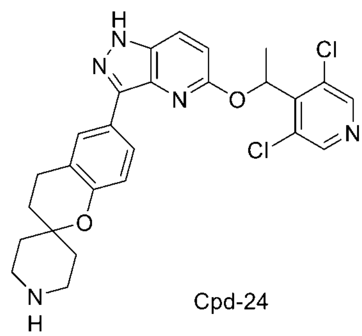
Cpd-24
,

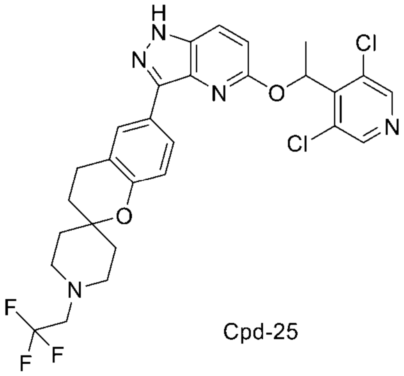
Cpd-25,

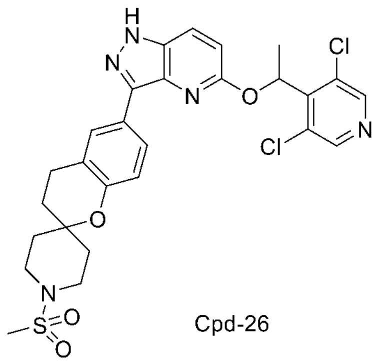
Cpd-26,

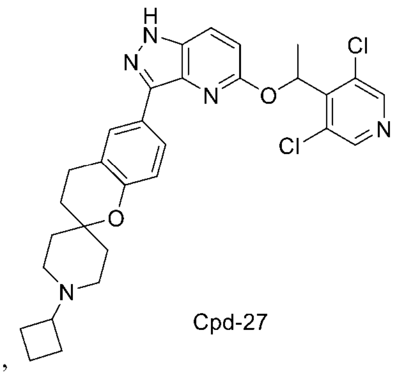
Cpd-27,

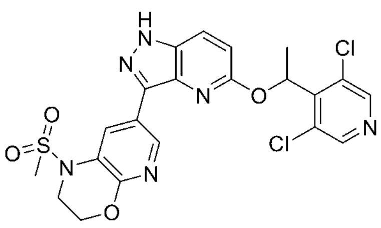
Cpd-28,

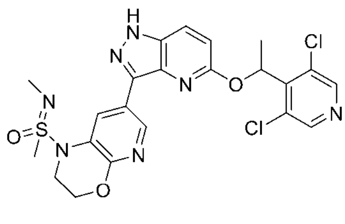
Cpd-29,

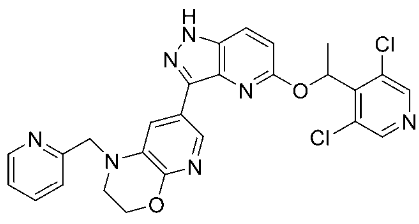
Cpd-30,

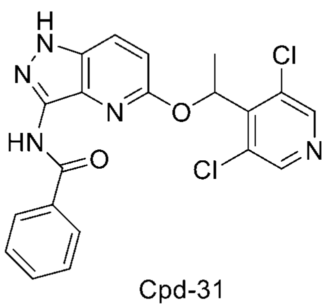
Cpd-31,

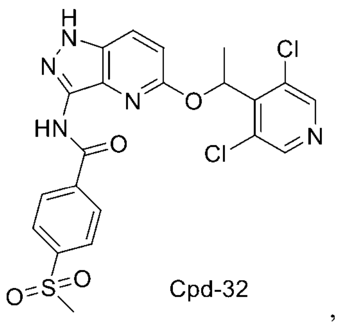
Cpd-32,

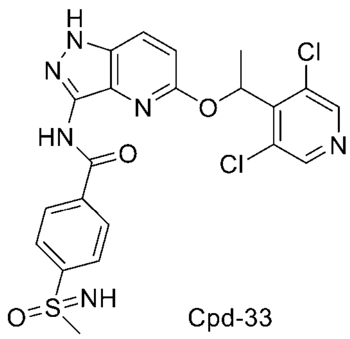
Cpd-33,

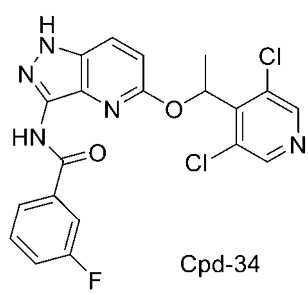
Cpd-34,

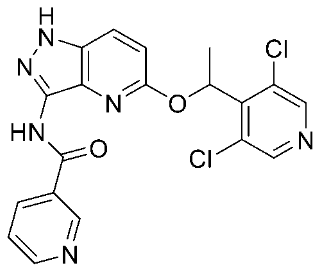
Cpd-35,

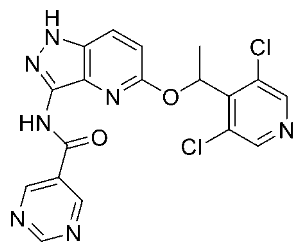
Cpd-36,

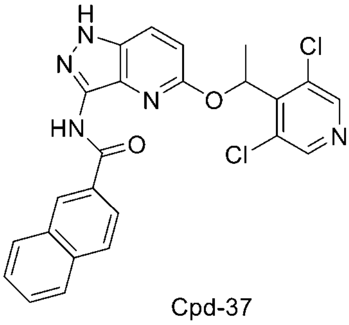
Cpd-37 ,

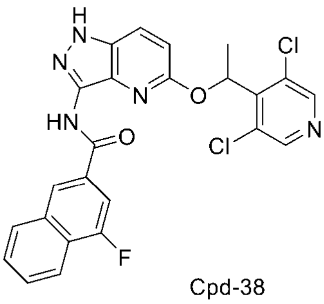
Cpd-38 ,

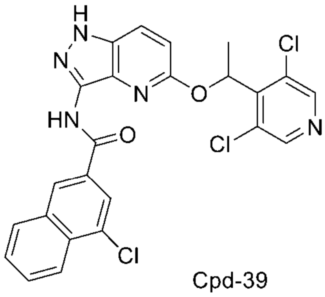
Cpd-39 ,

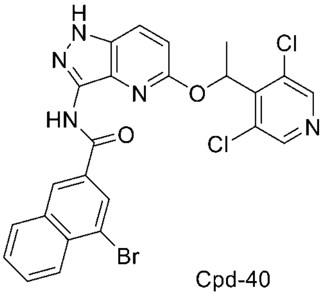
Cpd-40 ,

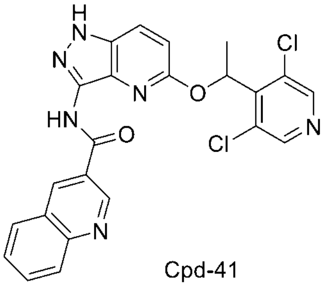
Cpd-41 ,

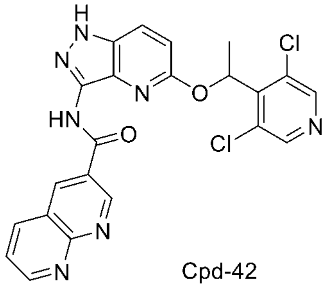
Cpd-42 ,

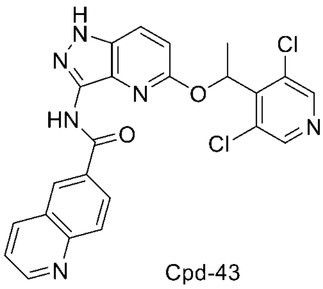
Cpd-43 ,

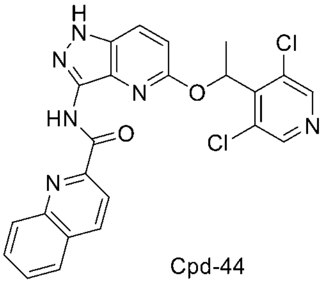
Cpd-44 ,

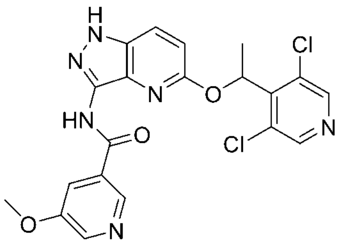
Cpd-45 ,

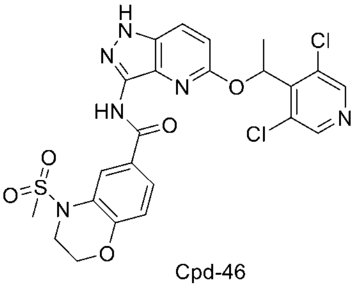
Cpd-46 ,

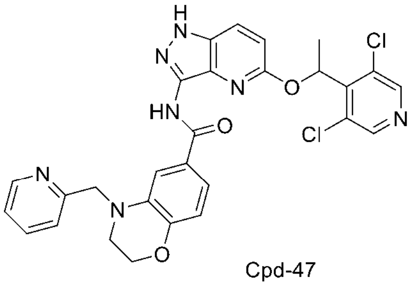
Cpd-47 ,

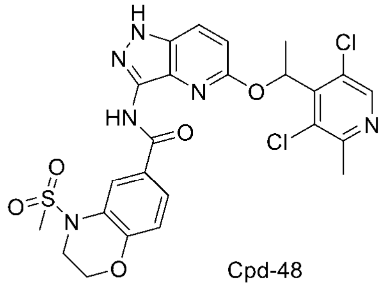
Cpd-48 ,

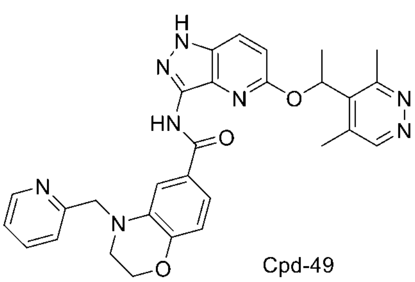
Cpd-49 ,

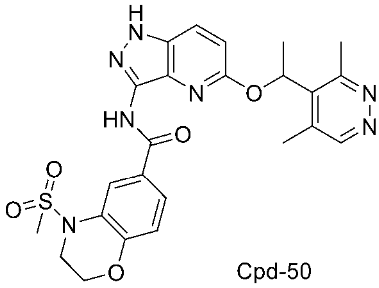
Cpd-50 ,

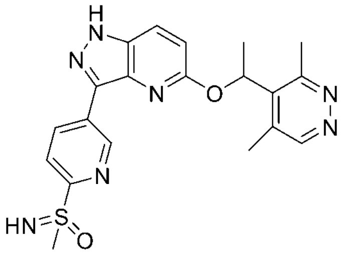
Cpd-51 ,

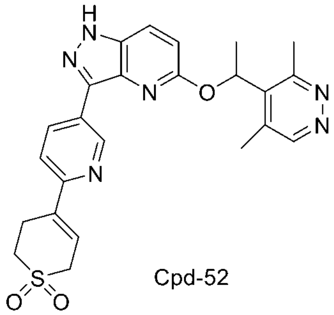
Cpd-52,

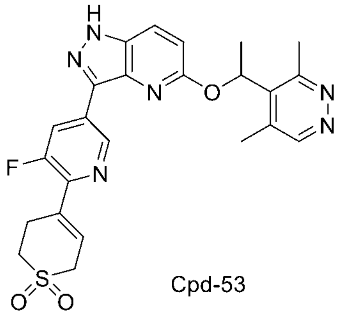
Cpd-53,

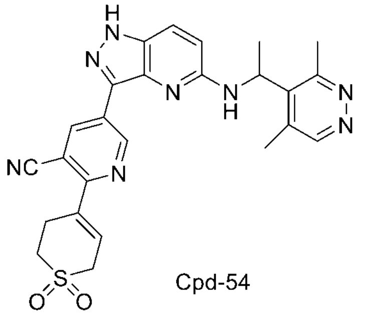
Cpd-54,

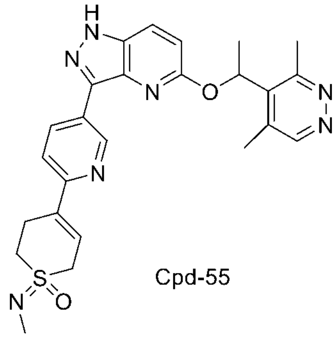
Cpd-55,

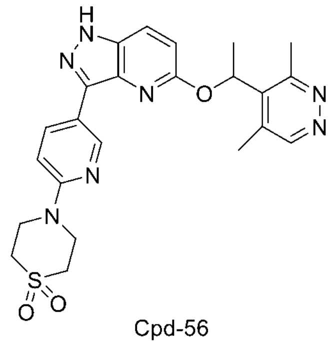
Cpd-56,

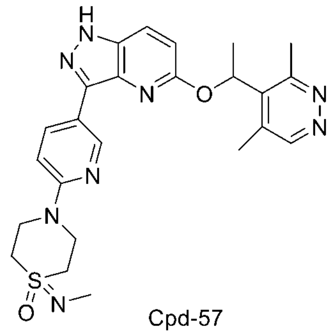
Cpd-57,

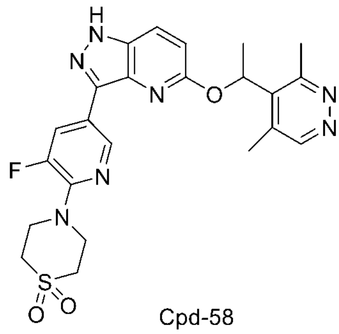
Cpd-58,

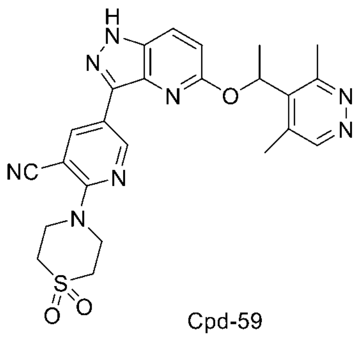
Cpd-59,

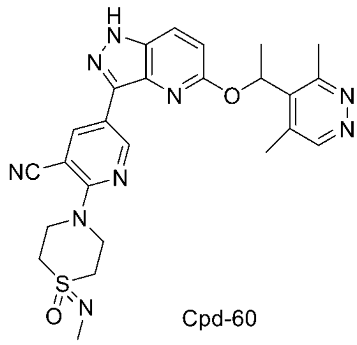
Cpd-60,

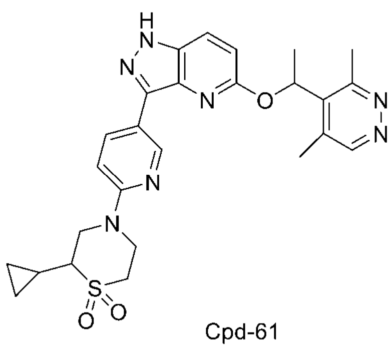
Cpd-61,

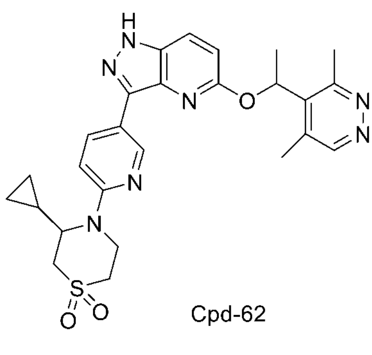
Cpd-62,

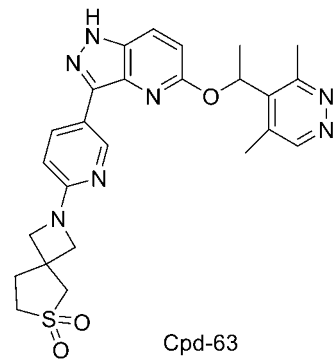
Cpd-63,

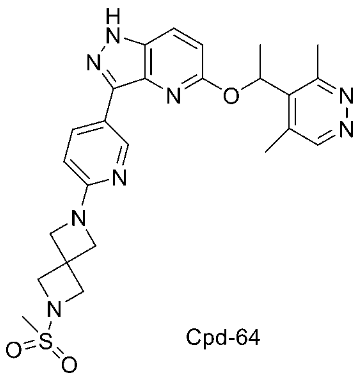
Cpd-64 ,

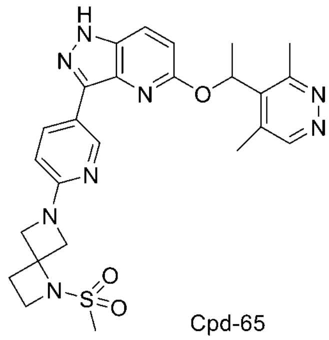
Cpd-65 ,

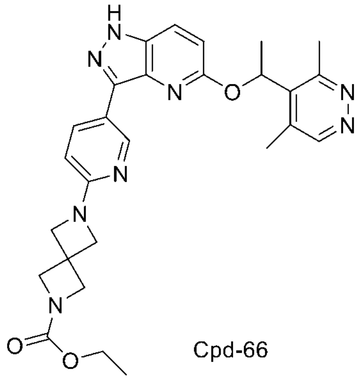
Cpd-66 ,

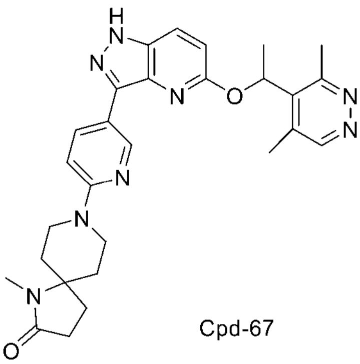
Cpd-67 ,

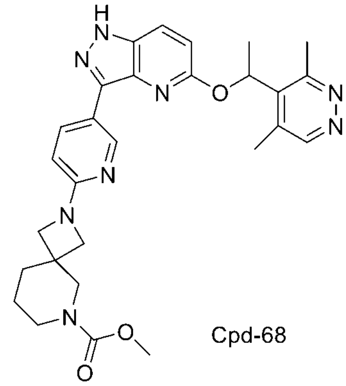
Cpd-68 ,

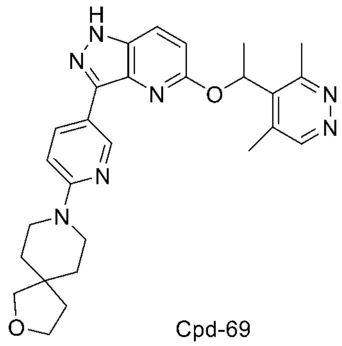
Cpd-69 ,

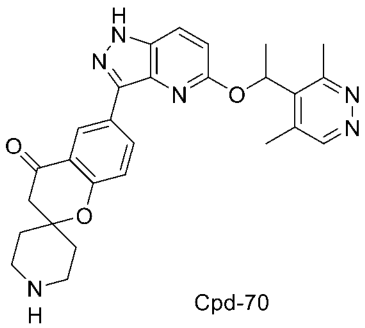
Cpd-70 ,

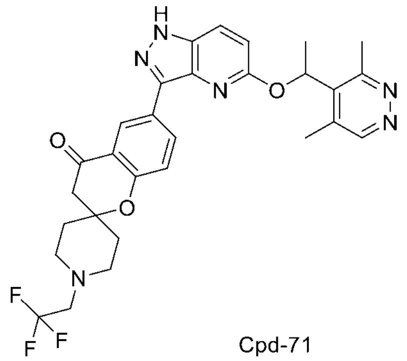
Cpd-71 ,

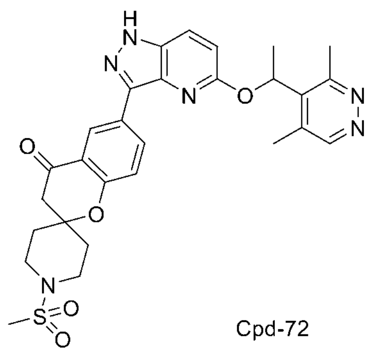
Cpd-72 ,

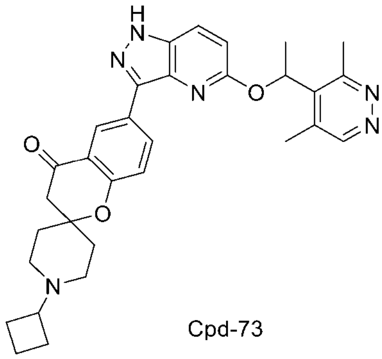
Cpd-73 ,

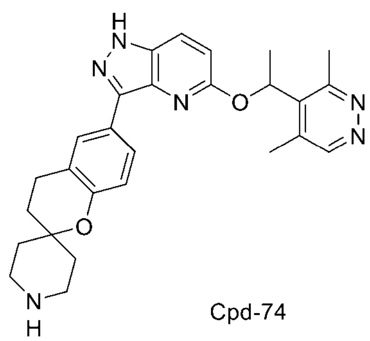
Cpd-74 ,

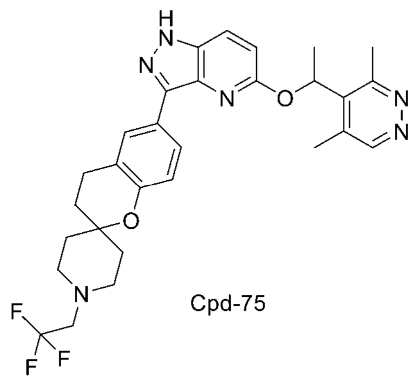
Cpd-75 ,

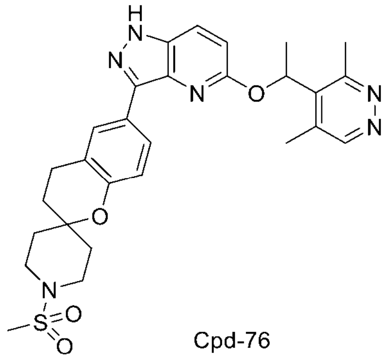
Cpd-76 ,

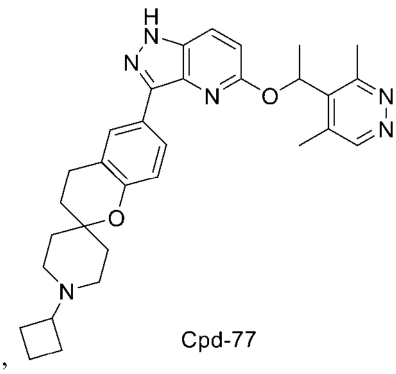
Cpd-77 ,

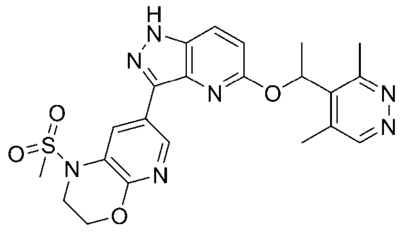
Cpd-78 ,

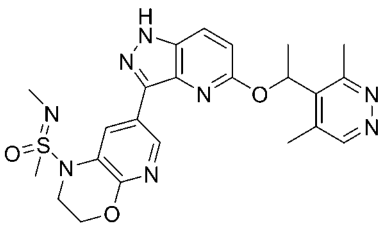
Cpd-79 ,

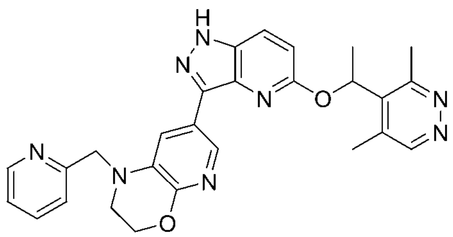
Cpd-80 ,

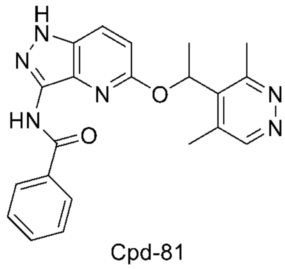
Cpd-81 ,

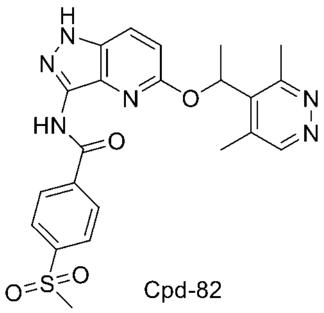
Cpd-82 ,

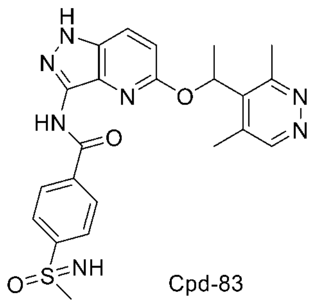
Cpd-83 ,

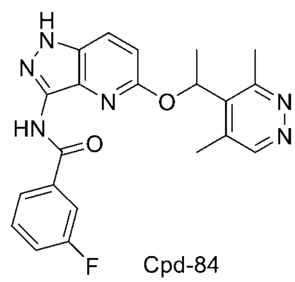
Cpd-84 ,

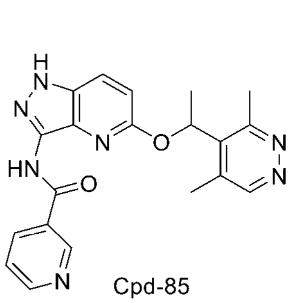
Cpd-85 ,

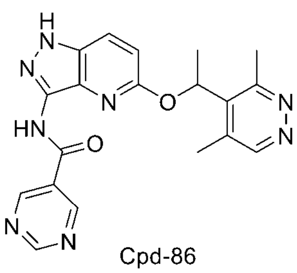
Cpd-86 ,

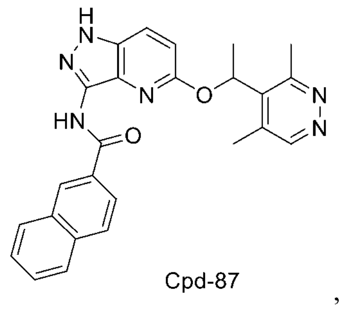
Cpd-87 ,

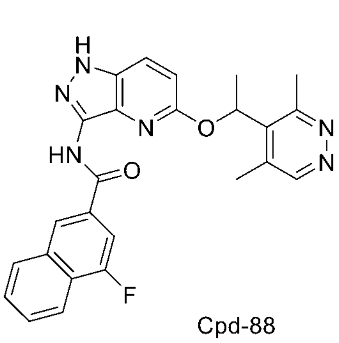
Cpd-88 ,

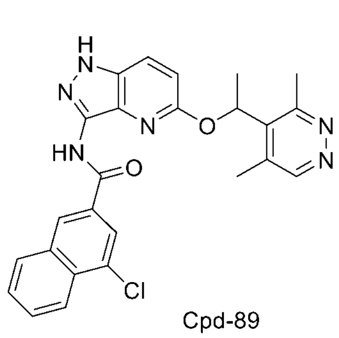
Cpd-89 ,

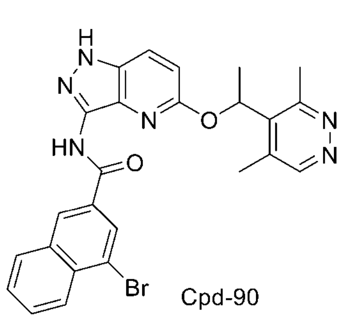
Cpd-90 ,

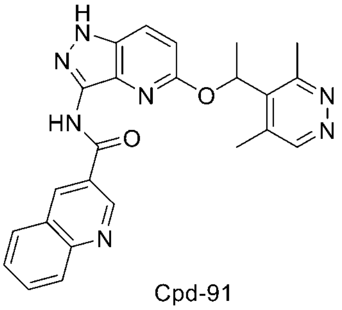
Cpd-91 ,

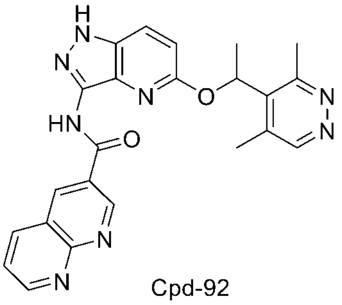
Cpd-92 ,

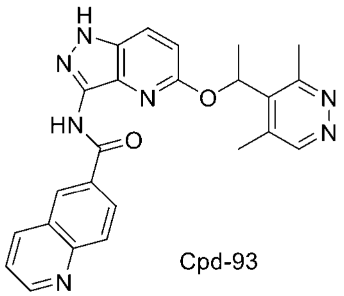
Cpd-93 ,

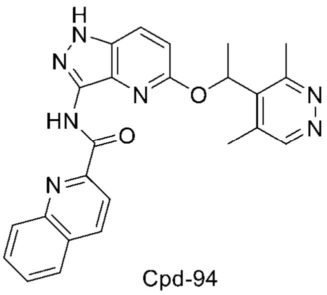
Cpd-94 ,

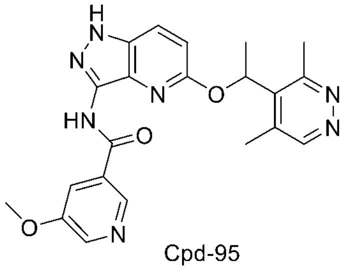
Cpd-95 ,

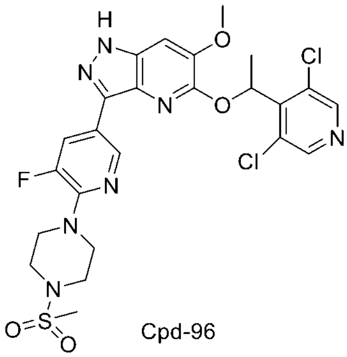
Cpd-96 ,

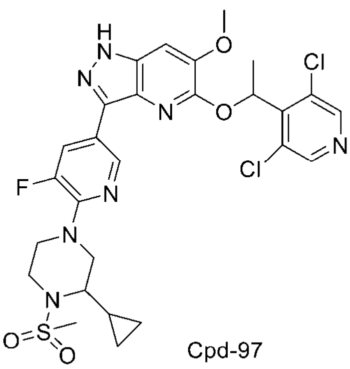
Cpd-97 ,

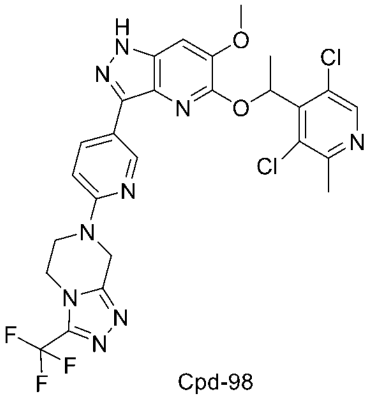
Cpd-98 ,

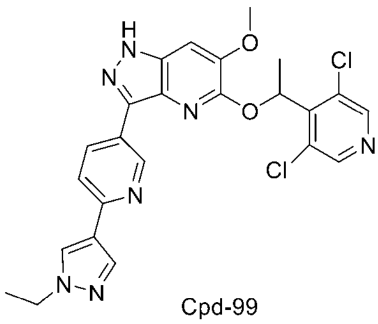
Cpd-99 ,

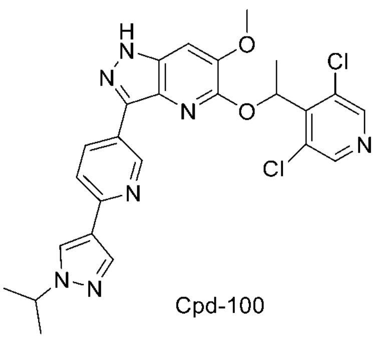
Cpd-100 ,

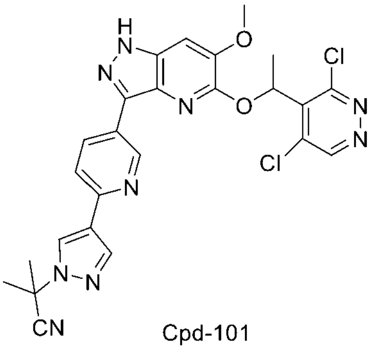
Cpd-101 ,

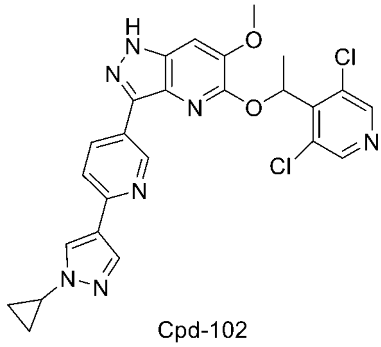
Cpd-102 ,

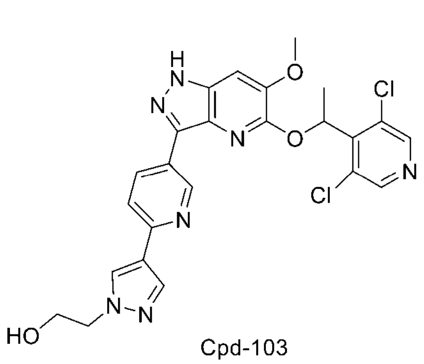
Cpd-103 ,
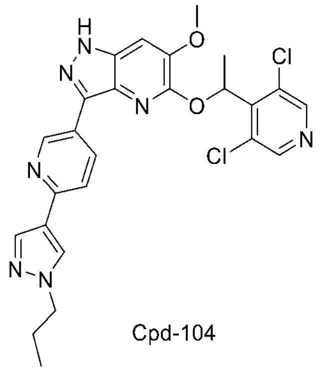
Cpd-104 ,
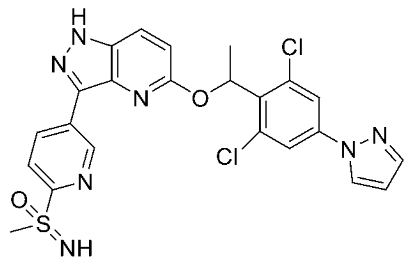
Cpd-105 ,

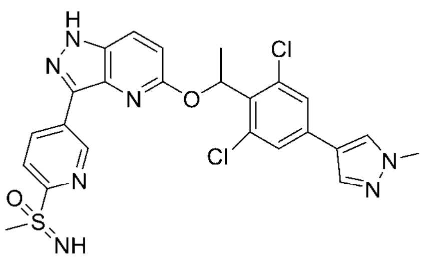
Cpd-106 ,
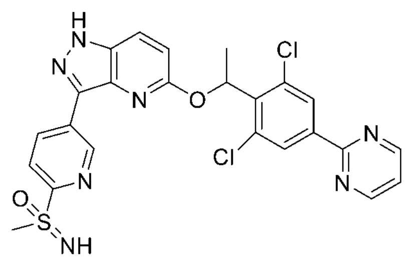
Cpd-107 ,
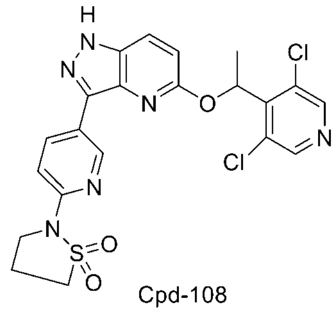
Cpd-108 ,

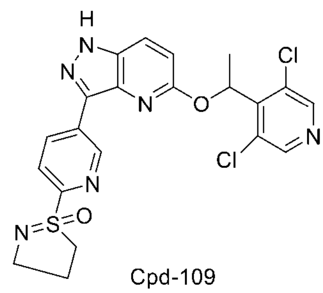
Cpd-109 ,
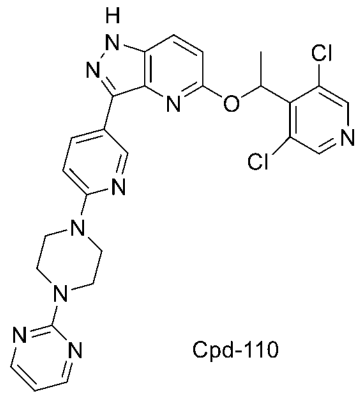
Cpd-110 ,
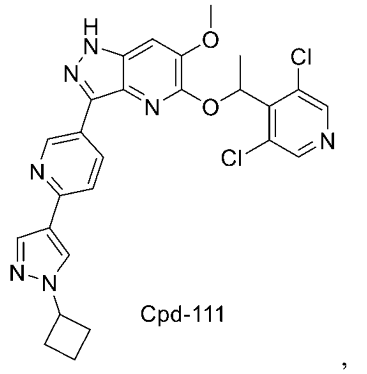
Cpd-111 ,

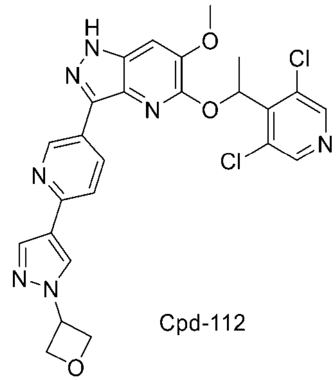
Cpd-112 ,
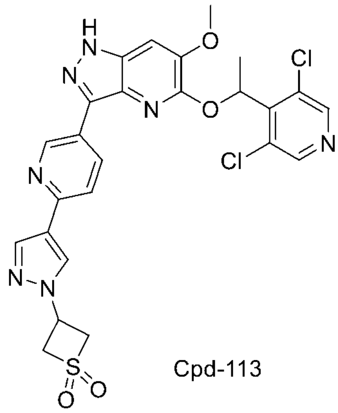
Cpd-113 ,
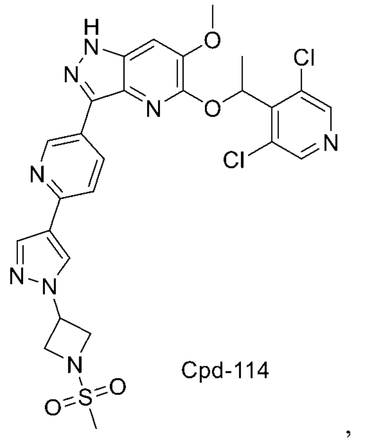
Cpd-114 ,

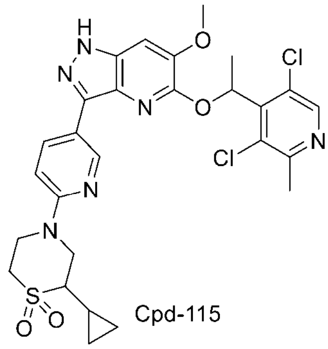

Cpd-115 ,

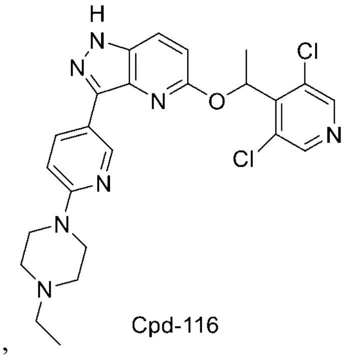

Cpd-116 ,

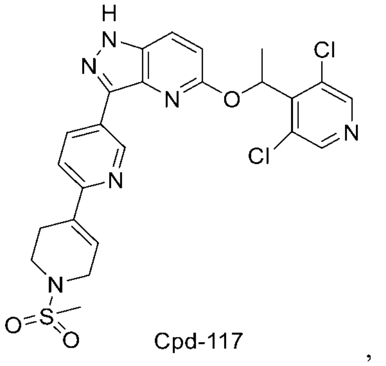

Cpd-117 ,

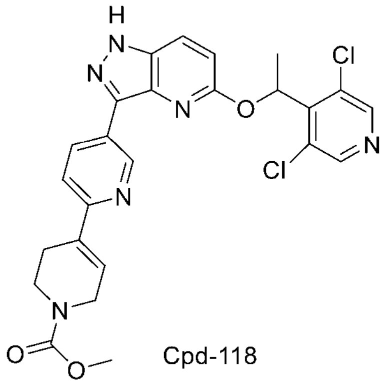

Cpd-118 ,

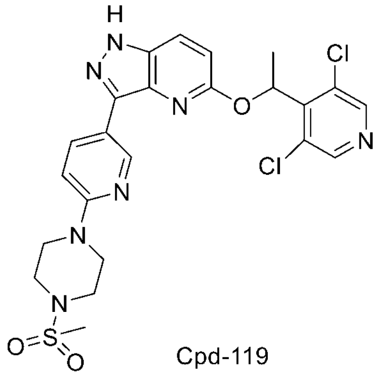

Cpd-119 ,

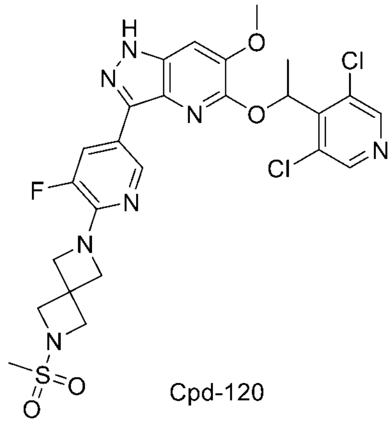

Cpd-120 ,

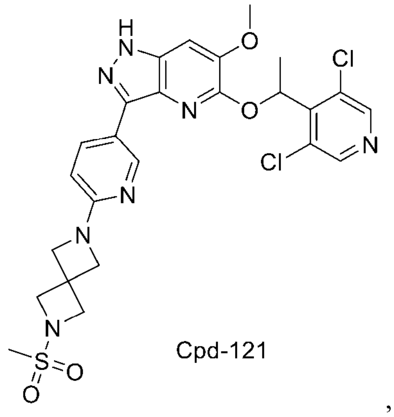

Cpd-121 ,

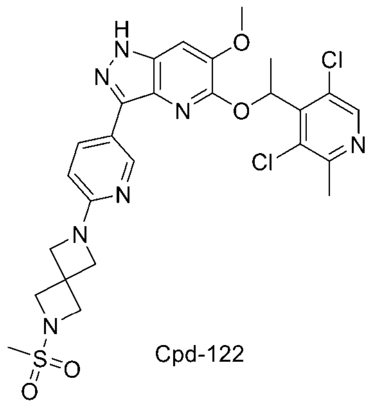

Cpd-122 ,

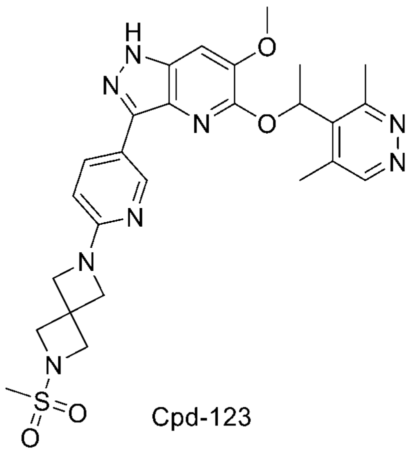

Cpd-123 ,

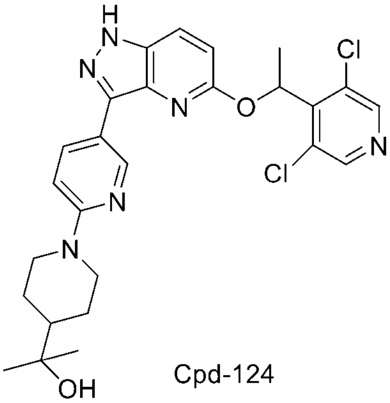

Cpd-124 ,

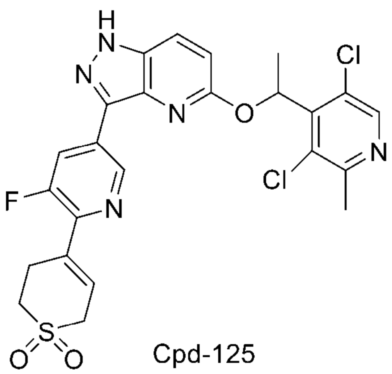

Cpd-125 ,

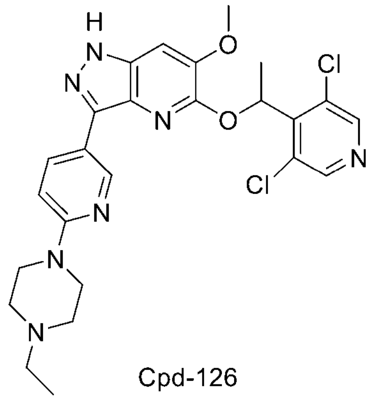

Cpd-126 ,

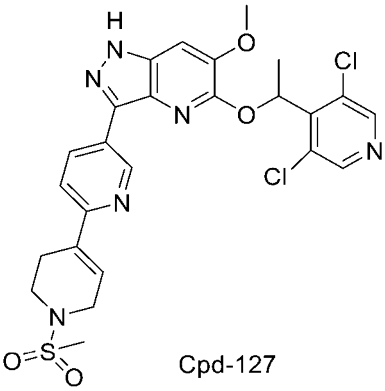
Cpd-127 ,

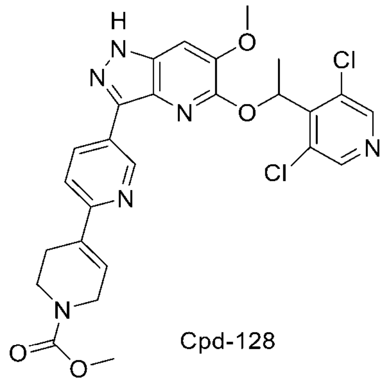
Cpd-128 ,

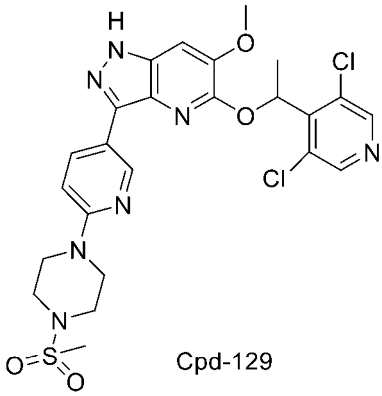
Cpd-129 ,

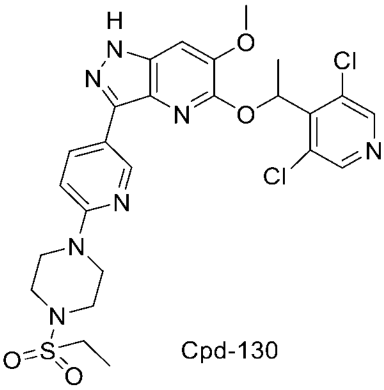
Cpd-130 ,

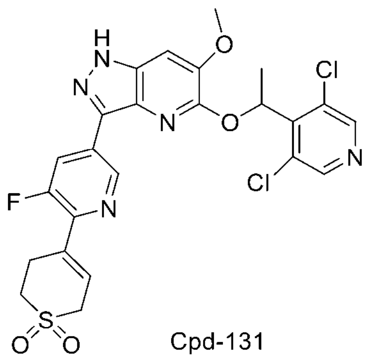
Cpd-131 ,

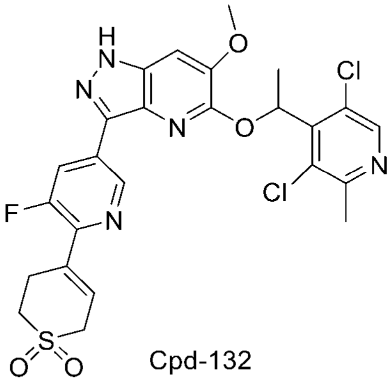
Cpd-132 ,

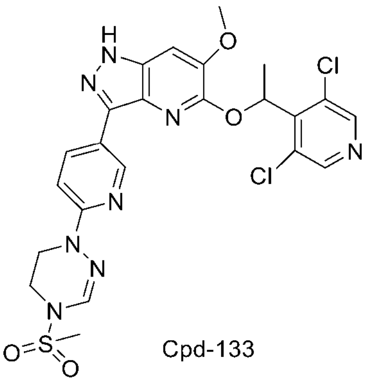
Cpd-133 ,

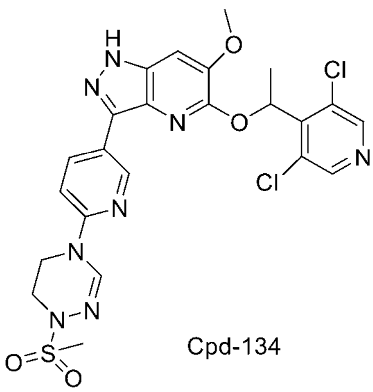
Cpd-134 ,

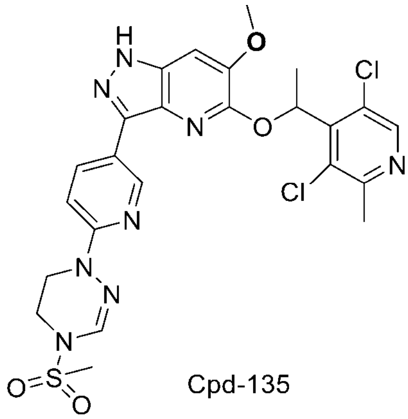
Cpd-135 ,

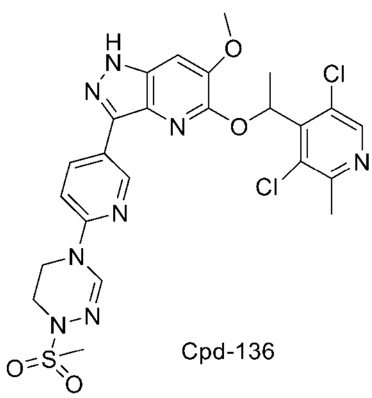
Cpd-136 ,

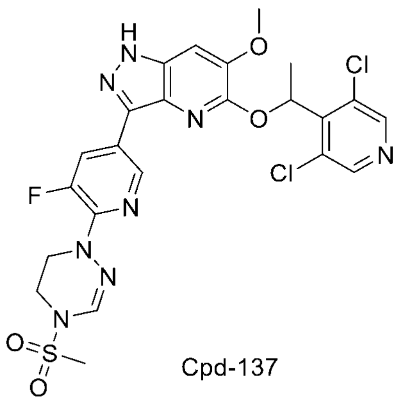
Cpd-137 ,

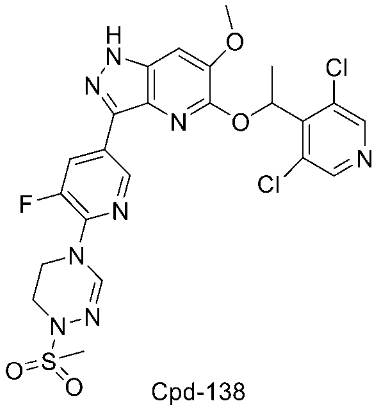
Cpd-138 ,

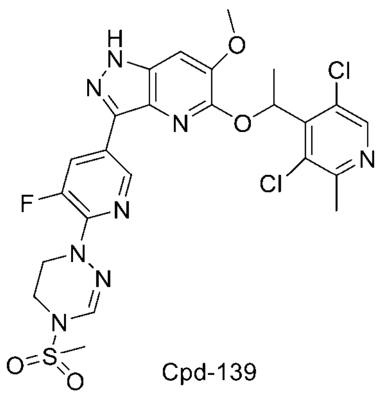

Cpd-139 ,

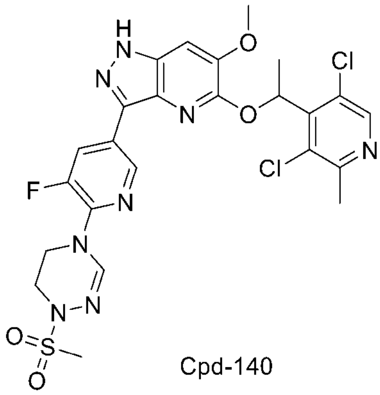

Cpd-140 ,

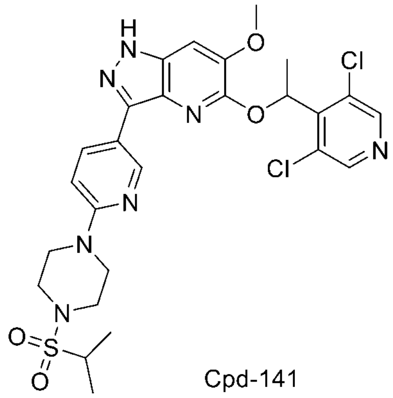

Cpd-141 ,

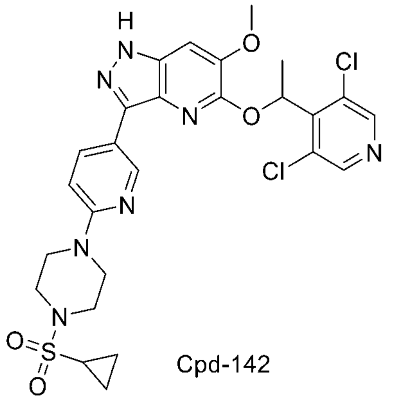

Cpd-142 ,

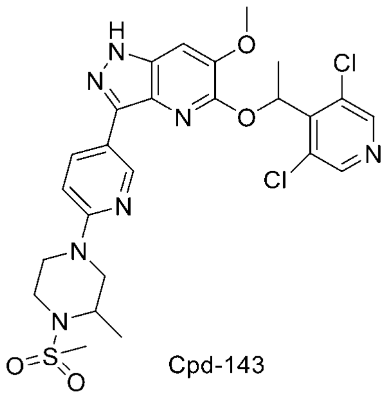

Cpd-143 ,

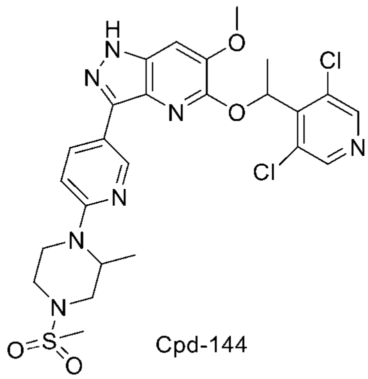

Cpd-144 ,

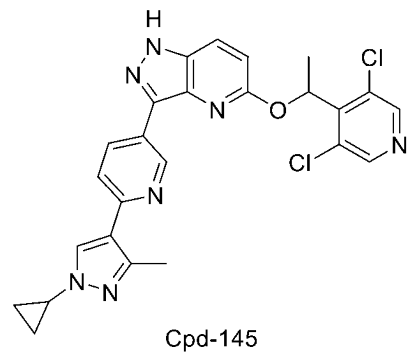

Cpd-145 ,

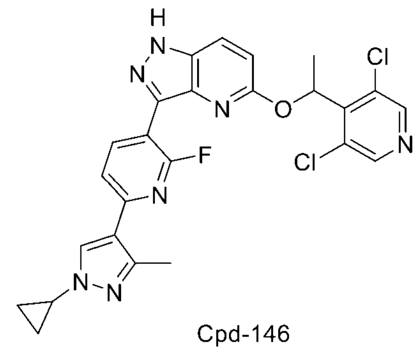

Cpd-146 ,

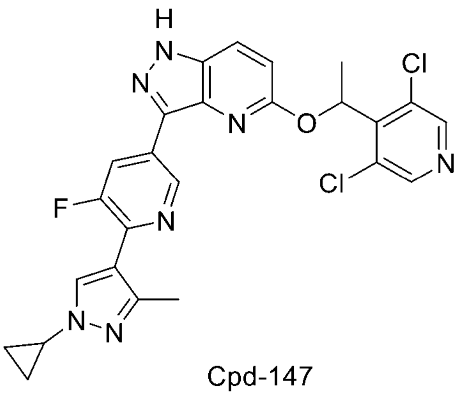

Cpd-147 ,

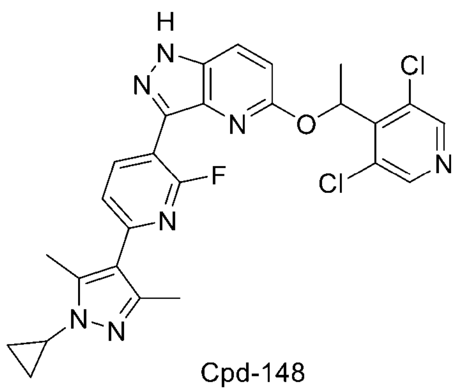

Cpd-148 ,

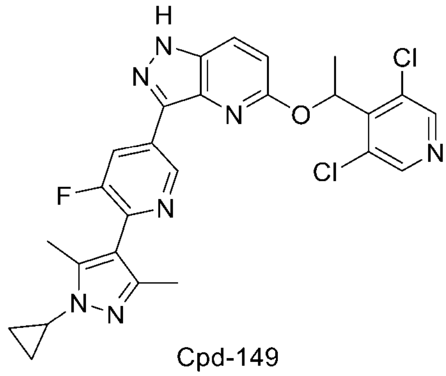

Cpd-149 ,

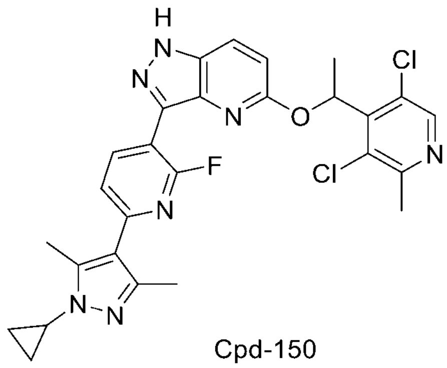

Cpd-150 ,

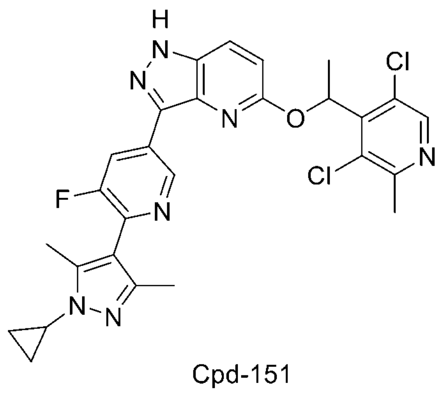

Cpd-151 ,

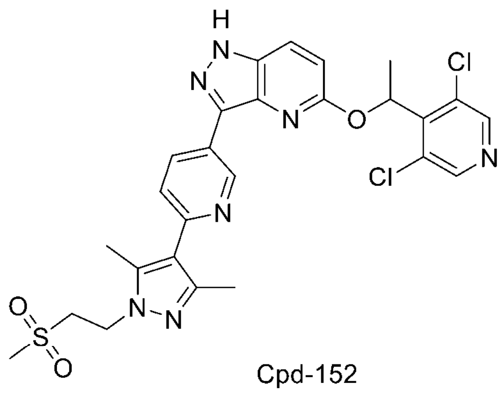

Cpd-152 ,

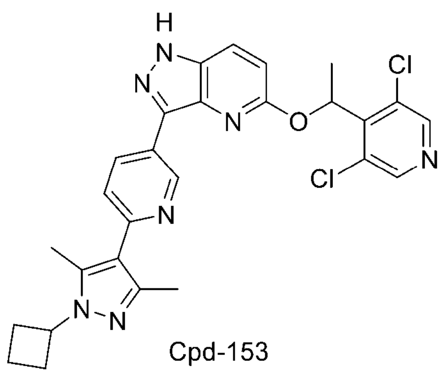

Cpd-153 ,

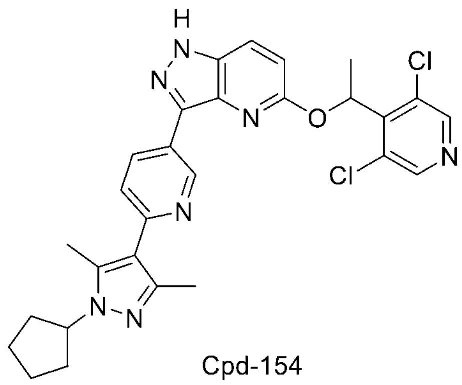

Cpd-154 ,

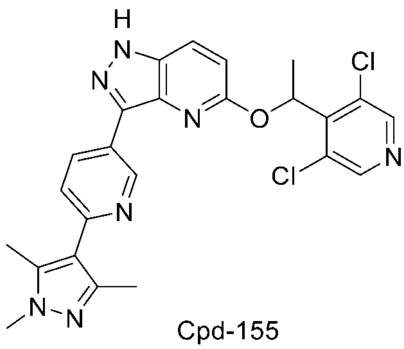

Cpd-155

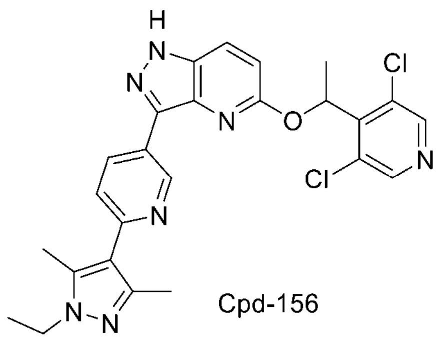

Cpd-156

,

,

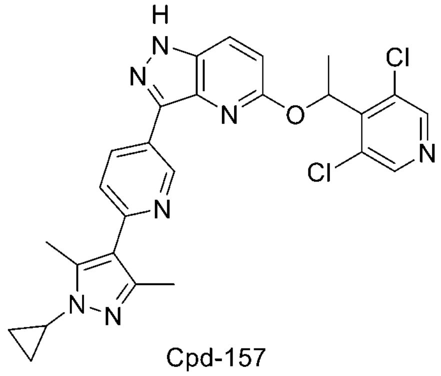

Cpd-157

,

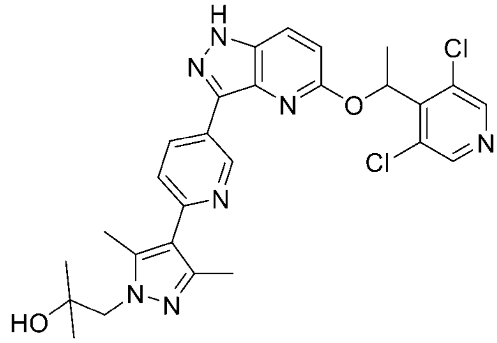

Cpd-158

,

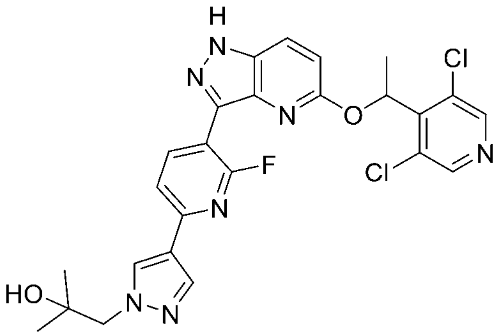

Cpd-159

,

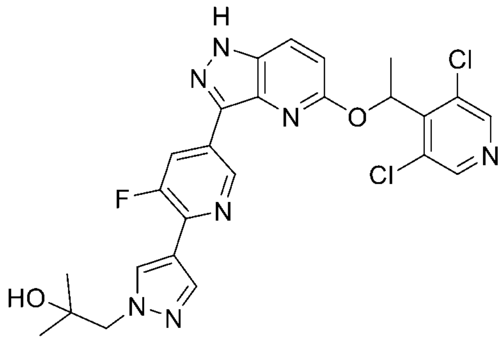

Cpd-160

,

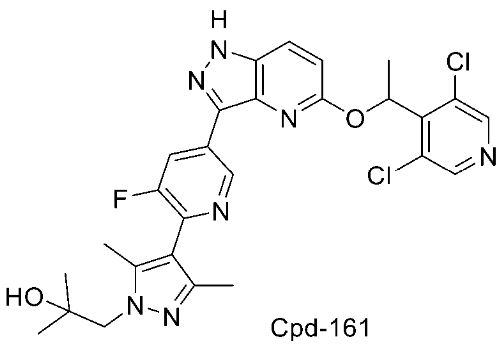

Cpd-161

,

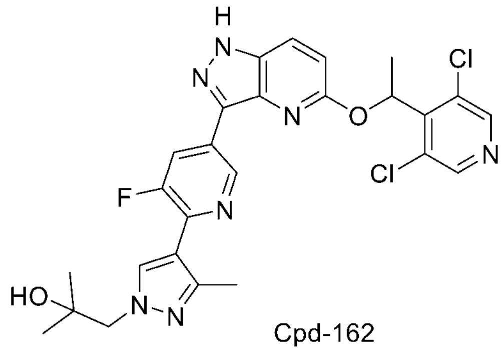

Cpd-162

,

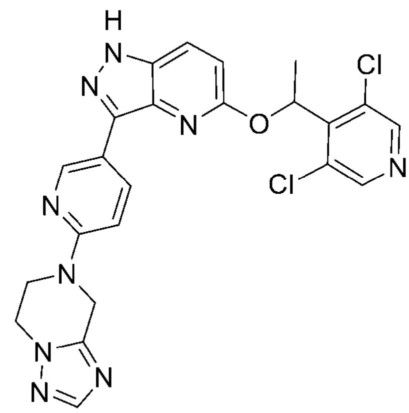

Cpd-163

,

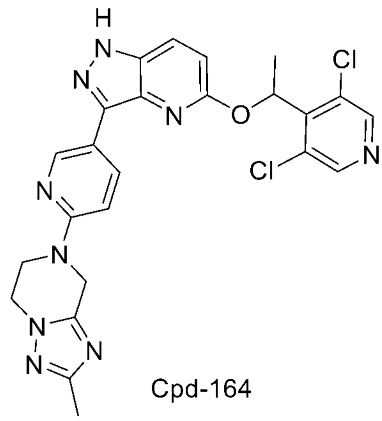

Cpd-164

,

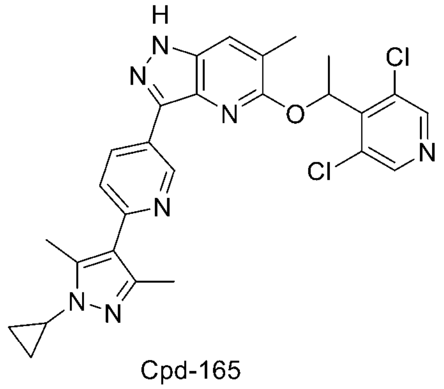
Cpd-165
,

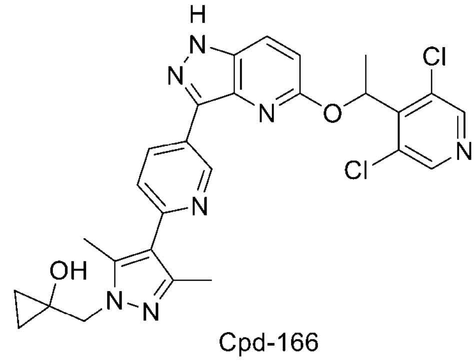
Cpd-166
,

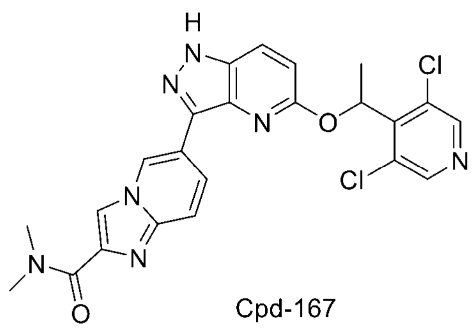
Cpd-167
,

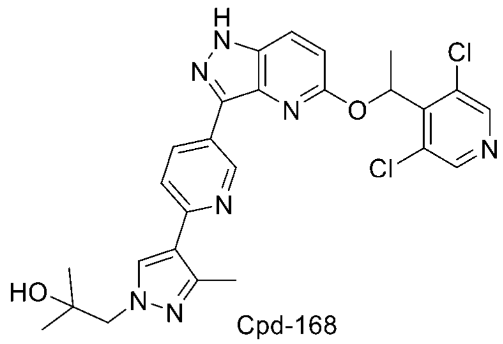
Cpd-168
,

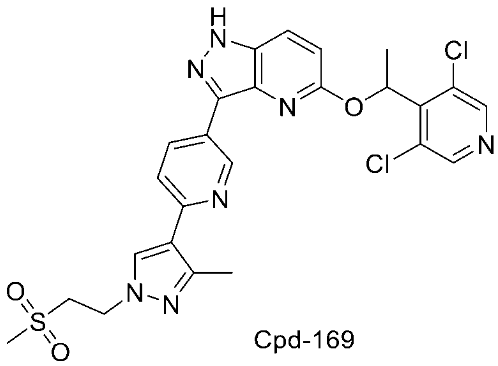
Cpd-169
,

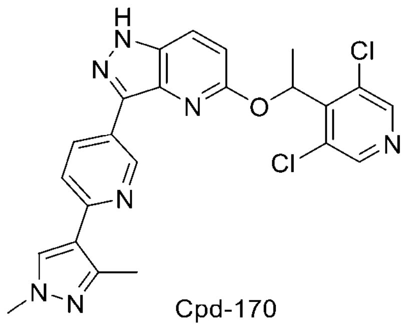
Cpd-170
,

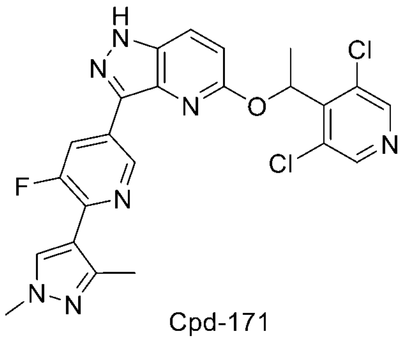
Cpd-171
,

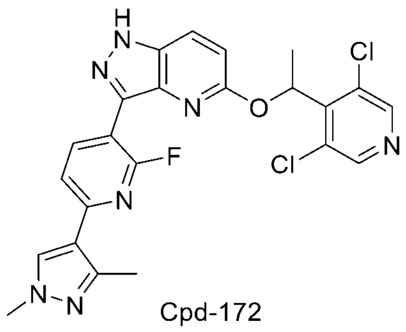
Cpd-172
,

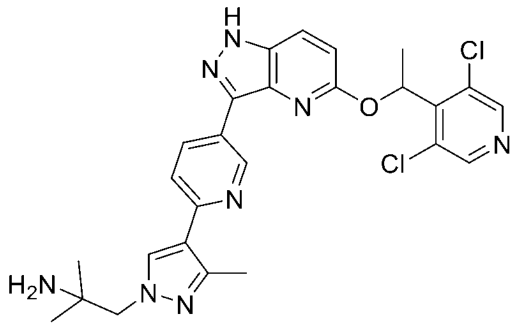

Cpd-173 ,

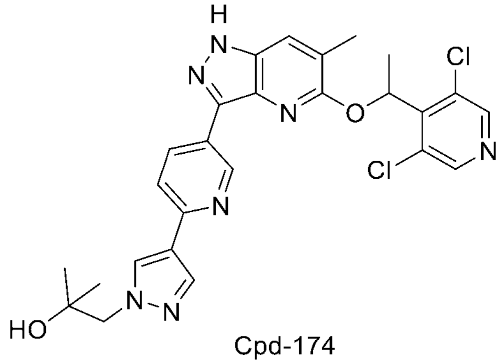

Cpd-174 ,

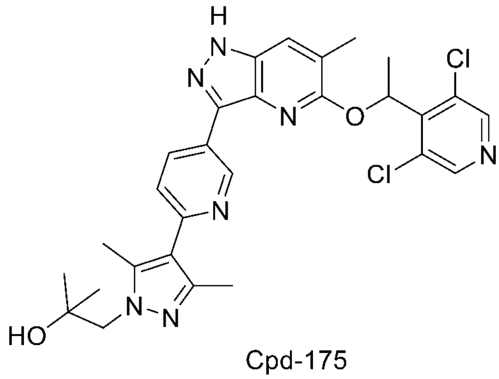

Cpd-175 ,

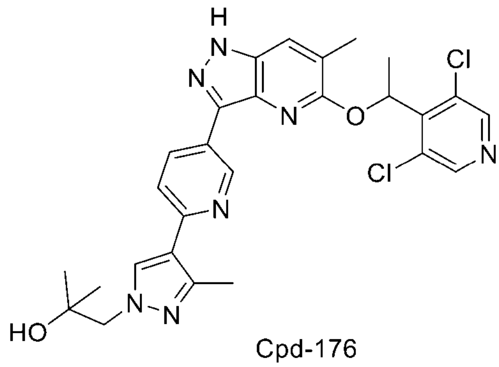

Cpd-176 ,

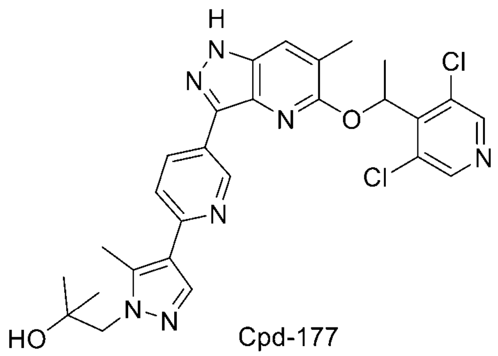

Cpd-177 ,

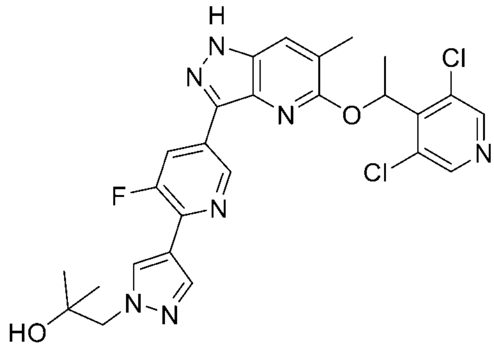

Cpd-178 ,

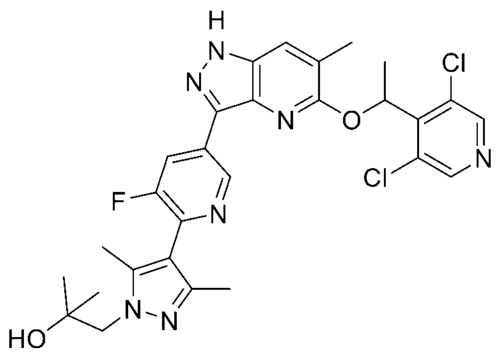

Cpd-179 ,

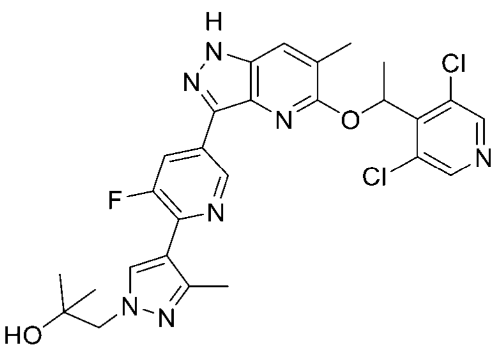

Cpd-180 ,

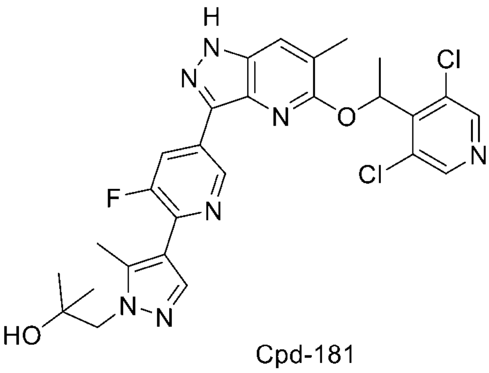

Cpd-181 ,

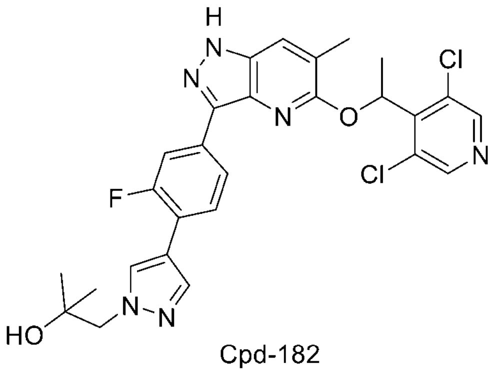

Cpd-182 ,

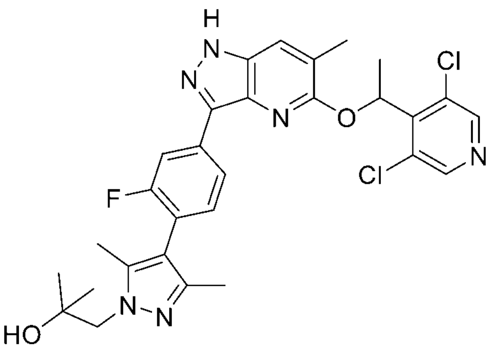

Cpd-183 ,

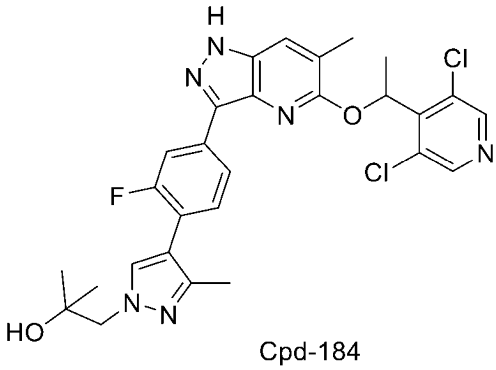

Cpd-184 ,

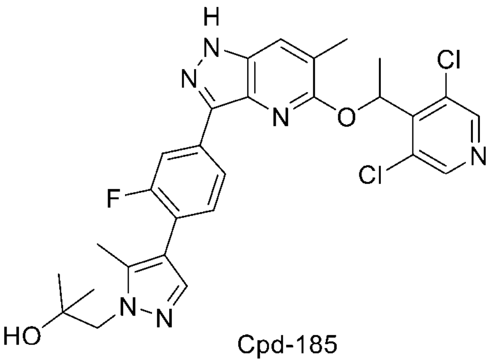

Cpd-185 ,

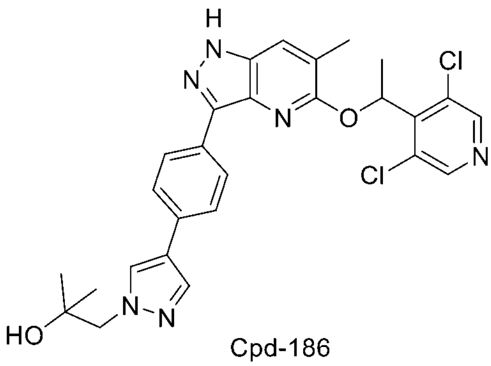

Cpd-186 ,

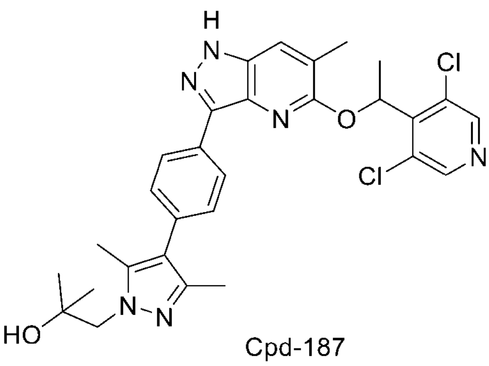

Cpd-187 ,

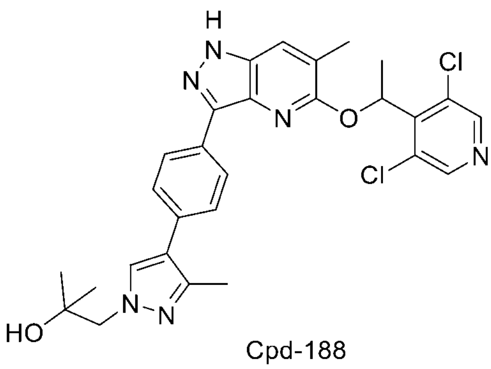

Cpd-188 ,

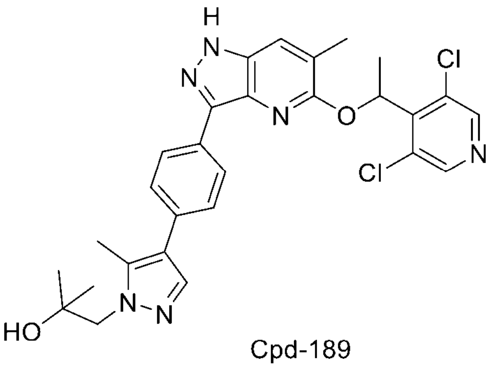

Cpd-189 ,

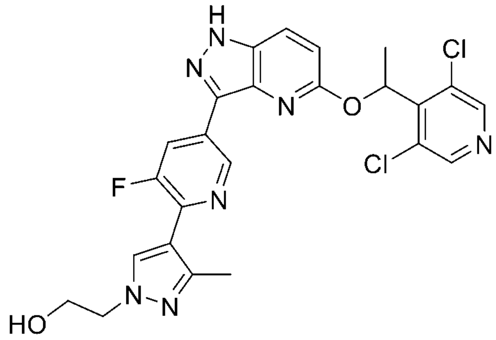

Cpd-190 ,

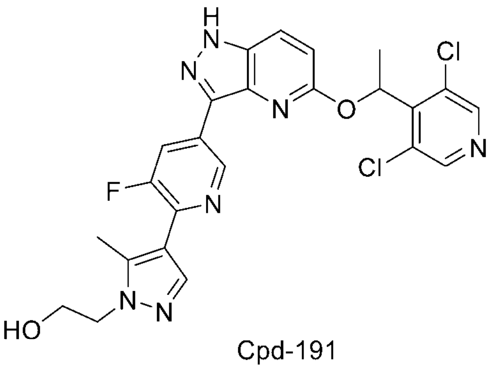

Cpd-191 ,

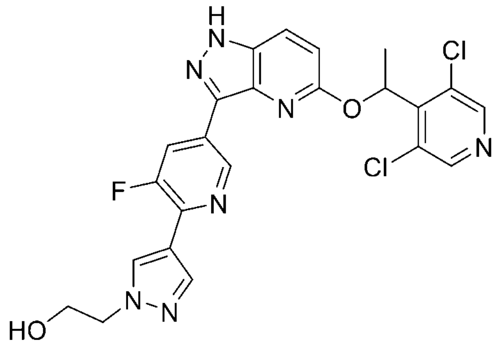

Cpd-192 ,

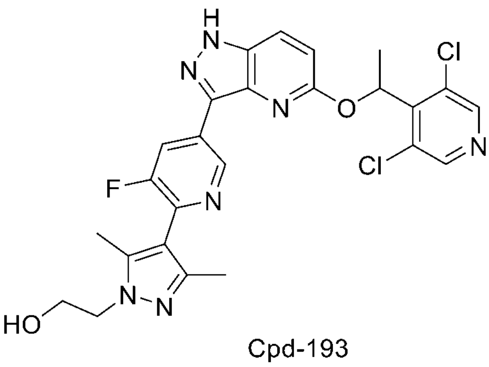

Cpd-193 ,

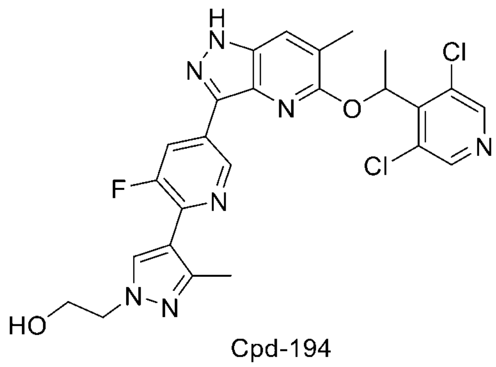

Cpd-194 ,

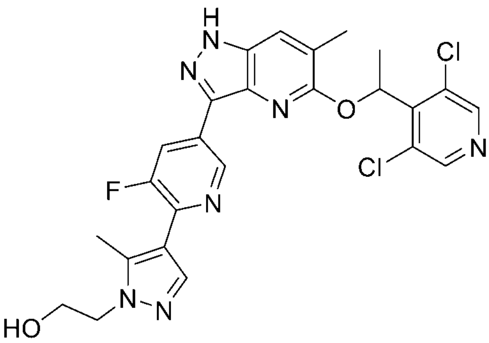

Cpd-195 ,

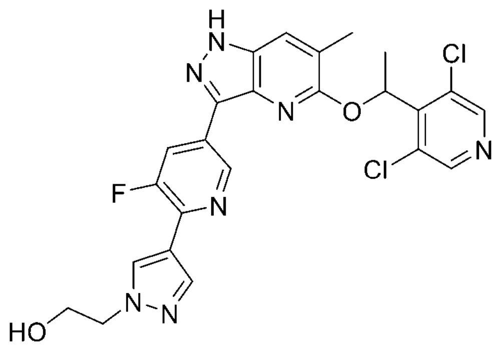

Cpd-196 ,

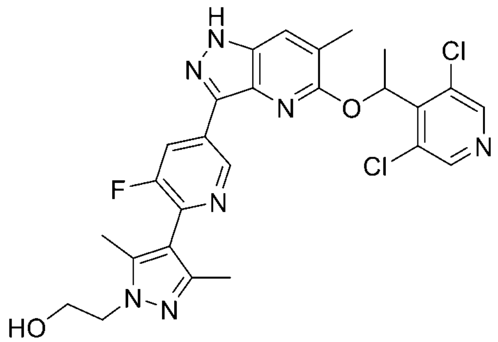

Cpd-197 ,

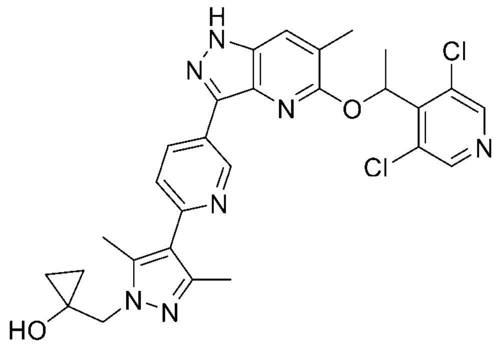

Cpd-198 ,

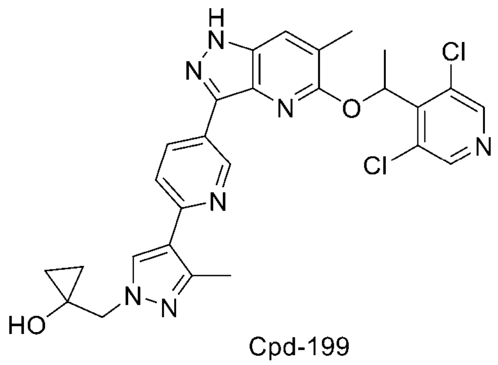

Cpd-199 ,

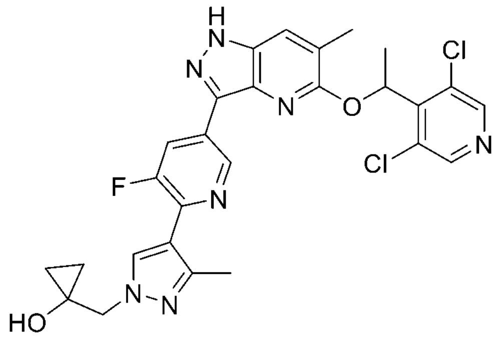

Cpd-200 ,

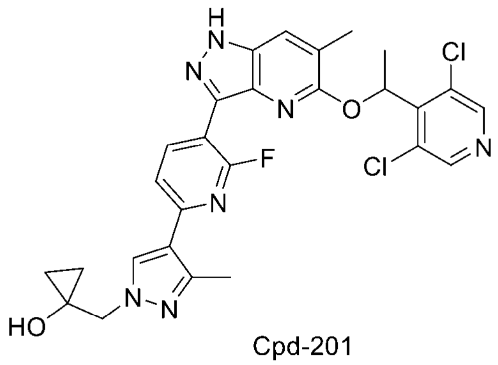

Cpd-201 ,

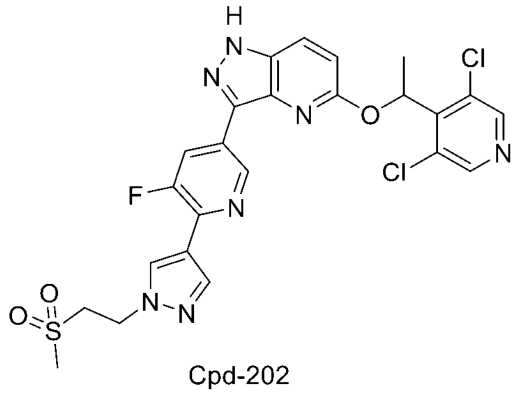

Cpd-202 ,

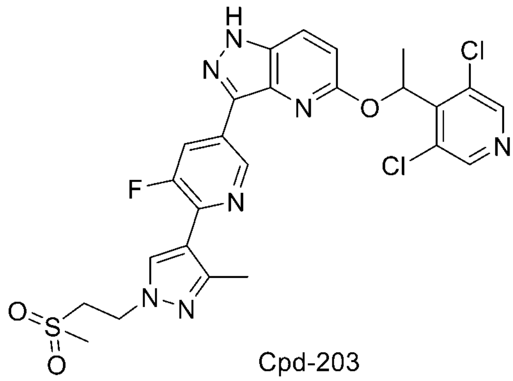

Cpd-203 ,

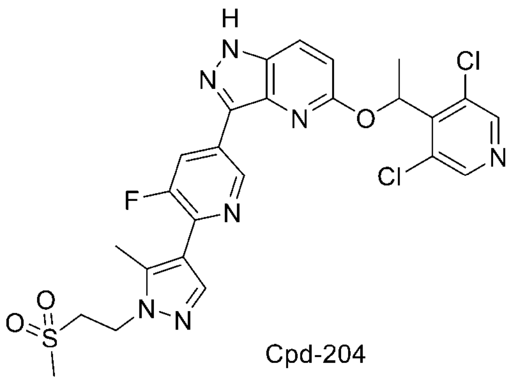

Cpd-204 ,

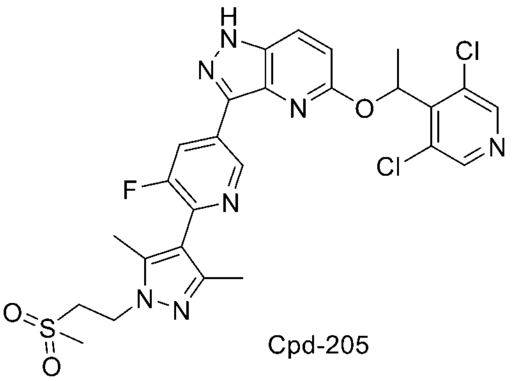

Cpd-205 ,

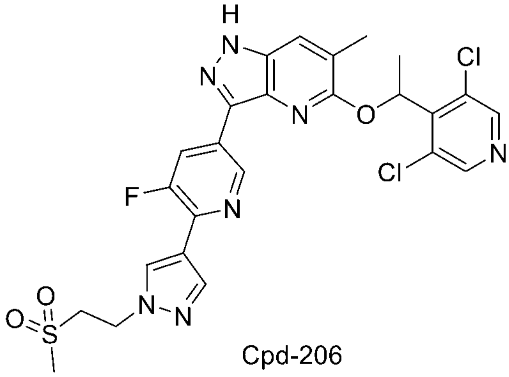

Cpd-206 ,

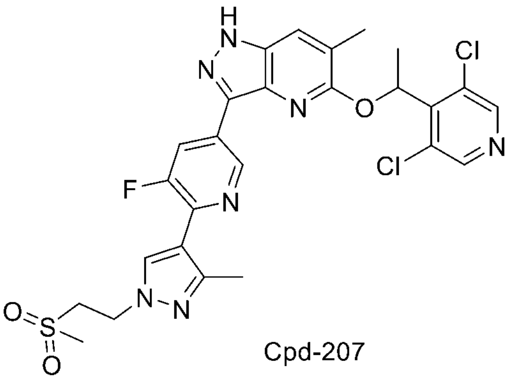

Cpd-207 ,

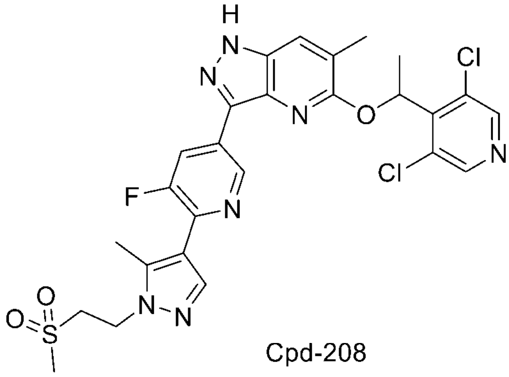

Cpd-208 ,

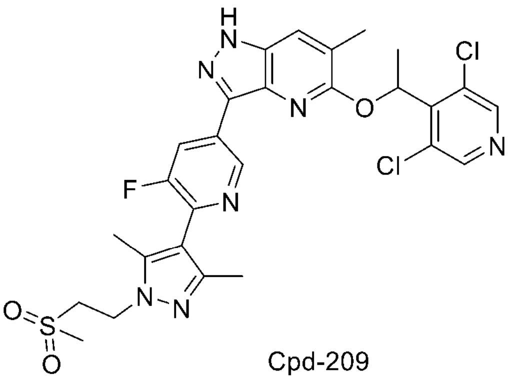

Cpd-209 ,

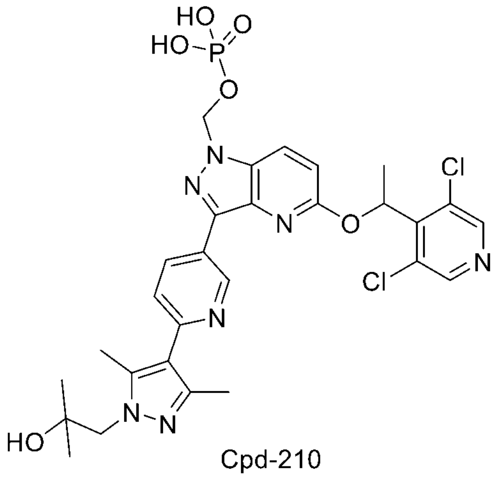

Cpd-210 ,

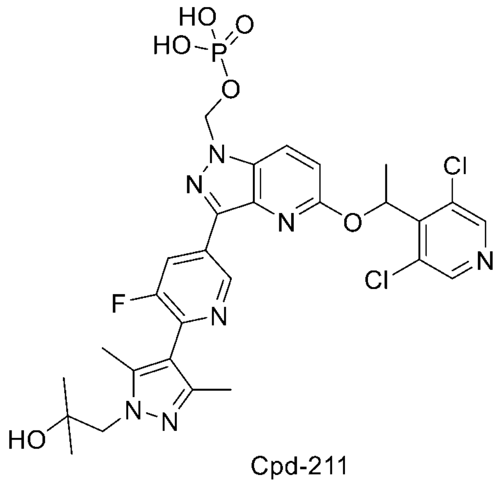

Cpd-211 ,

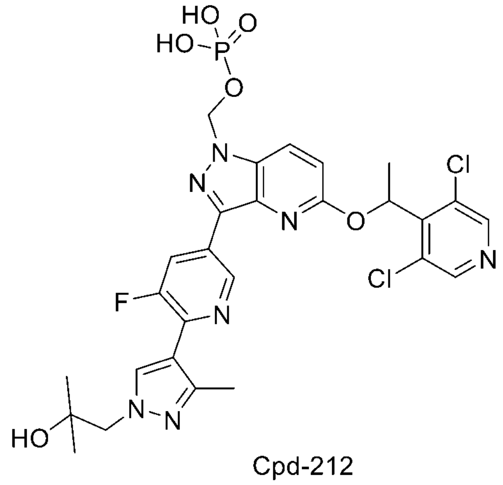

Cpd-212 ,

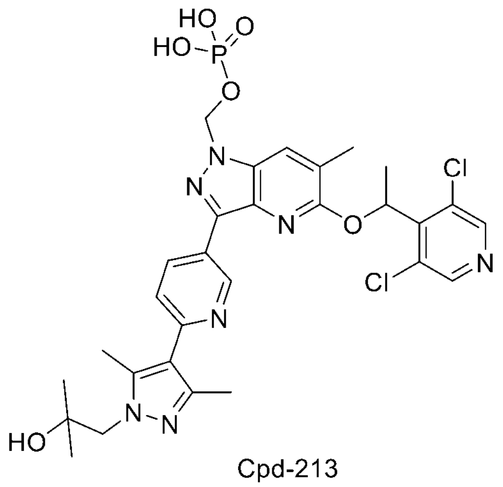

Cpd-213 ,

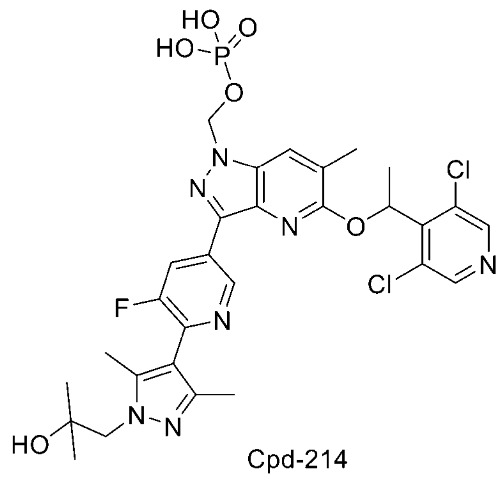

Cpd-214 ,

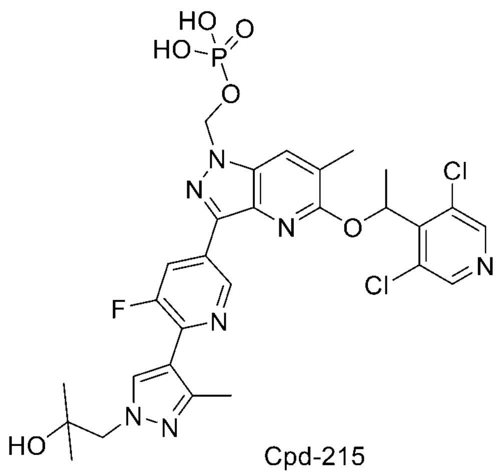

Cpd-215 ,

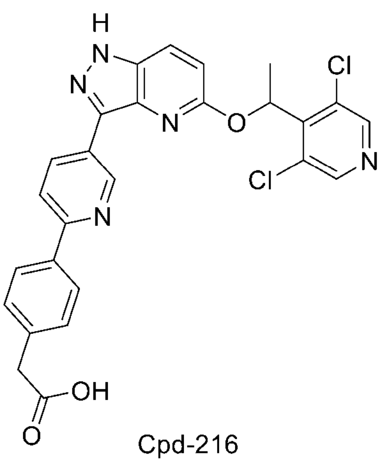

Cpd-216 ,

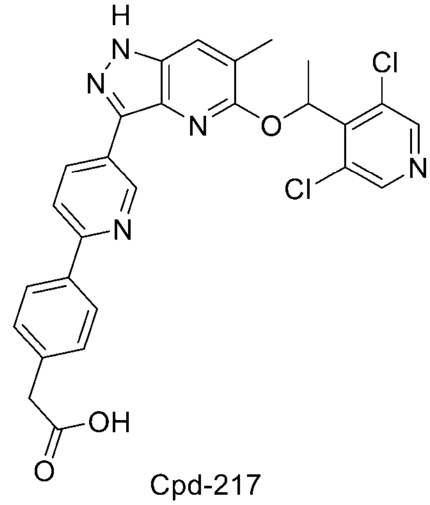

Cpd-217 ,

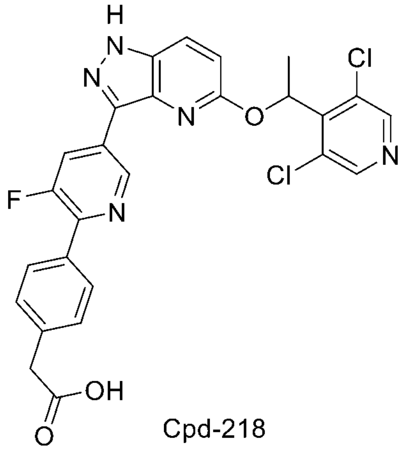

Cpd-218 ,

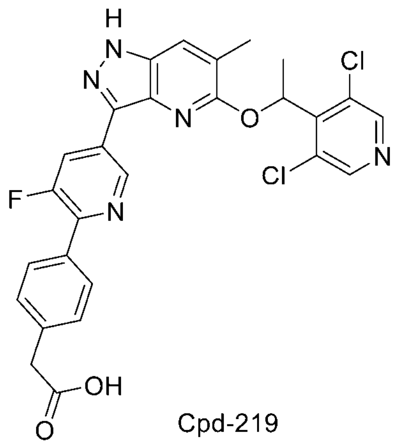

Cpd-219 ,

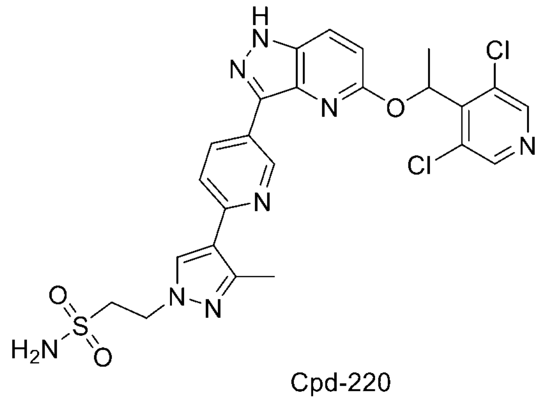

Cpd-220 ,

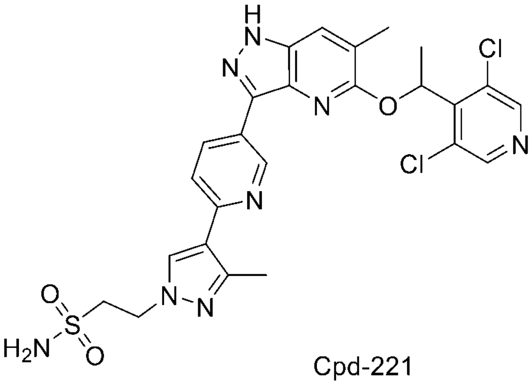

Cpd-221 ,

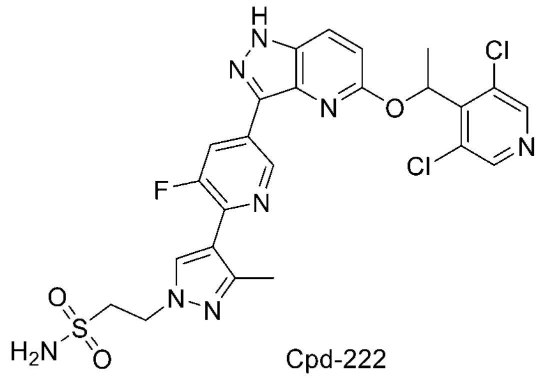

Cpd-222 ,

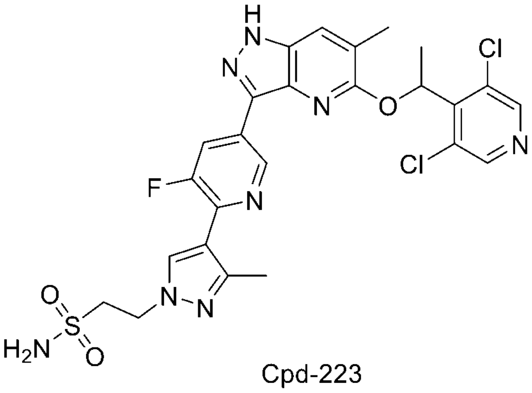

Cpd-223 ,

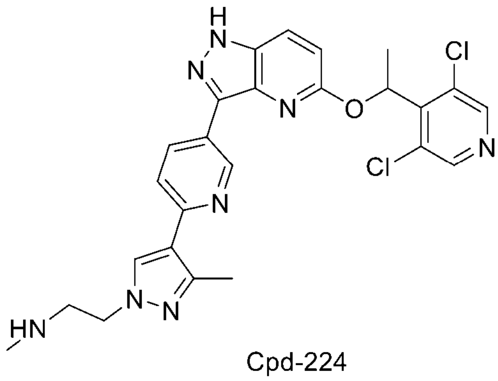

Cpd-224 ,

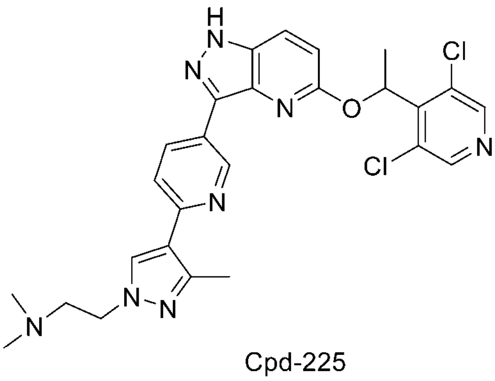

Cpd-225 ,

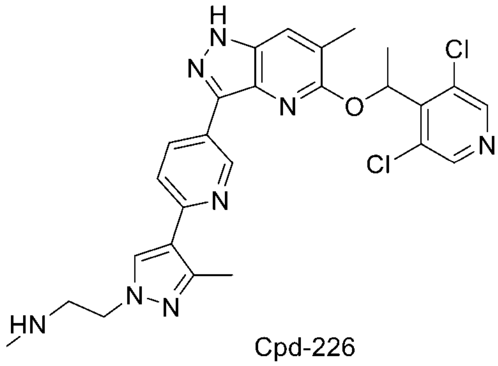

Cpd-226 ,

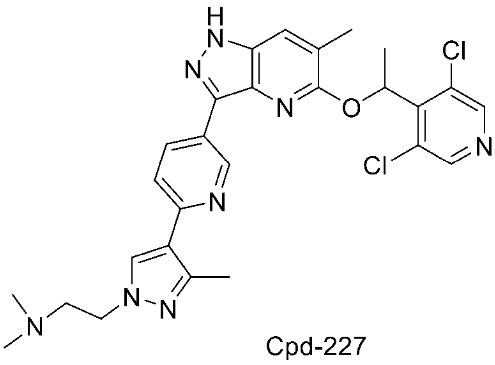

Cpd-227 ,

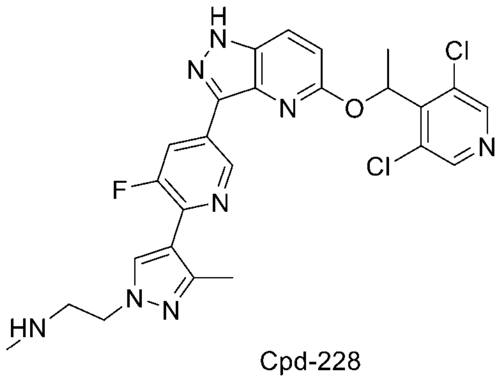

Cpd-228 ,

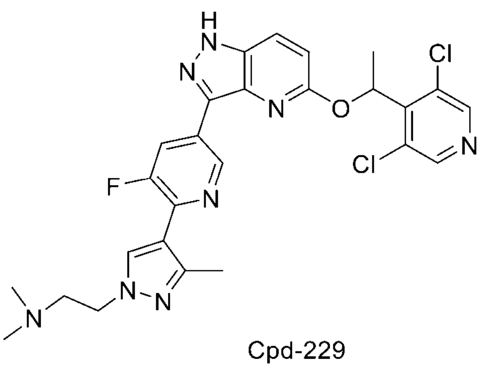

Cpd-229 ,

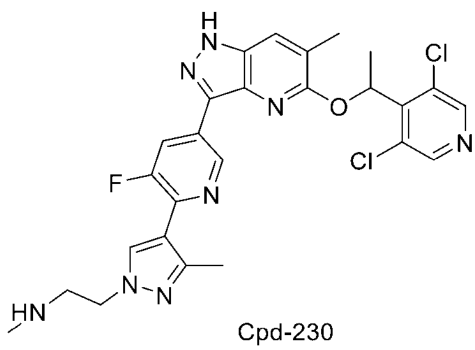

Cpd-230 ,

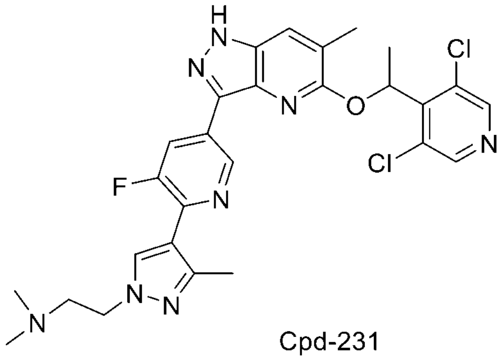

Cpd-231 .

According to some embodiments, the compound of Formula (I) is selected from:

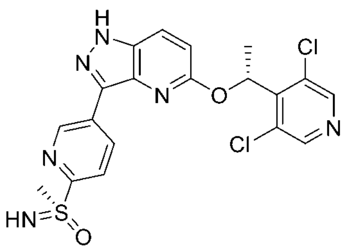

,

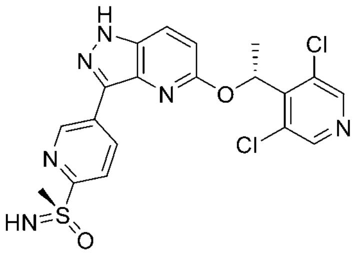

,

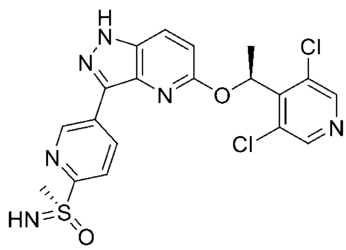

,

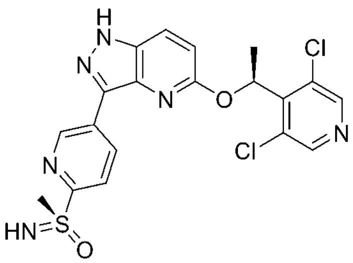

,

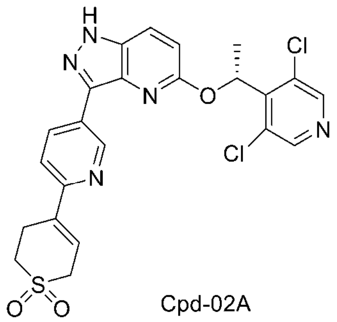

Cpd-02A ,

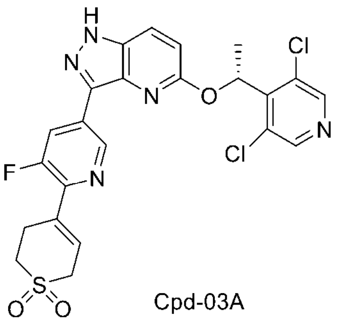

Cpd-03A ,

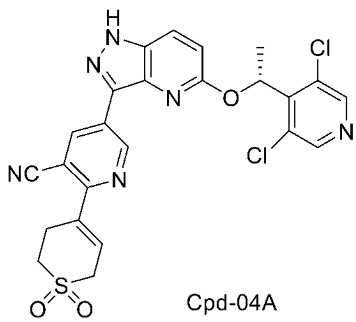

Cpd-04A ,

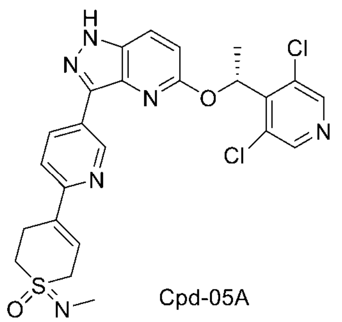

Cpd-05A ,

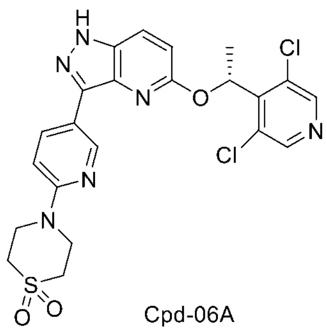

Cpd-06A ,

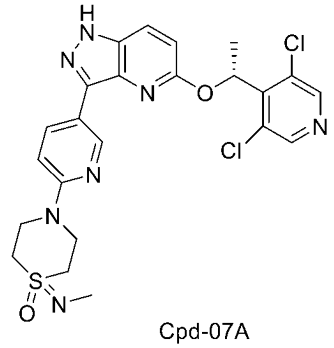
Cpd-07A ,

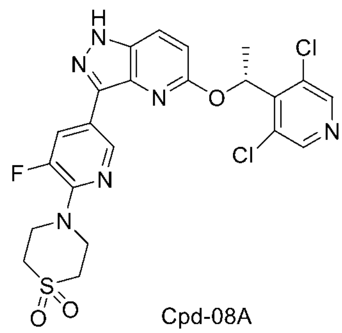
Cpd-08A ,

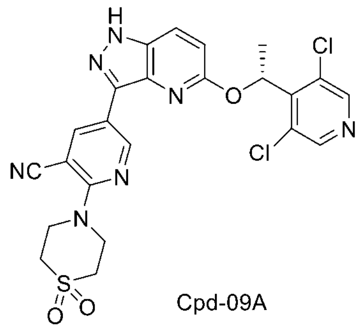
Cpd-09A ,

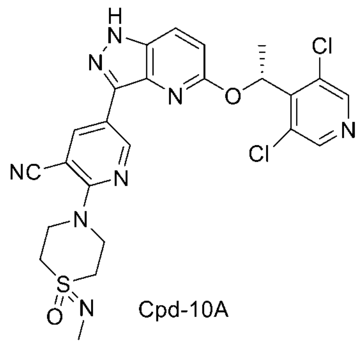
Cpd-10A ,

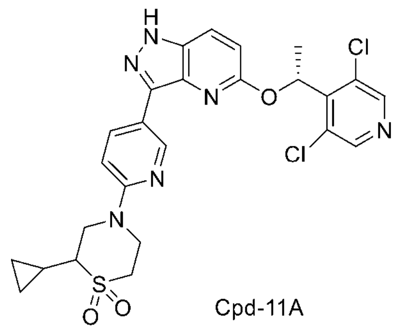
Cpd-11A ,

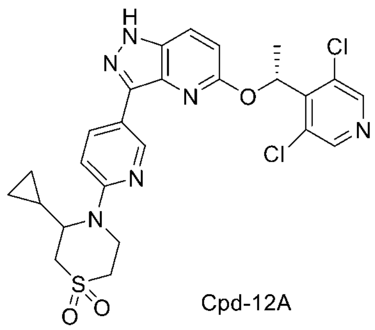
Cpd-12A ,

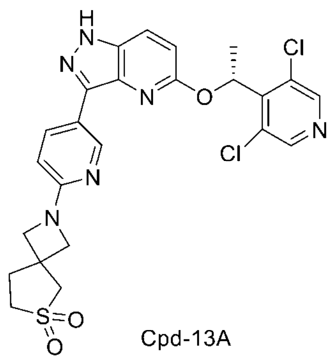
Cpd-13A ,

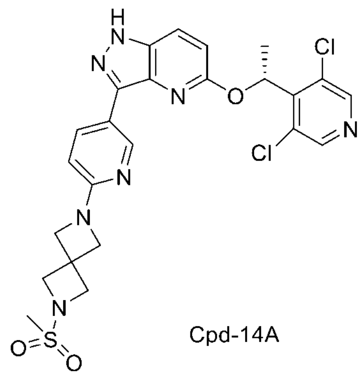
Cpd-14A ,

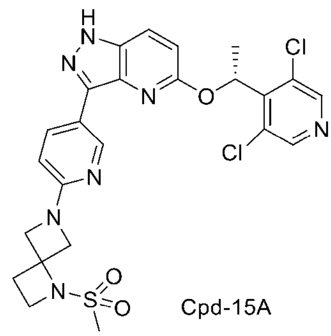
Cpd-15A ,

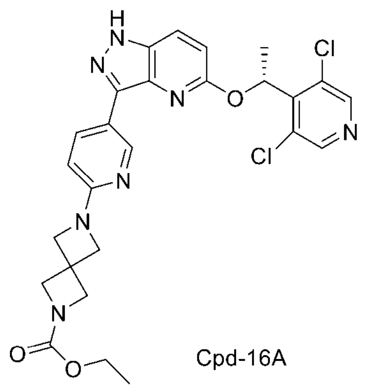
Cpd-16A ,

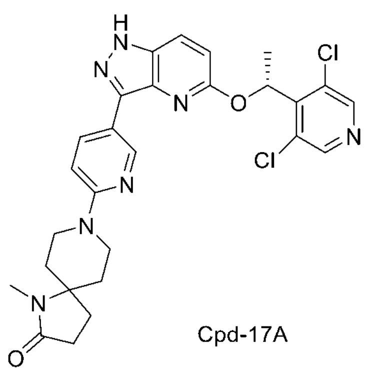
Cpd-17A ,

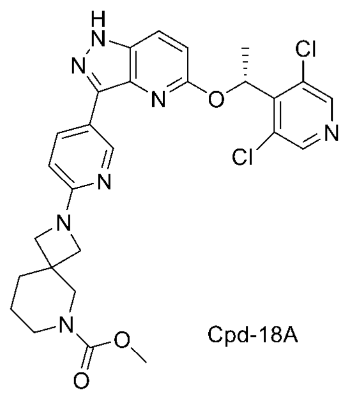
Cpd-18A ,

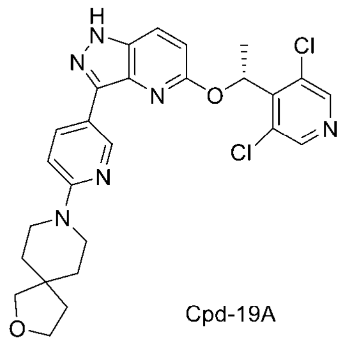

Cpd-19A ,

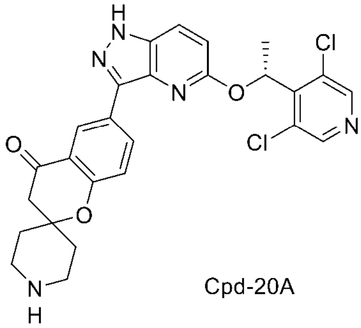

Cpd-20A ,

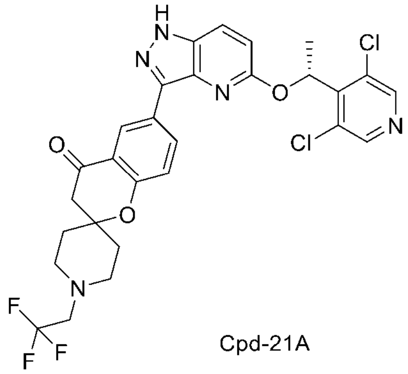

Cpd-21A ,

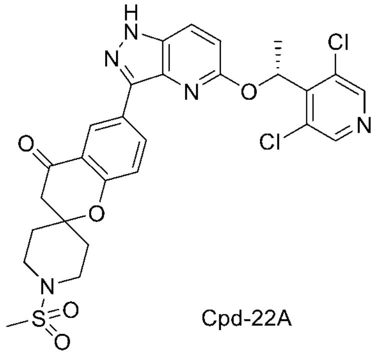

Cpd-22A ,

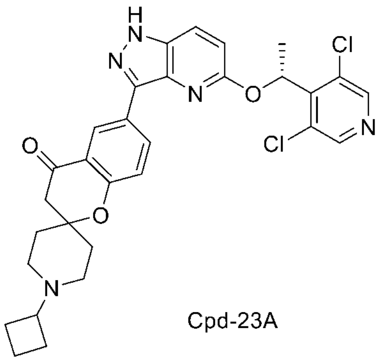

Cpd-23A ,

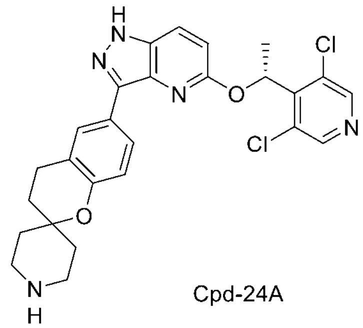

Cpd-24A ,

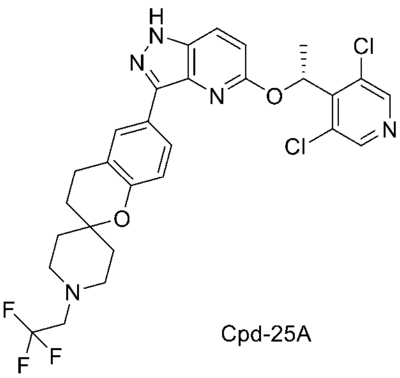

Cpd-25A ,

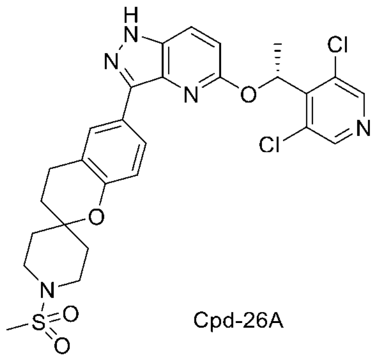

Cpd-26A ,

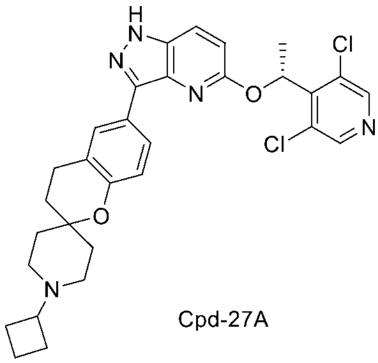

Cpd-27A ,

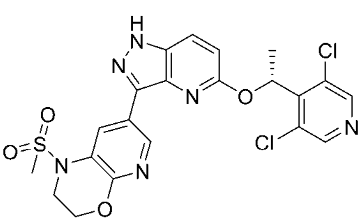

Cpd-28A ,

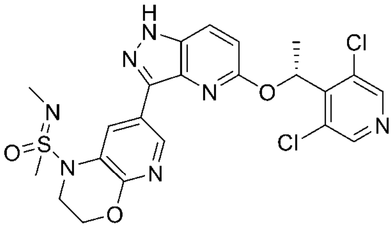

Cpd-29A ,

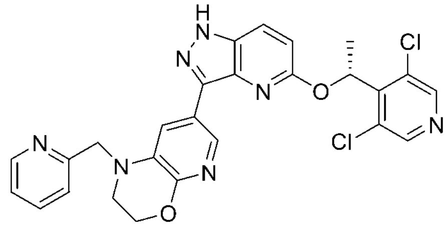

Cpd-30A ,

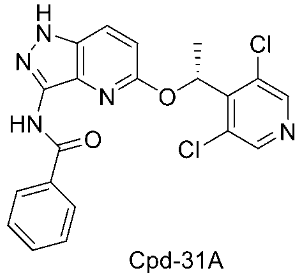
Cpd-31A ,

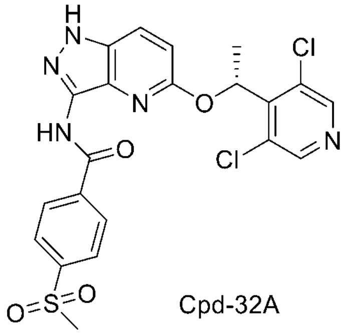
Cpd-32A ,

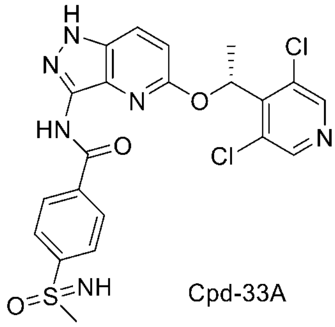
Cpd-33A ,

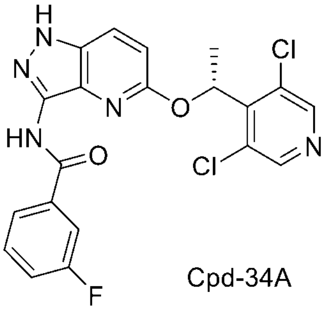
Cpd-34A ,

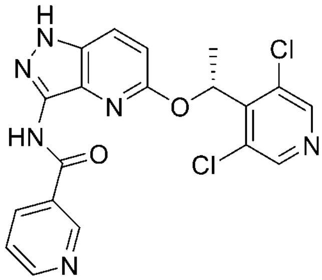
Cpd-35A ,

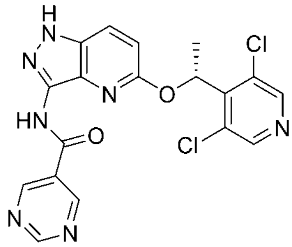
Cpd-36A ,

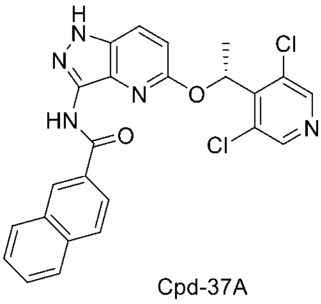
Cpd-37A ,

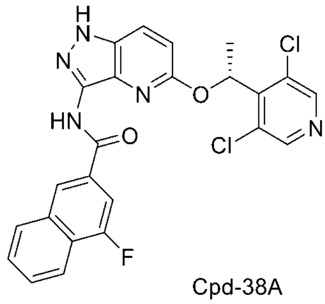
Cpd-38A ,

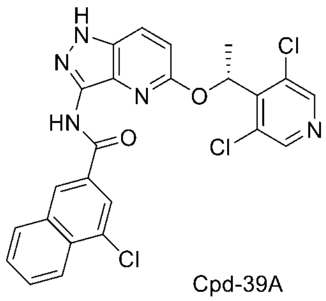
Cpd-39A ,

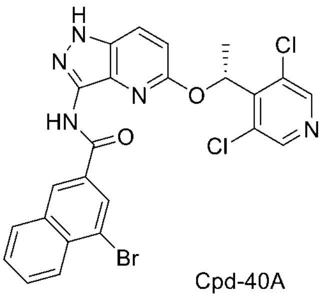
Cpd-40A ,

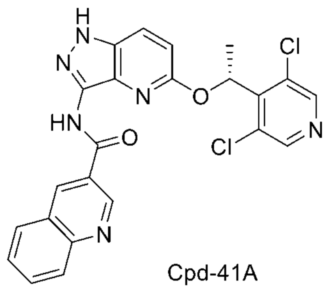
Cpd-41A ,

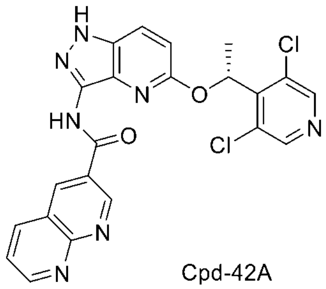
Cpd-42A ,

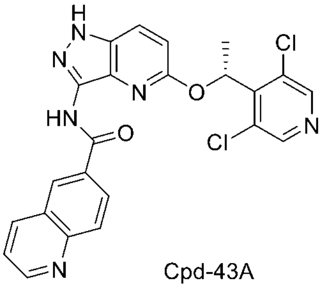
Cpd-43A ,

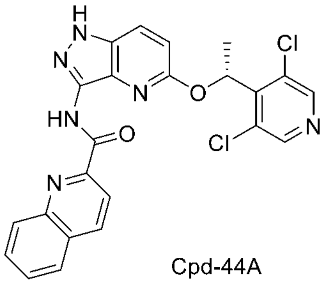
Cpd-44A ,

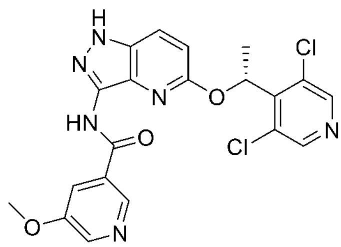
Cpd-45A ,

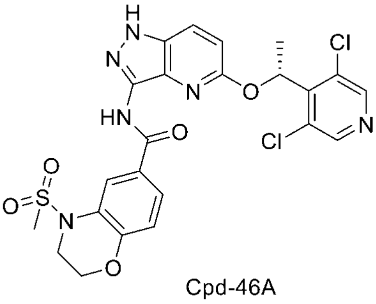
Cpd-46A ,

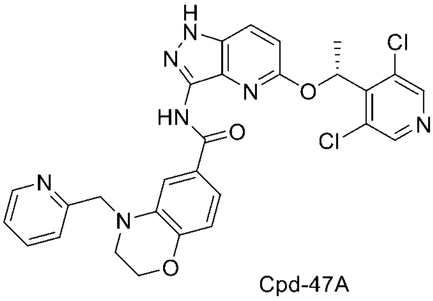
Cpd-47A ,

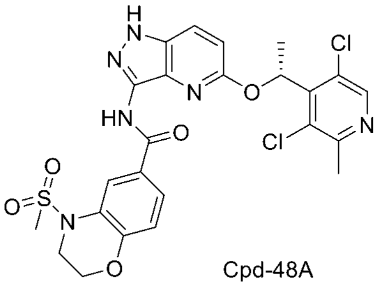
Cpd-48A ,

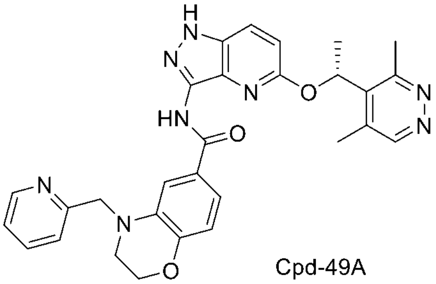
Cpd-49A ,

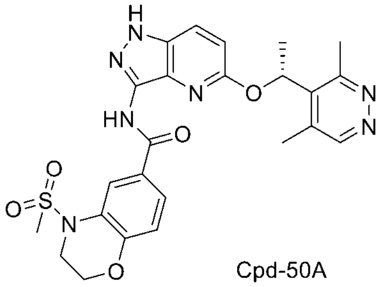
Cpd-50A ,

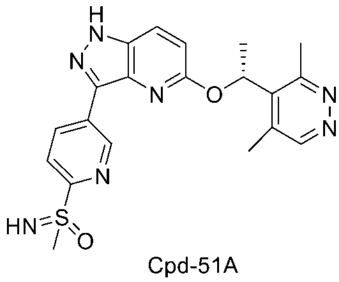
Cpd-51A ,

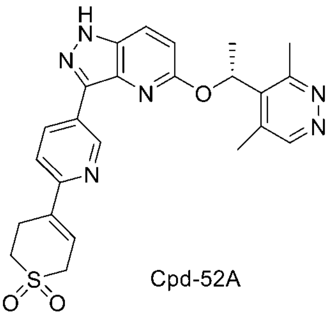
Cpd-52A ,

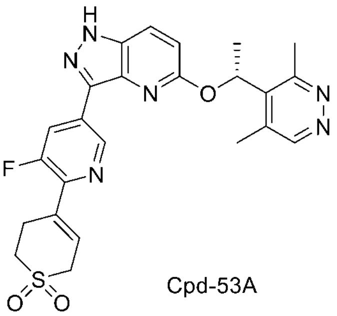
Cpd-53A ,

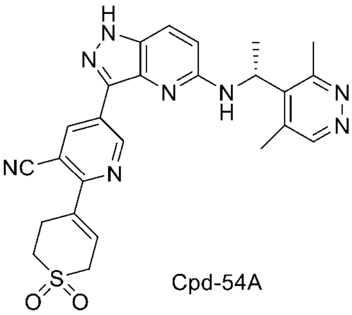
Cpd-54A ,

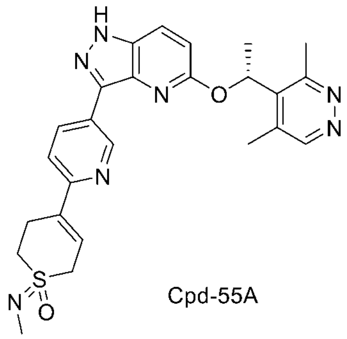
Cpd-55A ,

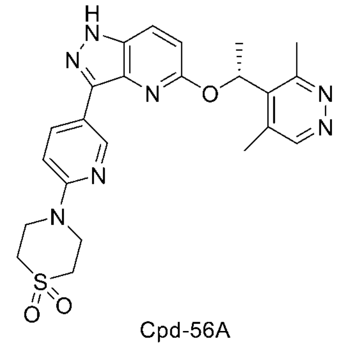
Cpd-56A ,

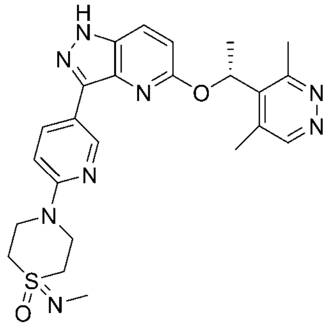
Cpd-57A ,

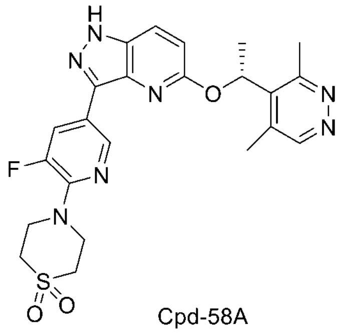
Cpd-58A ,

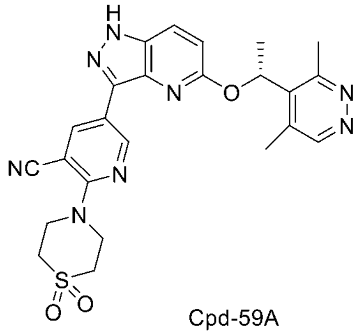
Cpd-59A ,

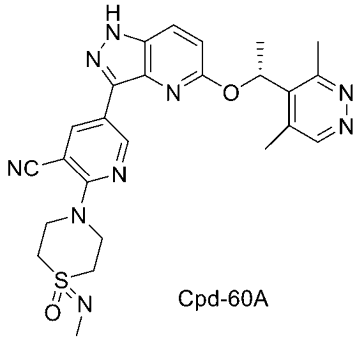
Cpd-60A ,

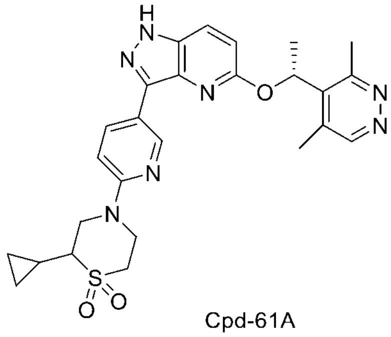
Cpd-61A ,

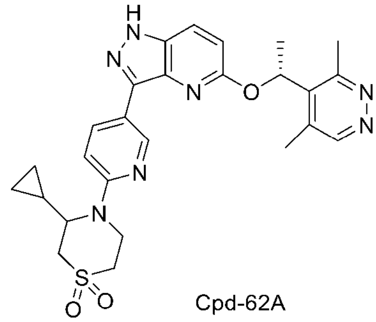
Cpd-62A ,

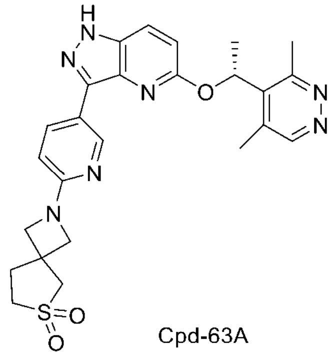
Cpd-63A ,

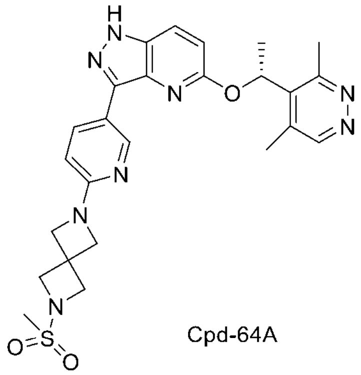
Cpd-64A ,

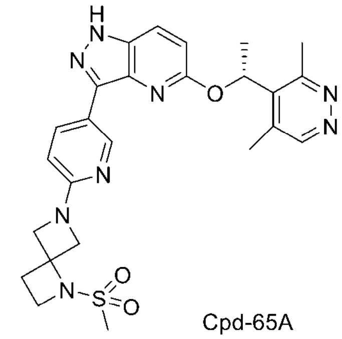
Cpd-65A ,

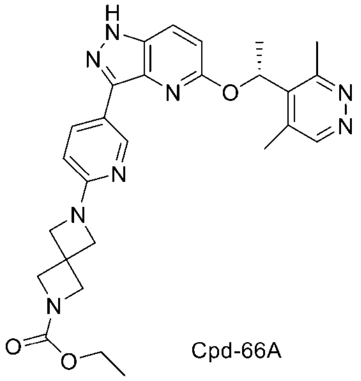
Cpd-66A ,

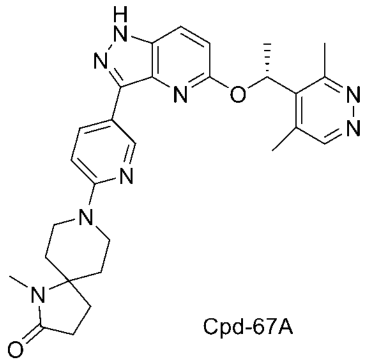
Cpd-67A ,

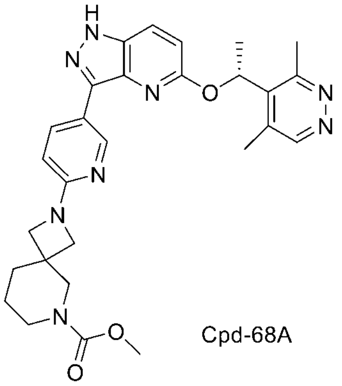
Cpd-68A ,

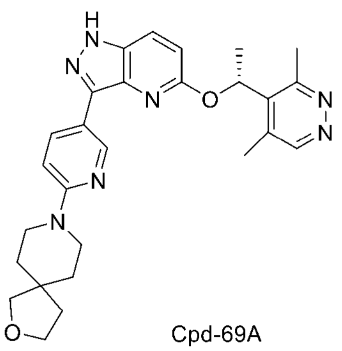
Cpd-69A ,

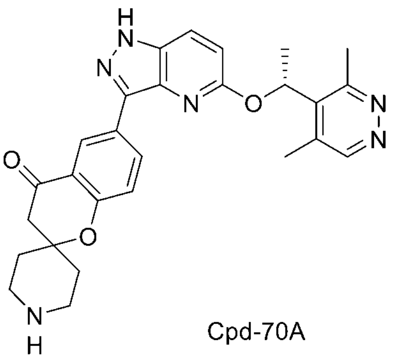

Cpd-70A ,

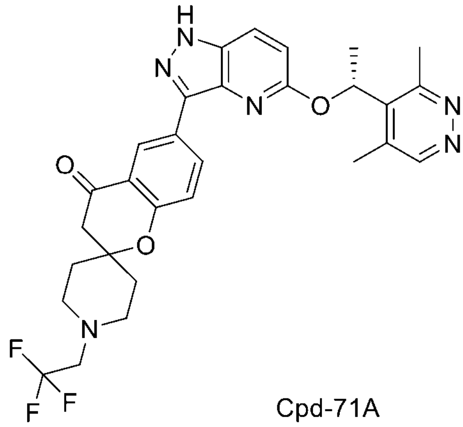

Cpd-71A ,

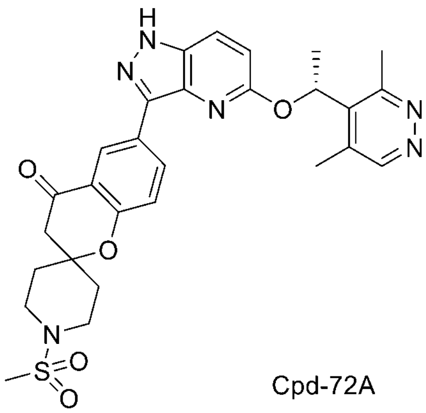

Cpd-72A ,

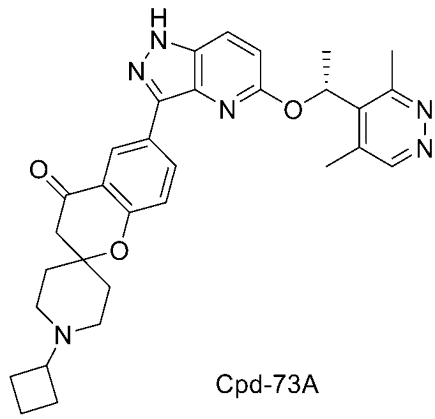

Cpd-73A ,

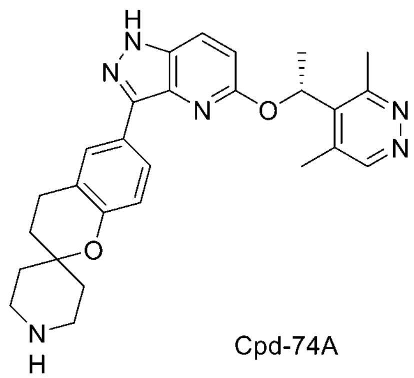

Cpd-74A ,

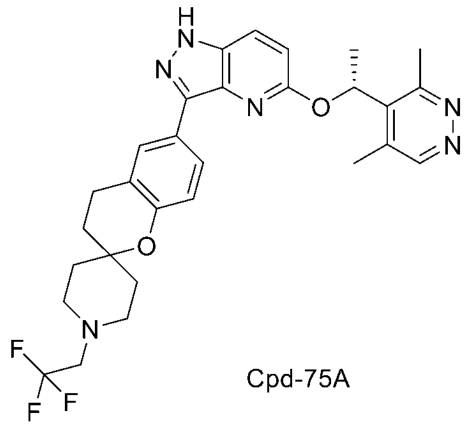

Cpd-75A ,

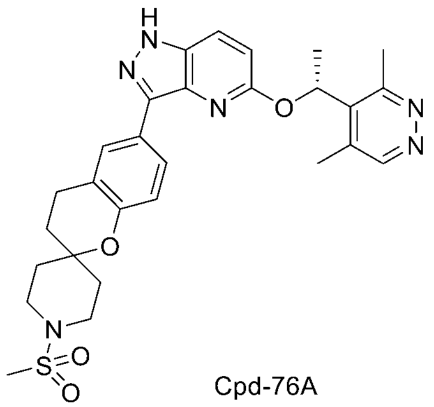
Cpd-76A ,

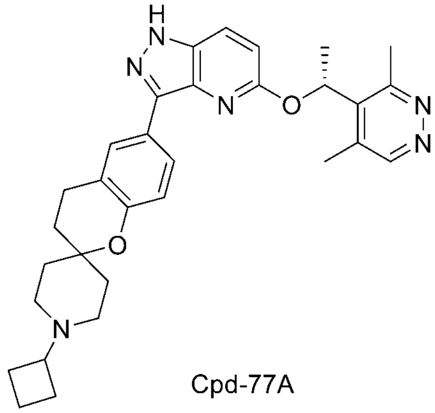
Cpd-77A ,

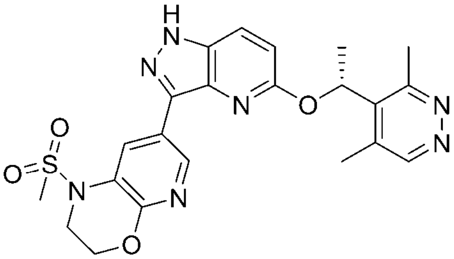
Cpd-78A ,

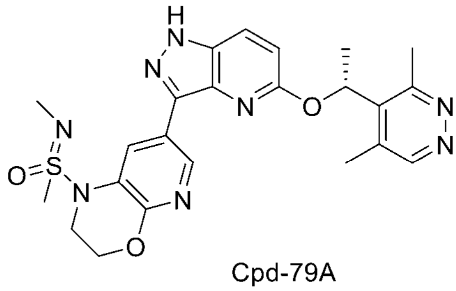
Cpd-79A ,

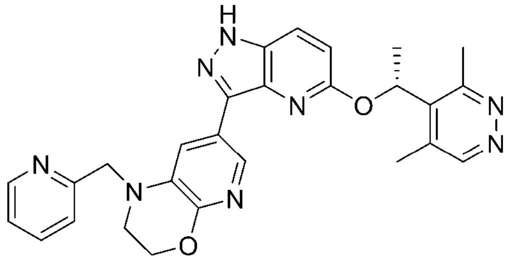
Cpd-80A ,

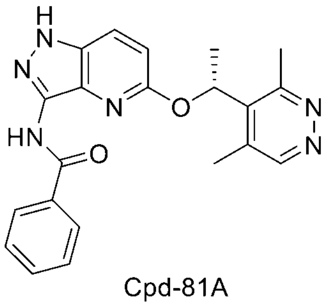
Cpd-81A ,

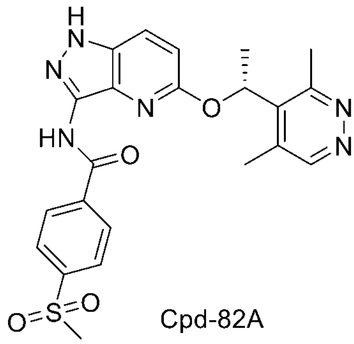
Cpd-82A ,

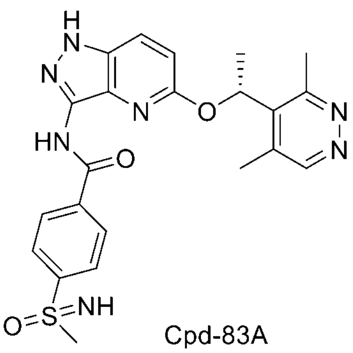
Cpd-83A ,

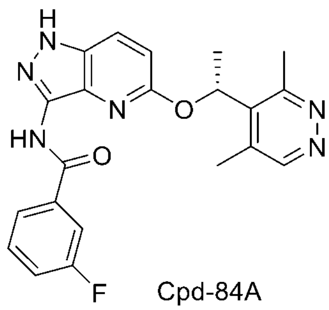
Cpd-84A ,

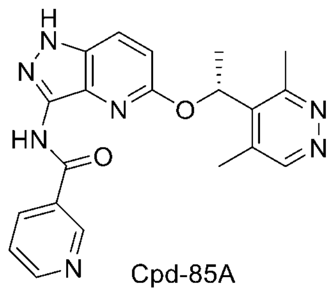
Cpd-85A ,

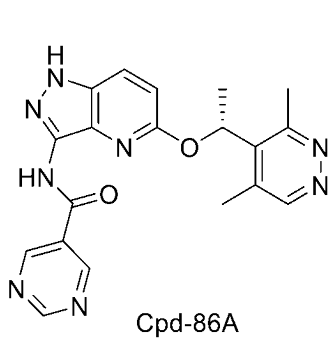
Cpd-86A ,

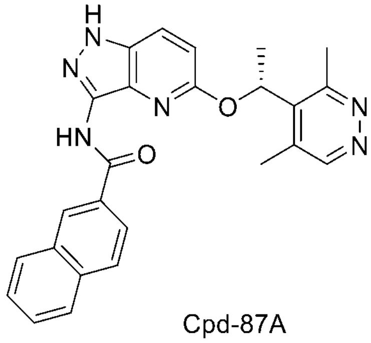
Cpd-87A ,

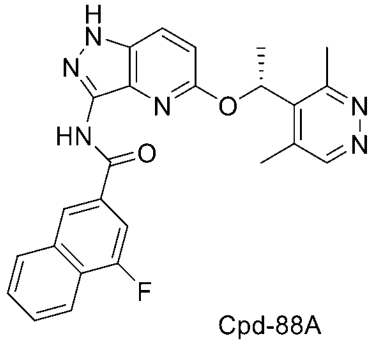
Cpd-88A ,

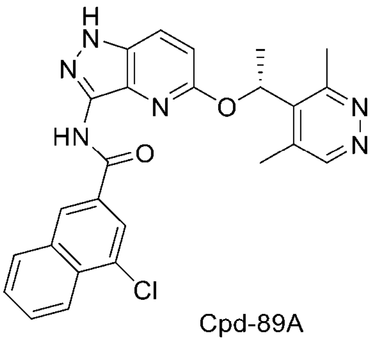
Cpd-89A ,

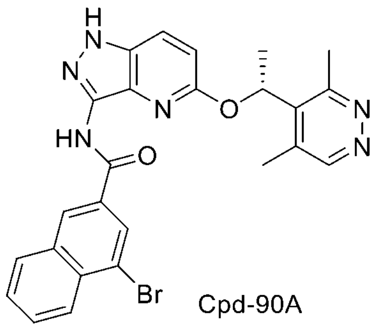
Cpd-90A ,

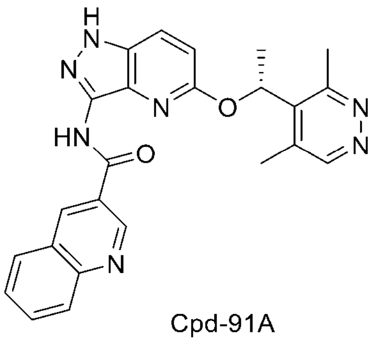
Cpd-91A ,

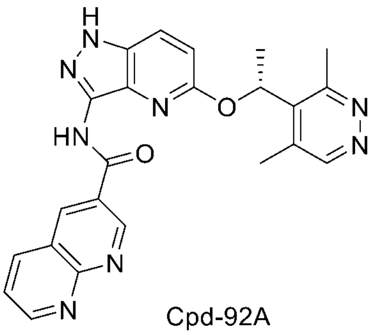
Cpd-92A ,

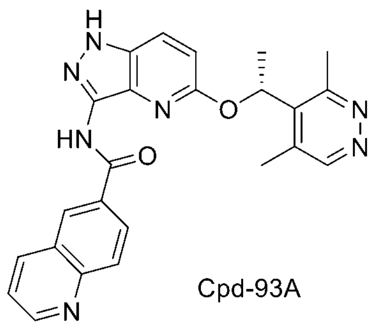
Cpd-93A ,

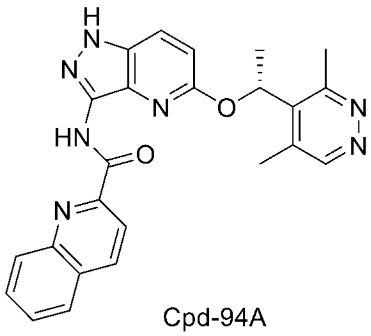
Cpd-94A ,

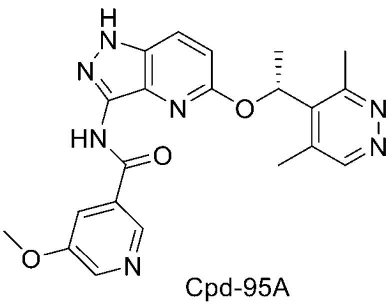
Cpd-95A ,

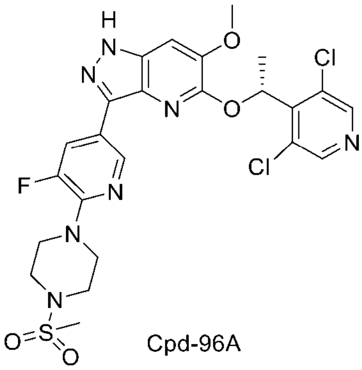
Cpd-96A ,

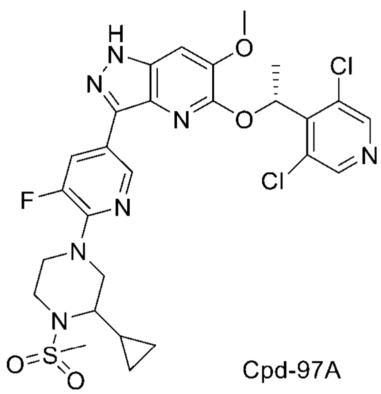
Cpd-97A ,

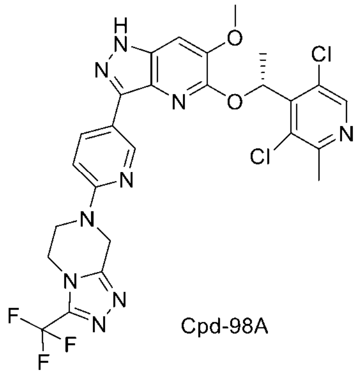
Cpd-98A ,
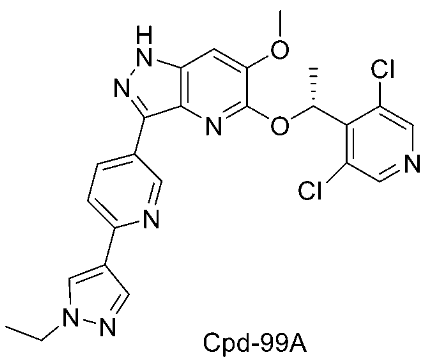
Cpd-99A ,
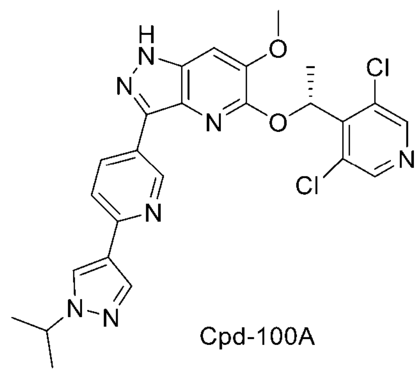
Cpd-100A ,

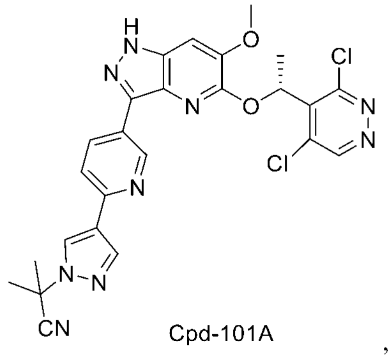
Cpd-101A ,
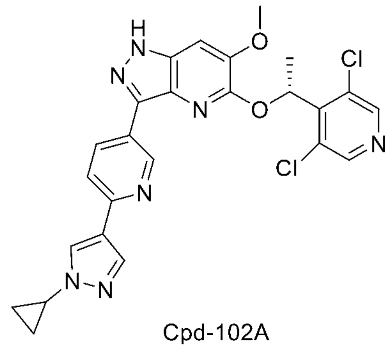
Cpd-102A ,
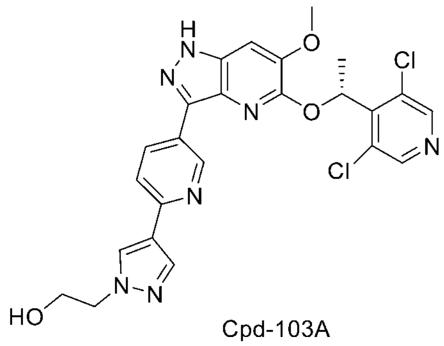
Cpd-103A ,

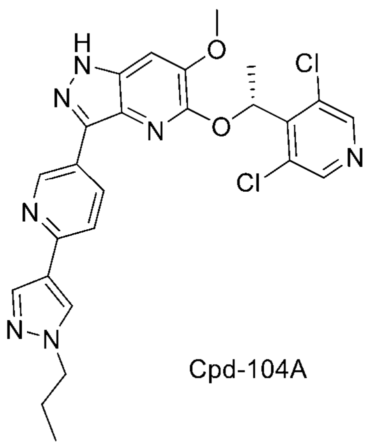
Cpd-104A ,
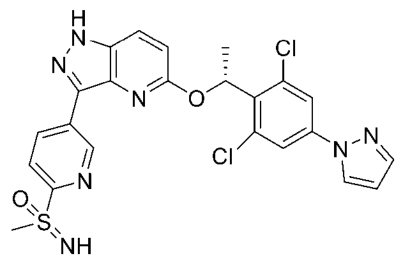
Cpd-105A ,
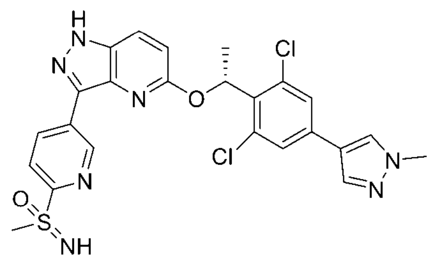
Cpd-106A ,

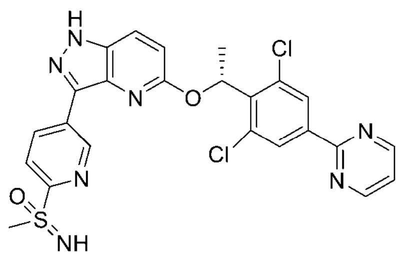
Cpd-107A ,
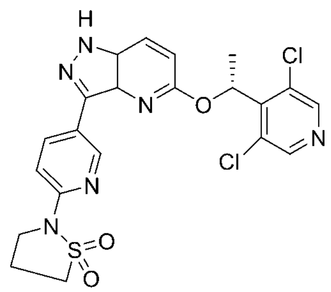
Cpd-108A ,
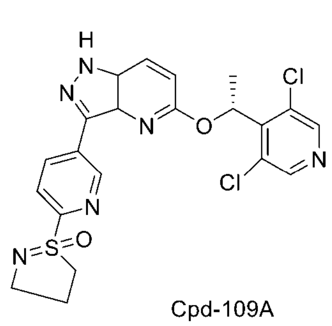
Cpd-109A ,

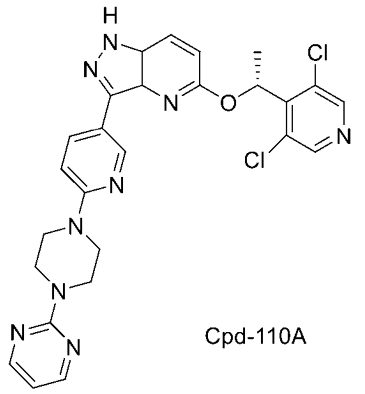
Cpd-110A
,

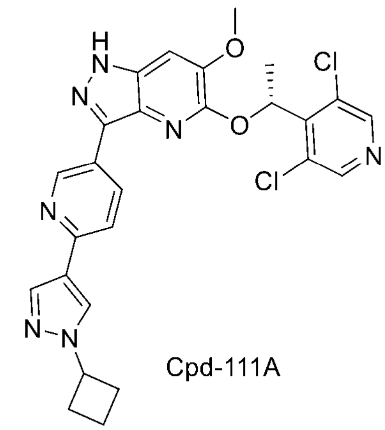
Cpd-111A
,

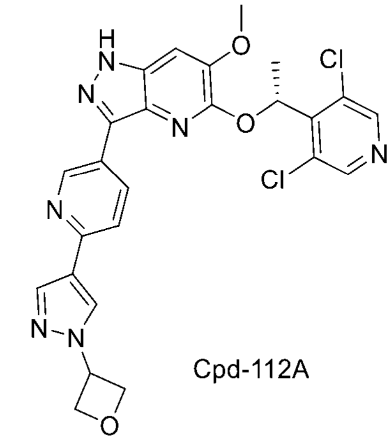
Cpd-112A
,

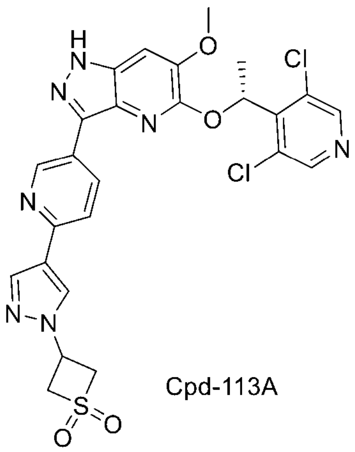
Cpd-113A
,

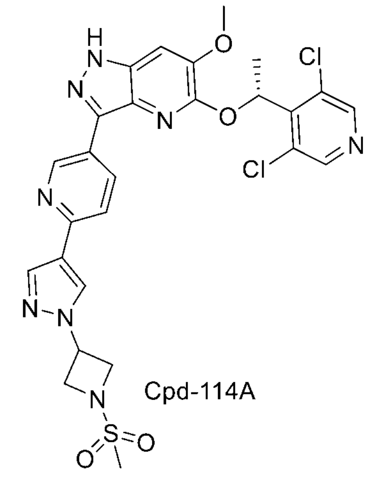
Cpd-114A
,

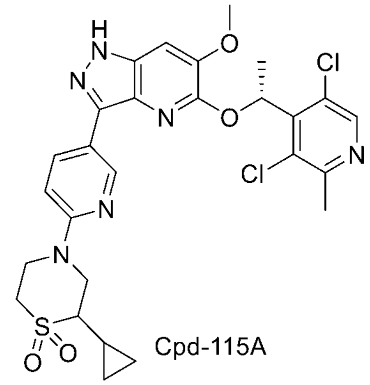
Cpd-115A
,

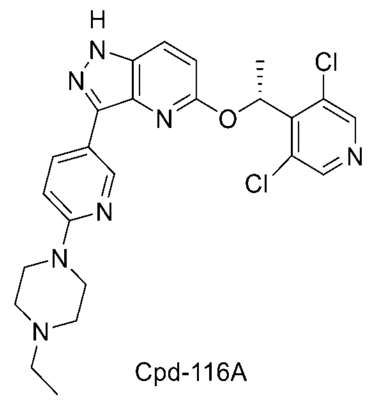
Cpd-116A
,

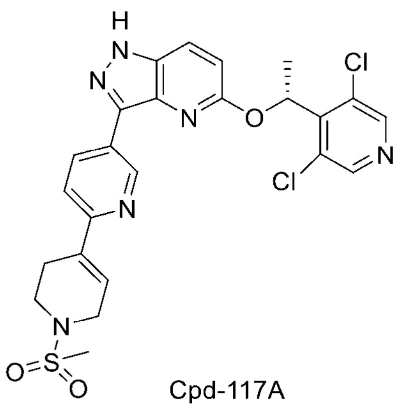
Cpd-117A
,

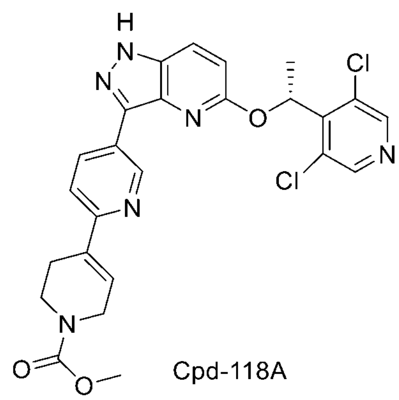
Cpd-118A
,

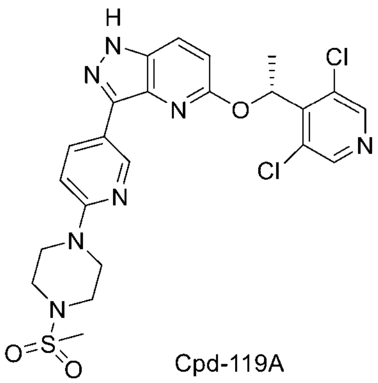
Cpd-119A ,

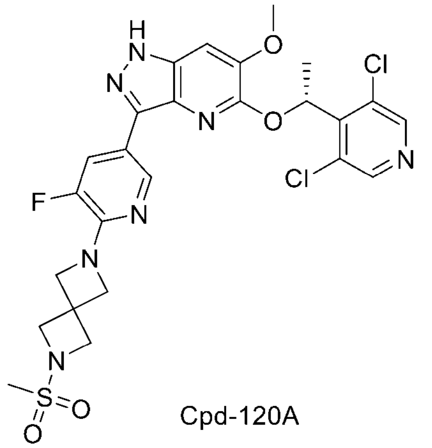
Cpd-120A ,

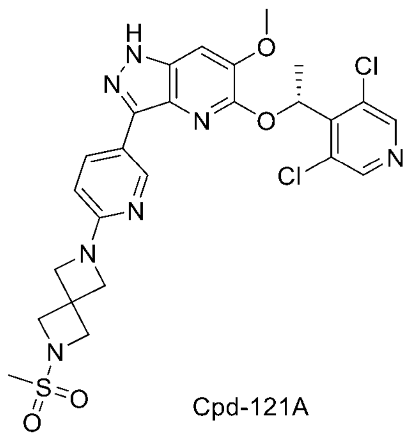
Cpd-121A ,

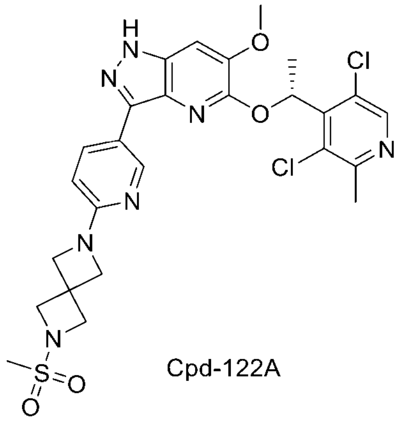
Cpd-122A ,

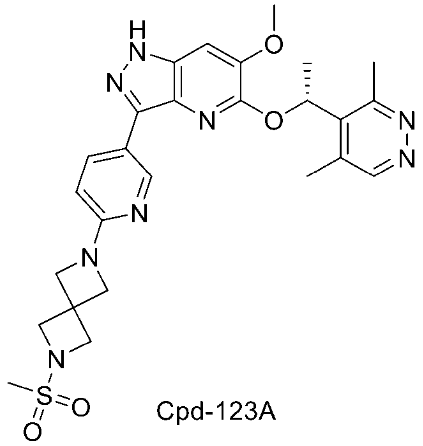
Cpd-123A ,

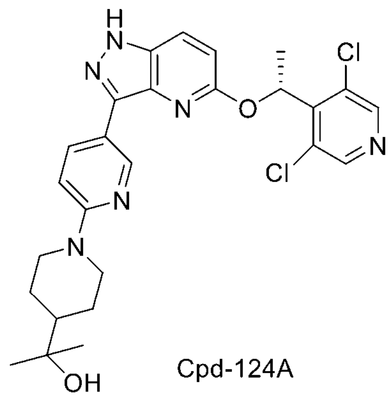
Cpd-124A ,

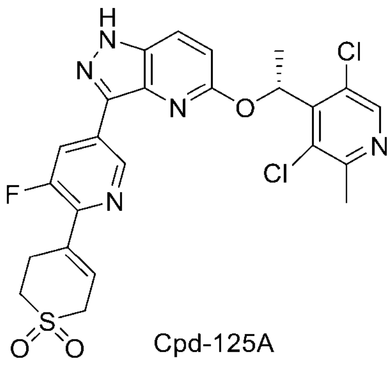
Cpd-125A ,

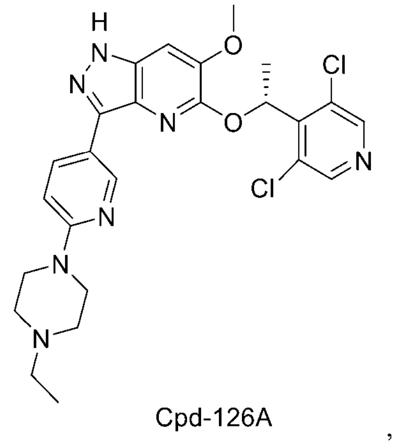
Cpd-126A ,

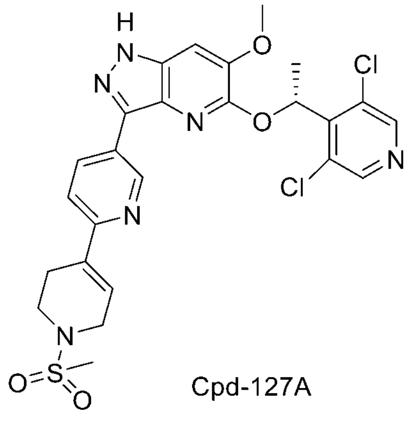
Cpd-127A ,

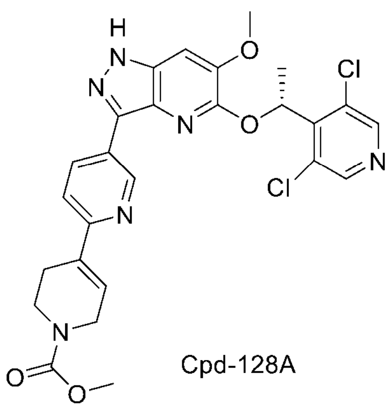
Cpd-128A ,

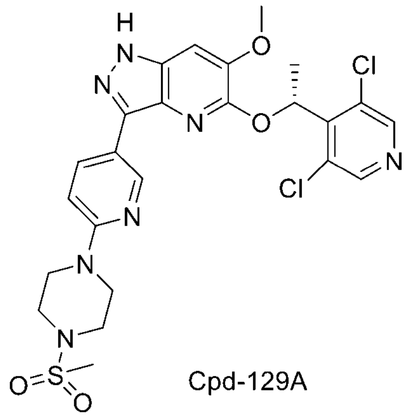
Cpd-129A ,

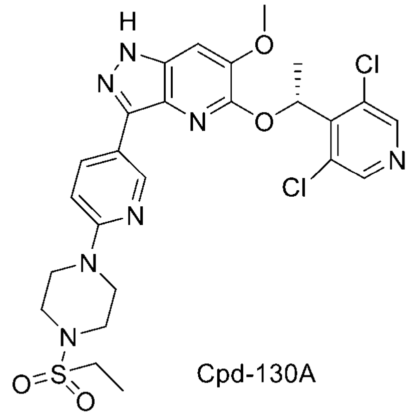
Cpd-130A ,

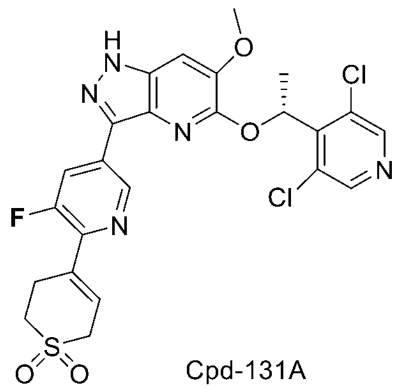
Cpd-131A ,

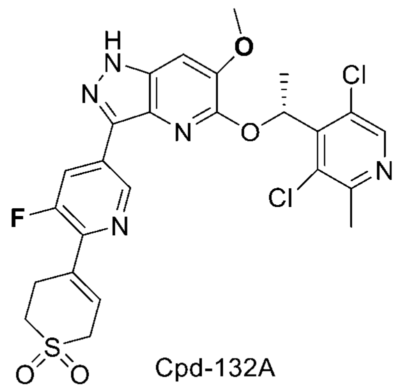
Cpd-132A ,

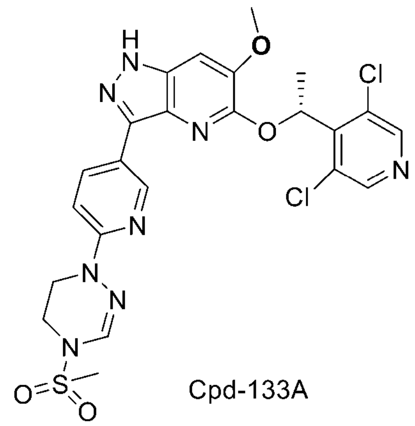
Cpd-133A ,

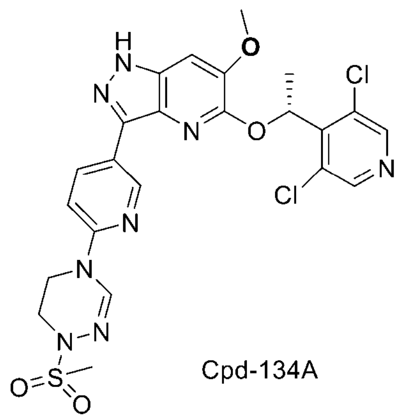
Cpd-134A ,

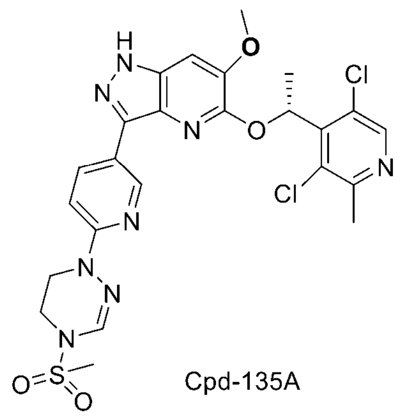
Cpd-135A ,

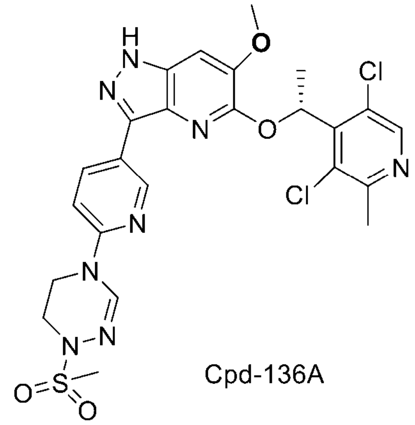
Cpd-136A ,

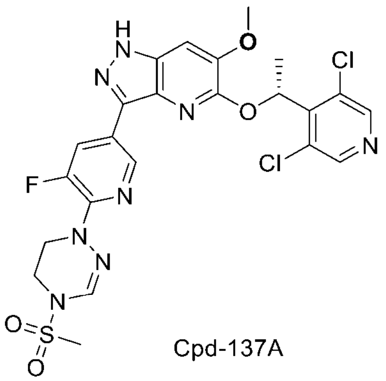
Cpd-137A ,

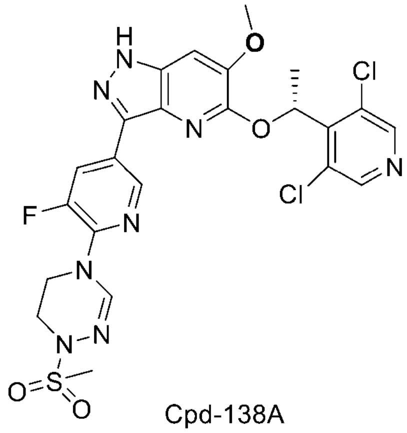
Cpd-138A ,

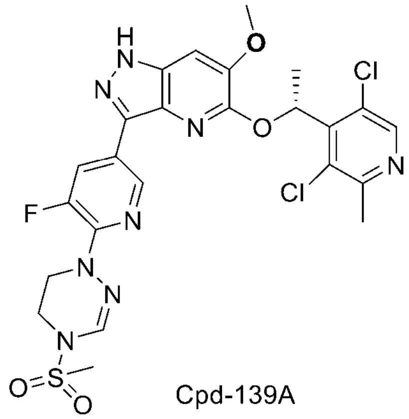
Cpd-139A ,

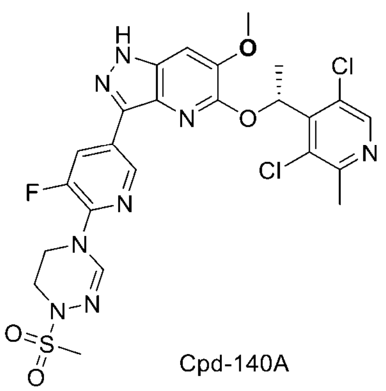
Cpd-140A ,

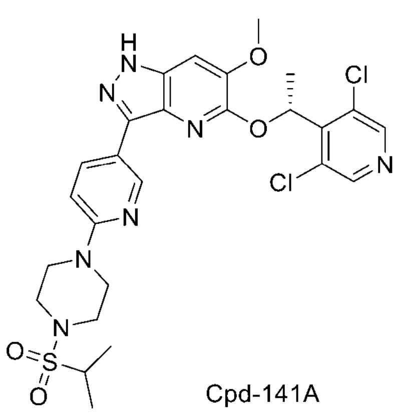
Cpd-141A ,

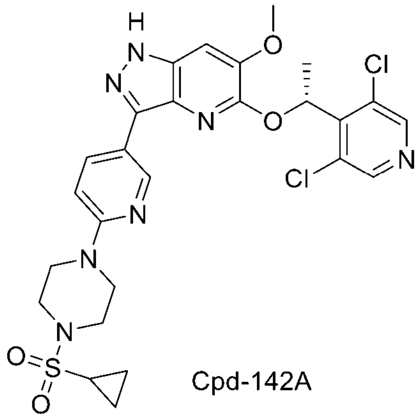
Cpd-142A ,

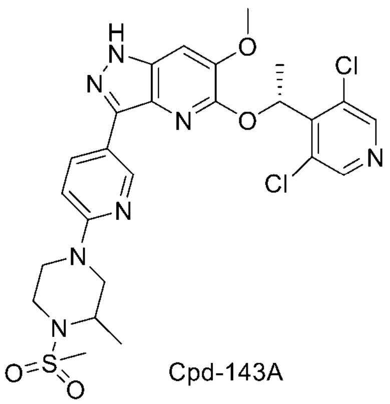
Cpd-143A ,

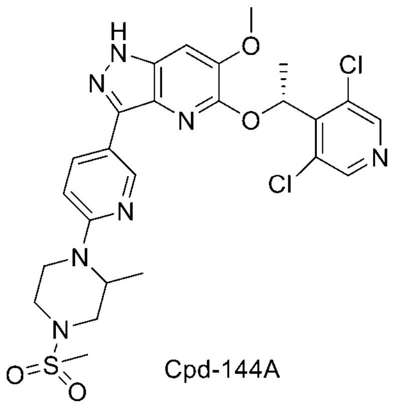
Cpd-144A ,

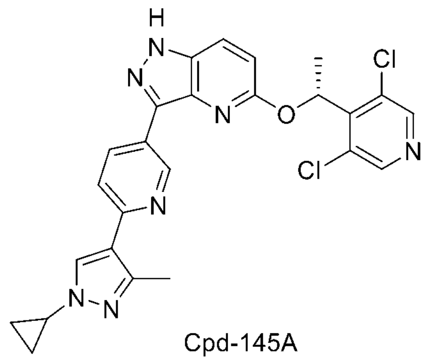
Cpd-145A ,

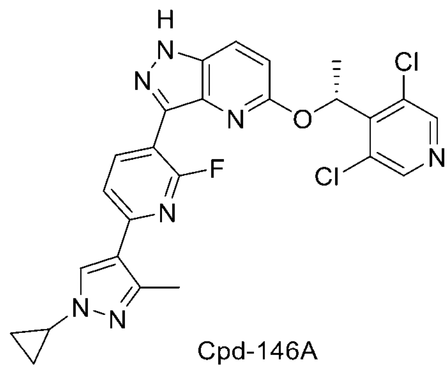
Cpd-146A ,

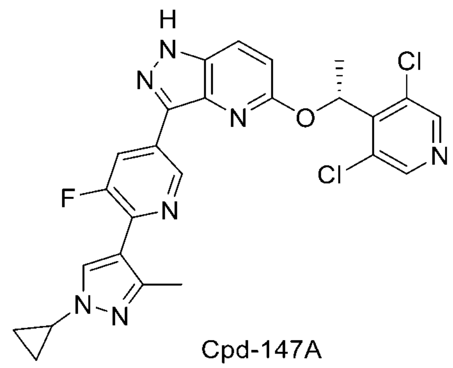
Cpd-147A ,

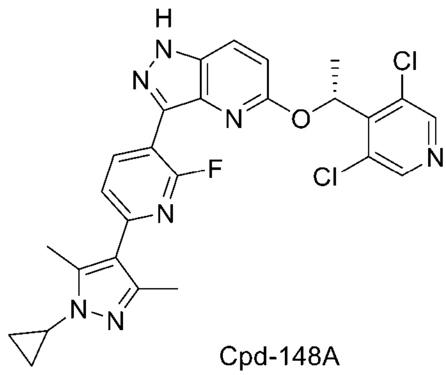

Cpd-148A

,

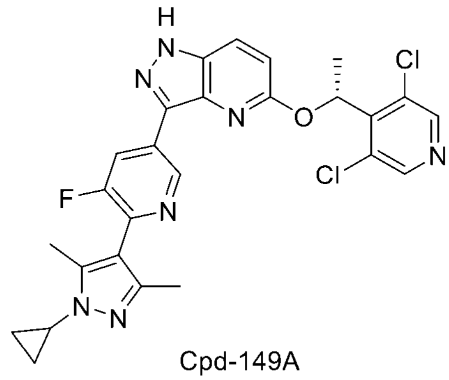

Cpd-149A

,

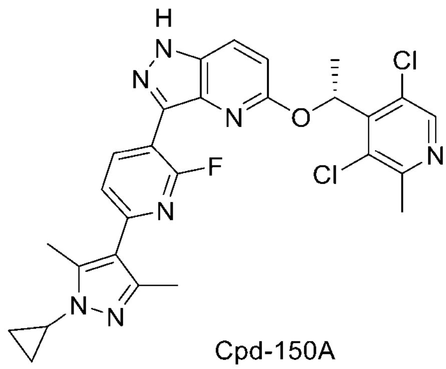

Cpd-150A

,

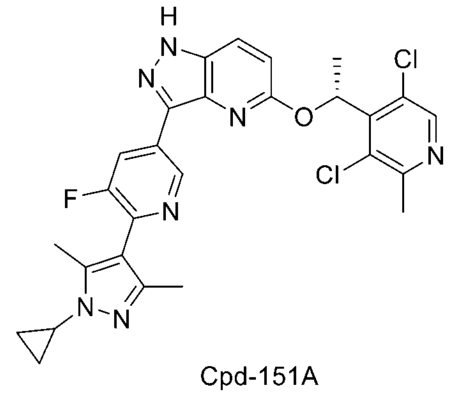

Cpd-151A

,

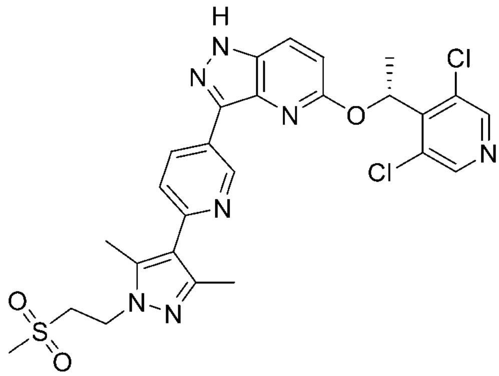

Cpd-152A

,

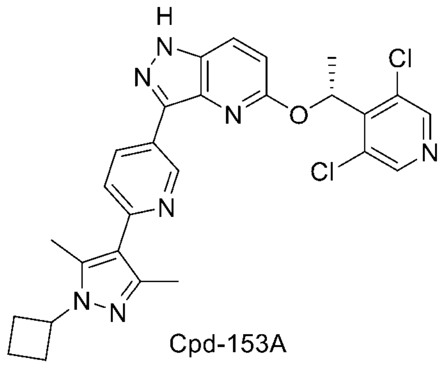

Cpd-153A

,

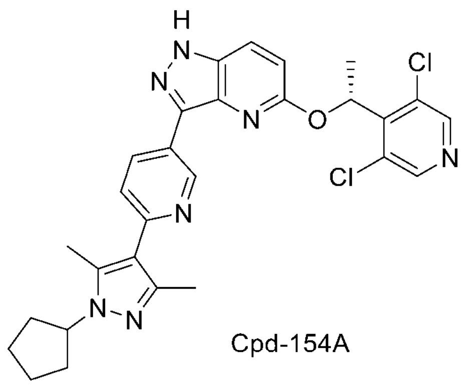

Cpd-154A

,

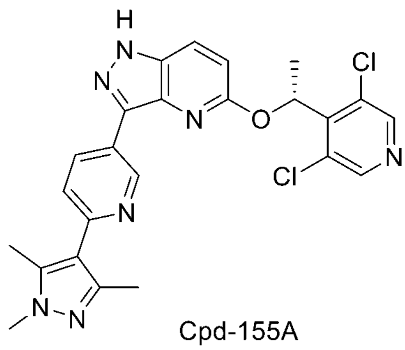

Cpd-155A

,

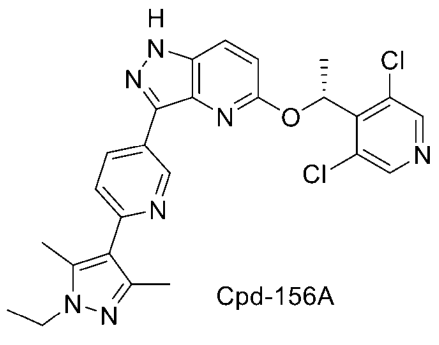
Cpd-156A
,

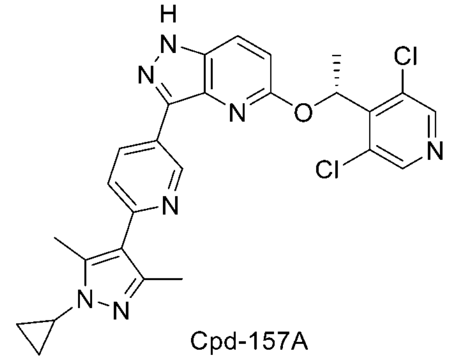
Cpd-157A
,

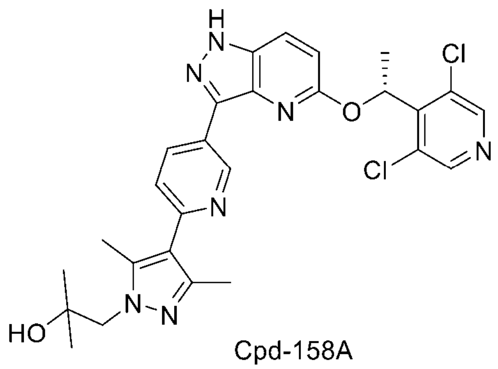
Cpd-158A
,

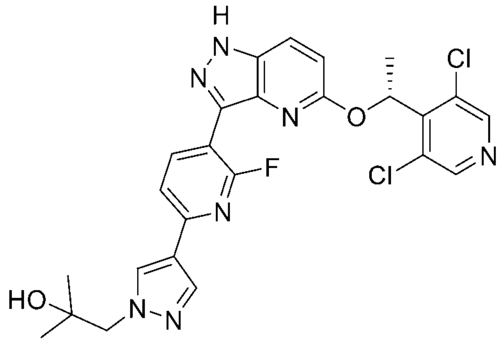
Cpd-159A
,

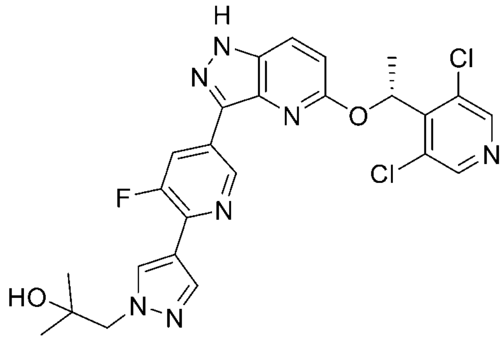
Cpd-160A
,

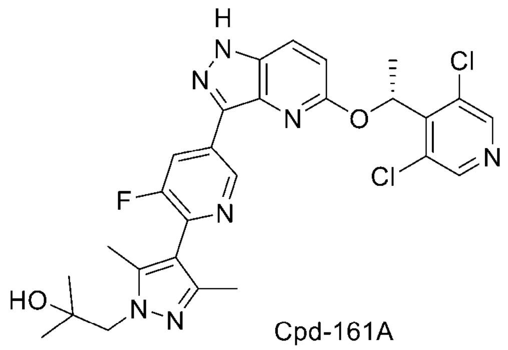
Cpd-161A
,

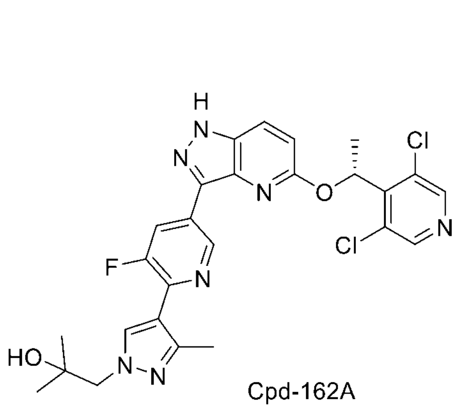
Cpd-162A
,

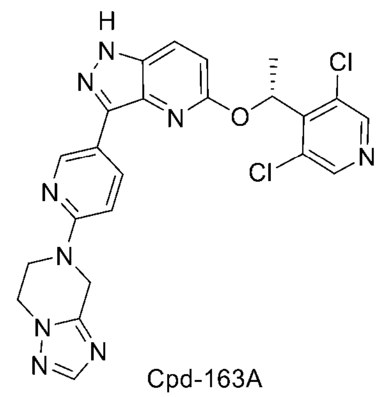
Cpd-163A
,

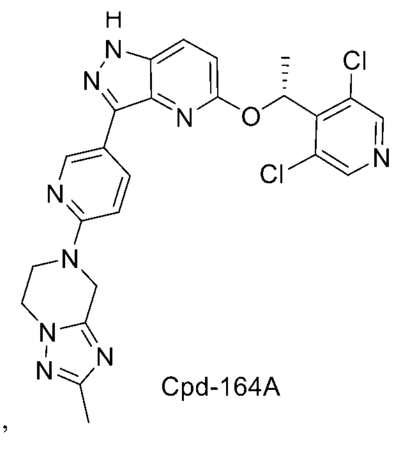
Cpd-164A
,

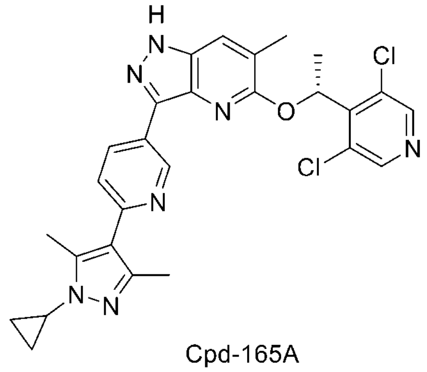
Cpd-165A
,

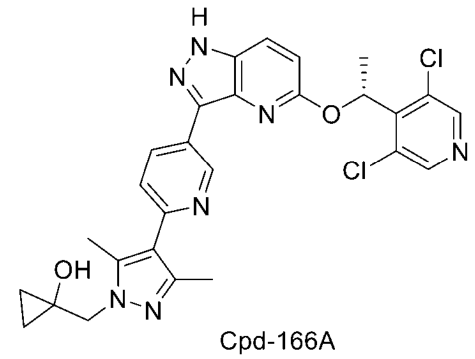
Cpd-166A
,

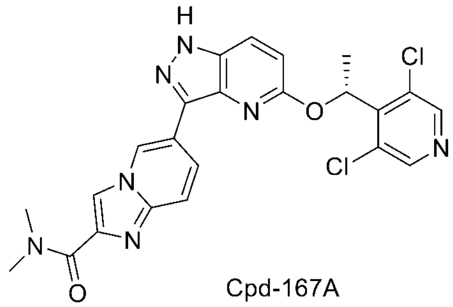
Cpd-167A
,

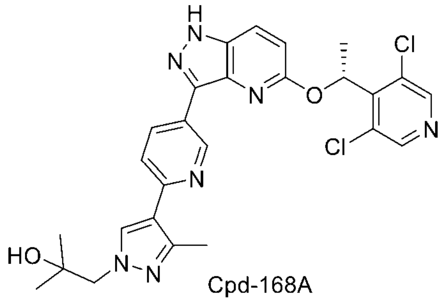
Cpd-168A
,

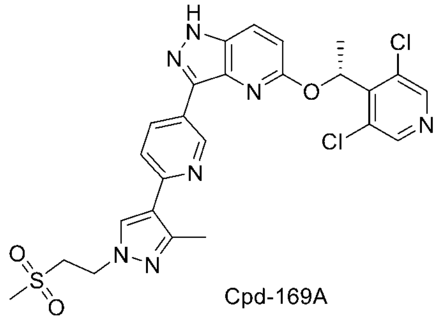
Cpd-169A
,

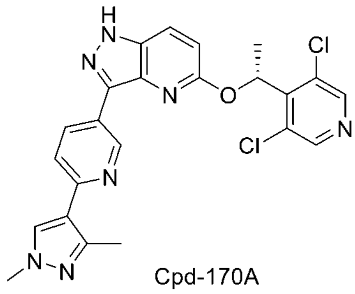
Cpd-170A
,

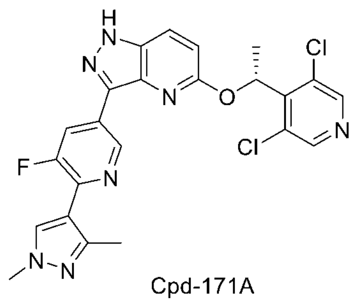
Cpd-171A
,

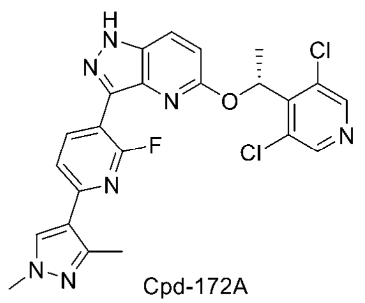
Cpd-172A
,

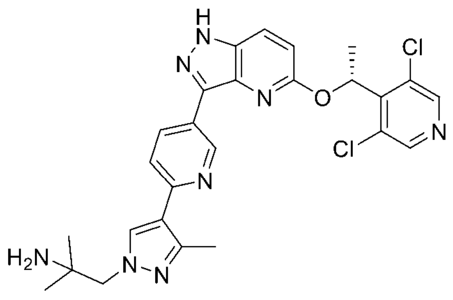
Cpd-173A
,

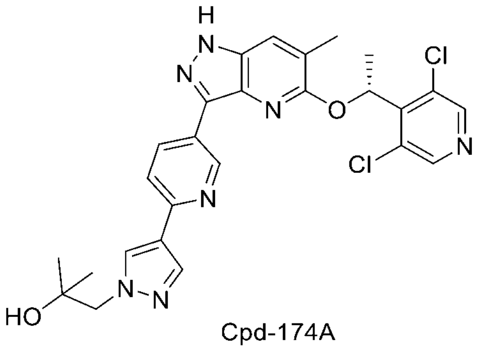

Cpd-174A ,

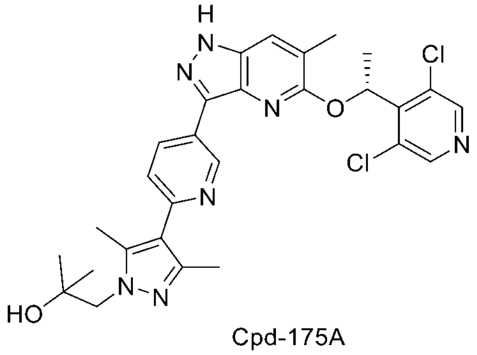

Cpd-175A ,

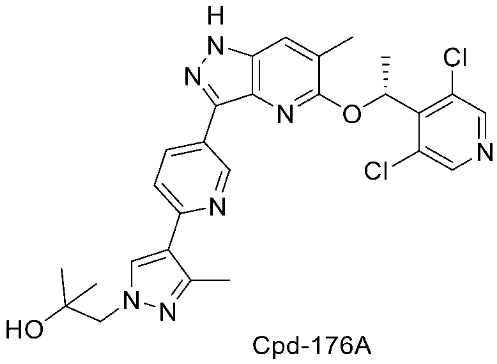

Cpd-176A ,

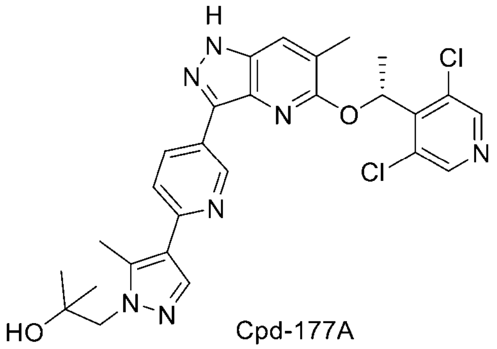

Cpd-177A ,

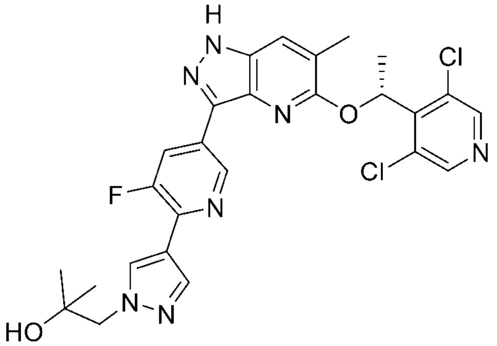

Cpd-178A ,

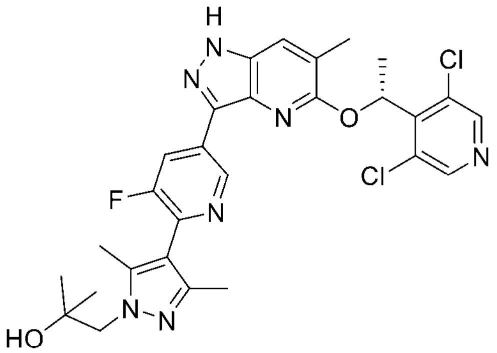

Cpd-179A ,

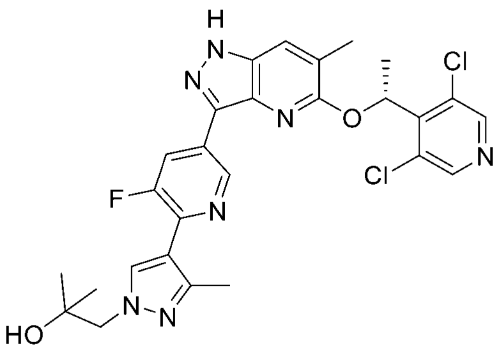

Cpd-180A ,

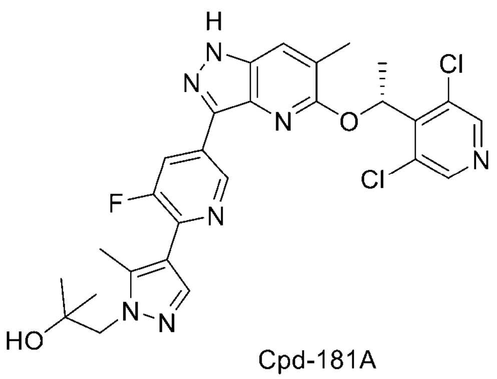

Cpd-181A ,

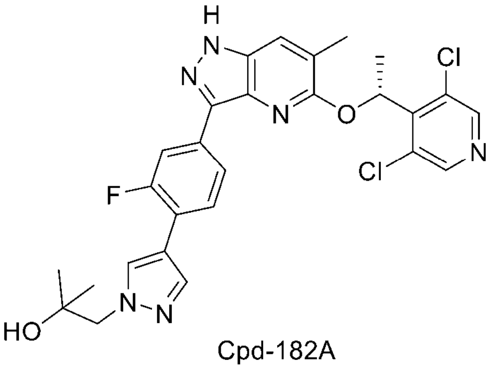

Cpd-182A ,

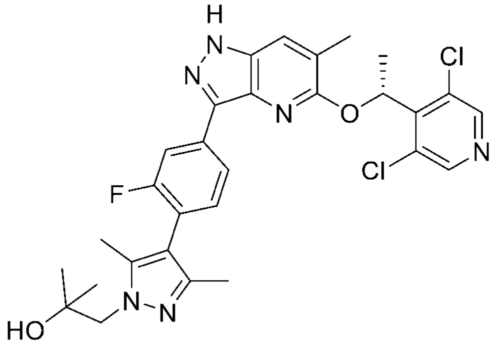

Cpd-183A ,

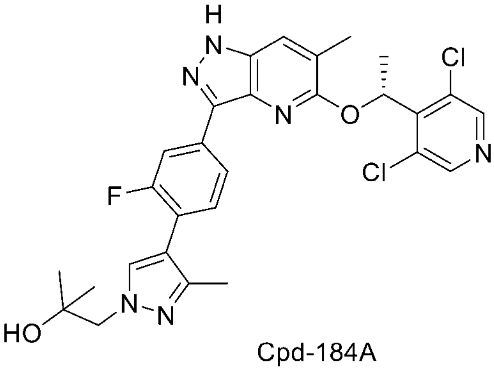

Cpd-184A ,

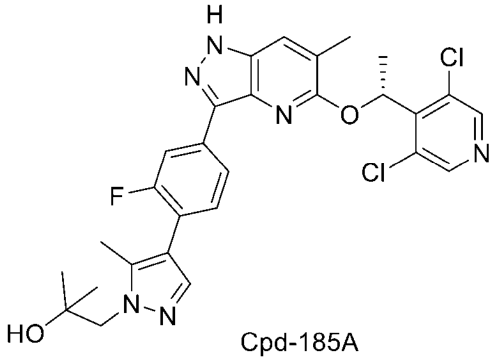

Cpd-185A ,

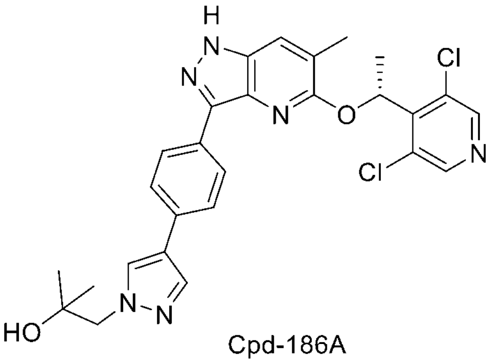

Cpd-186A ,

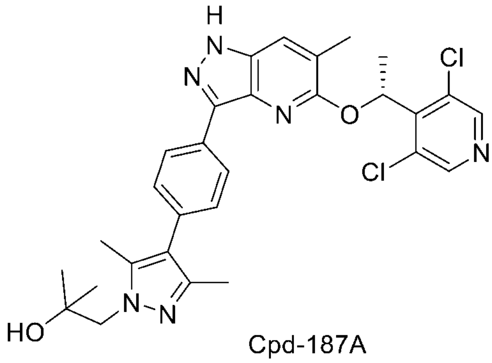

Cpd-187A ,

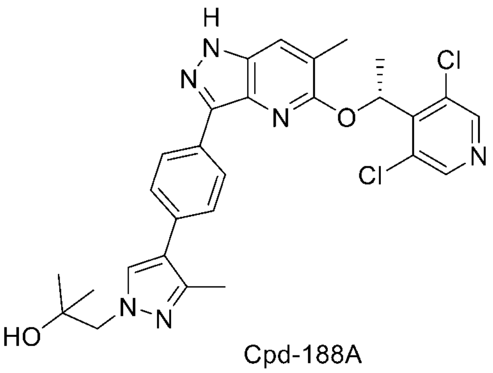

Cpd-188A ,

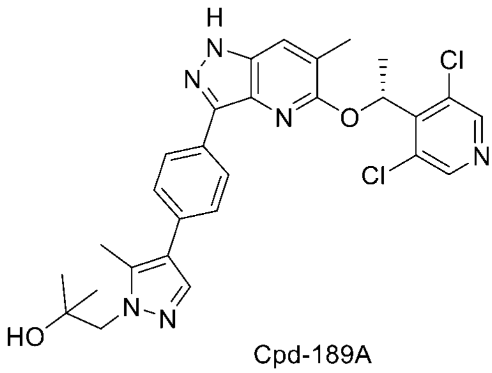

Cpd-189A ,

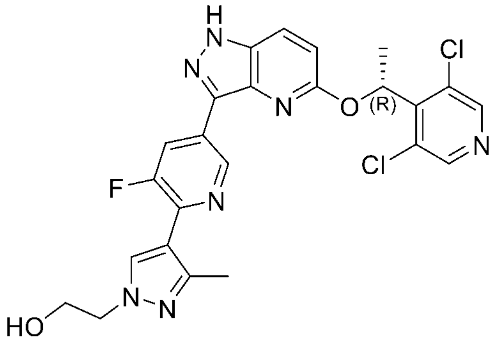

Cpd-190A ,

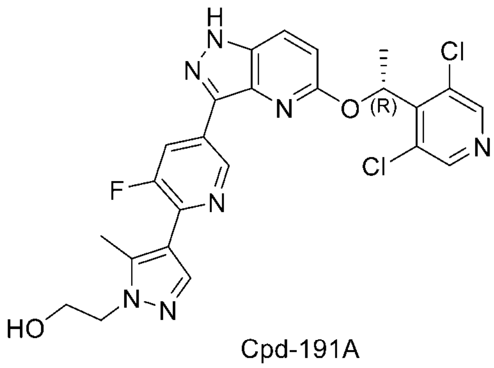

Cpd-191A ,

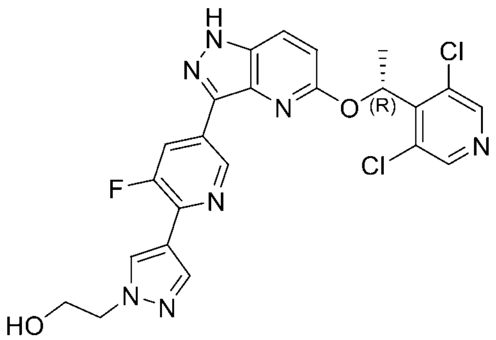

Cpd-192A ,

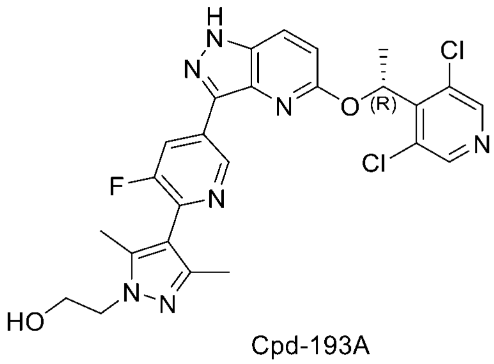

Cpd-193A ,

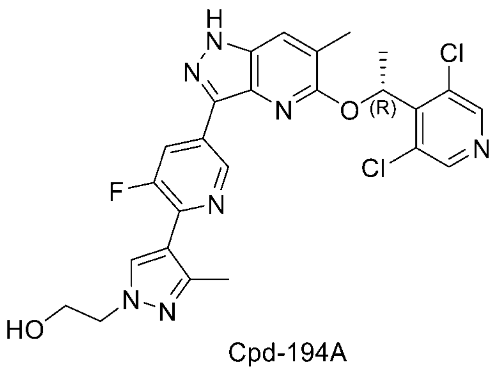

Cpd-194A ,

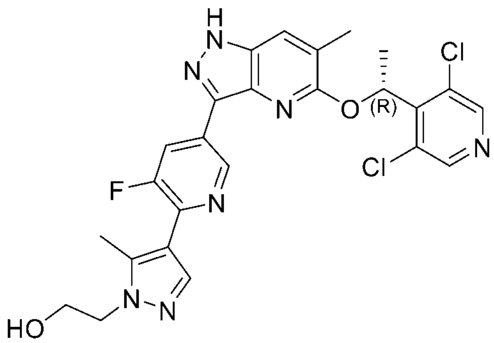

Cpd-195A ,

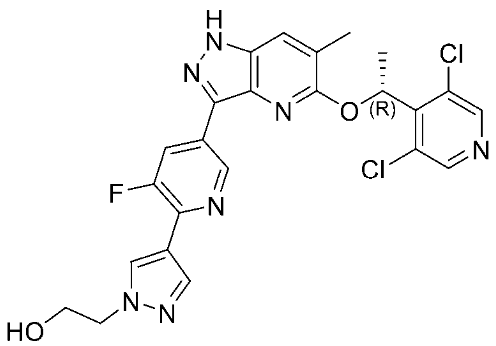

Cpd-196A ,

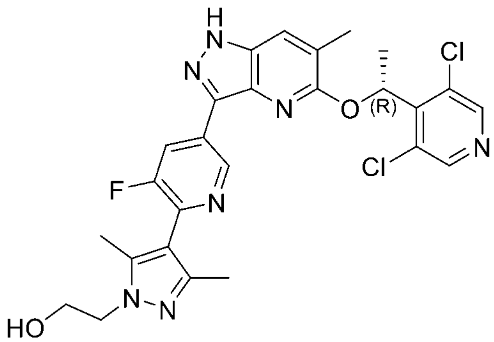

Cpd-197A ,

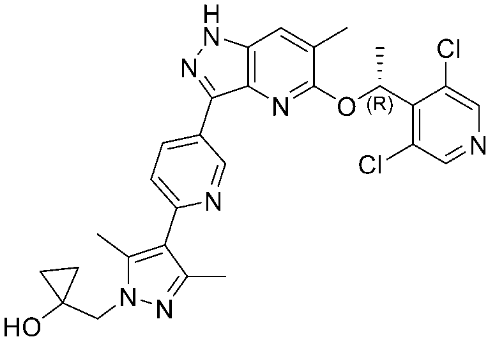

Cpd-198A ,

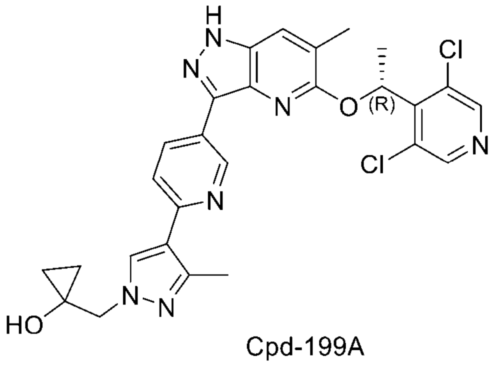

Cpd-199A ,

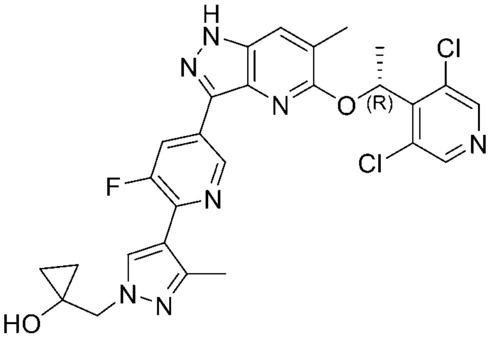

Cpd-200A ,

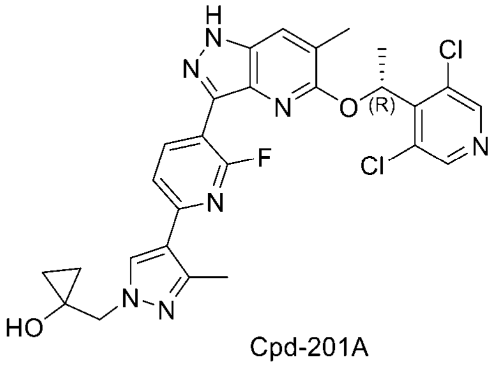

Cpd-201A ,

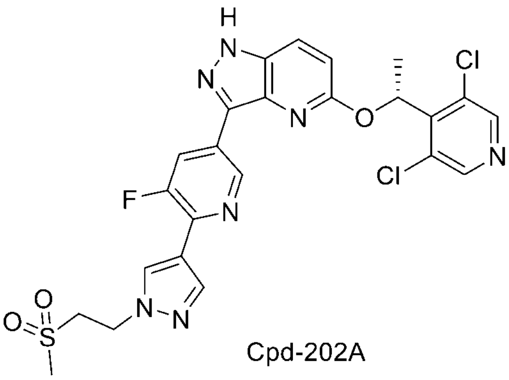

Cpd-202A ,

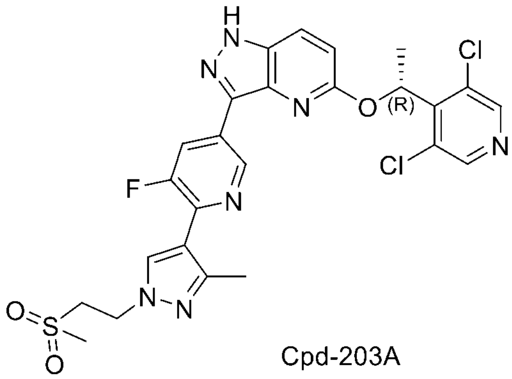

Cpd-203A ,

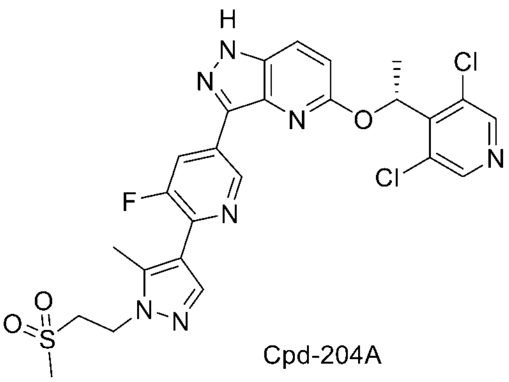

Cpd-204A ,

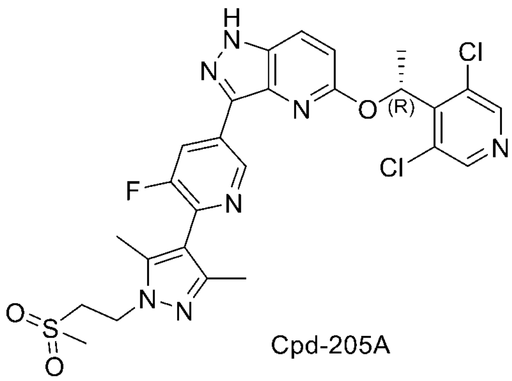

Cpd-205A ,

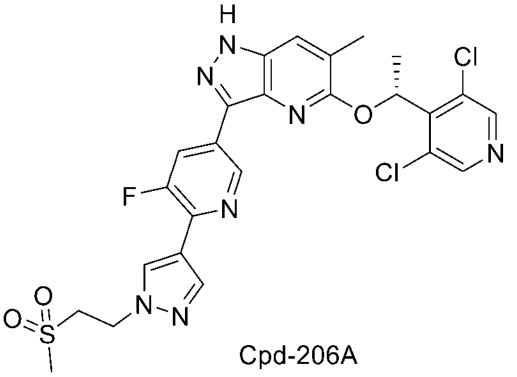
Cpd-206A
,

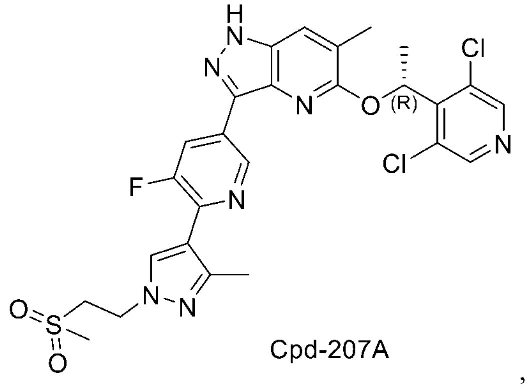
Cpd-207A
,

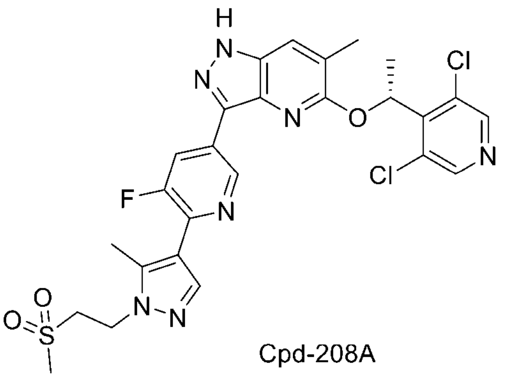
Cpd-208A
,

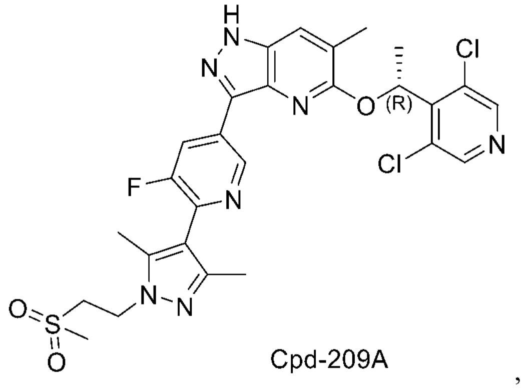
Cpd-209A
,

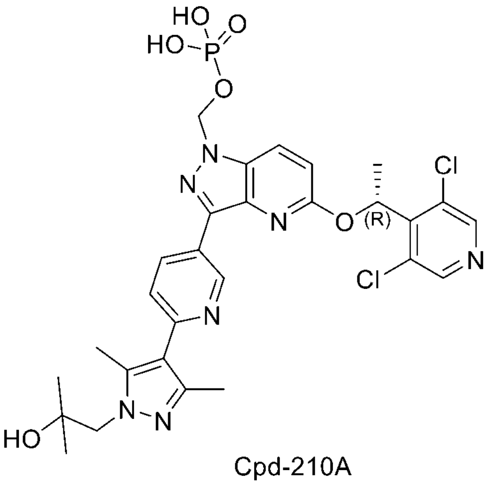
Cpd-210A
,

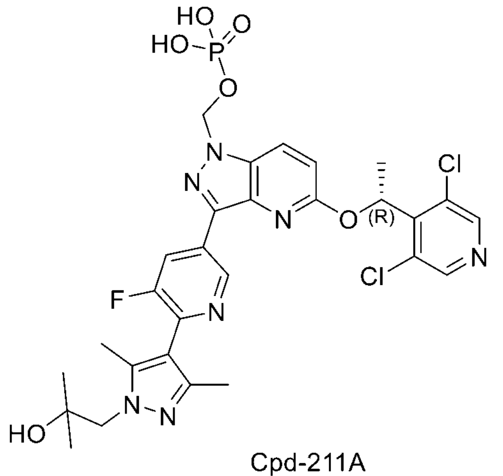
Cpd-211A
,

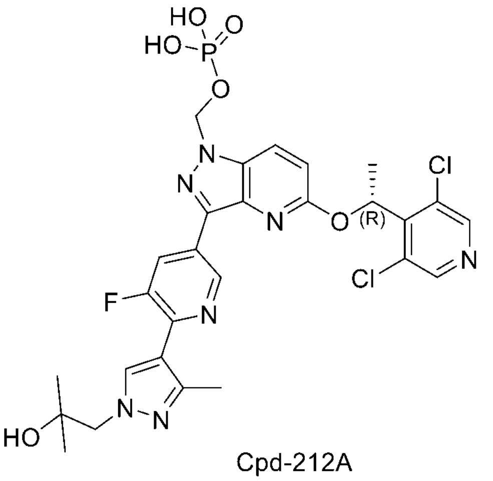

Cpd-212A ,

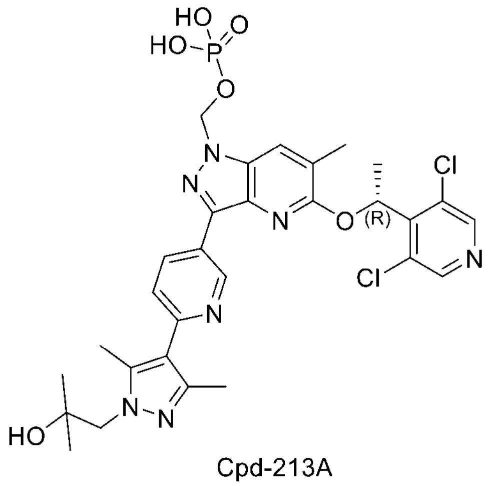

Cpd-213A ,

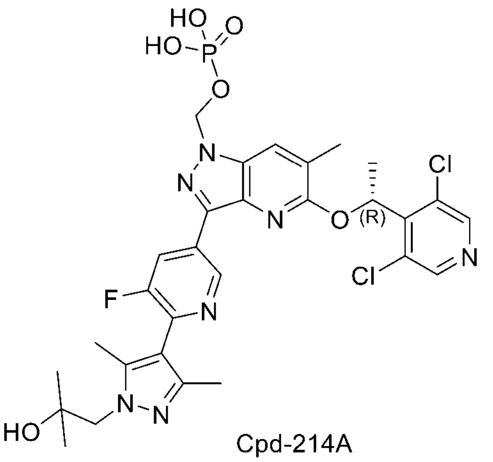

Cpd-214A ,

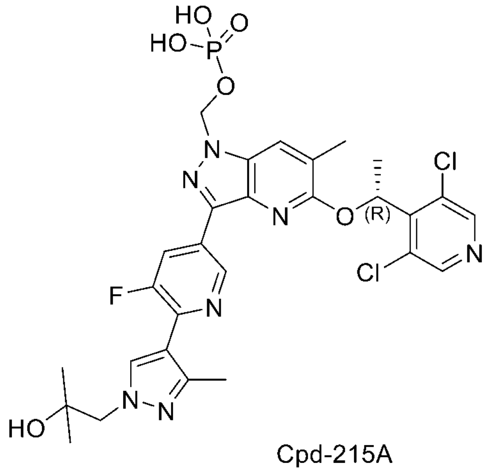

Cpd-215A ,

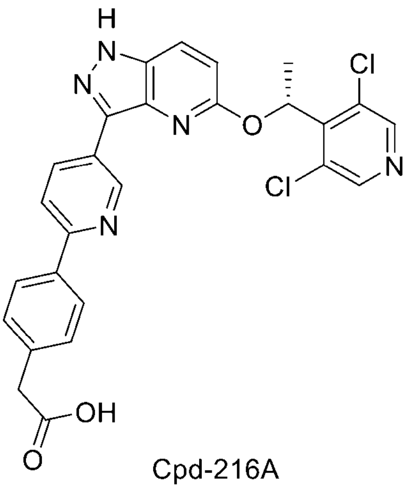

Cpd-216A ,

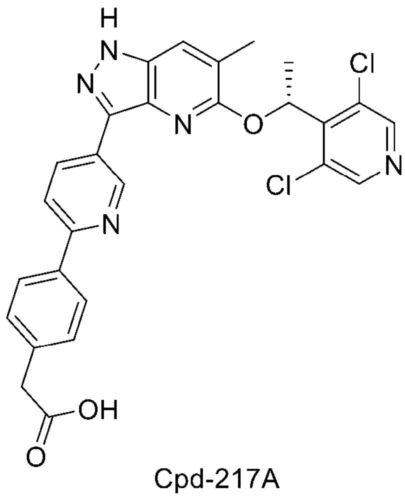

Cpd-217A ,

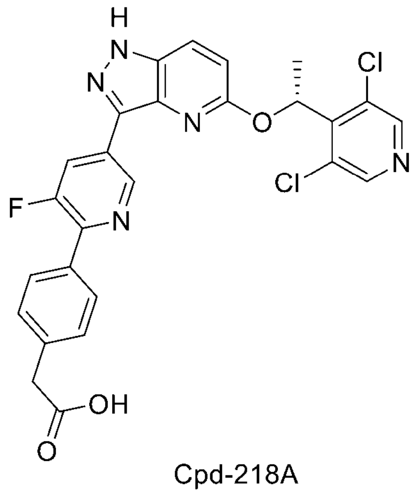

Cpd-218A ,

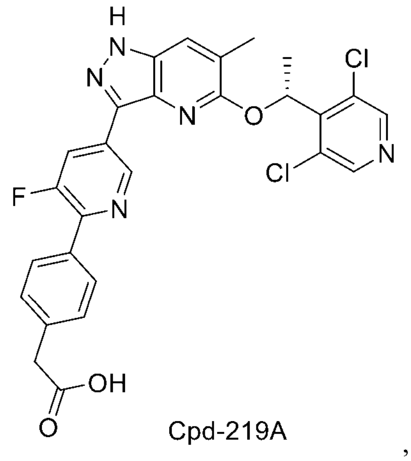

Cpd-219A ,

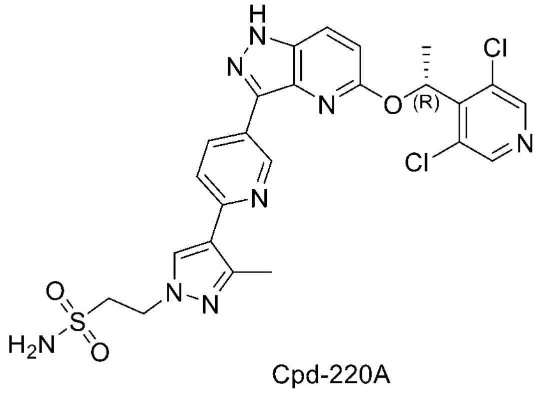

Cpd-220A ,

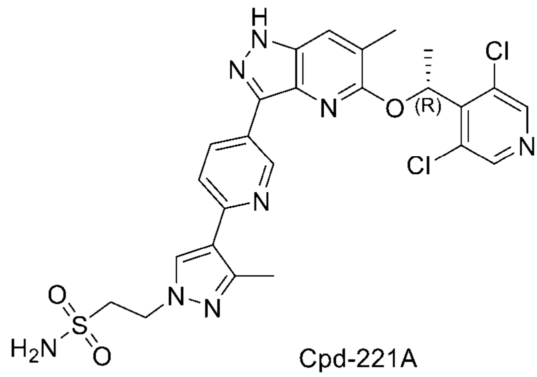

Cpd-221A ,

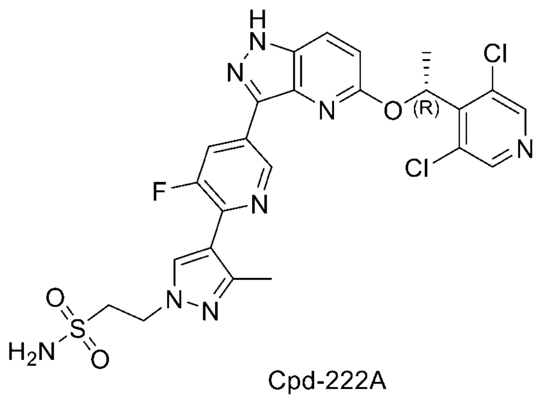

Cpd-222A ,

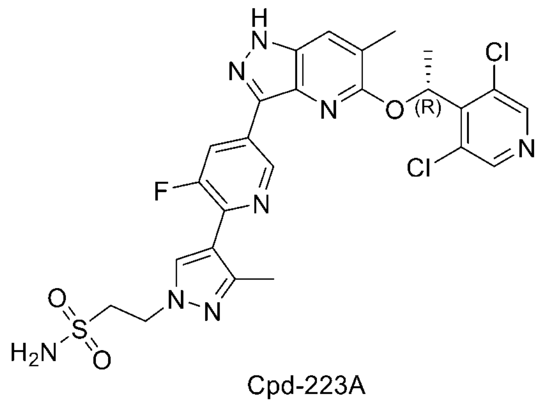

Cpd-223A ,

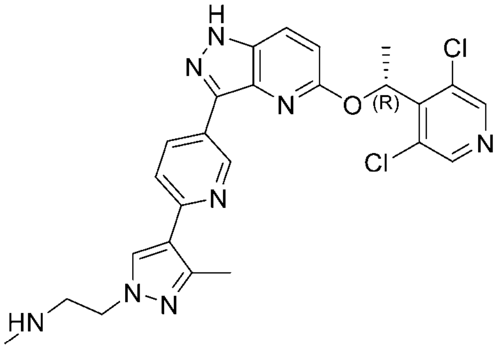

Cpd-224A ,

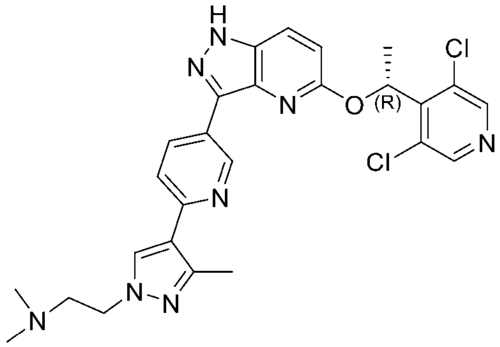

Cpd-225A ,

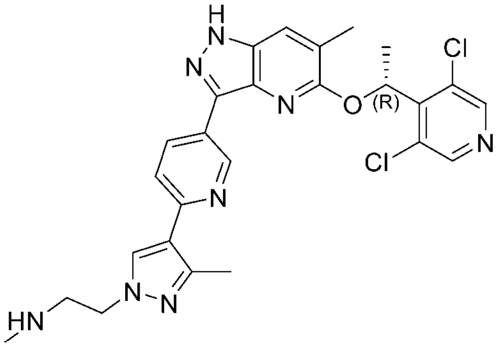

Cpd-226A ,

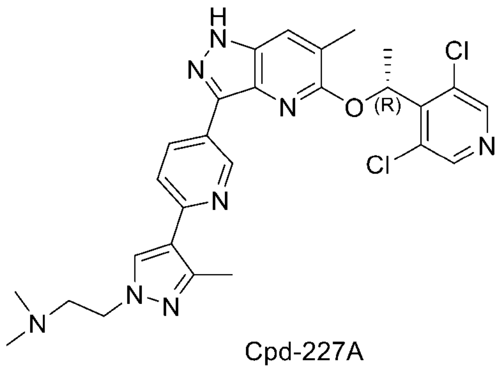

Cpd-227A ,

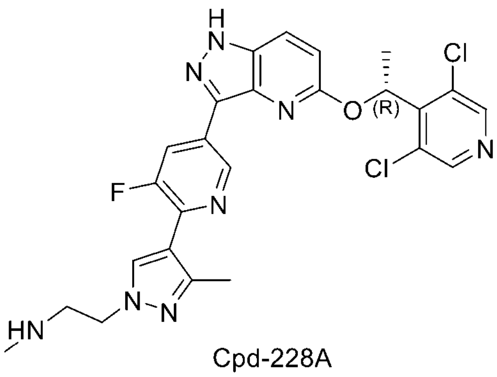

Cpd-228A ,

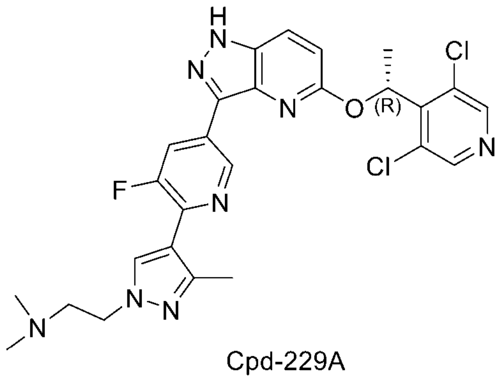

Cpd-229A ,

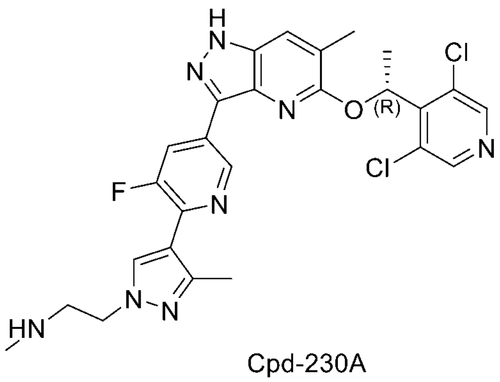

Cpd-230A ,

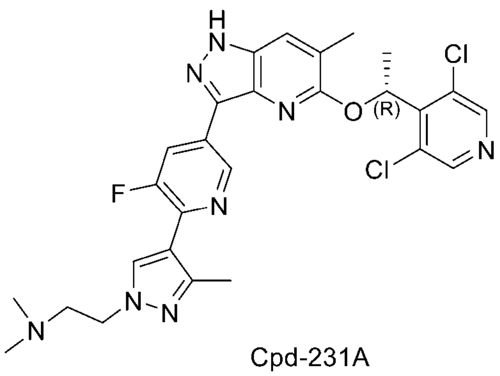

Cpd-231A .

The present invention further provides a pharmaceutical composition comprising a therapeutically effective amount of at least one of the compound of Formula (I), and a racemate, a stereoisomer, a tautomer, an isotopically labeled compound, an N-oxide, a solvate, a polymorph, a metabolite, an ester, a prodrug or a pharmaceutically acceptable salt

thereof.

According to embodiments of the present invention, the pharmaceutical composition further comprises one or more pharmaceutically acceptable excipients.

The excipient in the pharmaceutical composition is "acceptable", compatible with (and preferably able to stabilize) the active ingredient in the composition, and not harmful to the subject treated. One or more pharmaceutical excipients can be used to deliver the active compound.

According to some embodiments of the present invention, the pharmaceutical composition may further comprise one or more additional therapeutic agents.

The present invention further provides use of at least one of the compound of Formula (I), or a racemate, a stereoisomer, a tautomer, an isotopically labeled compound, an N-oxide, a solvate, a polymorph, a metabolite, an ester, a prodrug or a pharmaceutically acceptable salt thereof, or the pharmaceutical composition in the preparation of a drug.

According to some embodiments, the drug is a drug for diagnosing, preventing and/or treating diseases or disorders mediated by FGFR2 and/or FGFR3.

According to some embodiments, the drug is an FGFR2 and/or FGFR3 inhibitor.

According to some embodiments, the disease is an FGFR-related cancer.

According to some embodiments, the disease or disorder is selected from bladder cancer, brain cancer, breast cancer, cholangiocarcinoma, head and neck cancer, lung cancer, multiple myeloma, rhabdomyosarcoma, urethral carcinoma, and uterine cancer.

According to some embodiments, the disease is FGFR-related chondrodysplasia or achondroplasia.

According to some embodiments, at least one of the compound of Formula (I), and a racemate, a stereoisomer, a tautomer, an isotopically labeled compound, an N-oxide, a solvate, a polymorph, a metabolite, an ester, a prodrug or a pharmaceutically acceptable salt thereof can be formulated into a form suitable for administration through any appropriate route, formulated by a conventional method with one or more pharmaceutically acceptable carriers. Therefore, at least one of the compound of Formula (I), and a racemate, a stereoisomer, a tautomer, an isotopically labeled compound, an N-oxide, a solvate, a polymorph, a metabolite, an ester, a prodrug or a pharmaceutically acceptable salt thereof can be formulated into various dosage forms for oral administration, or administration by injection (such as intravenous, intramuscular or subcutaneous), inhalation or insufflation; or formulated into sustained-release dosage forms, such as tablets, hard or soft capsules, aqueous or oily suspensions, emulsions, injections, dispersible powders or granules, suppositories, lozenges or syrups.

The present invention further provides a method for diagnosing, preventing and/or treating diseases or disorders mediated by FGFR2 and/or FGFR3. The method comprises: administering a therapeutically effective amount of at least one compound of Formula (I) and a racemate, a stereoisomer, a tautomer, an isotopically labeled compound, an N-oxide, a solvate, a polymorph, a metabolite, an ester, a prodrug or a pharmaceutically acceptable salt thereof or the pharmaceutical composition of the present invention alone to a patient in need thereof, or optionally in combination with another compound of the present invention and/or at least one therapeutical agent of other type.

According to some embodiments, the disease or disorder mediated by FGFR2 and/or FGFR3 is selected from bladder cancer, brain cancer, breast cancer, cholangiocarcinoma, head and neck cancer, lung cancer, multiple myeloma, rhabdomyosarcoma, urethral carcinoma, uterine cancer, and achondroplasia.

In some embodiments, the patient is a mammal, preferably a human.

## Beneficial Effects

The compound provided in the present invention has good FGFR2 and/or FGFR3 inhibiting effects, and can be used for treating or preventing disorders and diseases associated with FGFR2 and/or FGFR3 and for preparing drugs for treating or preventing such disorders and diseases.

## Definition and Explanation of Terms

Unless otherwise indicated, the definitions of groups and terms recorded in the specification and claims of the present application, including their definitions as examples, exemplary definitions, preferred definitions, definitions recorded in tables, definitions of specific compounds in examples, etc., may be arbitrarily combined and associated with each other. Such combined and associated group definitions and compound structures should be understood to be within the scope of the specification and/or claims of the present application.

The term "optional" (or "optionally") in the general formula definitions of the present application means a situation of being substituted with zero, one or more substituents, for example "optionally substituted with one, two or more R" means that it may not be substituted with R (unsubstituted) or may be selectively substituted with one, two or more R.

"More" means three or more, for example, 3, 4, 5, 6, 7, 8, 9 or 10.

Unless otherwise stated, the numerical ranges recited in the specification and claims are equivalent to reciting at

least each specific integer value therein. For example, the numerical range "1-12" is equivalent to reciting each integer value in the numerical range "1-12," namely 1, 2, 3, 4, 5, 6, 7, 8, 9, 10, 11, and 12.

The term "halogen" refers to fluorine, chlorine, bromine and iodine.

"HO-$C_{1-6}$ alkyl" refers to a $C_{1-6}$ alkyl substituted with hydroxyl.

The term "$C_{1-6}$ alkyl" refers to a linear or branched alkyl having 1, 2, 3, 4, 5 or 6 carbon atoms. The alkyl is, for example, methyl, ethyl, propyl, butyl, pentyl, hexyl, iso-propyl, iso-butyl, s-butyl, t-butyl, iso-pentyl, 2-methylbutyl, 1-methylbutyl, 1-ethylpropyl, 1,2-dimethylpropyl, neo-pentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl, or isomers thereof.

The term "$C_{3-8}$ cycloalkyl" should be construed to represent a saturated monovalent monocyclic or bicyclic (e.g., bridged cyclic or spirocyclic) hydrocarbon ring having 3, 4, 5, 6, 7, or 8 carbon atoms. The $C_{3-8}$ cycloalkyl may be a monocyclic hydrocarbon radical such as cyclopropyl, cyclobutyl, cyclopentyl, cyclohexyl, cycloheptyl, cyclooctyl, cyclononyl, or cyclodecyl, or may be a bicyclic hydrocarbon radical such as bornyl, indolyl, hexahydroindolyl, tetrahydronaphthyl, decahydronaphthyl, bicyclo[2.1.1]hexyl, bicyclo[2.2.1]heptyl, bicyclo[2.2.1]heptenyl, 6,6-dimethylbicyclo[3.1.1]heptyl, 2,6,6-trimethylbicyclo[3.1.1]heptyl, bicyclo[2.2.2]octyl, 2,7-diazaspiro[3,5]nonanyl, or 2,6-diazaspiro[3,4]octanyl.

The term "3- to 10-membered heterocyclyl" means a saturated or unsaturated non-aromatic ring or ring system and comprises at least one heteroatom selected from O, S and N. The heterocyclyl may be attached to the rest of the molecule via any of the carbon atoms or the nitrogen atom, if present. The heterocyclyl may include a fused or bridged ring and a spirocyclic ring. In particular, the heterocyclyl may include, but is not limited to: a 4-membered ring, such as azetidinyl, oxetanyl; a 5-membered ring, such as tetrahydrofuranyl, dioxolyl, pyrrolidinyl, imidazolidinyl, pyrazolidinyl, pyrrolinyl; or a 6-membered ring, such as tetrahydropyranyl, piperidinyl, morpholinyl, dithianyl, thiomorpholinyl, piperazinyl or trithianyl; or a 7-membered ring, such as diazepanyl. Optionally, the heterocyclyl may be benzo-fused. The heterocyclyl may be bicyclic, such as but not limited to a 5,5-membered ring, such as a hexahydrocyclopenta[c]pyrrol-2(1H)-yl ring, or a 5,6-membered bicyclic ring, such as a hexahydropyrrolo[1,2-a]pyrazin-2(1H)-yl ring. The heterocyclyl may be partially unsaturated, that is, it may comprise one or more double bonds, such as, but not limited to, dihydrofuranyl, dihydropyranyl, 2,5-dihydro-1H-pyrrolyl, 4H-[1,3,4]thiadiazinyl, 1,2,3,5-tetrahydrooxazolyl or 4H-[1,4]thiazinyl, or it may be benzo-fused, such as, but not limited to, dihydroisoquinolinyl. When the 3- to 10-membered heterocyclyl is attached to other groups to constitute the compound of the present invention, either the carbon atom on the 3- to 10-membered heterocyclyl is attached to other groups, or the heteroatom on the 3- to 10-membered heterocyclyl is attached to other groups. For example, when the 3- to 10-membered heterocyclyl is selected from piperazinyl, the nitrogen atom on the piperazinyl may be attached to other groups. Alternatively, when the 3- to 10-membered heterocyclyl is selected from piperidinyl, the nitrogen atom on the piperidinyl ring and the carbon atom at the para position thereof may be attached to other groups.

The term "5- to 10-membered heteroaryl" should be understood to comprise such a monovalent monocyclic or bicyclic ring system that has 5, 6, 7, 8, 9 or 10 ring atoms, and comprises 1-5, and preferably 1-3 heteroatoms independently selected from N, O and S, which, in addition, may be benzo-fused in each case. Examples of monocyclic "heteroaryl" comprise, for example, pyridyl, pyridazinyl, pyrimidinyl, pyrazinyl, thiazinyl, oxazinyl, triazinyl, thiadiazinyl or oxadiazinyl, and the like. "Heteroaryl" also means a group in which a heteroaromatic ring is fused with one or more aryl, alicyclic, or heterocyclic rings, wherein the site of attachment is on the heteroaromatic ring. Non-limiting examples comprise 1-, 2-, 3-, 5-, 6-, 7- or 8-indolizinyl, 1-, 3-, 4-, 5-, 6-, or 7-isoindolyl, 2-, 3-, 4-, 5-, 6- or 7-indolyl, 2-, 3-, 4-, 5-, 6-, or 7-indazolyl, 2-, 4-, 5-, 6-, 7- or 8-purinyl, 1-, 2-, 3-, 4-, 6-, 7-, 8- or 9-quinolizinyl, 2-, 3- , 4-, 5-, 6-, 7- or 8-quinolinyl, 1-, 3-, 4-, 5-, 6-, 7- or 8-isoquinolinyl, 1-, 4-, 5-, 6-, 7- or 8-phthalazinyl, 2-, 3-, 4-, 5- or 6-naphthyridinyl, 2-, 3-, 5-, 6-, 7- or 8-quinazolinyl, 3-, 4-, 5-, 6-, 7- or 8-cinnolinyl, 2-, 4-, 6- or 7-pteridinyl, 1-, 2-, 3-, 4-, 5-, 6-, 7- or 8-4aH carbazolyl, 1-, 2-, 3-, 4-, 5-, 6-, 7- or 8-carbazolylcarbazolyl, 1-, 3-, 4-, 5-, 6-, 7-, 8- or 9-carbolinyl, 1-, 2-, 3-, 4-, 6-, 7-, 8-, 9- or 10-phenanthridinyl, 1-, 2-, 3-, 4-, 5-, 6-, 7-, 8- or 9-acridinyl, 1-, 2-, 4-, 5-, 6-, 7-, 8- or 9-pyridyl, 2-, 3-, 4-, 5-, 6-, 8-, 9- or 10-phenanthrolinyl, 1-, 2-, 3-, 4-, 6-, 7-, 8- or 9-phenazinyl, 1-, 2-, 3-, 4-, 6-, 7-, 8-, 9- or 10-phenothiazinyl, 1-, 2-, 3-, 4-, 6-, 7-, 8-, 9- or 10-phenazinyl , 2-, 3-, 4-, 5-, 6- or 1-, 3-, 4-, 5-, 6-, 7-, 8-, 9- or 10-benzoisoquinolyl, 2-, 3-, 4- or thieno[2,3-b]furanyl, 2-, 3-, 5-, 6-, 7-, 8-, 9-, 10- or 11-7H-pyrazino[2,3-c]carbazolyl, 2-, 3-, 5-, 6- or 7-2H-furo[3,2-b]pyranyl , 2-, 3-, 4-, 5-, 7-, or 8-5H-pyrido[2,3-d]-o-oxazinyl, 1-, 3-, or 5-1H-pyrazolo[4,3-d]-oxazolyl, 2-, 4-, or 5-4H-imidazo[4,5-d]thiazolyl, 3-, 5-, or 8-pyrazino[2,3-d]pyridazinyl, 2-, 3-, 5-, or 6-imidazo[2,1-b]thiazolyl, 1-, 3-, 6-, 7-, 8-, or 9 -furo[3,4-c]cinnolinyl, 1-, 2-, 3-, 4-, 5-, 6-, 8-, 9-, 10 or 11-4H-pyrido[2,3-c]carbazolyl, 2-, 3-, 6- or 7-imidazo[1,2-b][1,2,4]triazinyl, 7-benzo[b]thienyl, 2-, 4-, 5-, 6- or 7-benzoxazolyl, 2-, 4-, 5-, 6- or 7-benzimidazolyl, 2-, 4- , 4-, 5-, 6- or 7-benzothiazolyl, 1-, 2-, 4-, 5-, 6-, 7-, 8- or 9-benzoxapinyl, 2-, 4-, 5-, 6-, 7- or 8-benzoxazinyl, 1-, 2-, 3-, 5-, 6-, 7-, 8-, 9-, 10- or 11-4H-pyrrolo[1,2-b][2]benzazapinyl. A typical fused heteroaryl comprises, but is not limited to, 2-, 3-, 4-, 5-, 6-, 7-, or 8-quinolinyl, 1-, 3-, 4-, 5-, 6-, 7-, or 8-isoquinolinyl, 2-, 3-, 4-, 5-, 6-, or 7-indolyl, 2-, 3-, 4-, 5-, 6-, or 7-benzo[b]thienyl, 2-, 4-, 5-, 6-, or 7-benzoxazolyl, 2-, 4-, 5-, 6-, or 7-benzimidazolyl, and 2-, 4-, 5-, 6-, or 7-benzothiazolyl. When the 5- to 10-membered heteroaryl is linked to other groups to constitute the compound of the present invention, either the carbon atom on the 5- to 10-membered heteroaryl ring can be linked to other groups, or the heteroatom on the 5- to 10-membered heteroaryl ring can be linked to other groups. When the 5- to 10-membered heteroaryl is

substituted, it can be monosubstituted or polysubstituted. Further, the substitution site is not limited. For example, hydrogen attached to a carbon atom on the heteroaryl ring may be substituted, or hydrogen attached to a heteroatom on the heteroaryl ring may be substituted.

The term "N-oxide" refers to a compound formed by oxidation of a nitrogen atom in the structure of tertiary amine or nitrogen-containing (aromatic) heterocyclic compound.

The term "spirocyclic ring" refers to a ring system in which two rings share 1 ring-forming atom.

The term "fused ring" refers to a ring system in which two rings share 2 ring-forming atoms.

The term "bridged ring" refers to a ring system in which two rings share more than 3 ring-forming atoms.

Unless otherwise specified, the heterocyclyl, heteroaryl, or heteroarylene comprises all possible isomeric forms thereof, such as positional isomers thereof. Therefore, for some illustrative non-limiting examples, the substitution or bonding to other radicals may be comprised at, e.g., 1, 2, or more of its 1-, 2-, 3-, 4-, 5-, 6-, 7-, 8-, 9-, 10-, 11-, 12-C (if present), comprising pyridin-2-yl, pyridyliden-2-yl, pyridin-3-yl, pyridyliden-3-yl, pyridin-4-yl, and pyridyliden-4-yl; and the thienyl or thienylenyl comprises thien-2-yl, thienylen-2-yl, thien-3-yl, and thienylen-3-yl; pyrazol-1-yl, pyrazol-3-yl, pyrazol-4-yl, and pyrazol-5-yl.

The term "oxo" refers to a substitution with oxygen (=O) formed when a carbon atom, nitrogen atom or sulfur atom in a substituent is oxidized.

The term "alkylamino" refers to -NH-(alkyl) or $-N-(alkyl)_2$, where the alkyl is as defined above. Non-limiting examples of alkylamino comprise: methylamino, ethylamino, propylamino, iso-propylamino, butylamino, dimethylamino, methylethylamino, diethylamino, dipropylamino, methylpropylamino, di-iso-propylamino, dibutylamino, and the like.

The term "alkoxy" refers to -O-(alkyl), where the alkyl is as defined above. Non-limiting examples of alkoxy comprise: methoxy, ethoxy, propoxy, and butoxy. The alkoxy may be optionally substituted or unsubstituted, and when it is substituted, the substituent is preferably one or more radicals independently selected from alkyl, alkenyl, alkynyl, alkoxy, alkylamino, halogen, sulfhydryl, hydroxyl, nitro, cyano, cycloalkyl, heterocycloalkyl, aryl, heteroaryl, cycloalkoxy, or heterocycloalkoxy.

The terms "alkyleneoxy" and "oxyalkylene" refer to -alkylene-O- or -O-alkylene-, where the alkylene represents a linear or branched saturated divalent hydrocarbon radical. For the definition of the number of carbon atoms in the "alkylene", the above definition for the "alkyl" applies. Those skilled in the art can understand that alkyleneoxy or oxyalkylene can be attached to the rest of the molecule containing it in any direction, that is, they can be used interchangeably.

"Haloalkyl" refers to an alkyl substituted with one or more halogens, where the alkyl is as defined above.

When L is selected from $-C(=O)-N(R^a)-$, it indicates that the radical attached to Ring A can be either the carbonyl in L or N in L. When the carbonyl is attached to Ring A, N in L is attached to the pyrazolopyridine ring, and when N is attached to the carbonyl, the carbonyl in L is attached to the pyrazolopyridine ring.

In the present invention, the compound involved also comprises an isotopically labeled compound, which is the same as the compound of Formula I, but in which one or more atoms are replaced by atoms having an atomic mass or mass number different from the atomic mass or mass number usually occurring in nature. Exemplary isotopes that can be incorporated into the compound of the present invention comprise isotopes of H, C, N, O, S, F and Cl, such as $^{2}H$, $^{3}H$, $^{13}C$, $^{11}C$, $^{14}C$, $^{15}N$, $^{18}O$, $^{17}O$, $^{32}P$, $^{35}S$, $^{18}F$ and $^{36}Cl$. The compound of the present invention containing the aforementioned isotopes and/or other isotopes of other atoms and a prodrug thereof, or a pharmaceutically acceptable salt of the compound or the prodrug are covered in the scope of the present invention. Some isotopically labeled compounds of the present invention, for example, compounds incorporated with radioactive isotopes (such as $^{3}H$ and $^{14}C$) can be used in the distribution determination of drugs and/or substrates in tissues. The isotopes tritium (that is, $^{3}H$) and carbon 14 (that is, $^{14}C$) are particularly preferred because of their easy preparation and detectability. Furthermore, the substitution with a heavier isotope (such as deuterium, that is, $^{2}H$ or D) can provide some therapeutic advantages due to the greater metabolic stability (for example, increased in-vivo half-life or reduced dosage requirement), and is thus preferred in some cases. The compound of the present invention as claimed in the claims can be particularly defined to be substituted with deuterium or tritium. In addition, in case that hydrogen in a substituent is not explicitly indicated to be deuterium or tritium, it does not mean that deuterium or tritium is excluded, but deuterium or tritium can be contained as well.

Those skilled in the art will understand that the compound represented by Formula (I) may exist in the form of various pharmaceutically acceptable salts. If the compounds have basic centers, they can form acid addition salts; if they have acidic centers, they can form base addition salts; if the compounds contain both acidic centers (e.g. carboxyl) and basic centers (e.g. amino), they can also form inner salts.

The compounds of the present invention may be present in the form of solvates, such as hydrates, wherein the compounds of the present invention comprise polar solvents, such as water, methanol or ethanol, as structural elements of the lattice of the compounds. The polar solvent, especially water, may be present in a stoichiometric or non-stoichiometric amount.

Depending on their molecular structure, the compounds of the present invention may be chiral and may therefore exist in various enantiomeric forms. These compounds may therefore exist in racemic or optically active forms. The

compounds of the present invention encompass isomers in which the chiral carbons are each in R or S configuration, or mixtures and racemates thereof. The compounds of the present invention or their intermediates can be separated into enantiomeric compounds by chemical or physical methods known to those skilled in the art, or used in synthesis in this form. In the case of racemic amines, diastereomers are prepared from the mixture by reaction with an optically active resolving reagent. Examples of suitable resolving reagents are optically active acids, such as the R and S forms of tartaric acid, diacetyltartaric acid, dibenzoyltartaric acid, mandelic acid, malic acid, lactic acid, appropriate N-protected amino acids (e.g. N-benzoylproline or N-phenylsulfonylproline) or various optically active camphorsulfonic acids. Chromatographic enantiomeric resolution can also be advantageously carried out by means of optically active resolving reagents, such as dinitrobenzoylphenylglycine, cellulose triacetate or other carbohydrate derivatives or chirally derivatized methacrylate polymers immobilized on silica gel. Suitable eluents for this purpose are aqueous or alcoholic solvent mixtures, for example, hexane/isopropanol/acetonitrile.

The corresponding stable isomers can be separated according to known methods, such as by extraction, filtration or column chromatography.

The term "patient" refers to any animal, including mammals, preferably mice, rats, other rodents, rabbits, dogs, cats, pigs, cows, sheep, horses or primates, and most preferably humans.

The term "therapeutically effective amount" refers to that amount of an active compound or drug which causes a biological or medical response sought by a researcher, veterinarian, physician or other clinician in a tissue, system, animal, individual or human, and which comprises one or more of the following: (1) prevention of disease: for example, prevention of a disease, disorder or condition in an individual who is susceptible to the disease, disorder or condition but has not yet experienced or developed the pathology or symptoms of the disease. (2) Inhibition of disease: for example, inhibition of a disease, disorder or condition (i.e., prevention of further progression of pathology and/or symptoms) in an individual undergoing or exhibiting the pathology or symptoms of the disease, disorder or condition. (3) Alleviation of disease: for example, alleviation of a disease, disorder or condition (i.e., reversion of pathology and/or symptoms) in an individual undergoing or exhibiting the pathology or symptoms of the disease, disorder or condition.

## DETAILED DESCRIPTION

The technical solution of the present invention will be further described in detail below in conjunction with specific embodiments. It should be understood that the following embodiments are only intended to exemplify and explain the present invention and should not be construed as limiting the scope of protection of the present invention. All technologies realized based on the above contents of the present invention are included in the scope that the present invention intends to protect.

Unless otherwise indicated, the raw materials and reagents used in the following examples are commercially available or may be prepared by known methods.

The structures of the compounds in the present invention are determined by nuclear magnetic resonance (NMR) or/and liquid chromatography-mass spectrometry (LC-MS). NMR chemical shifts (δ) are given in parts per million (ppm). The NMR measurement is performed by using Bruker AVANCE-400 nuclear magnetic resonance spectrometer, with deuterated dimethyl sulfoxide (DMSO-$d_6$), deuterated methanol (CD3OD), and deuterated chloroform (CDC13) as determination solvents, and with tetramethylsilane (TMS) as an internal standard.

An Agilent 1200 Infinity Series mass spectrometer is used for liquid chromatography-mass spectrometry (LC-MS) measurements. The HPLC measurements are performed by using Agilent 1200DAD high pressure liquid chromatograph and Waters 2695-2996 high pressure liquid chromatograph.

Yantai Huanghai HSGF254 or Qingdao GF254 silica gel plate is used as the silica gel plate for thin layer chromatography, the specification used for TLC is from 0.15 mm to 0.20 mm, and the specification of the thin layer chromatography for separation and purification of products is from 0.4 mm to 0.5 mm. Yantai Huanghai silica gel of 200-300 mesh is generally used as the carrier for column chromatography.

Unless otherwise specified, all reactions in the present invention are carried out under continuous magnetic stirring in a dry nitrogen or argon atmosphere in a dry solvent at a reaction temperature expressed in °C.

### Example 1

(5-(5-(1-(3,5-dichloropyridin-4-yl)ethoxy)-1H-pyrazolo[4,3-b]pyridin-3-yl)pyridin-2-yl)(imino)(methyl)- $\lambda^6$-sulfonyl ketone

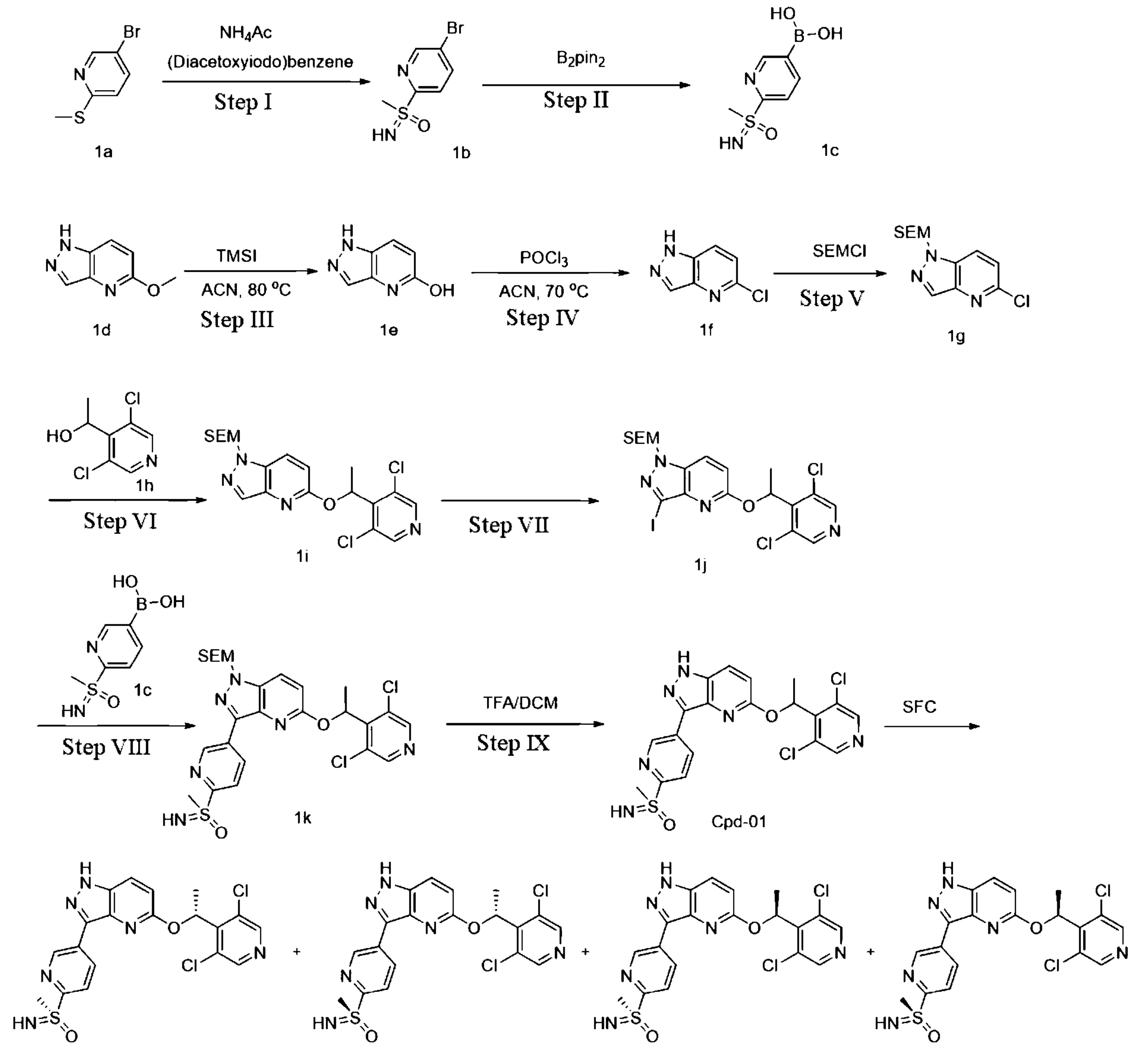


1a 1b 1c 1d 1e 1f 1g 1h 1i 1j 1c 1k Cpd-01

Step I

Compound 1a (10 g, 4.9 mmol), ammonium acetate (0.6 g, 7.8 mmol) and iodobenzene diacetate (3.2 g, 9.9 mmol) were weighed, and methanol (100 ml) was added, which were stirred at room temperature for three hours. The organic solvent was removed by concentration under reduced pressure, and the residue was purified by reverse-phase preparative HPLC (ACN/$H_2O$=5% to 80%) to give Compound 1b (8.7 g, yield 75%)

MS: m/z=234.9 $(M+H)^+$.

Step II

Compound 1b (8.7 g, 37 mmol), bis(pinacolato)diboron (18 g, 70.9 mmol), potassium carbonate (7.5 g, 54.3 mmol), and [1,1'-bis(diphenylphosphino)ferrocene]dichloropalladium (2.6 g, 3.6 mmol) were weighed and dissolved in 1,4-dioxane/water (5/1, 150 mL), and heated to 90°C and reacted for 16 hrs under a nitrogen atompshere. After cooling to room temperature, ethyl acetate (100 ml) was added, and the reaction solution was extracted. After separation, the organic layer was washed three times with dilute hydrochloric acid (10%, 100ml). The organic layers were combined. The organic phase was dried over anhydrous sodium sulfate, and filtered. The filtrate was concentrated, and the residue was separated by column chromatography (mobile phase: ethyl acetate/n-hexane =1/10 to 10/1) to give Compound 1c (7 g, yield 94.5%).

MS: m/z=201.1 $(M+H)^+$.

Step III

Commecially available Compound 1d (5 g, 33.5 mol), sodium iodide (15.0 g, 100.7 mmol), and trimethylchlorosilane (5.5 g, 50.6 mmol) were weighed and dissolved in anhydrous acetonitrile (100 ml), heated to 80°C, and reacted for 3 hrs. After cooling to room temperature, ethyl acetate (150 ml) and water (150 ml) were added, and the mixture was stirred vigorously for 1 hr. The organic layer was separated out, and the aqueous phase was extracted with ethyl acetate (150 ml). The organic phases were combined, and concentrated under reduced pressure, to give Compound 1e (3.6 g, yield 80%).
MS: m/z=136.0 $(M+H)^+$.

Step IV

Compound 1e (3.6 g, 26.6 mmol) was weighed and dissolved in phosphorus oxychloride (50 ml), heated to 70°C, and reacted for 3 hrs with heat preservation. Phosphorus oxychloride was removed by concentration. Ethyl acetate (50 ml) and water (50 ml) were added, and the reaction solution was extracted. After separation, the organic phase was dried, and the residue was separated by column chromatography (mobile phase: ethyl acetate/n-hexane =1/10 to 1/1) to give Compound 1f (3.5 g, yield 85%).
MS: m/z=154.0 $(M+H)^+$.

Step V

Compound 1f (3.5 g, 22.8 mmol) and t-potassium butoxide (2.5g, 22.0 mmol) were weighed, dissolved in tetrahydrofuran (50 ml), cooled to 0 to 5°C in an ice bath, and stirred for one hour. 2-(Trimethylsilyl)ethoxymethyl chlorode (4.2g, 25.2mmol) was added, the mixture was continuously reacted for 1 hr in an ice bath, and then stirred at room temperature for 2 hrs. The reaction solution was poured into water (100 ml), extracted with ethyl acetate (150 ml), and separated. The organic layers were combined, concentrated, and separated by column chromatography (mobile phase: ethyl acetate/n-hexane =1/10 to 1/2) to tive Compound 1g (5.8 g, yield 89.6%).
MS: m/z=284.1 $(M+H)^+$.

Step VI

Commercially available Compound 1h (1.0 g, 5.2 mmol) was weighed, dissolved in anhydrous tetrahydrofuran (50 ml), and stirred for half an hour in an ice bath. Sodium hydride (125 mg) was added, and the mixture was continuously stirred for half an hour in the ice bath. Compound 1g (1.5 g, 5.3 mmol) was added, the mixture was stirred for 2 hrs in the ice bath, and then stirred for one hour at room temperature. The reaction solution was poured into iced water (100 ml). The mixture was extracted with ethyl acetate (100 ml*3). The organic layers were combined, concentrated, and purified by column chromatography (mobile phase: ethyl acetate/n-hexane =1/10 to 1/1) to give Compound 1i (2.2 g, yield 94.7%)
MS: m/z=439.1 $(M+H)^+$.

Step VII

Compound 1i (2.2 g, 11.5 mmol) was weighed and dissolved in N,N-dimethylformamide (50ml). N-iodosuccinimide (2.8 g, 12.4 mmol) was added, the mixture was heated to 60°C, and reacted for 3 hrs under a nitrogen atmosphere. After cooling to room temperature, the reaction solution was pured into iced water (100 ml), and extracted with ethyl acetate (100 ml*3). The organic layers were combined, concentrated, and purified by column chromatography (mobile phase: ethyl acetate/n-hexane =1/10 to 1/1) to give Compound 1j (2.0 g, yield 70.6%).
MS: m/z=565.0 $(M+H)^+$.

Step VIII

Compound 1j (200 mg, 0.35 mmol) was weighed and dissolved in 1,4-dioxane/water (10/1, 10 mL). Then, Compound 1c (77 mg, 0.39 mmol), potassium carbonate (70 mg, 0.5 mmol), and [1,1'-bis(diphenylphosphino)ferrocene] dichloropalladium (10 mg) were added, and the mixture was reacted at 90°C for 2 hrs. After the reaction was completed, the reaction solution was extracted. The organic phase was collected, dried, and enriched. The residue was purified by column chromatography on silica gel (mobile phase: petroleum ether/ethyl acetate =10/1 to 1/1) to give Compound 1k (150 mg, yield 54.2%).
MS: m/z=593.1 $(M+H)^+$.

Step IX

Compound 1k (150 mg, 0.25 mmol) was weighed and dissolved in dichloromethane (10 mL). Trifluoroacetic acid (729 mg, 6.39 mmol) was added, and the reaction solution was stirred at 25°C for 16 hrs. After the reaction was completed, the reaction solution was concentrated, adjusted to pH 7-8 with a 10% sodium bicarbonate aqueous solution, extracted with ethyl acetate (50 ml), and concentrated. The residue was separated and purified by high performance liquid chromatography (mobile phase: acetonitrile/water =44/56) to give Cpd-01 (110 mg).

Cpd-01 was subjected to a first chiral resolution to give an isomer mixture Cpd-01M (30 mg, $t_R$ = 2.604 min), and optically pure compounds Cpd-01C (10 mg, $t_R$ = 2.691 min) and Cpd-01D (12 mg, $t_R$ = 3.352 min). Cpd-01M was subjected to a second chiral resolution to give optically pure compounds Cpd-01A (10 mg, $t_R$ = 1.836 min) and Cpd-01B (8 mg, $t_R$ = 2.300 min).

First resolution condition:

instrument brand: SFC 150;
preparative column model: Daicel CHIRALCEL OZ, 250 mm × 30 mm I.D., 10 μm;
mobile phase: $CO_2$/MeOH [0.2% $NH_3$ (7M Solution in MeOH)]= 65/35;
flow rate: 80 g/min;
column temperature: 35°C.

Second resolution condition:

instrument brand: SFC 150;
preparative column model: Daicel CHIRALCEL AD, 250 mm × 30 mm I.D., 10 μm;
mobile phase: $CO_2$/MeOH [0.2% $NH_3$ (7M Solution in MeOH)]= 65/35;
flow rate: 80 g/min;
column temperature: 35°C.

Cpd-01A
MS: m/z=463.0 $(M+H)^+$.
$^1$H NMR (400 MHz, $CDCl_3$) δ 9.51 (s, 1H), 8.81 (d, J = 7.6 Hz, 1H), 8.42 (s, 2H), 8.18 (s, 1H), 7.86 (d, J = 9.0 Hz, 1H), 7.04 (d, J = 9.0 Hz, 1H), 6.59 (dd, J = 13.8, 6.8 Hz, 1H), 3.36 (s, 3H), 1.81 (d, J = 6.8 Hz, 3H).

Cpd-01B
MS: m/z=463.0 $(M+H)^+$.
$^1$H NMR (400 MHz, $CDCl_3$) δ 9.49 (s, 1H), 8.80 (d, J = 8.0 Hz, 1H), 8.43 (s, 2H), 8.18 (s, 1H), 7.86 (d, J = 9.0 Hz, 1H), 7.04 (d, J = 9.0 Hz, 1H), 6.58 (q, J = 7.0 Hz, 1H), 3.35 (s, 3H), 1.81 (d, J = 6.8 Hz, 3H).

Cpd-01C
MS: m/z=463.0 $(M+H)^+$.
$^1$H NMR (400 MHz, $CDCl_3$) δ 9.51 (s, 1H), 8.82 (d, J = 8.2 Hz, 1H), 8.42 (s, 2H), 8.18 (d, J = 8.2 Hz, 1H), 7.85 (d, J = 9.2 Hz, 1H), 7.04 (d, J = 9.0 Hz, 1H), 6.60 (dd, J = 13.8, 6.8 Hz, 1H), 3.35 (s, 3H), 1.81 (d, J = 7.0 Hz, 3H).

Cpd-01D
MS: m/z=463.0 $(M+H)^+$.
$^1$H NMR (400 MHz, $CDCl_3$) δ 9.49 (s, 1H), 8.79 (s, 1H), 8.44 (s, 2H), 8.17 (s, 1H), 7.86 (d, J = 8.8 Hz, 1H), 7.04 (d, J = 9.0 Hz, 1H), 6.56 (d, J = 6.6 Hz, 1H), 3.38 (s, 3H), 1.81 (d, J = 6.8 Hz, 3H).

**Synthesis of Intermediate INT-1**

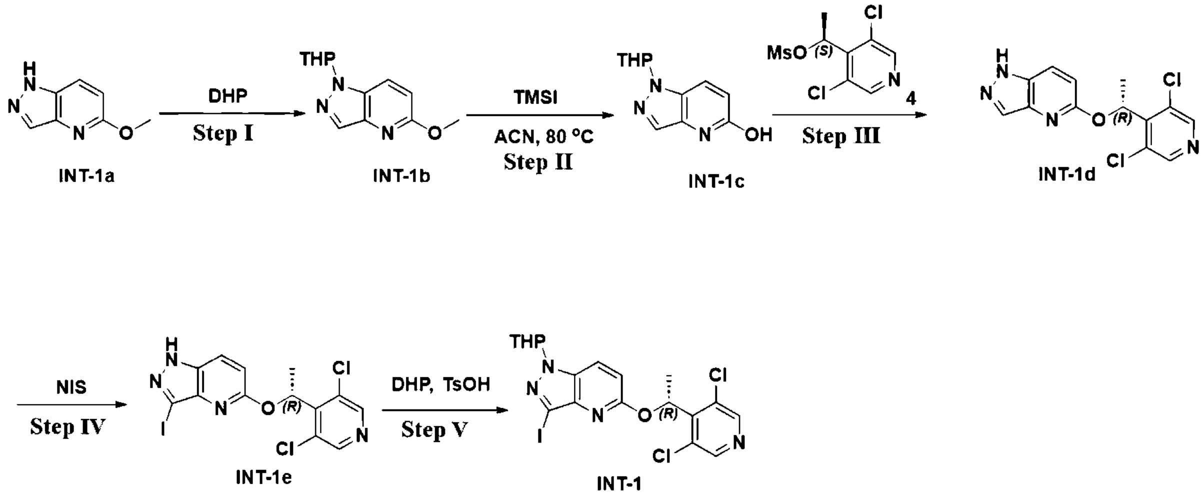


Step I

Tetrahydrofuran (100 mL) was added to 5-methoxy-4-azaindazole **INT-1a**(10 g, 0.067 mol), DHP(18.1 g, 0.216 mol) and PTSA (1.2 g, 0.0072 mol). The reaction solution was stirred at room temperature for 16 hrs. After the reaction was completed, the reaction solution was concentrated, washed with saturated ammonium chloride, extracted with ethyl acetate, dried over anhydrous sodium sulfate, and concentrated. The residue was separated and purified by column chromatography on silica gel (petroleum ether/ethyl acetate =5/1) to give 5-methoxy-1-(tetrahydro-2H-pyran-2-yl)-1*H*-4-azaindazole **INT-1b** (14.0 g, yield 89%).

MS m/z (ESI): 234.1 $(M+H)^+$.

Step II

5-methoxy-1-(tetrahydro-2*H*-pyran-2-yl)-1*H*-4-azaindazole **INT-1b** (14.0 g, 0.06 mol) and trimethyliodosilane (36.7 g, 0.18 mol) were weighed. Then, acetonitrile (100 ml) was added, and the mixture was heated to 80°C. After 6 hrs of reaction, the reaction solution was cooled to room temperature, and washed and extracted with ethyl acetate (100 ml) and water (100 ml). The organic layer was concentrated and purified by column chromatography (ethyl acetate /petroleum ether=1/5 to 1/1) to give Compound **INT-1c**(9.2 g, yield 70%).

MS m/z (ESI): 220.1 $(M+H)^+$.

Step III

Compound **INT-1c** (9.0 g, 0.041 mol) was weighed and dissolved in DMF (50 mL). Then, cesium carbonate (3.25 g, 10.0 mmol) and ethyl (R) -1-(3,5-dichloropyridin-4-yl)methansulfonate (16.7 g, 0.062 mol) were added. The reaction solution was stirred for 16 hrs under nitrogen atmosphere at 80°C. Then, water (50 mL) was added. The reaction solution was extracted with ethyl acetate and rotary evaporated to dryness. Trifluoroacetic acid (5 ml) and dichloromethane (50.0 mL) were added, and the reaction was conducted at room temperature for 6 hrs. After the reaction was completed, the reaction solution was added with water (50 mL). The organic layer was separated, extracted with ethyl acetate (50 ml), concentrated, and purified by column chromatography (mobile phase: ethyl acetate/petroleum ether=1/10 to 10/1) to give Compound **INT-1d** (10.0 g, yield 78%).

MS m/z (ESI): 309.0 $(M+H)^+$.

Step IV

Compound **INT-1d** (3.0 g, 9.7 mmol) was weighed and dissolved in anhydrous DMF (30.0 mL). NIS (2.18 g, 9.7 mmol) was added into the DMF (30 mL) solution, and the reaction solution was stirred at room temperature for 16 hrs. After the reaction was completed, the reaction solution was concentrated, quenched with a saturated sodium thiosulfate solution, concentrated, and separated and purified by column chromatography on silica gel (petroleum ether/ethyl acetate =5/1) to give Compound **INT-1e**(3.1 g, yield 73%).

MS m/z (ESI): 434.9 $(M+H)^+$.

$^1$H NMR (400 MHz, $CDCl_3$) δ 8.41 (s, 2H), 7.77 (d, *J*= 9.0 Hz, 1H), 6.99 (d, *J*= 9.0 Hz, 1H), 6.59 (q, *J*= 7.0 Hz, 1H), 1.80 (d, *J*= 7.0 Hz, 3H).

Step V

Compound **INT-1e** (8 g, 0.018 mol), DHP (1.8 g, 0.02 mol) and PTSA (0.3 g, 0.002 mol) were weighed, to which tetrahydrofuran (50 mL) was added. The reaction solution was stirred at room temperature for 16 hrs. After the reaction was completed, the reaction solution was concentrated, washed with saturated ammonium chloride, extracted with ethyl acetate, dried over anhydrous sodium sulfate, concentrated, and separated and purified by column chromatography on silica gel (petroleum ether/ethyl acetate =5/1) to give Compound **INT-1**(7.4 g, yield 80%).

MS m/z (ESI): 518.9 $(M+H)^+$.

**Example 2**

(*R*)-4-(5-(5-(1-(3,5-dichloropyridin-4-yl) ethoxy)-1*H*-pyrazolo[4,3-*b*]pyridin-3-yl) pyridin-2-yl)-3,6-dihydro-2*H*-thiopyran 1,1-dioxide

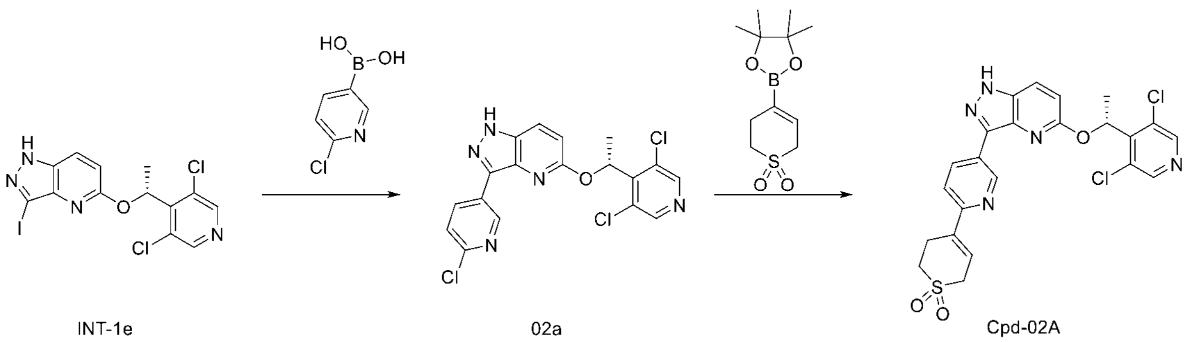

INT-1e 02a Cpd-02A

Step I

(*R*)-5-(1-(3,5-dichloropyridin-4-yl) ethoxy)-3-iodo-1*H*-pyrazolo[4,3-*b*] pyridine **INT-1e** (60 mg, 0.1379 mmol), (6-chloropyridin-3-yl)boric acid (28.21 mg, 0.17927 mmol), potassium acetate (40.6 mg, 0.4137 mmol) and Pd(AMPhos)$Cl_2$ (10.09 mg, 0.01379 mmol) were dissolved in ethanol/water (2/0.5 mL), and purged three times. The reaction solution was stirred at 90°C for 1 hr. After the reaction was completed, the reaction solution was filtered through diatomaceous earth, extracted with ethyl acetate, dried, concentrated, and separated and purified by column chromatography on silica gel (petroleum ether/ethyl acetate =1/1) to give (*R*)-3-(6-chloropyridin-3-yl)-5-(1-(3,5-dichloropyridin-4-yl) ethoxy)-1*H*-pyrazolo[4,3-*b*] pyridine **02a**(50 mg, yield 86%).

Step II

(*R*)-3-(6-chloropyridin-3-yl)-5-(1-(3,5-dichloropyridin-4-yl) ethoxy)-1*H*-pyrazolo[4,3-*b*] pyridine **02a** (60 mg, 0.1426 mmol), 4-(4,4,5,5-tetramethyl-1,3,2-dioxaborolan-2-yl)-3,6-dihydro-2*H*-thiopyran 1,1-dioxide (40.49 mg, 0.15686 mmol), potassium carbonate (39.42 mg, 0.2852 mmol) and Pd(dppf)$Cl_2$ (10.43 mg, 0.01426 mmol) were dissolved in 1, 4-dioxane/water (1/0.2 mL), and purged three times. The reaction solution was stirred at 90°C for 1 hr. After the reaction was completed, the reaction solution was filtered through diatomaceous earth, extracted with ethyl acetate, dried, concentrated, and separated and purified by column chromatography on silica gel (petroleum ether/ethyl acetate =1/1) to give a crude product. Finally, the crude product was purified by preparative high performance liquid chromatography (Waters MS-triggered Prep-LC with QDA detector, chromatographic column: Xbridge 5u C18 150 x 19 mm; mobile phase 1: water (with 0.1% formic acid); mobile phase 2: acetonitrile; over 9 min of a gradient of acetonitrile phase 50%-60%, flow rate: 25 mL/min), to give (*R*)-4-(5-(5-(1-(3,5-dichloropyridin-4-yl)ethoxy)-1*H*-pyrazolo[4,3-*b*]pyridin-3-yl)pyridin-2-yl)-3,6-dihydro-2*H*-thiopyran 1,1-dioxide **Cpd-02A** (8 mg, yield 11%).

$^1$HNMR(400MHz, DMSO-$d_6$) δ 13.51 (s, 1H), 9.16 (d,*J*= 1.8Hz, 1H), 8.63 (s, 2H), 8.36 (dd,*J*= 8.3, 2.1Hz, 1H), 8.07 (d,*J*= 9.0Hz, 1H), 7.72 (d,*J*= 8.4Hz, 1H), 7.07 (d,*J*= 9.0Hz, 1H), 6.74 (t,*J*= 4.5Hz, 1H), 6.41 (q,*J*= 6.8Hz, 1H), 4.02 (s, 2H), 3.42 (t,*J*= 6.1Hz, 2H), 3.25 (d,*J* = 5.6Hz, 2H), 1.75 (d,*J*= 6.8Hz, 3H).

**Example 3**

(*R*)-5-(1-(3,5-dichloropyridin-4-yl)ethoxy)-3-(6-(4-methylsulfonyl)piperidin-3-yl)-1*H*-pyrazolo[4,3-b]pyridine

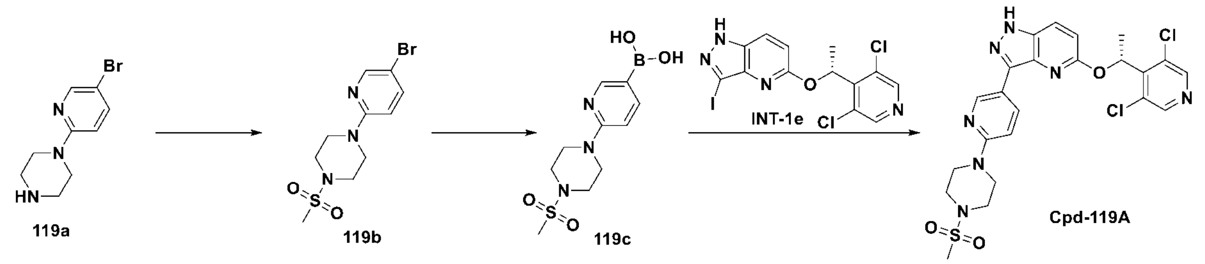


119a 119b 119c Cpd-119A

Step I

1-(5-bromopyridin-2-yl)piperazine **119a** (500 mg, 2.065 mmol), methanesulfonyl chloride (261 mg, 2.272 mmol), and triethylamine (418 mg, 4.130 mmol) were dissolved in dichloromethane (10 mL). The reaction was stirred at room temperature for 2 hrs. After the reaction was completed, the reaction solution was concentrated to give a crude product. The crude product was purified by column chromatography on silica gel (petroleum ether/ethyl acetate =1/1), to give 1-(5-bromopyridin-2-yl)-4-(methylsulfonyl)piperazine **119b** (400 mg), yield: 54.4%.

MS m/z (ESI): 320.0 $(M+H)^+$.

Step II

1-(5-bromopyridin-2-yl)-4-(methylsulfonyl)piperazine **119b** (200 mg, 0.625 mmol), bis(pinacolato)diboron (238 mg, 0.937 mmol), [1,1'-bis(diphenylphosphino)ferrocene]dichloropalladium (46 mg, 0.0625 mmol) and potassium acetate (123 mg, 1.249 mmol) were dissolved in 1.4-dioxane (5 mL) and stirred for 2 hrs under a nitrogen atmosphere at 80°C. After the reaction was completed, the reaction solution was filtered and concentrated. The crude product was purified by column chromatography on silica gel (petroleum ether/ethyl acetate =3/1) to give (6-(4-(methylsulfonyl)piperazine -1-yl)pyridin-3-yl)boric acid **119c** (100 mg), yield: 50.5%.

MS m/z (ESI): 286.1 $(M+H)^+$.

Step III

(6-(4-methylsulfonyl)piperazine -1-yl)pyridin-3-yl)boric acid **119c** (79 mg, 0.28 mmol), (*R*)-5-(1-(3,5-dichloropyridin-4-yl)ethoxy)-3-iodo-1*H*-pyrazolo[4,3-b]pyridine **INT-1e** (80 mg, 0.18 mmol), [1,1'-bis(diphenylphosphino)ferrocene]dichloropalladium (13 mg, 0.02 mmol) and potassium acetate (36 mg, 0.37 mmol) were dissolved in ethanol:water (5 mL), and stirred for 18 hrs under a nitrogen atmosphere at 90°C. After the reaction was completed, the reaction solution was filtered and concentrated. The crude product was purified by preparative HPLC (FA, mobile phase: ACN:$H_2O$ (0.1% $NH_3$)=50% : 50%), to give (*R*)-5-(1-(3,5-dichloropyridin-4-yl)ethoxy)-3-(6-(4-methylsulfonyl)piperidin-3-yl)-1*H*-pyrazolo [4,3-b]pyridine Cpd-**119A** (4.13 mg), yield: 3.92%.

MS m/z (ESI): 548.0 $(M+H)^+$.

$^1H$ NMR (400 MHz, $CDCl_3$) δ 8.95 (s, 1H), 8.41 (s, 2H), 8.30 (d, J = 8.4 Hz, 1H), 7.75 (d, J = 8.8 Hz, 1H), 6.98 (d, J = 9.0 Hz, 1H), 6.74 (d, J = 8.8 Hz, 1H), 6.56 (q, J = 7.0 Hz, 1H), 3.83 - 3.78 (m, 4H), 3.41 - 3.37 (m, 4H), 2.84 (s, 3H), 1.80 (d, J = 6.8 Hz, 3H).

Example 4

(*R*)-2-(1-(5-(1-(3,5-dichloropyridin-4-yl)ethoxy)-1*H*-pyrazolo[4,3-b]pyridin-3-yl)pyridin-2-yl)piperidin-4-yl)propan-2-ol

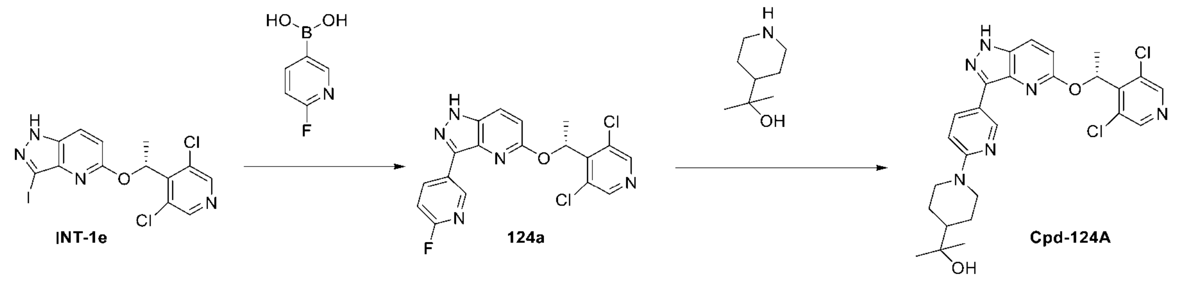

INT-1e, 124a, Cpd-124A

Step I

Preparation of (R)-5-(1-(3,5-dichloropyridin-4-yl)ethoxy)-3-(6-fluoropyridin-3-yl)-1*H*-pyrazolo[4,3-b]pyridine

(R)-5-(1-(3,5-dichloropyridin-4-yl)ethoxy)-3-iodo-1*H*-pyrazolo[4,3-b]pyridine **INT-1e** (300 mg, 0.58 mmol) was dissolved in dioxane/water (5/1, 5 mL). (6-Fluoropyridin-3-yl)boric acid (97 mg, 0.58 mmol), potassium carbonate (240 mg, 1.773 mmol), and [1,1'-bis(diphenylphosphino)ferrocene]dichloropalladium (II) (84 mg, 0.11 mmol) were added, and the mixture was stirred at 90°C for 3 hrs. After the reaction was completed, the reaction solution was quenched with water and extracted with ethyl acetate (3 x 30 mL). The organic phase was dried over anhydrous sodium sulfate, and filtered. The filtrate was concentrated and purified by column chromatography on silica gel (mobile phase: petroleum ether/ethyl acetate =5/1), to give (R)-5-(1-(3,5-dichloropyridin-4-yl)ethoxy)-3-(6-fluoropyridin-3-yl)-1H-pyrazolo[4,3-b]pyridine **124a** (200 mg, yield: 60.99%).

MS m/z (ESI): 404.0 $(M+H)^+$.

Step II

(R)-5-(1-(3,5-dichloropyridin-4-yl)ethoxy)-3-(6-fluoropyridin-3-yl)-1*H*-pyrazolo[4,3-b]pyridine **124a** (30 mg, 0.07 mmol) was dissolved in N, N-dimethylacetamide (1 mL). N,N-di-isopropylethylamine (28 mg, 0.22 mmol) and 2-(piperidin-4-yl)propan-2-ol (21 mg, 0.14 mmol) were added. The reaction mixture was stirred at 80°C for 3 hrs. After the reaction was completed, the reaction solution was purified by preparative high performance liquid chromatography (Waters MS-triggered Prep-LC with QDA detector, chromatographic column: WELCH Xtimate C18 21.2*250 mm 10 um ; mobile phase 1: water (with 0.1% 0.1FA); mobile phase 2: acetonitrile; over 18 min of a gradient of acetonitrile phase 5%-100%, flow rate: 30 mL/min), to give (R)-2-(1-(5-(1-(3,5-dichloropyridin-4-yl)ethoxy)-1*H*-pyrazolo[4,3-b]pyridin-3-yl)pyridin-2-yl)piperidin-4-yl)propan-2-ol **Cpd-124A** (16.19 mg, yield: 41.37%).

MS m/z (ESI): 527.1 $(M+H)^+$.

$^1$H NMR (400 MHz, DMSO-$d_6$) δ 13.11 (s, 1H), 8.71 (s, 1H), 8.59 (s, 2H), 8.06 (d, *J*= 7.2 Hz, 1H), 7.98 (d, *J* = 9.2 Hz, 1H), 7.00 (d, *J* = 9.2 Hz, 1H), 6.86 (d, *J* = 9.2 Hz, 1H), 6.38 (d, *J* = 6.8 Hz, 1H), 4.51 (d, *J*= 12.8 Hz, 2H), 4.14 (s, 1H), 2.75 (t, *J* = 12.0 Hz, 2H), 1.81 (d, *J*= 12.0 Hz, 2H), 1.73(d, *J*= 6.8 Hz, 3H), 1.47 (t, *J*= 12.0 Hz, 1H), 1.38 - 1.19 (m, 2H), 1.07 (s, 6H).

Example 5

(R)-4-(5-(5-(1-(3,5-dichloropyridin-4-yl)ethoxy)-1H-pyrazolopyridin-3-yl)-3-fluoropyridin-2-yl)-3,6-dihydro-2H-thiapyran 1,1-dioxide

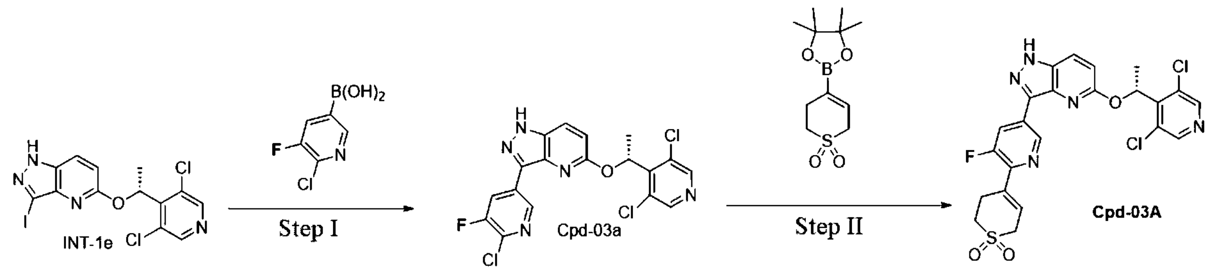


INT-1e, Cpd-03a, Cpd-03A

Step I

Under a nitrogen atmosphere, Compound INT-1e (150 mg, 0.34 mmol) was dissolved in ethanol (3 mL) and water (0.5 mL), to which (6-chloro-5-fluoropyridin-3-yl)boric acid (91 mg, 0.52 mmol), [1,1-bis(diphenylphosphino)ferrocene] dichloropalladium (25 mg, 0.03 mmol), and potassium acetate (68 mg, 0.69 mmol) were sequentially added. The reaction mixture was stirred at 90°C for 5 hrs, until the reaction was monitored to be completed by LC-MS. The reaction solution was filtered. The filtrate was collected and concentrated under reduced pressure to obtain a crude product. The crude product was purified by column chromatography (eluting with petroleum ether: ethyl acetate = 4:1) to give (R)-3-(6-chloro-5-fluoropyridin-3-yl)-5-(1-(3,5-dichloropyridin-4-yl)ethoxy)-1H-pyrazolopyridine **Cpd-03a** (100 mg), yield: 66%.

MS m/z (ESI): 438.0 $(M+H)^+$.

Step II

Under a nitrogen atmosphere, (R)-3-(6-chloro-5-fluoropyridin-3-yl)-5-(1-(3,5-dichloropyridin-4-yl)ethoxy)-1H-pyrazolopyridine **Cpd-03a** (100 mg, 0.23 mmol) was dissolved in 1,4-dioxane (5 mL) and water (1 mL), to which 1,1-dioxy-3,6-dihydro-2H-thiapyran-4-pinacol borate (71 mg, 0.28 mmol), [1,1'-bis(diphenylphosphino)ferrocene]dichloropalladium (II) (17 mg, 0.02 mmol), and potassium carbonate (63 mg, 0.46 mmol) were added. The reaction mixture was stirred at 90°C for 3 hrs. After the raw material was detected to be disappeared by LC-MS, the reaction was terminated. The reaction solution was filtered, and the filtrate was collected and concentrated under reduced pressure to give a crude product. The crude product was purified by column chromatography (eluting with petroleum ether: ethyl acetate = 10: 1 to 1:10) to give (R)-4-(5-(5-(1-(3,5-dichloropyridin-4-yl)ethoxy)-1H-pyrazolopyridin-3-yl)-3-fluoropyridin-2-yl)-3,6-dihydro-2H-thiapyran 1,1-dioxide **Cpd-03A** (56 mg, yield: 46%).

MS m/z (ESI): 534.0 $(M+H)^+$.

$^1$H NMR (400 MHz, DMSO-$d_6$) δ 13.67 (s, 1H), 9.05 (s, 1H), 8.56 (s, 2H), 8.16 (d, *J* = 12.6 Hz, 1H), 8.09 (d, *J* = 9.0 Hz, 1H), 7.09 (d, *J* = 9.0 Hz, 1H), 6.52 - 6.38 (m, 2H), 4.05 (s, 2H), 3.44 (t, *J*= 6.0 Hz, 2H), 3.24 (t, *J*= 6.0 Hz, 2H), 1.74 (d, *J*= 6.8 Hz, 3H).

Example 6

(*R*)-3-(6-(1-cyclopropyl-3-methyl-1*H*-pyrazol-4-yl)pyridin-3-yl)-5-(1-(3,5-dichloropyridin-4-yl)ethoxy)-1*H*-pyrazolo[4,3-*b*] pyridine

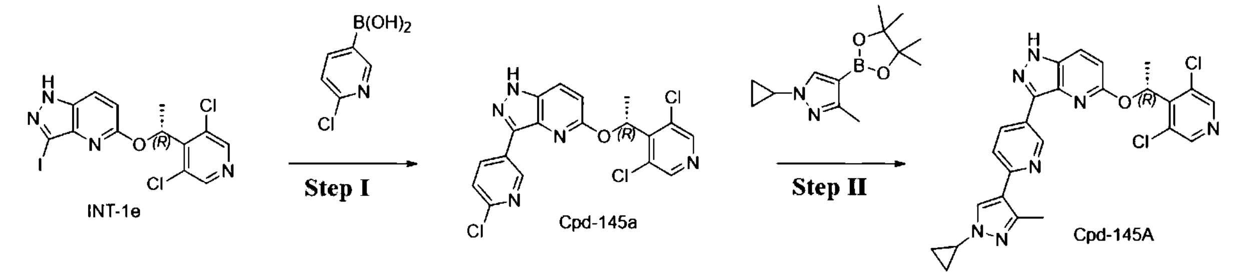


Step I

Under a nitrogen atmosphere, Compound INT-1e (150 mg, 0.34 mmol) was dissolved in ethanol (3 mL) and water (0.5 mL), to which (6-chloropyridin-3-yl)boric acid (82 mg, 0.52 mmol), [1,1-bis(diphenylphosphino)ferrocene]dichloropalladium (25 mg, 0.03 mmol), and potassium acetate (68 mg, 0.69 mmol) were sequentially added. The reaction mixture was stirred at 90°C for 5 hrs, until the reaction was monitored to be completed by LC-MS. The reaction solution was filtered. The filtrate was collected and concentrated under reduced pressure to obtain a crude product. The crude product was purified by column chromatography (eluting with petroleum ether: ethyl acetate = 4 : 1) to give (R)-3-(6-chloropyridin-3-yl)-5-(1-(3,5-dichloropyridin-4-yl)ethoxy)-1H-pyrazolopyridine **Cpd-145a** (110 mg, yield: 75%).

MS m/z (ESI): 420.0 $(M+H)^+$.

Step II

To (*R*)-3-(6-chloropyridin-3-yl)-5-(1-(3,5-dichloropyridin-4-yl)ethoxy)-1*H*-pyrazolo[4,3-*b*]pyridine **Cpd-145a**

(100 mg, 0.2 mmol) in a mixed solvent (6 mL) of 1,4-dioxane/water (having a volume ratio of 5/1) were added 1-cyclopropyl-3-methyl-1*H*-pyrazol-4-pinacol borate (100 mg, 0.4 mmol), potassium carbonate (66 mg, 0.5 mmol), and 1,1'-bis(diphenylphosphino)ferrocene (34 mg, 0.04 mmol). The reaction mixture was stirred at 90°C for 1 hr. After the reaction was completed, the reaction was quenched with water and extracted with ethyl acetate (3 x 10 mL). The organic phase was dried over anhydrous sodium sulfate, and filtered, and the filtrate was concentrated. The concentrate was purified by column chromatography (eluting with petroleum ether/ethyl acetate = 10/1 to 1/10) to give (R)-3-(6-(1-cyclopropyl-3-methyl-1*H*-pyrazol-4-yl)pyridin-3-yl)-5-(1-(3,5-dichloropyridin-4-yl)ethoxy)-1*H*-pyrazolo[4,3-*b*]pyridine **Cpd-145A** (35 mg, yield: 27.98%).

MS m/z (ESI): 506.1 $(M+H)^+$.

$^1$H NMR (400 MHz, $CDCl_3$) δ 9.80 (dd, *J*= 5.8, 2.9 Hz, 1H), 9.34 (s, 1H), 8.46 (d, *J* = 9.9 Hz, 1H), 8.42 (s, 1H), 7.96 (s, 1H), 7.79 (d, *J* = 9.0 Hz, 1H), 7.50 (d, *J* = 8.2 Hz, 1H), 7.01 (d, *J*= 9.1 Hz, 1H), 6.60 (dd, *J* = 13.7, 6.8 Hz, 1H), 3.61 (ddd, *J*= 10.9, 7.2, 3.6 Hz, 1H), 2.64 (s, 3H), 1.81 (d, *J* = 6.9 Hz, 3H), 1.20 - 1.16 (m, 2H), 1.08 - 1.04 (m, 2H).

Example 7

5-((R)-1-(3,5-dichloropyridin-4-yl)ethoxy)-3-iodo-6-methyl-1-(tetrahydro-2H-pyran-2-yl)-1*H*-pyrazolo[4,3-b]pyridine **INT-2**

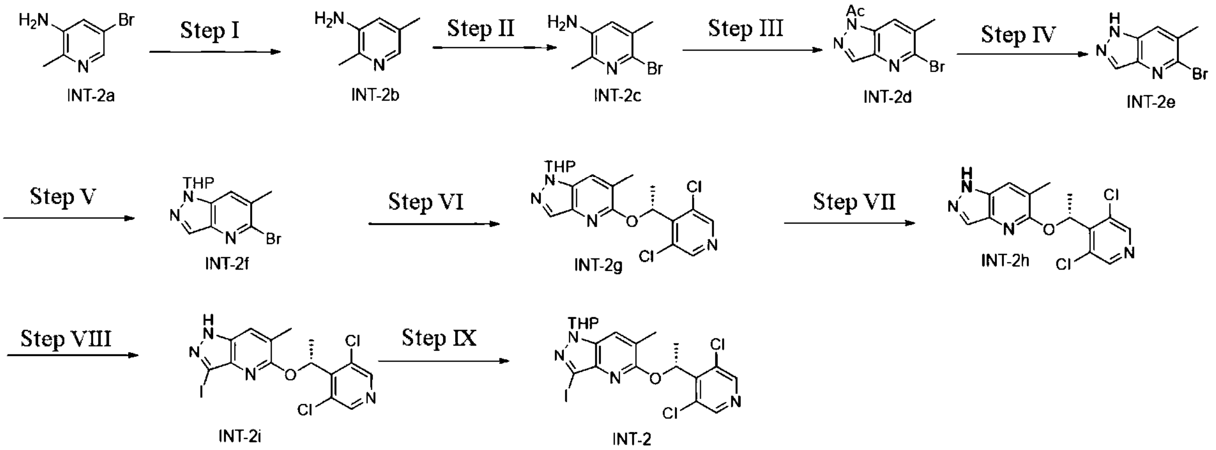


Step I

5-Bromo-2-methylpyridin-3-amine **INT-2a** (10 g, 0.0535 mol) was dissolved in 1,4-dioxane (75 mL) and water (10 mL). Methylboric acid (14.41 g, 0.24 mol), potassium carbonate (22.18 g, 0.16 mmol), and [1,1-bis(diphenylphosphino) ferrocene]dichloropalladium (2.9 g, 4 mmol) were added. Under a nitrogen atmosphere, the reaction mixture was stirred at 100°C for 16 hrs. After the reaction was completed, water (30 mL) was added, and the reaction solution was extracted with ethyl acetate (60 mL). The organic phase was washed with brine, dried over anhydrous sodium sulfate, concentrated, pretreated with silica gel, and purified by column chromatography (mobile phase: dichloromethane/methanol =40/1), to give 2,5-dimethylpyridin-3-amine **INT-2b** (6.2 g, yield: 94%).

MS m/z (ESI): 123.2 $(M+H)^+$.

Step II

2,5-Dimethylpyridin-3-amine **INT-2b** (6.2 g, 0.0507 mol) was dissolved in N,N-dimethylformamide (20 mL). N-bromosuccinimide (9.02 g, 0.0507 mol) was added, and the mixture was stirred at 0°C for 3 hrs. After the reaction was completed, water (30 mL) was added, and then the reaction solution was extracted with ethyl acetate (50 mL x 4). The organic phase was washed with brine (30 mL x 3), dried over anhydrous sodium sulfate, concentrated, pretreated with silica gel, and purified by column chromatography (mobile phase: petroleum ether/ethyl acetate =68/32), to give 6-bromo-2,5-dimethylpyridin-3-amine **INT-2c** (5.1 g, yield: 50%).

MS m/z (ESI): 201.0 $(M+H)^+$.

Step III

6-Bromo-2,5-dimethylpyridin-3-amine **INT-2c** (5.1 g, 25.40 mmol) was dissolved in chloroform (60 mL), to which potassium acetate (2.99 g, 30.48 mmol) and acetic anhydride (10.37 g, 101.60 mmol) were added. The reaction mixture was stirred at 55°C for 2 hrs. Then, the reaction solution was cooled to 0°C, to which 18-crown ether-6 (0.67 g, 2.54 mmol) and iso-pentyl nitrite (5.95 g, 50.8 mmol) were added. The reaction mixture was stirred at 80°C for 16 hrs. After the reaction was completed, the reaction was quenched with a sodium bicarbonate solution, and extracted with dichloromethane (100 ml). The organic phase was washed with brine (100 ml), dried over anhydrous sodium sulfate, concentrated, pretreated with silica gel, and purified by column chromatography on silica gel (mobile phase: petroleum ether/dichloromethane =75/25 to 70/30) to give 1-(5-bromo-6-methyl-1*H*-pyrazolo[4,3-b]pyridin-1-yl)ethan-1-one **INT-2d** (4.9 g, yield: 76%).
MS m/z (ESI): 254.0 $(M+H)^+$.

Step IV

An ammonia/methanol solution (40 mL) was added to 1-(5-bromo-6-methyl-*1H*-pyrazolo[4,3-b]pyridin-1-yl)ethan-1-one **INT-2d** (4.9 g, 19.29 mmol), and the reaction was stirred at room temperature for 2 hrs. After the reaction was completed, the reaction solution was concentrated to give 5-bromo-6-methyl-1*H*-pyrazolo[4,3-b]pyridine **INT-2e** (4.6 g), which was directly used in the next reaction without further purification.

Step V

5-Bromo-6-methyl-1*H*-pyrazolo[4,3-b]pyridine **INT-2e** (4.6 g, 21.70 mmol) was dissolved in dichloromethane (40 mL), to which 3,4-dihydro-2*H*-pyran (5.48 g, 65.10 mmol) and p-toluenesulfonic acid (0.37 g, 2.17 mmol) were added, and stirred at room temperature for 16 hrs. After the reaction was completed, the reaction solution was concentrated, pretreated with silica gel, and purified by column chromatography (mobile phase: petroleum ether/ethyl acetate =80/20) to give 5-bromo-6-methyl-1-(tetrahydro-2*H*-pyran-2-yl)-1*H*-pyrazolo[4,3-b]pyridine **INT-2f** (5.1 g, yield: 79%).
MS m/z (ESI): 296.0 $(M+H)^+$.

Step VI

5-Bromo-6-methyl-1-(tetrahydro-2*H*-pyran-2-yl)-*1H*-pyrazolo[4,3-b]pyridine **INT-2f** (5.1 g, 17.20 mmol) was dissolved in toluene (50 mL), to which (R)-1-(3,5-dichloropyridin-4-yl)ethan-1-ol (3.3 g, 17.20 mmol), sodium t-butoxide (3.31 g, 34.40 mmol), S-(-)-1,1'-binaphthyl-2,2'-bis(diphenylphosphine) (2.14 g, 3.44 mmol) and tris(dibenzylideneacetone)dipalladium (790 mg, 0.86 mmol) were added, and stirred for 12 hrs under a nitrogen atmosphere at 110°C. After the reaction was completed, the reaction solution was concentrated, pretreated with silica gel, and purified by column chromatography on silica gel (mobile phase: petroleum ether/ethyl acetate =91/9) to give 5-((R)-1-(3,5-dichloropyridin-4-yl)ethoxy)-6-methyl-1-(tetrahydro-2*H*-pyran-2-yl)-1*H*-pyrazolo[4,3-b]pyridine **INT-2g** (6.4 g, yield: 91%).
MS m/z (ESI): 407.1 $(M+H)^+$.

Step VII

To 5-((R)-1-(3,5-dichloropyridin-4-yl)ethoxy)-6-methyl-1-(tetrahydro-2*H*-pyran-2-yl)-1*H*-pyrazolo[4,3-b]pyridine **INT-2g** (6.4 g, 15.70 mmol) were added hydrochloric acid in methanol (4M, 30 mL) and methanol (5 mL), and the mixture was reacted at 50°C for 8 hrs. The reaction solution was concentrated and dissolved in ethyl acetate (50 mL). The reaction was neutralized with a sodium bicarbonate solution. The aqueous phase was extracted with ethyl acetate (50 ml), and then the organic phase was washed with a saturated sodium chloride solution, and dried over anhydrous sodium sulfate, to give (R)-5-(1-(3,5-dichloropyridin-4-yl)ethoxy)-6-methyl-1*H*-pyrazolo[4,3-b]pyridine **INT-2h**(4.7 g, yield: 93%).
MS m/z (ESI): 323.0 $(M+H)^+$.

Step VIII

(R)-5-(1-(3,5-dichloropyridin-4-yl)ethoxy)-6-methyl-1H-pyrazolo[4,3-b]pyridine **INT-2h** (4.7 g, 14.50 mmol) was dissolved in dichloromethane (100 mL), and N-iodosuccinimide (3.26 g, 14.50 mmol) was added. The reaction mixture was stirred at room temperature for 2 hrs. After the reaction was completed, the reaction solution was quenched with a sodium sulfite solution (80 mL). The organic layer was separated, washed with water and brine, and directly concentrated to give crude (R)-5-(1-(3,5-dichloropyridin-4-yl)ethoxy)-3-iodo-6-methyl-1H-pyrazolo[4,3-b]pyridine **INT-2i** (6.25 g), which was directly used in the next step.

Step IX

Crude (R)-5-(1-(3,5-dichloropyridin-4-yl)ethoxy)-3-iodo-6-methyl-1H-pyrazolo[4,3-b]pyridine **INT-2i** was dissolved in dichloromethane (100 mL), and 3,4-dihydro-2H-pyran (3.5 g, 41.70 mmol) and p-toluenesulfonic acid (0.24 g, 1.39 mmol) were added. The reaction mixture was stirred at room temperature for 16 hrs, and reacted at 40°C for 3 hrs. After the reaction was completed, the reaction solution was concentrated, pretreated with silica gel, and purified by column chromatography on silica gel (mobile phase: petroleum ether/ethyl acetate =86/14) to give 5-((R)-1-(3,5-dichloropyridin-4-yl)ethoxy)-3-iodo-6-methyl-1-(tetrahydro-2*H*-pyran-2-yl)-1*H*-pyrazolo[4,3-b]pyridine **INT-2** (6 g, yield: 81%).

MS m/z (ESI): 533.0 (M+H)$^+$.

Example 8

(*R*)-1-(4-(5-(1-(3,5-dichloropyridin-4-yl)ethoxy)-1*H*-pyrazolo[4,3-b]pyridin-3-yl)pyridin-2-y1)-3,5-dimethyl-1*H*-pyrazol-1-yl)-2-methyl-2-propanol **Cpd-158A**

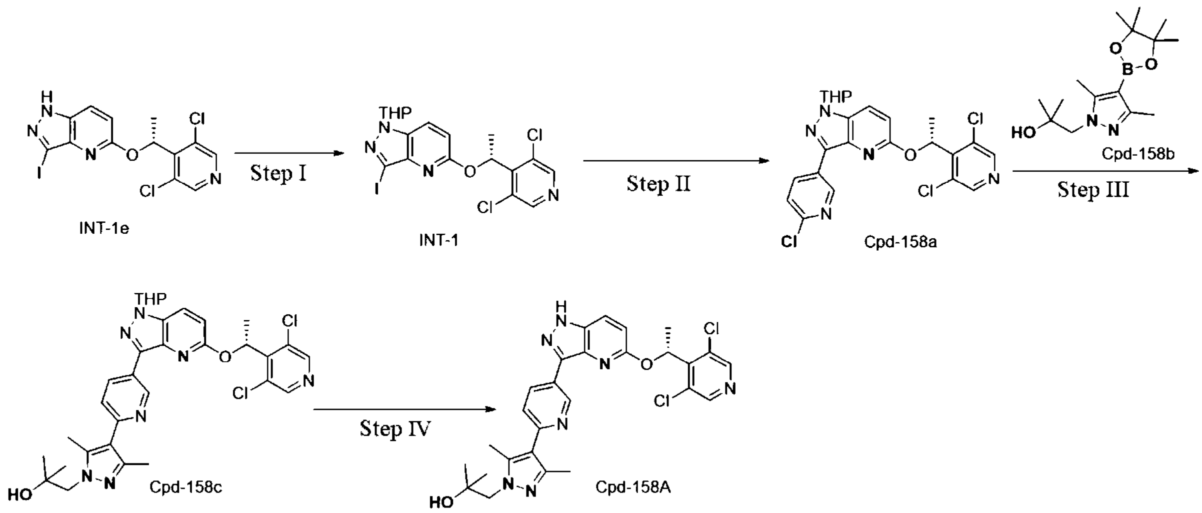

INT-1e INT-1 Cpd-158a Cpd-158b Cpd-158c Cpd-158A

Step I

(R)-5-(1-(3,5-dichloropyridin-4-yl)ethoxy)-3-iodo-1*H*-pyrazolo[4,3-b]pyridine **INT-1e** (6.2 g, 14.3 mmol) was dissolved in dichloromethane (20 mL). 3,4-Dihydro-2*H*-pyran (3.5 g, 41.70 mmol) and p-toluenesulfonic acid (0.24 g, 1.39 mmol) were added to the reaction flask, and the mixture was stirred at 25°C for 16 hrs. After the reaction was completed, the reaction solution was concentrated. The crude product was purified by column chromatography on silica gel (ethyl acetate /petroleum ether=13/100), to give 5-((*R*)-1-(3,5-dichloropyridin-4-yl)ethoxy)-3-iodo-1-(tetrahydro-2*H*-pyran-2-yl)-1*H*-pyrazolo[4,3-b]pyridine **INT-1** (6.1 g, yield: 81%).

MS m/z (ESI): 519.0 (M+H)$^+$.

Step II

5-((*R*)-1-(3,5-dichloropyridin-4-yl)ethoxy)-3-iodo-1-(tetrahydro-2*H*-pyran-2-yl)-1*H*-pyrazolo[4,3-b]pyridine **INT-1** (3 g, 5.8 mmol), (6-chloropyridin-3-yl)-boric acid (commercially available) (1 g, 6.4 mmol), [1,1'-bis(diphenylphosphino)ferrocene]dichloropalladium (420 mg, 0.6 mmol), and potassium carbonate (1.6 g, 11.6 mmol) were dissolved in a mixed solvent of 1,4-dioxane and water (25 mL), and the mixture was stirred for 16 hrs under a nitrogen atmosphere at 90°C. After the reaction was completed, the reaction solution was filtered and concentrated. The crude product was purified by column chromatography on silica gel (ethyl acetate/petroleum ether=8/100) to give 3-(6-chloropyridin-3-yl) -5-((*R*) -1-(3,5-dichloropyridin-4-yl)ethoxy) -1-(tetrahydro-2*H*-pyran-2-yl) -1*H*-pyrazolo[4,3-b ]pyridine **Cpd-158a** (2.2 g, yield: 75%).

MS m/z (ESI): 504.0 (M+H)$^+$.

Step III

3-(6-Chloropyridin-3-yl)-5-((*R*)-1-(3,5-dichloropyridin-4-yl)ethoxy)-1-(tetrahydro-2*H*-pyran-2-yl)-1*H*-pyrazolo [4,3-b]pyridine **Cpd-158a** (2.1 g, 4.2 mmol), 1-(3,5-dimethyl-4-(4,4,5,5-tetramethyl-1,3,2-dioxaborol-2-yl)-1*H*-pyrazol-1-yl)-2-methyl-2-propanol **Cpd-158b** (commercially available) (1.48 g, 5.0 mmol), [1,1'-bis(diphenylphosphino)ferrocene] dichloropalladium (300 mg, 0.4 mmol), and potassium carbonate (1.2 g, 8.4 mmol) were dissolved in a mixed solvent of 1,4-dioxane and water (25 mL), and the mixture was stirred for 16 hrs under a nitrogen atmosphere at 110°C. After the reaction was completed, the reaction solution was extracted with ethyl acetate. The organic phase was collected and concentrated. The crude product was purified by column chromatography on silica gel (ethyl acetate /petroleum ether-50/100) to give 1-(4-(5-(5-(*R*)-1-(3,5-dichloropyridin-4-yl)ethoxy)-1-(tetrahydro-2*H*-pyran-2-yl)-1*H*-pyrazolo[4,3-b]pyridin-3-yl)pyridin-2-yl)-3,5-dimethyl-1*H*-pyrazol-1-yl)-2-methyl-2-propanol **Cpd-158c** (1.7 g, yield: 64%).

MS m/z (ESI): 636.2 $(M+H)^+$.

Step IV

1-(4-(5-(5-(*R*)-1-(3,5-dichloropyridin-4-yl)ethoxy)-1-(tetrahydro-2*H*-pyran-2-yl)-1*H*-pyrazolo[4,3-b]pyridin-3-yl) pyridin-2-yl)-3,5-dimethyl-1*H*-pyrazol-1-yl)-2-methyl-2-propanol **Cpd-158c** (1.7 g, 2.7 mmol) was dissolved in hydrogen chloride/methanol (20 mL). The reaction solution was stirred at 50°C for 5 hrs. After the reaction was completed, the reaction solution was adjusted to pH 7-9 with a saturated sodium bicarbonate solution, and extracted with dichloromethane (100 ml). The organic phase was collected and concentrated. The crude product was concentrated and purified by column chromatography on silica gel (methanol/dichloromethane =8/100) to give the product (*R*)-1-(4-(5-(1-(3,5-dichloropyridin-4-yl)ethoxy)-1*H*-pyrazolo[4,3-b]pyridin-3-yl)pyridin-2-yl)-3,5-dimethyl-1*H*-pyrazol-1-yl)-2-methyl-2-propanol **Cpd-158A** (1.1 g), yield: 74%.

MS m/z (ESI): 552.2 $(M+H)^+$.

$^1$H NMR (400 MHz, $CDCl_3$) δ 9.50 (s, 1H), 8.73 (s, 1H), 8.40 (s, 2H), 7.87 (d, J = 8.8 Hz, 1H), 7.62 - 7.43 (m, 1H), 7.01 (d, J = 9.2 Hz, 1H), 6.58 (q, J = 6.8 Hz,1H), 4.66 (s, 1H), 4.03 (s, 2H), 2.55 (s, 3H), 2.46 (s, 3H), 1.80 (d, J = 6.8 Hz, 3H), 1.26 (s, 6H).

Example 9

(*R*)-1-(4-(5-(5-(1-(3,5-dichloropyridin-4-yl)ethoxy)-1*H*-pyrazolo[4,3-*b*]pyridin-3-yl)-3-fluoropyridin-2-yl)-3,5-dimethyl-1*H*-pyrazol-1-yl)-2-methyl-2-propanol **Cpd-161A**

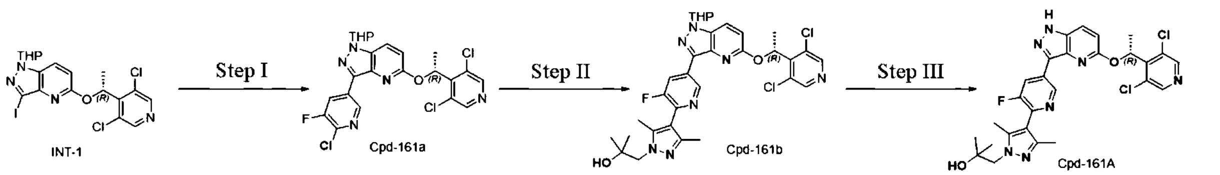


Step I

5-((*R*)-1-(3,5-dichloropyridin-4-yl)ethoxy)-3-iodo-1-(tetrahydro-2*H*-pyran-2-yl)-1*H*-pyrazolo[4,3-b]pyridine **INT-1** (4.8 g, 9.2 mmol) , (6-chloro-5-fluoropyridin-3-yl)-boric acid (commercially available) (1.94 g, 11.0 mmol), [1,1'-bis(diphenylphosphino)ferrocene]dichloropalladium (670 mg, 0.9 mmol) and potassium carbonate (2.54 g, 18.4 mmol) were dissolved in a mixed solvent (150 mL) of 1,4-dioxane and water, and stirred for 16 hrs under a nitrogen atmosphere at 90°C. After the reaction was completed, the reaction solution was filtered and concentrated. The crude product was purified by column chromatography on silica gel (ethyl acetate /petroleum ether=8/100), to give 3-(6-chloro-5-fluoropyridin-3-yl)-5-((*R*)-1-(35-dichloropyridin-4-yl)ethoxy)-1-(tetrahydro-2*H*-pyran-2-yl)-1*H*-pyrazolo[4,3-b]pyridine **Cpd-161a** (4.6 g, yield: 95%).

MS m/z (ESI): 522.5 $(M+H)^+$.

Step II

3-(6-chloro-5-fluoropyridin-3-yl)-5-((*R*)-1-(35-dichloropyridin-4-yl)ethoxy)-1-(tetrahydro-2H-pyran-2-yl)-1*H*-pyrazolo[4,3-b]pyridine **Cpd-161a** (4.6 g, 8.8 mmol), 1-(3,5-dimethyl-4-(4,4,5,5-tetramethyl-1,3,2-dioxaborol-2-yl)-1*H*-

pyrazol-1-yl)-2-methyl-2-propanol **Cpd-158b** (3.1 g, 10.6 mmol), [1,1'-bis(diphenylphosphino)ferrocene]dichloropalladium (640 mg, 0.9 mmol), and potassium carbonate (2.4 g, 17.6 mmol) were dissolved in a mixed solvent (150 mL) of 1,4-dioxane and water, and stirred for 16 hrs under a nitrogen atmosphere at 90°C. After the reaction was completed, the reaction solution was extracted with ethyl acetate, and the organic phase was collected and concentrated. The crude product was purified by column chromatography on silica gel (ethyl acetate /petroleum ether-60/100) to give 1-(4-(5-(5-((*R*)-1-(3,5-dichloropyridin-4-yl)ethoxy)-1-(tetrahydro-2*H*-pyran-2-yl)-1*H*-pyrazolo[4,3-b]pyridin-3-yl)-3-fluoropyridin-2-yl)-3,5-dimethyl-1*H*-pyrazol-1-yl)-2-methyl-2-propanol **Cpd-161b** (3.9 g, yield: 68%).

MS m/z (ESI): 654.2 $(M+H)^+$.

Step III

1-(4-(5-(5-((*R*)-1-(3,5-dichloropyridin-4-yl)ethoxy)-1-(tetrahydro-2*H*-pyran-2-yl)-1*H*-pyrazolo[4,3-b]pyridin-3-yl)-3-fluoropyridin-2-yl)-3,5-dimethyl-1*H*-pyrazol-1-yl)-2-methyl-2-propanol **Cpd-161b** (3.9 g, 6.0 mmol) was dissolved in hydrogen chloride/methanol (40 mL). The reaction solution was stirred at 50°C for 16 hrs. After the reaction was completed, the reaction was adjusted to pH 7-9 with a saturated sodium bicarbonate solution, and extracted with ethyl acetate. The organic phase was collected, and concentrated. The crude product was purified by column chromatography on silica gel (ethyl acetate /petroleum ether=60/100 to 100/1) to give the product (*R*)-1-(4-(5-(5-(1-(3,5-dichloropyridin-4-yl)ethoxy)-1*H*-pyrazolo[4,3-*b*]pyridin-3-yl)-3-fluoropyridin-2-yl)-3,5-dimethyl-1*H*-pyrazol-1-yl)-2-methyl-2-propanol **Cpd-161A** (2.5 g), yield: 70%.

MS m/z (ESI): 570.4 $(M+H)^+$.

$^1$H NMR (400 MHz, DMSO-$d_6$) δ 13.59 (s, 1H), 9.08 (s, 1H), 8.51 (s, 2H), 8.13 (dd, *J* = 11.0, 1.6 Hz, 1H), 8.07 (d, *J* = 9.0 Hz, 1H), 7.06 (d, *J* = 9.0 Hz, 1H), 6.40(t, *J* = 6.8 Hz, 1H), 4.75 (s, 1H), 3.98 (s, 2H), 2.33 (s, 3H), 2.22 (s, 3H), 1.72 (d, *J* = 6.8 Hz, 3H), 1.18 (s, 6H).

Example 10

(*R*)-1-(4-(5-(5-(1-(3,5-dichloropyridin-4-yl)ethoxy)-1*H*-pyrazolo[4,3-*b*]pyridin-3-yl)-3-fluoropyridin-2-yl)-3-methyl-1*H*-pyrazol-1-yl)-2-methyl-2-propanol **Cpd-162A**

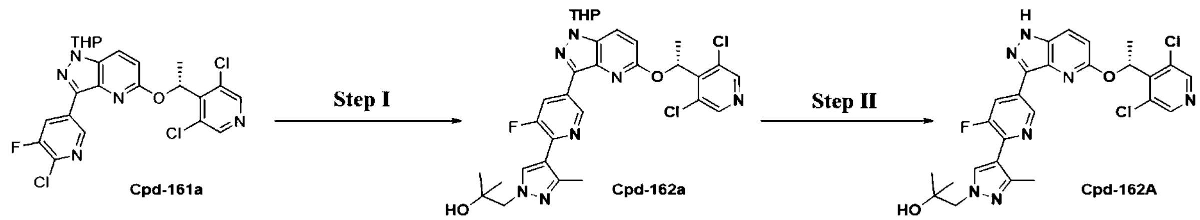


Cpd-161a Cpd-162a Cpd-162A

Step I

Under a nitrogen atmosphere, a mixed solution of 3-(6-chloro-5-fluoropyridin-3-yl)-5-((*R*)-1-(35-dichloropyridin-4-yl)ethoxy)-1-(tetrahydro-2*H*-pyran-2-yl)-1*H*-pyrazolo[4,3-b]pyridine **Cpd-161a** (10.0 g, 0.02 mol), 2-methyl-1-(3-methyl-4-(4,4,5,5-tetramethyl-1,3,2-dioxaborol-2-yl)-1*H*-pyrazol-1-yl)propan-2-ol (6.96 g, 0.025 mol), potassium carbonate (7.9 g, 0.06 mol), and [1,1'-bis(diphenylphosphino)ferrocene]dichloropalladium (2.37 g, 0.003 mol) in 1,4-dioxane/water (10/1, 88 mL) was stirred at 90°C for 10 hrs. After the reaction was completed, the reaction solution was concentrated, and extracted with ethyl acetate. The organic phase was combined, concentrated, and separated and purified by column chromatography on silica gel (petroleum ether/ethyl acetate =1/1) to give 1-(4-(5-(5-((*R*)-1-(3,5-dichloropyridin-4-yl)ethoxy)-1-(tetrahydro-2*H*-pyran-2-yl)-1*H*-pyrazolo[4,3-*b*]pyridin-3-yl)-3-fluoropyridin-2-yl)-3-methyl-1*H*-pyrazol-1-yl)-2-methyl-2-propanol **Cpd-162a** (8 g, yield 39%).

MS m/z (ESI): 640.2 $(M+H)^+$.

Step II

1-(4-(5-(5-((*R*)-1-(3,5-dichloropyridin-4-yl)ethoxy)-1-(tetrahydro-2H-pyran-2-yl)-1*H*-pyrazolo[4,3-*b*]pyridin-3-yl)-3-fluoropyridin-2-yl)-3-methyl-1*H*-pyrazol-1-yl)-2-methyl-2-propanol **Cpd-162a** (8 g, 0.012 mol) was dissolved in hydrogen chloride in methanol (4M, 80 mL). The mixed solution was stirred at 55°C for 2 hrs. After the reaction was completed, the reaction solution was concentrated, adjusted to pH 7-8 with a saturated sodium bicarbonate solution, and

extracted with dichloromethane. The organic phase was combined, concentrated, and separated and purified by column chromatography on silica gel (dichloromethane/methanol=1/20) to give a relatively pure product, which was chirally resolved (column: Daicel CHIRALCEL IB-N;mobile phase: $CO_2$/MeOH[0.2%$NH_3$(7M Solution in MeOH)]= 50/50), to give (R)-1-(4-(5-(5-(1-(3,5-dichloropyridin-4-yl)ethoxy)-1*H*-pyrazolo[4,3-b]pyridin-3-yl)-3-fluoropyridin-2-yl)-3-methyl-1*H*-pyrazol-1-yl)-2-methyl-2-propanol **Cpd-162A** (2.3 g, yield 33%).

MS m/z (ESI): 556.1 $(M+H)^+$.

$^1$H NMR (400 MHz, $CDCl_3$) $\delta$ 9.34 (s, 1H), 8.51-8.42 (m, 3H), 8.18 (s, 1H), 7.90 (d, *J* = 9.2 Hz, 1H), 7.02 (d, *J* = 9.2 Hz, 1H), 6.59 (q, *J*= 6.8 Hz, 1H), 4.15 (s, 2H), 2.63 (s, 3H), 1.80 (d, *J* = 6.8 Hz, 3H), 1.27 (s, 6H).

Example 11

(*R*)-1-(4-(5-(5-(1-(3,5-dichloropyridin-4-yl)ethoxy)-6-methyl-1*H*-pyrazolo[4,3-b]pyridin-3-yl)pyridin-2-yl)-3,5-dimethyl-1*H*-pyrazol-1-yl)-2-methyl-2-propanol **Cpd-175A**

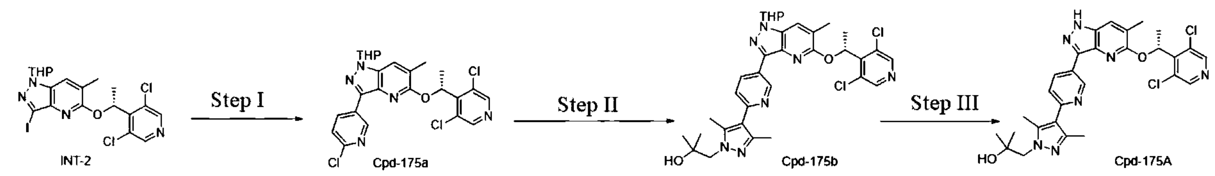


Step I

5-((R)-1-(3,5-dichloropyridin-4-yl)ethoxy)-3-iodo-6-methyl-1-(tetrahydro-2H-pyran-2-yl)-1H-pyrazolo[4,3-b] pyridine **INT-2** (3 g, 5.6 mmol), (6-chloropyridin-3-yl)-boric acid (commercially available) (1 g, 6.4 mmol), [1,1'-bis(diphenylphosphino)ferrocene]dichloropalladium (420 mg, 0.6 mmol) and potassium carbonate (1.6 g, 11.6 mmol) were dissolved in a mixed solution (25 mL) of 1,4-dioxane and water, and stirred for 16 hrs under a nitrogen atmosphere at 90°C. After the reaction was completed, the reaction solution was filtered and concentrated. The crude product was purified by column chromatography on silica gel (ethyl acetate /petroleum ether=8/100), to give 3-(6-chloropyridin-3-yl)-5-((*R*)-1-(3,5-dichloropyridin-4-yl)ethoxy)-6-methyl-1-(tetrahydro-2*H*-pyran-2-yl)-1*H*-pyrazolo[4,3-b]pyridine **Cpd-175a** (2.2 g, yield: 75%).

MS m/z (ESI): 518.0 $(M+H)^+$.

Step II

3-(6-chloropyridin-3-yl)-5-((*R*)-1-(3,5-dichloropyridin-4-yl)ethoxy)-6-methyl-1-(tetrahydro-2*H*-pyran-2-yl)-1*H*-pyrazolo[4,3-b]pyridine **Cpd-175a** (100 mg, 0.19 mmol, 1.0 eq) was dissolved in 1,4-dioxane (8 mL) and water (1.5 mL). 1-(3,5-dimethyl-4-(4,4,5,5-tetramethyl-1,3,2-dioxaborol-2-yl)-1H-pyrazol-1-yl)-2-methyl-2-propanol (commercially available) (68 mg, 0.23 mmol, 1.2 eq), potassium carbonate (80 mg, 0.58 mmol, 3 eq), and [1,1-bis(diphenylphosphino) ferrocene]dichloropalladium (14 mg, 0.02 mmol, 0.1 eq) were added. The reaction mixture was stirred for 8 hrs under a nitrogen atmosphere at 90°C. After the reaction was completed, the reaction solution was concentrated, pretreated with silica gel, and purified by column chromatography on silica gel (mobile phase: petroleum ether/ethyl acetate =37/63) to give 1-(4-(5-(5-((*R*)-1-(3,5-dichloropyridin-4-yl)ethoxy)-6-methyl-1-(tetrahydro-2H-pyran-2-yl)-1H-pyrazolo[4,3-b]pyridin-3-yl)pyridin-2-yl)-3,5-dimethyl-1H-pyrazol-1-yl)-2-methyl-2-propanol **Cpd-175b** (50 mg, yield: 40%).

MS m/z (ESI): 650.2 $(M+H)^+$.

Step III

1-(4-(5-(5-((R)-1-(3,5-dichloropyridin-4-yl)ethoxy)-6-methyl-1-(tetrahydro-2H-pyran-2-yl)-1H-pyrazolo[4,3-b] pyridin-3-yl)pyridin-2-yl)-3,5-dimethyl-1H-pyrazol-1-yl)-2-methyl-2-propanol **Cpd-175b** (50 mg, 0.08 mmol) was added to hydrogen chloride in dioxane (4M, 3 mL), and then reacted for 2 hrs at 50 °C. After the reaction was completed, the reaction solution was concentrated and purified by preparative high performance liquid chromatography (Waters MS-triggered Prep-LC with SQD2 detector, chromatographic column: Xbridge 5u C18 150 x 19 mm;mobile phase 1: water (with 0.1% FA); mobile phase 2: acetonitrile; over 65%-75% gradient of acetonitrile phase in 16 min, flow rate: 20mL/min), to give (R)-1-(4-(5-(5-(1-(3,5-dichloropyridin-4-yl)ethoxy)-6-methyl-1*H*-pyrazolo[4,3-b]pyridin-3-yl)pyridin-2-yl)-3,5-dimethyl-1H-pyrazol-1-yl)-2-methyl-2-propanol **Cpd-175A**(21.7 mg, yield: 47%).

MS m/z (ESI): 566.1(M+H)$^+$.

$^1$H NMR (400 MHz, DMSO-$d_6$) δ 13.30 (s, 1H), 9.19 (d, *J* = 2.2 Hz, 1H), 8.58 (s, 2H), 8.33 (dd, *J* = 8.2, 2.2 Hz, 1H), 7.90 (d, *J*= 1.0 Hz, 1H), 7.46 (d, *J*= 8.4 Hz, 1H), 6.43 (q, *J*= 6.8 Hz, 1H), 4.74 (s, 1H), 3.98 (s, 2H), 2.53 (s, 3H), 2.44 (s, 3H), 2.39 (s, 3H), 1.77 (d, *J*= 6.8 Hz, 3H), 1.17 (s, 6H).

The compounds in Table 1 below were prepared using conditions similar to those in the above examples. The structural characterization data of these compounds are listed in Table 1.

Table 1

| Compound No. | $^1$H NMR data |
|---|---|
| Cpd-14A | $^1$H NMR (400 MHz, DMSO-$d_6$) δ 9.21 (s, 1H), 8.59 (s, 2H), 8.46 (s, 1H), 8.08 (d, J = 9.2 Hz, 1H), 7.57 (s, 1H), 7.08 (d, J = 9.2 Hz, 1H), 6.47 - 6.41 (m, 1H), 4.49(t, J = 7.2 Hz, 2H), 3.72 (t, J = 7.2 Hz, 2H), 2.99 (s, 3H), 2.55 (s, 3H), 2.38 (s, 3H), 1.75 (d, J = 6.8 Hz, 3H). |
| Cpd-152A | $^1$H NMR (400 MHz, DMSO-$d_6$) δ 9.21 (s, 1H), 8.59 (s, 2H), 8.46 (s, 1H), 8.08 (d, J = 9.2 Hz, 1H), 7.57 (s, 1H), 7.08 (d, J = 9.2 Hz, 1H), 6.47 - 6.41 (m, 1H), 4.49(t, J = 7.2 Hz, 2H), 3.72 (t, J = 7.2 Hz, 2H), 2.99 (s, 3H), 2.55 (s, 3H), 2.38 (s, 3H), 1.75 (d, J = 6.8 Hz, 3H). |
| Cpd-155A | $^1$H NMR (400 MHz, DMSO-$d_6$) δ 13.47 (s, 1H), 9.18 (d, J = 1.8 Hz, 1H), 8.58 (s, 2H), 8.33 (dd, J = 8.4, 2.2 Hz, 1H), 8.06 (d, J = 9.0 Hz, 1H),7.45 (d, J = 8.4 Hz, 1H), 7.07 (d, J = 9.0 Hz, 1H), 6.41 (q, J = 6.8 Hz, 1H), 3.75 (s, 3H), 3.33 (s, 3H), 2.36 (s, 3H), 1.75 (d, J = 6.8 Hz, 3H). |
| Cpd-156A | $^1$HNMR(400MHz, DMSO-$d_6$) δ 13.44 (s, 1H), 9.18 (d,J = 2.0Hz, 1H), 8.59 (s, 2H), 8.33 (dd, J = 8.2, 2.2Hz, 1H), 8.06 (d, J = 9.0Hz, 1H), 7.46(d, J = 8.0Hz, 1H), 7.07 (d, J = 8.8Hz, 1H), 6.41 (q, J = 6.8Hz, 1H), 4.09 (d, J = 7.2Hz, 2H), 2.51 (s, 3H), 2.37 (s, 3H), 1.75 (d, J = 6.8Hz, 3H),1.35 (t ,J = 7.2Hz, 3H). |
| Cpd-159A | $^1$H NMR (400 MHz, $CDCl_3$) δ 8.76 - 8.61 (m, 1H), 8.42 (s, 2H), 8.10 (d, J = 4.9 Hz, 2H), 7.94 - 7.86 (m, 1H), 7.49 - 7.42 (m, 1H), 7.05 - 7.00 (m, 1H), 6.62 - 6.47 (m, 1H), 4.16 (s, 2H), 1.80 (d, J = 6.9 Hz, 3H), 1.25 (s, 6H) |
| Cpd-160A | $^1$H NMR (400 MHz, $CDCl_3$) δ 9.26 (s, 1H), 8.44 (s, 4H), 8.35 (s, 1H), 7.95 - 7.79 (m, 1H), 7.11 - 6.95 (m, 1H), 6.61 (d, J = 6.7 Hz, 1H), 4.22 (s, 2H), 1.81 (d, J= 6.7 Hz, 3H), 1.27 (s, 6H) |
| Cpd-168A | $^1$H NMR (400 MHz, DMSO-$d_6$) δ 9.19 (d, J = 2.0 Hz, 1H), 8.76 (d, J = 7.6 Hz, 1H), 8.61 (s, 2H), 8.52 (s, 1H), 8.11 (d, J = 9.0 Hz, 1H), 7.96 (d, J = 8.6 Hz, 1H),7.10 (d, J = 9.0 Hz, 1H), 6.54 (q, J = 6.8 Hz, 1H), 4.07 (s, 2H), 2.58 (s, 3H), 1.77 (d, J = 7.0 Hz, 3H), 1.15 (d, J = 1.0 Hz, 6H). |
| Cpd-169A | $^1$H NMR (400 MHz, DMSO-$d_6$) δ 9.17 (d, J = 1.6 Hz, 1H), 8.61 (s, 2H), 8.51 - 8.42 (m, 2H), 8.08 (d, J = 9.2 Hz, 1H), 7.72 (d, J = 8.4 Hz, 1H), 7.07 (d, J = 9.2 Hz, 1H), 6.44 (d, J = 6.8 Hz, 1H), 4.57 (t, J = 6.8 Hz, 2H), 3.77 (t, J = 6.8 Hz, 2H), 2.98 (s, 3H), 2.58 (s, 3H), 1.75 (d, J = 6.8 Hz, 3H). |
| Cpd-170A | $^1$H NMR (400 MHz, DMSO-$d_6$) δ 13.42 (s, 1H), 9.14 (s, 1H), 8.61 (s, 2H), 8.29 (s, 2H), 8.05 (d, J = 9.0 Hz, 1H), 7.61 (d, J = 8.2 Hz, 1H),7.06 (d, J = 9.0 Hz, 1H), 6.41 (dd, J = 13.8, 7.0 Hz, 1H), 3.84 (s, 3H), 2.56 (s, 3H), 1.75 (d, J = 7.0 Hz, 3H). |
| Cpd-176A | $^1$HNMR(400MHz, DMSO-$d_6$) δ 13.34 (s, 1H), 9.17 - 9.14 (m, 1H), 8.61 (s, 2H), 8.39 (d,J = 7.6Hz, 1H), 8.28 (s, 1H), 7.90 (d, J = 0.8Hz, 1H), 7.69(d, J = 8.4Hz, 1H), 6.46 (q, J = 6.8Hz, 1H), 4.02 (s, 2H), 2.57 (s, 3H), 2.44 (d, J = 0.8Hz, 3H), 1.78 (d, J = 6.8Hz, 3H), 1.13 (s, 6H). |
| Cpd-177A | $^1$HNMR(400MHz, DMSO-$d_6$) δ 13.30 (s, 1H), 9.16 - 9.13 (m, 1H), 8.59 (s, 2H), 8.34 (d, J = 8.0Hz, 1H), 8.04 (s, 1H), 7.88 (d, J = 1.0Hz, 1H), 7.70(d, J = 8.4Hz, 1H), 6.43 (q, J = 6.8Hz, 1H), 4.05 (s, 2H), 2.75 (s, 3H), 2.42 (d, J = 0.6Hz, 3H), 1.76 (d, J = 6.8Hz, 3H), 1.16 (s, 6H). |
| Cpd-190A | $^1$H NMR (400 MHz, DMSO-$d_6$) δ 13.58 (s, 1H), 9.08 - 9.07 (m, 1H), 8.55 (s, 2H), 8.19 (d, J = 4.0 Hz, 1H), 8.13 (dd, J = 12.2, 1.8 Hz, 1H), 8.08 (d, J = 9.0 Hz,1H), 7.08 (d, J = 9.0 Hz, 1H), 6.45 (q, J = 8.0 Hz, 1H), 4.94 (t, J = 5.0 Hz, 1H), 4.18 (t, J = 5.6 Hz, 2H), 3.78 (q, J = 8.0 Hz, 2H), 2.60 (s, 3H), 1.75 (d, J = 7.0Hz, 3H) |
| Cpd-191A | $^1$H NMR (400 MHz, DMSO-$d_6$) δ 13.59 (s,lH), 9.07 (s, 1H), 8.55 (s, 2H), 8.13 (dd, J =12.2, 1.6 Hz, 1H), 8.08 (d, J = 9.0 Hz, 1H),7.88 (d, J = 4.0 Hz, 1H), 7.08 (d, J = 9.0 Hz,1H), 6.44 (q, J = 6.8 Hz, 1H), 4.21 (t, J = 5.8Hz, 2H), 3.79 (t, J = 5.8 Hz, 2H), 2.74 (s,3H), 1.75 (d, J = 6.8 Hz, 3H) |

(continued)

| Compound No. | $^1$H NMR data |
|---|---|
| Cpd-202A | $^1$H NMR (400 MHz, DMSO-$d_6$) δ 9.03 (s, 1H), 8.59 (s, 2H), 8.53 (d, J = 1.5 Hz, 1H), 8.18 (d, J = 13.4 Hz, 2H), 8.09 (d, J = 9.0 Hz, 1H), 7.08 (d, J = 9.0 Hz, 1H),6.47 (d, J = 6.8 Hz, 1H), 4.70 (t, J = 6.9 Hz, 2H), 3.80 (t, J = 6.9 Hz, 2H), 2.97 (s, 3H), 1.75 (d, J = 6.8 Hz, 3H) |
| Cpd-203A | $^1$H NMR (400 MHz, DMSO-$d_6$) δ 13.59 (s, 1H), 9.09 (s, 1H), 8.55 (s, 2H), 8.34 (d, J = 3.7 Hz, 1H), 8.18 - 8.12 (m, 1H), 8.08 (d, J = 9.3 Hz, 1H), 7.09 (d, J = 9.1Hz, 1H), 6.46 (d, J = 6.8 Hz, 1H), 4.60 (t, J = 6.9 Hz, 2H), 3.76 (t, J = 7.1 Hz, 2H), 2.98 (s, 3H), 2.61 (s, 3H), 1.75 (d, J = 6.8 Hz, 3H). |

**Biological Evaluation**

**Test Example 1: Inhibitory effect of representative compounds of the present invention on FGFR1 and FGFR3 kinases**

The inhibitory activity of the compounds on FGFR1 and FGFR3 kinases was tested by HTRF method. The highest concentration tested was 3000 nM, which was three-fold diluted to give 10 concentrations. The test was duplicated.

1.1. Experimental Materials

1.1.1. Reagents and Materials

| Reagent Name | Supplier | Art. No. | Lot No. |
|---|---|---|---|
| Dimethyl sulfoxide (DMSO) | Sigma | D8418-1L | 102420455 |
| Adenosine triphosphate (ATP) | Sigma | A7699 | 0000490252 |
| Dithiothreitol (DTT) | Sigma | 43816 | BCCF1365 |
| Magnesium chloride | Sigma | M1028 | 102477211 |
| FGFR3 | Invitrogen | PR4631B | 194617BD |
| FGFR1 | Invitrogen | PR4660A | 19681847 |
| HTRF KinEASE-TK kit | Cisbio | 62TKOPEC | 13A |
| 384 shallow-well white polystyrene plate | Greiner | 784075 | E211237T |

1.1.2. Instruments

| Instrument Name | Supplier | Model |
|---|---|---|
| Vortex Mixer | Thermo | 4625-1CECN/THZ Q |
| Centrifuge | Eppendorf | 5810R |
| Envision 2104 Multi-Mode Plate Reader | PerkinElmer | 2104 |
| Echo Liquid Handling Station | Labcyte | 655 |

1.2. Experimental Steps

a) The compound was diluted into a test plate (784075, Greiner) by using ECHO Liquid Handling Station, and centrifuged at 1000 rpm for 30 s.
b) A FGFR kinase solution of twice the final concentration was prepared with a 1X kinase buffer.
c) 5 μL of the FGFR kinase solution of twice the final concentration was added to the 384-well test plate, centrifuged and allowed to stand at room temperature for 10 min.
d) A mixture of Biotin-conjugated tyrosine kinase substrate and ATP of twice the final concentration was prepared with the 1X kinase buffer.
e) 5 μL of the mixture of Biotin-conjugated tyrosine kinase substrate and ATP of twice the final concentration was added to the test plate, to start the reaction.
f) The 384-well test plate was centrifuged at 1000g for 30s, mixed well and reacted at room temperature for 50 min.
g) 4X Sa-XL665 was prepared with a HTRF detection buffer.
h) 5 μL 4X Sa-XL665 and 5 μL $Eu^3$-Cryptate-conjugated TK antibody were added to the reaction plate.
i) The system was centrifuged at 1000 g for 30 s and reacted at room temperature for 1 hr.
j) The fluorescence signals at 665 nM and 615 nM of each well were read on the Envision2014 microplate reader, and the ratio was calculated.

$$\%inhibition=100\%-(Ratio_{cmpd}-Ratio_{noATP}/Ratio_{DMSO}-Ratio_{noATP})*100\%$$

1.3. Experimental Results

Table 2. Test results of kinase inhibitory activity of representative compound of the present invention

| Compound No. | FGFR1 ($IC_{50}$ nM) | FGFR3 ($IC_{50}$ nM) |
|---|---|---|
| Cpd-01C | 34.8 | 4.8 |
| Cpd-01D | 36.1 | 6.2 |

Experimental results show that the representative compound of the present invention has good kinase inhibitory activity.

**Test Example 2: Inhibitory Effect and Selectivity of Compound of the Present Invention on the Proliferation of Ba/F3-FGFR1/2/3/4 cells**

The CTG method was used to detect the inhibitory effect of the compound on the proliferation of Ba/F3-FGFR1, Ba/F3-FGFR2, Ba/F3-FGFR3, and Ba/F3-FGFR4 cells. The highest concentration tested was 3000 nM, which was three-fold diluted to give 10 concentrations. The test was duplicated.

2.1. Experimental Materials

2.1.1. Reagents and Materials

| Reagent Name | Supplier | Art. No. | Lot No. |
|---|---|---|---|
| Dimethyl sulfoxide (DMSO) | Sigma | D8418-1L | 102420455 |
| Heparin sodium salt | Sigma | H4784 | 1003534766 |
| Celltiter Glo assay kit | Promega | G7573 | 496323 |
| Recombinant Human FGF acidic/FGF1 aa 16-155 | R&D | 232-FA-025 | CQ3620021 |

2.1.2. Instruments

| Instrument Name | Supplier | Model |
|---|---|---|
| Vortex Mixer | Thermo | 4625-1CECN/THZ Q |
| Centrifuge | Eppendorf | 5810R |
| Envision 2104 Multi-Mode Plate Reader | PerkinElmer | 2104 |
| Echo Liquid Handling Station | Labcyte | 655 |

2.2. Experimental Steps

a) Ba/F3-FGFR1, Ba/F3-FGFR2, Ba/F3-FGFR3, and Ba/F3-FGFR4 were cultured to exponential growth period for later use.
Complete medium: RPMI1640 medium, 10% FBS, 1% GlutaMAX and P/S, 10 ng/mL FGF1, 10 μg/mL Heparin,10 μM 2-mercaptoethanol, 2 μg/mL puromycin.
b) The stock solution of the compound of 3000 nM was diluted into a 384 cell culture plate using the ECHO liquid handling station, and centrifuged at 1000 g for 1 min.
c) The cells were digested. 30 μL of Ba/F3-FGFR1, Ba/F3-FGFR2, Ba/F3-FGFR3, or Ba/F3-FGFR4 (500/well) was uniformly seeded in a 384-well black-edge cell culture plate containing the compound, having the wells at the edge blocked with 50 μL PBS, and cultured in an incubator with $CO_2$ at 37°C for 72 hrs.
d) 30 μL of Cell-Titer-Glo reagent was added to each well, shaken and mixed evenly on a shaker, and then incubated at 37°C for 30 min. Then the signal was detected on a microplate reader after the signal was stable.

2.3. Experimental Results

Table 3. Test results of inhibitory activity of representative compounds of the present invention on Ba/F3 cells

| Compound No. | Ba/F3-FGFR1 ($IC_{50}$ nM) | Ba/F3-FGFR2 ($IC_{50}$ nM) | Ba/F3-FGFR3 ($IC_{50}$ nM) | Ba/F3-FGFR4 ($IC_{50}$ nM) |
|---|---|---|---|---|
| Infigratinib | 8.1 | 4.0 | 11.9 | 277.2 |
| Cpd-02A | 23.9 | / | 5.06 | / |
| Cpd-03A | 14.2 | 8.4 | 4.2 | 33.8 |
| Cpd-14A | 141.9 | 24.7 | 34.1 | / |
| Cpd-119A | 45.9 | / | 7.3 | / |
| Cpd-124A | 122.6 | 16.7 | 13.6 | 80.4 |
| Cpd-145A | 31.4 | 10.6 | 9.7 | 76.3 |
| Cpd-152A | 27.9 | 2.9 | 4.0 | 43.9 |
| Cpd-158A | 23.6 | 2.0 | 2.6 | 55.4 |
| Cpd-123A | 45.9 | / | 7.3 | / |
| Cpd-155A | 79.6 | 7.7 | 10.0 | 165.3 |
| Cpd-156A | 120.3 | 10.4 | 17.8 | 193.0 |
| Cpd-160 | 72.9 | 8.2 | 9.6 | 62.2 |
| Cpd-161A | 98.3 | 11.7 | 15.4 | 236.6 |
| Cpd-162A | 32.6 | 3.6 | 4.0 | 29.8 |
| Cpd-168A | 34.9 | 7.3 | 7.0 | 87.6 |
| Cpd-169A | 24.5 | 4.6 | 4.8 | 23.7 |
| Cpd-170A | 48.2 | 7.7 | 7.7 | 165.3 |

(continued)

| Compound No. | Ba/F3-FGFR1 ($IC_{50}$ nM) | Ba/F3-FGFR2 ($IC_{50}$ nM) | Ba/F3-FGFR3 ($IC_{50}$ nM) | Ba/F3-FGFR4 ($IC_{50}$ nM) |
|---|---|---|---|---|
| Cpd-175A | 31.5 | 3.5 | 4.9 | 72.9 |
| Cpd-176A | 21.1 | 2.6 | 3.0 | 30.0 |
| Cpd-177A | 21.4 | 3.6 | 3.6 | 41.5 |
| Cpd-178A | 71.8 | 8.0 | 12.2 | 73.3 |
| Cpd-190A | 26.8 | 3.9 | 4.7 | 23.0 |
| Cpd-191A | 24.3 | 2.9 | 3.4 | 16.2 |
| Cpd-202A | 62.2 | 7.3 | 8.1 | 62.8 |
| Cpd-203A | 32.1 | 4.6 | 5.7 | 23.7 |

Experimental results show that the representative compounds of the present invention have potent inhibitory activity and good selectivity on Ba/F3-FGFR2/3 cells.

**Test example 3. Pharmacokinetic study of representative compounds of the present invention**

3.1 Experimental Purpose

The SD rats were used as test animals. The drug concentration in plasma at different time points was determined by LC/MS/MS method after the compound of the present invention was administered to the SD rats by oral gavage. The pharmacokinetic behavior of the compound of the present invention in SD rats was studied and the pharmacokinetic characteristics were evaluated.

3.2. Experimental Scheme

(1) Experimental Animals

24 SD Rat rats (female:male 1:1), purchased from Shanghai Ji Hui Laboratory Animal Breeding Co., Ltd., animal production license SCXK (Shanghai) 2017-0012, were divided into 6 groups.

(2) Drug Preparation

The prescription was 5% DMSO + 60% PEG300 + 35% glucose aqueous solution. A proper amount of the representative compound of the present invention (converted according to the purity and salt coefficient) was weighed. A prescribed amount of DMSO was added, and a clear and transparent solution was obtained by vortex. Then a prescribed amount of PEG300 was added, and mixed by vortex. Then, a prescribed amount of a glucose aqueous solution was added. A 0.6 mg/mL or 2 mg/mL solution was obtained. During the preparation, if a solution cannot be obtained, ultrasonication in a water bath at a temperature not higher than 60°C can be performed to facilitate the dissolution.

(3) Administration

Rats were administered by oral gavage (2mpk) after fasting for one night.

(4) Sample Collection

At 15 min, 30 min, 1 hr, 2 hrs, 4 hrs, 6 hrs, 8 hrs, and 24 hrs after administration, about 30 μL blood samples were collected into an anticoagulation tube containing the anticoagulant EDTA-K2, and centrifuged within 30 minutes to obtain the plasma. The whole blood sample was placed on wet ice before centrifugation. All of the collected plasma samples were stored on dry ice or at a temperature not higher than -70°C before analysis and detection. Liquid chromatography-tandem mass spectrometry (LC/MS/MS) was used to determine the original drug concentration in the plasma and the administered solution.

3.3. Experimental Results

The pharmacokinetic parameters of the representative compounds Cpd-03A/Cpd-145A/Cpd-168A/Cpd-175A/Cpd-203A of the present invention and Infigratinib in SD Rat are shown in Table 4.

Table 4. Experimental results of pharmacokinetics of the compounds of the present invention in rats

| Compound No. | Infigratinib | Cpd-03A | Cpd-145A | Cpd-168A | Cpd-175A | Cpd-203A |
|---|---|---|---|---|---|---|
| $T_{max}$ (hr) | 3.0 | 1.00 | 2.0 | 5.3 | 2.0 | 2.7 |
| $C_{max}$ (ng/mL) | 22.2 | 24.4 | 186.3 | 71.1 | 82.6 | 52.3 |
| $T_{1/2}$ (hr) | 1.1 | 1.5 | 4.0 | 4.5 | 3.2 | 4.7 |
| $AUC_{last}$ (hr*ng/mL) | 103.4 | 86.2 | 1568.5 | 949 | 359 | 426 |
| $AUC_{INF}$_pred (hr*ng/mL) | 106.1 | 91.5 | 1606.1 | 977 | 508 | 439 |
| $MRT_{INF}$_pred (h) | 3.1 | 2.0 | 5.6 | 7.0 | 3.5 | 5.9 |

Conclusion: The half-life ($T_{1/2}$), area under curve (AUC), in-vivo residence time, and other pharmacokinetic parameters of the representative compounds of the present invention in rats are good, and can meet the requirements of oral administration.

The embodiments of the technical solution of the present invention are described in an illustrative manner above. It is to be understood that the scope of protection of the present invention is not limited to the above-mentioned embodiments. Any modifications, equivalent substitutions, improvements, etc. made by those skilled in the art within the spirit and principles of the present invention should be included in the scope of protection of the claims of the present application.

## Claims

1. A compound of Formula (I), or a racemate, a stereoisomer, a tautomer, an isotopically labeled compound, an N-oxide, a solvate, a polymorph, a metabolite, an ester, a prodrug or a pharmaceutically acceptable salt thereof:

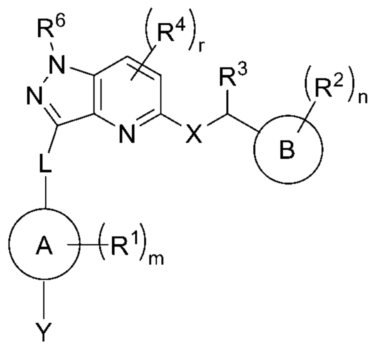

Formula (I)

wherein

Ring A and Ring B are the same or different and are each independently selected from a $C_{6-14}$ aromatic ring, a 5-to 14-membered heteroaromatic ring or a 5- to 14-membered heterocyclic ring;
L is absent or selected from -N($R^a$)-C(=O)- or -$CR^b$=$CR^c$-; and when L is absent, Ring A is directly attached to the pyrazole ring via a chemical bond;
$R^a$ is selected from hydrogen, or the following groups which are unsubstituted or optionally substituted with one, two or more $R^{a1}$: a $C_{1-6}$ alkyl and a $C_{3-6}$ cycloalkyl, where each $R^{a1}$ is the same or different, and independently selected from hydroxyl, cyano, halogen, a $C_{1-6}$ alkyl, a $C_{1-6}$ alkoxy, and a $C_{3-6}$ cycloalkyl;
$R^b$ and $R^c$ are the same or different, and each independently selected from hydrogen, halogen, or the following groups which are unsubstituted or optionally substituted with one, two or more $R^{b1}$: a $C_{1-6}$ alkyl, a $C_{1-6}$ alkoxy, and

a $C_{3-6}$ cycloalkyl, where each $R^{b1}$ is the same or different, and independently selected from hydroxyl, cyano, halogen, a $C_{1-6}$ alkyl, a $C_{1-6}$ alkoxy, and a $C_{3-6}$ cycloalkyl;

X is selected from O, S or NH;

Y is absent, or selected from the following groups which are unsubstituted or optionally substituted with one, two or more $R^{y}$: $-S(=O)-R^{y1}$, $-S(=O)_2-R^{y2}$, $-S(=O)(=NR^{y3})-R^{y4}$, a $C_{3-12}$ cycloalkyl, a 3- to 14-membered heterocyclyl, a $C_{6-14}$ aryl, and a 5- to 14-membered heteroaryl, where $R^{y1}$, $R^{y2}$, $R^{y3}$, and $R^{y4}$ are the same or different, and are absent or each independently selected from hydrogen, a $C_{1-6}$ alkyl, or a $C_{3-6}$ cycloalkyl; each $R^{y}$ is the same or different, and independently selected from hydrogen, hydroxyl, cyano, halogen, oxo (=O), or the following groups which are unsubstituted or optionally substituted with one, two or more $R^{y'}$: a $C_{1-6}$ alkyl, a $C_{1-6}$ alkoxy, a $C_{3-6}$ cycloalkyl, a 3- to 8-membered heterocyclyl, a $C_{6-14}$ aryl, a 5- to 14-membered heteroaryl, $=N-R^{y5}$, $-C(=O)-R^{y6}$, $-C(=O)O-R^{y7}$, $-S(=O)_2-R^{y8}$, $-S(=O)(=NR^{y9})-R^{y10}$, and $-P(=O)(R^{y11})(R^{y12})$, in which $R^{y5}$, $R^{y6}$, $R^{y7}$, $R^{y8}$, $R^{y9}$, $R^{y10}$, $R^{y11}$, and $R^{y12}$ are the same or different, and each independently selected from hydrogen, a $C_{1-6}$ alkyl, and a $C_{3-6}$ cycloalkyl; each $R^{y'}$ is the same or different, and independently selected from hydrogen, hydroxyl, cyano, halogen, oxo (=O), or the following groups which are unsubstituted or optionally substituted with one, two or more $R^{y''}$: a $C_{1-6}$ alkyl, a $C_{1-6}$ alkoxy, a $C_{3-6}$ cycloalkyl, a 3- to 8-membered heterocyclyl, $-C(=O)-NR^{y13}R^{y14}$, $-C(=O)-R^{y15}$, $-C(=O)O-R^{y16}$, $-OR^{y17}$, $-S(=O)_2-R^{y18}$, $- S(=O)_2-NH_2$, $-S(=O)(=NR^{y19})-R^{y20}$, $-P(=O)(R^{y21})(R^{y22})$, and amino, where each $R^{y''}$ is the same or different, and independently selected from hydrogen, hydroxyl, cyano, halogen, oxo (=O), amino, a $C_{1-6}$ alkyl, a $C_{1-6}$ alkoxy, a $C_{3-6}$ cycloalkyl, a $C_{3-6}$ cycloalkyl, a 3- to 8-membered heterocyclyl, $-C(=O)-NR^{y23}R^{y24}$, $-C(=O)-R^{y25}$, $-C(=O)O-R^{y26}$, $-OR^{y27}$, $-S(=O)_2-R^{y28}$, $-S(=O)(=NR^{y29})-R^{y30}$, and $-P(=O)(R^{y31})(R^{y32})$, where $R^{y13}$, $R^{y14}$, $R^{y15}$, $R^{y16}$, $R^{y17}$, $R^{y18}$, $R^{y19}$, $R^{y20}$, $R^{y21}$, $R^{y22}$, $R^{y23}$, $R^{y24}$, $R^{y25}$, $R^{y26}$, $R^{y27}$, $R^{y28}$, $R^{y29}$, $R^{y30}$, $R^{y31}$, and $R^{y32}$ are the same or different, and each independently selected from hydrogen, a $C_{1-6}$ alkyl, or a $C_{3-6}$ cycloalkyl;

each $R^1$ is the same or different, and independently selected from hydrogen, halogen, cyano, hydroxyl, oxo (=O), or the following groups which are unsubstituted or optionally substituted with one, two or more $R^{11}$: amino, a $C_{1-6}$ alkyl, a $C_{1-6}$ alkoxy, a $C_{3-8}$ cycloalkyl, a 3- to 8-membered heterocyclyl, $-C(=O)-NH_2$, $-S(=O)_2-C_{1-6}$ alkyl, and $-S(=O)(=NH)-C_{1-6}$ alkyl; or two $R^1$ attached to the same atom, together with the atom to which they are attached, form a 3- to 12-membered heterocyclic ring or a $C_{3-12}$ alkyl ring that is unsubstituted or optionally substituted with one, two or more $R^{11}$; or two $R^1$ attached to different atoms, together with the atoms to which they are respectively attached, form a 3- to 12-membered heterocyclic ring or a $C_{3-12}$ alkyl ring that is unsubstituted or optionally substituted with one, two or more $R^{11}$, where each $R^{11}$ is the same or different, and independently selected from H, cyano, oxo (=O), halogen, or the following groups which are unsubstituted or optionally substituted with one, two or more $R^{12}$: a $C_{1-6}$ alkyl, a $C_{1-6}$ alkoxy, $-S(=O)_2-R^{y8}$, $-S(=O)(=NR^{y9})-R^{y10}$, a $C_{3-12}$ cycloalkyl, a 3- to 12-membered heterocyclyl, and a 5-to 14-membered heteroaryl, in which each $R^{12}$ is the same or different, and independently selected from hydrogen, hydroxyl, cyano, halogen, a $C_{1-6}$ alkyl, a $C_{1-6}$ alkoxy, a $C_{3-6}$ cycloalkyl, a $C_{6-14}$ aryl, or a 5- to 14-membered heteroaryl;

each $R^2$ and $R^4$ are the same or different, and independently selected from hydrogen, halogen, cyano, hydroxyl, oxo (=O), or the following groups which are unsubstituted or optionally substituted with one, two or more $R^{21}$: amino, a $C_{1-6}$ alkyl, a $C_{1-6}$ alkoxy, a $C_{3-8}$ cycloalkyl, a 3- to 8-membered heterocyclyl, $-C(=O)-NH_2$, $-S(=O)_2-C_{1-6}$ alkyl, a $C_{6-14}$ aryl, and a 5- to 14-membered heteroaryl, where each $R^{21}$ is the same or different, and independently selected from halogen, CN, amino, hydroxyl, oxo (=O), a $C_{1-6}$ alkyl, or a $C_{1-6}$ alkoxy;

$R^6$ is selected from hydrogen, or a $R^{6a}-C_{1-4}$ alkyl, where $R^{6a}$ is selected from

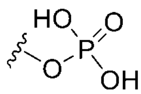
;

$R^3$ is selected from hydrogen, a $C_{1-6}$ alkyl, a $C_{1-6}$ haloalkyl, or a cyano-$C_{1-6}$ alkyl;

m and n are the same or different, and each independently selected from 0, 1, 2, 3, 4, 5 or 6; and

r is selected from 0, 1 or 2.

**2.** The compound according to claim 1, wherein Ring A is selected from a $C_{6-10}$ aromatic ring, a 5- to 10-membered heteroaromatic ring, or a 5- to 10-membered heterocyclic ring;

preferably, Ring A is selected from a benzene ring, a pyridine ring, a pyrimidine ring, a naphthalene ring, a quinoline ring, a 1,8-naphthyridine ring, a piperidine ring, a piperazine ring,

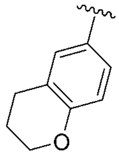, 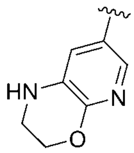, 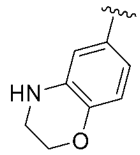,

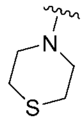, 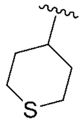 or 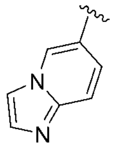;

preferably, Ring B is selected from a benzene ring or a 5- to 6-membered heteroaromatic ring;
preferably, Ring B is selected from a pyridine ring or a pyridazine ring;
preferably, L is absent or selected from -NH-C(=O)- or -CH=CH-;
preferably, X is selected from O;
preferably, each $R^1$ is the same or different, and independently selected from halogen, cyano, hydroxyl, oxo (=O), or the following groups which are unsubstituted or optionally substituted with one, two or more $R^{11}$: amino, a $C_{1-6}$ alkyl, a $C_{1-6}$ alkoxy, a $C_{3-6}$ cycloalkyl, a 3- to 6-membered heterocyclyl, -C(=O)-$NH_2$, a -S(=O)$_2$-$C_{1-3}$ alkyl, and a -S(=O)(=NH)-$C_{1-3}$ alkyl; or two $R^1$ attached to the same atom, together with the atom to which they are attached, form a 3- to 8-membered heterocyclic ring that is unsubstituted or optionally substituted with one, two or more $R^{11}$; or two $R^1$ attached to different atoms, together with the atoms to which they are respectively attached, form a 3- to 8-membered heterocyclic ring that is unsubstituted or optionally substituted with one, two or more $R^{11}$, where each $R^{11}$ is the same or different, and independently selected from H, cyano, oxo (=O), halogen, or the following groups which are unsubstituted or optionally substituted with one, two or more $R^{12}$: a $C_{1-6}$ alkyl, a $C_{1-6}$ alkoxy, -S(=O)$_2$-$CH_3$, - S(=O)(=NH)-$CH_3$, a $C_{3-6}$ cycloalkyl, a 3- to 8-membered heterocyclyl, and a 5- to 6-membered heteroaryl, where each $R^{12}$ is the same or different, and independently selected from hydrogen, hydroxyl, cyano, halogen, a $C_{1-6}$ alkyl, a $C_{1-6}$ alkoxy, a $C_{3-6}$ cycloalkyl, phenyl, or a 5- to 6-membered heteroaryl;
preferably, each $R^1$ is the same or different, and independently selected from F, Cl, Br, cyano, oxo (=O), methoxy, -S(=O)$_2$-$CH_3$, -S(=O)(=NH)-$CH_3$, -S(=O)(=N-$CH_3$)-$CH_3$,

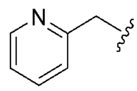

or -C(=O)-N($CH_3$)$_2$; or two $R^1$ attached to the same atom, together with the atom to which they are attached, form a piperidyl that is unsubstituted or optionally substituted with one, two or more $R^{11}$;
preferably, each $R^{11}$ is the same or different, and independently selected from hydrogen, 2,2,2-trifluoroethyl, -S(=O)$_2$-$CH_3$, or cyclobutyl; and
preferably,

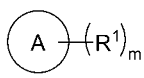

is selected from: phenyl,

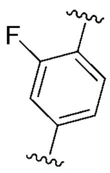, 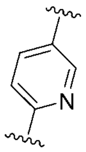, 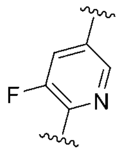, 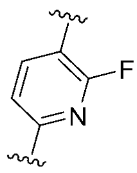, 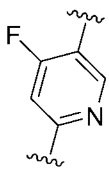,

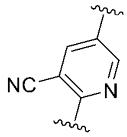, 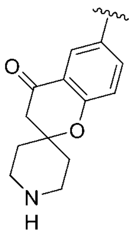, 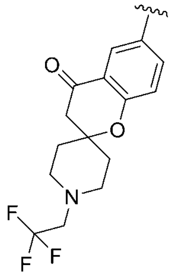, 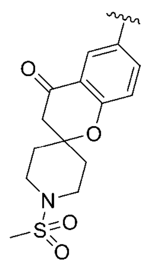, 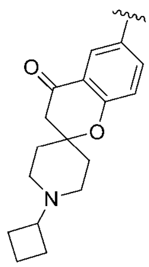, 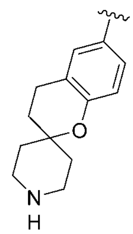, 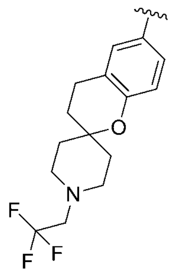,

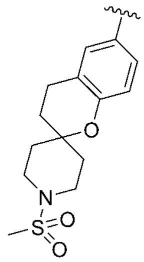, 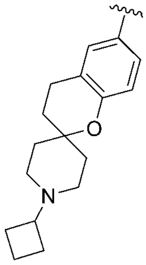, 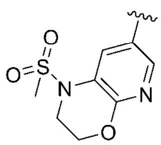, 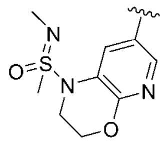, 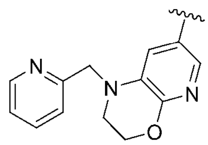, 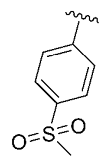, 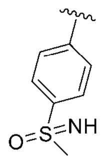,

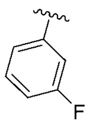, 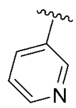, 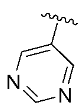, 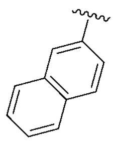, 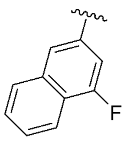, 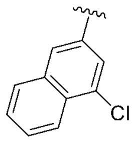, 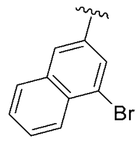, 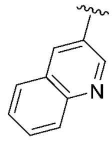, 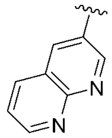,

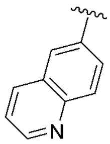, 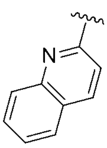, 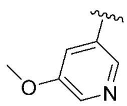, 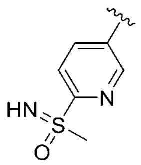, 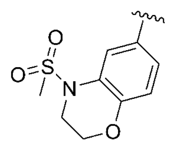, 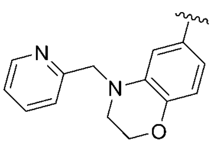, 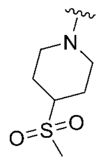, 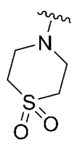,

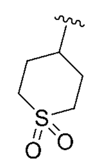, 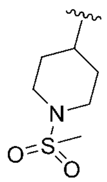, and 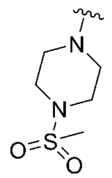.

3. The compound according to any one of claims 1 and 2, wherein Y is absent, or Y is selected from the following groups which are unsubstituted or optionally substituted with one, two or more $R^y$: $-S(=O)-R^{y1}$, $-S(=O)_2-R^{y2}$, $-S(=O)(=NR^{y3})-R^{y4}$, a $C_{3-10}$ cycloalkyl, a 3- to 10-membered heterocyclyl, and a 5- to 8-membered heteroaryl, where $R^{y1}$, $R^{y2}$, $R^{y3}$, and $R^{y4}$ are the same or different, and are absent or each independently selected from hydrogen, a $C_{1-6}$ alkyl, or a $C_{3-6}$ cycloalkyl;

preferably, Y is selected from the following groups which are unsubstituted or optionally substituted with one, two or more $R^y$: phenyl, piperazinyl, piperidyl, pyrazolyl,

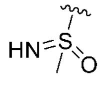, 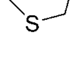, 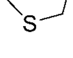, 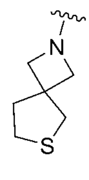, 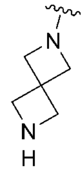, 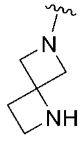,

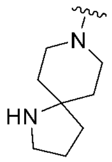, 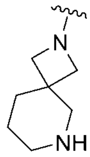, 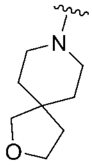, 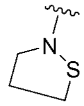, 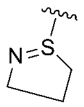, 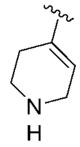, 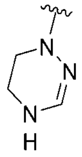, 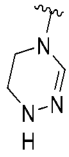, 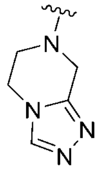 or 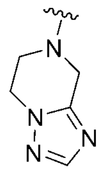;

preferably, each $R^y$ is the same or different, and independently selected from hydrogen, hydroxyl, cyano, halogen, oxo (=O), a $C_{1-6}$ alkyl, a $C_{1-6}$ haloalkyl, a hydroxy-$C_{1-6}$ alkyl, an amino-$C_{1-6}$ alkyl, a cyano-$C_{1-6}$ alkyl, a carboxy-$C_{1-6}$ alkyl, a $C_{1-6}$ alkyl-NH-$C_{1-6}$ alkyl, a $(C_{1-6}$ alkyl$)_2$N-$C_{1-6}$ alkyl, a $C_{1-6}$ alkoxy, a $C_{3-6}$ cycloalkyl, a -$C_{1-6}$ alkyl-(hydroxy substituted $C_{3-8}$ cycloalkyl), a -$C_{1-6}$ alkyl-S(=O)$_2$-$C_{1-6}$ alkyl, a -$C_{1-6}$ alkyl-S(=O)$_2$-NH$_2$, a $C_{6-10}$ aryl, a 5- to 10-membered heteroaryl, =N-$R^{y5}$, -C(=O)-$R^{y6}$, -C(=O)O-$R^{y7}$, -S(=O)$_2$-$R^{y8}$, -S(=O)(=N$R^{y9}$)-$R^{y10}$, -P(=O)($R^{y11}$)($R^{y12}$), or a 3- to 8-membered heterocyclyl that is optionally substituted with one, two or more $R^{y'}$, where each $R^{y'}$ is the same or different, and independently selected from cyano, halogen, oxo (=O) or a -S(=O)$_2$-$C_{1-6}$ alkyl; and $R^{y5}$, $R^{y6}$, $R^{y7}$, $R^{y8}$, $R^{y9}$, $R^{y10}$, $R^{y11}$, and $R^{y12}$ are the same or different, and each independently selected from hydrogen, a $C_{1-6}$ alkyl, or a $C_{3-6}$ cycloalkyl;

preferably, each $R^y$ is the same or different, and independently selected from hydroxyl, cyano, halogen, oxo (=O), a $C_{1-3}$ alkyl, a $C_{1-3}$ haloalkyl, a hydroxyl-$C_{1-4}$ alkyl, an amino-$C_{1-4}$ alkyl, a cyano-$C_{1-3}$ alkyl, a carboxy-$C_{1-4}$ alkyl, a $C_{1-4}$ alkyl-NH-$C_{1-4}$ alkyl, a $(C_{1-4}$ alkyl$)_2$N-$C_{1-4}$ alkyl, a $C_{1-3}$ alkoxy, a $C_{3-6}$ cycloalkyl, a -$C_{1-3}$ alkyl-(hydroxy substituted $C_{3-6}$ cycloalkyl), a -$C_{1-3}$ alkyl-S(=O)$_2$-$C_{1-3}$ alkyl, a -$C_{1-3}$ alkyl-S(=O)$_2$-NH$_2$, phenyl, a 5- to 6-membered heteroaryl, =N-$R^{y5}$, -C(=O)-$R^{y6}$, -C(=O)O-$R^{y7}$, -S(=O)$_2$-$R^{y8}$, -S(=O)(=N$R^{y9}$)-$R^{y10}$, -P(=O)($R^{y11}$)($R^{y12}$), or a 3- to 6-membered heterocyclyl that is optionally substituted with one, two or more $R^{y'}$, where each $R^{y'}$ is the same or different, and independently selected from oxo (=O) or a -S(=O)$_2$-$C_{1-3}$ alkyl; and $R^{y5}$, $R^{y6}$, $R^{y7}$, $R^{y8}$, $R^{y9}$, $R^{y10}$, $R^{y11}$, and $R^{y12}$ are the same or different, and each independently selected from hydrogen, a $C_{1-6}$ alkyl, or a $C_{3-6}$ cycloalkyl;

preferably, each $R^y$ is the same or different, and independently selected from methyl, ethyl, n-propyl, iso-propyl, cyclopropyl, cyclobutyl, cyclopentyl, trifluoromethyl, 2-hydroxylethyl, oxo (=O), =N-$CH_3$, -S(=O)$_2$-$CH_3$, -C(=O)O-$CH_3$, -C(=O)O-$C_2H_5$, pyrimidinyl, -C($CH_3$)$_2$OH, -C($CH_3$)$_2$CN, -S(=O)$_2$-$C_2H_5$, -S(=O)$_2$-CH($CH_3$)$_2$, -$CH_2$COOH,

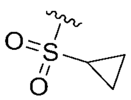, 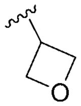, 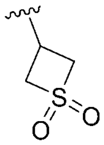, 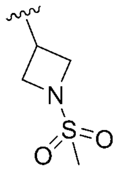 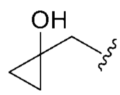, 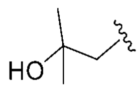, 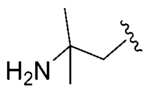, 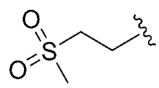

or

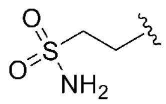;

and

more preferably, Y is selected from:

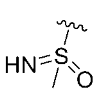, 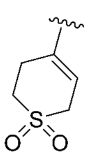, 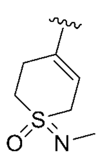, 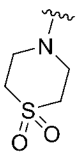, 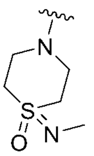, 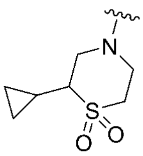, 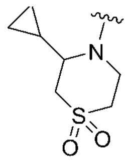,

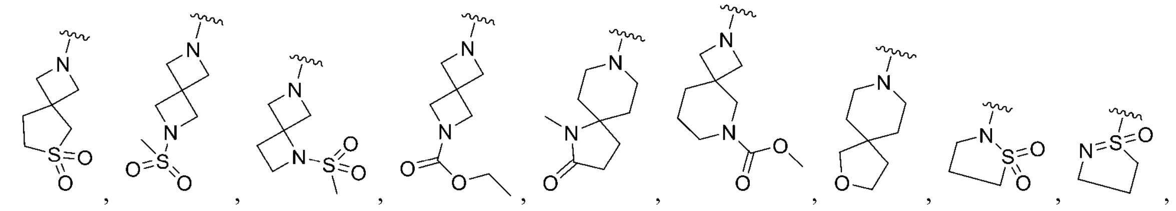
, , , , , , , , ,

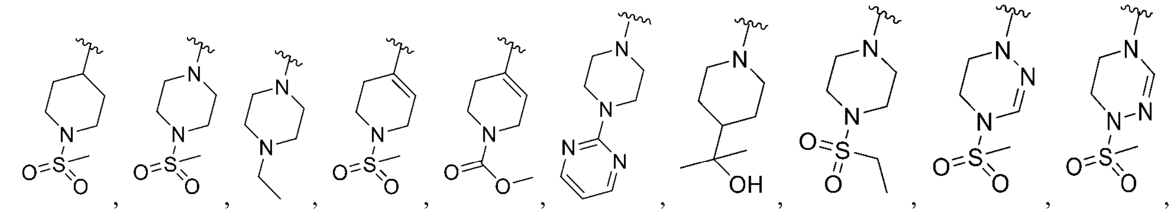
, , , , , , , , , ,

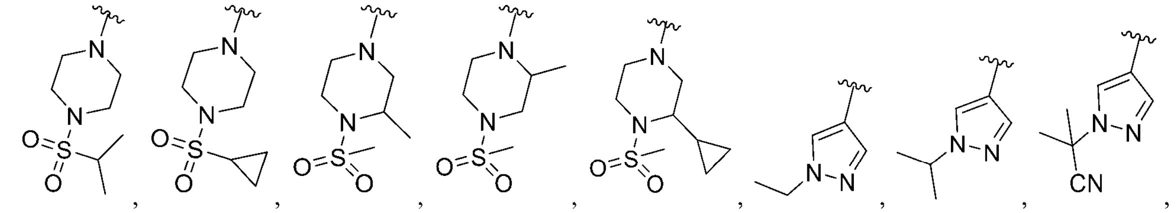
, , , , , , , ,

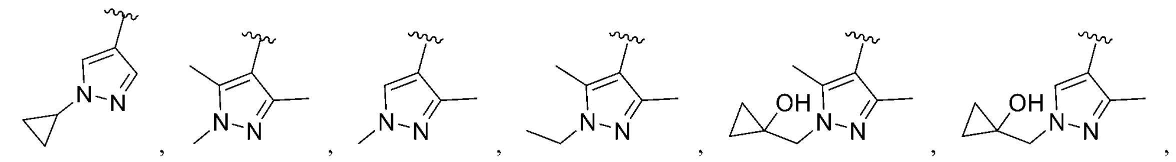
, , , , , ,

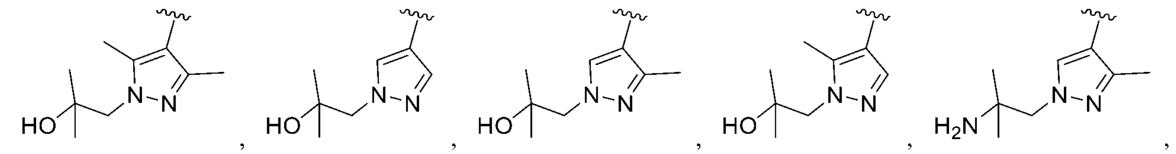
, , , , ,

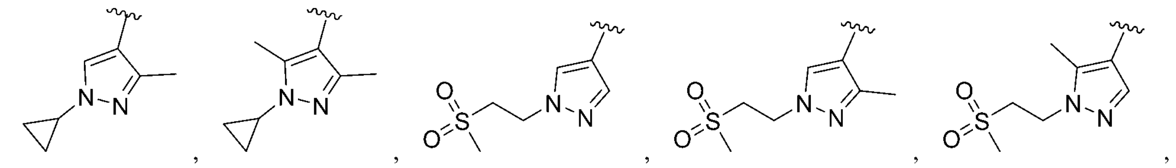
, , , , ,

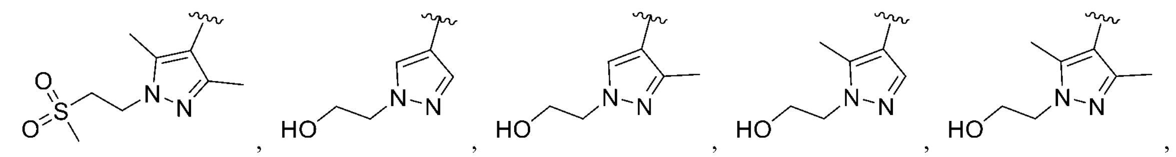
, , , , ,

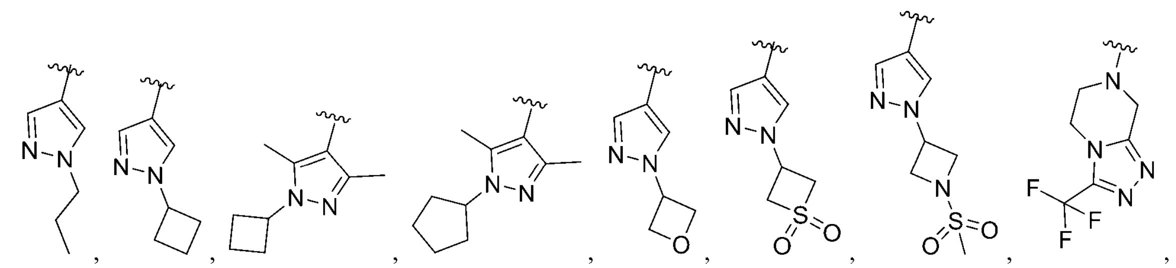
, , , , , , , ,

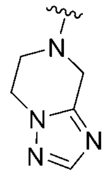, 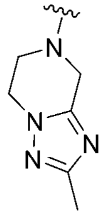, 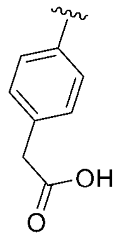, 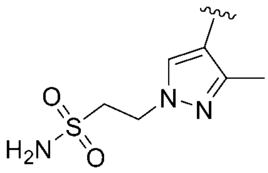, 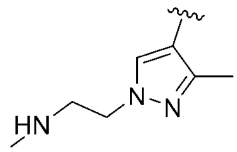, or 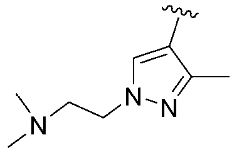.

**4.** The compound according to any one of claims 1 to 3, wherein each $R^2$ is the same or different, and independently selected from hydrogen, F, Cl, Br, cyano, or the following groups that are unsubstituted or optionally substituted with one, two or more $R^{21}$: a $C_{1-3}$ alkyl, a $C_{1-3}$ alkoxy, pyrazolyl, and pyrimidinyl;

preferably, each $R^{21}$ is the same or different, and independently selected from halogen, CN, or methyl;
preferably, each $R^2$ is the same or different, and independently selected from Cl, methyl,

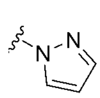, 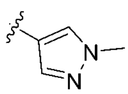,

or

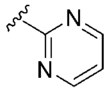;

preferably,

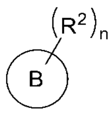

is selected from:

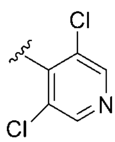, 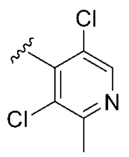, 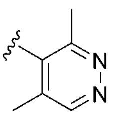, 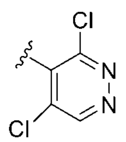, 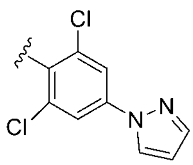,

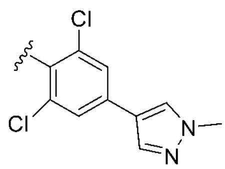, or 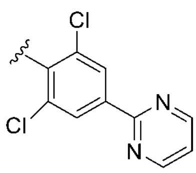;

preferably, each $R^4$ is the same or different, and independently selected from hydrogen, halogen, cyano, hydroxyl, amino, a $C_{1-6}$ alkyl, or a $C_{1-6}$ alkoxy;
preferably, each $R^4$ is the same or different, and independently selected from halogen, cyano, methyl, or methoxy;
preferably, $R^3$ is selected from hydrogen, a $C_{1-3}$ alkyl, or a $C_{1-3}$ haloalkyl;
preferably, $R^3$ is selected from methyl; and
preferably, $R^6$ is selected from hydrogen and

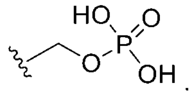

5. The compound according to any one of claims 1 to 4, wherein the compound of Formula (I) has a structure as shown below:

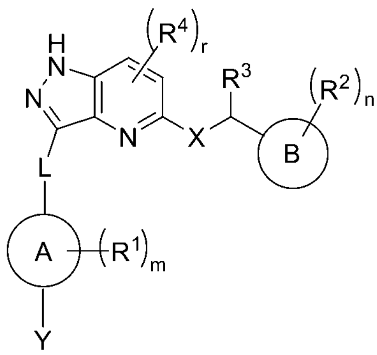

Formula II

where Ring A, Ring B, $R^1$, $R^2$, $R^3$, $R^4$, L, X, Y, m, n, and r are as defined in any one of claims 1 to 4; preferably, the compound of Formula (I) has the following structures:

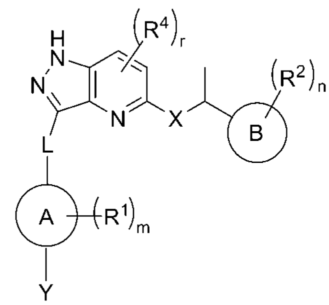

,

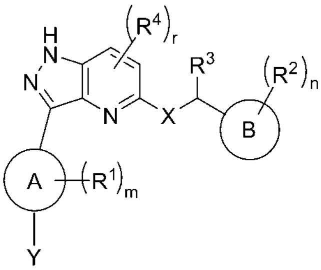

,

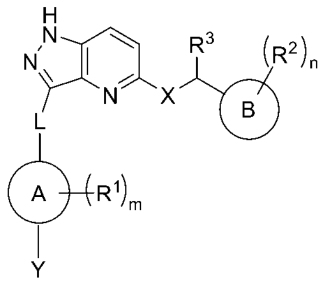

,

Formula II-1 Formula II-2 Formula II-3

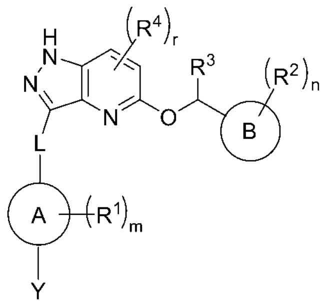

,

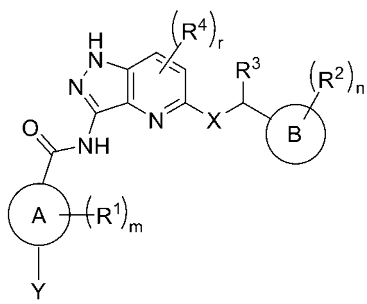

Formula II-4 Formula II-5

where Ring A, Ring B, $R^1$, $R^2$, $R^3$, $R^4$, L, X, Y, m, n, and r are as defined in any one of claims 1 to 4; preferably, the compound of Formula (I) has the following structures:

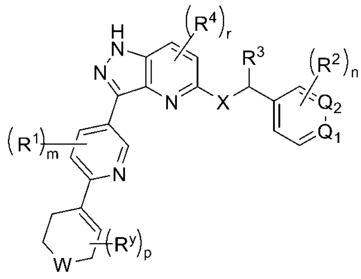,

Formula III-1

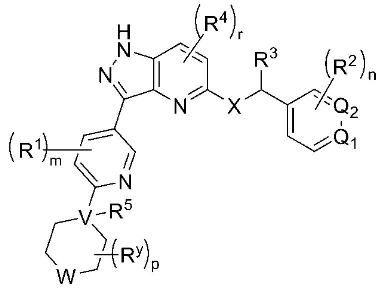,

Formula III-2

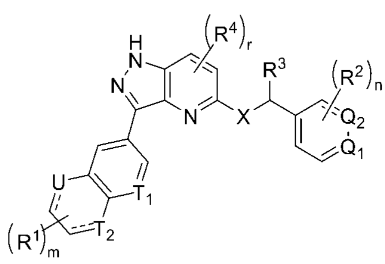,

Formula III-3

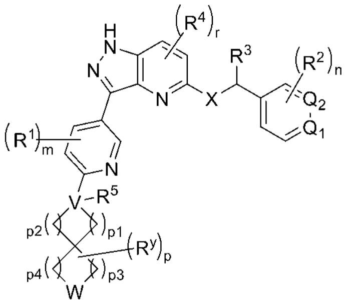,

Formula III-4

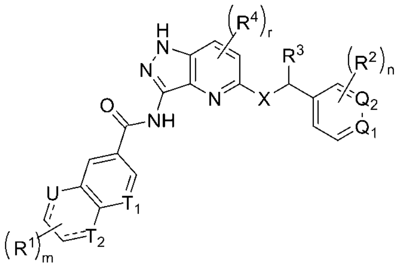,

Formula III-5

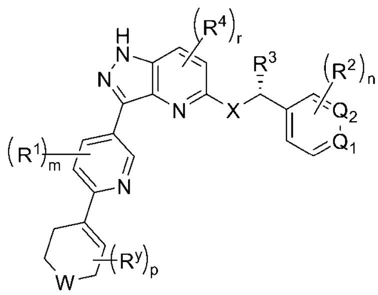,

Formula III-6

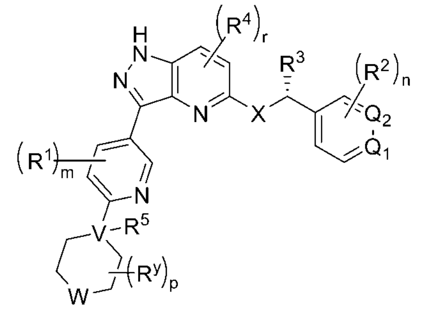,

Formula III-7

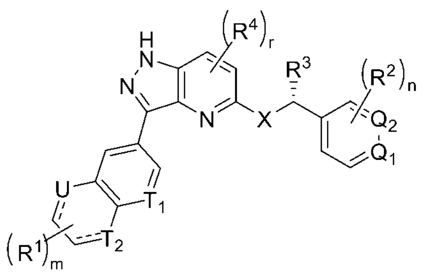,

Formula III-8

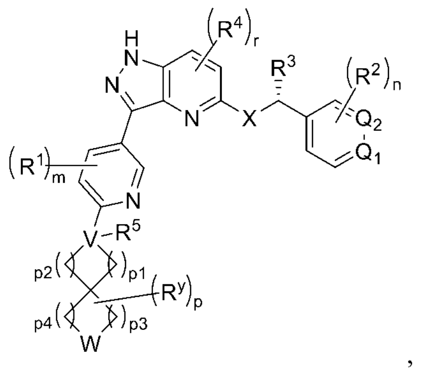,

Formula III-9

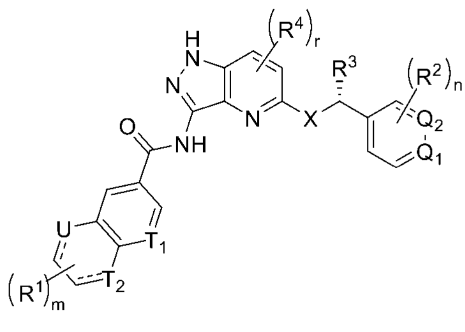;

Formula III-10

where $R^1$, $R^2$, $R^3$, $R^4$, $R^y$, X, m, n, and r are as defined in any one of claims 1 to 4, W is selected from O, S, $CH_2$, NH,

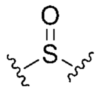, 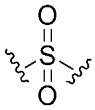, or 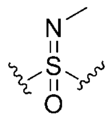;

$Q_1$ and $Q_2$ are the same or different, and each independently selected from CH or N; $T_1$ is selected from CH or N; $T_2$ and U are the same or different, and each independently selected from O, S, N, CH, NH or $CH_2$; V is selected from N or C, and when V is N, $R^5$ is absent; $R^5$ is absent or selected from hydrogen, hydroxyl, cyano, a $C_{1-6}$ alkyl, a $C_{1-6}$ alkoxy, a $C_{1-6}$ haloalkyl, a $C_{1-6}$ haloalkoxy, or a $C_{3-6}$ cycloalkyl; p, p1, p2, p3, and p4 are the same or different, and each independently selected from 0, 1, 2, 3, 4, or 5; and 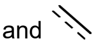 represents a single bond or a double bond; preferably, the compound of Formula (I) has the following structures:

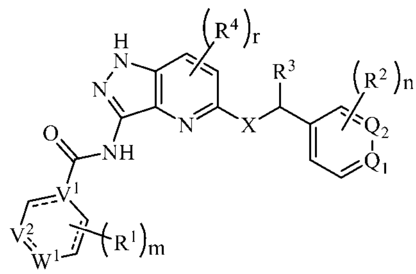,

Formula IV-1

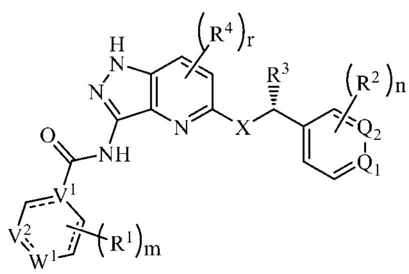;

Formula IV-2

where $R^1$, $R^2$, $R^3$, $R^4$, X, m, n, and r are as defined in any one of claims 1 to 4, $W^1$ is selected from O, S, $CH_2$, NH, CH, N,

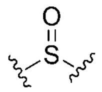, 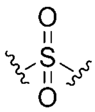, or 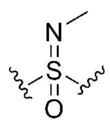;

$Q_1$ and $Q_2$ are the same or different, and each independently selected from CH or N; $V^1$ is selected from N, C or CH; $V^2$ is selected from N, NH, C, CH or $CH_2$; and 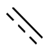 represents a single bond or a double bond; preferably, the compound of Formula (I) has the following structures:

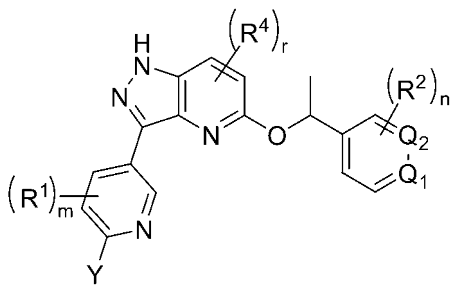,

Formula V-1

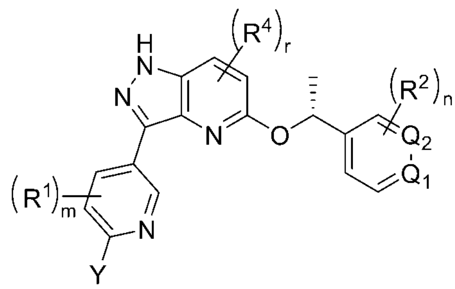;

Formula V-2

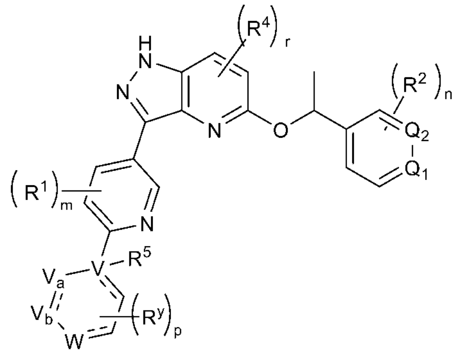
,

Formula V-3

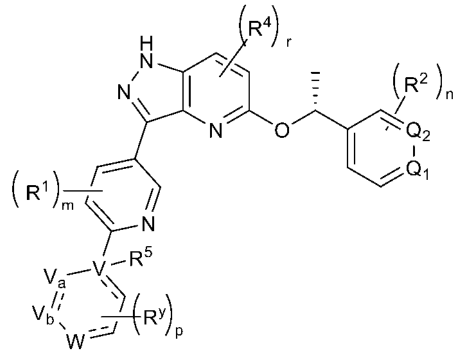
;

Formula V-4

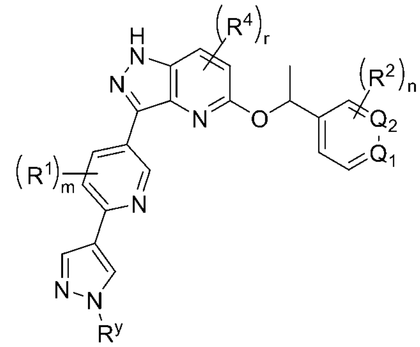
,

Formula V-5

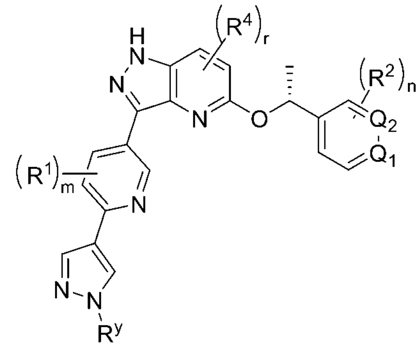
;

Formula V-6

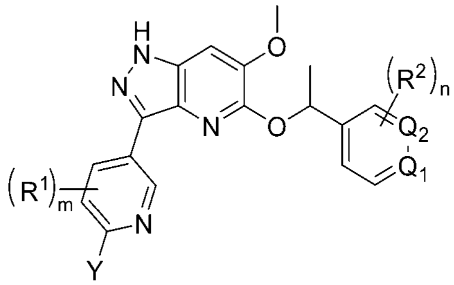
,

Formula V-7

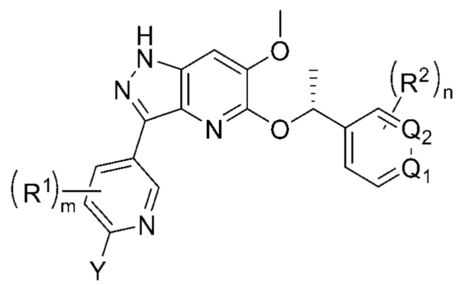
;

Formula V-8

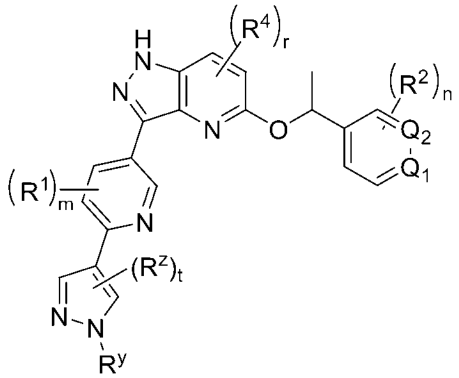
,

Formula V-9

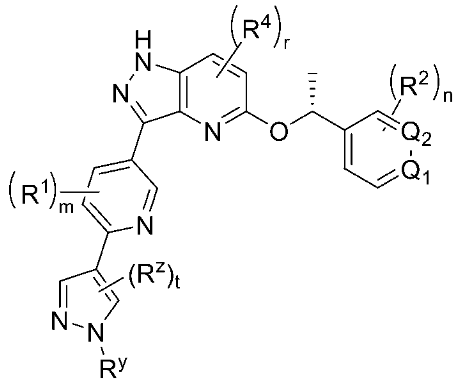
;

Formula V-10

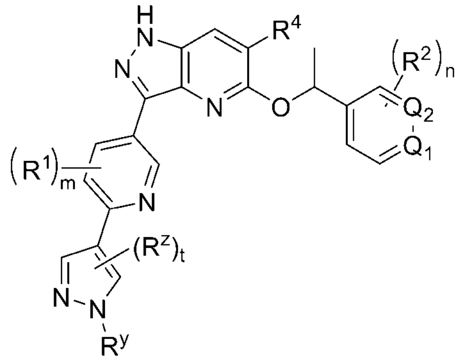
,
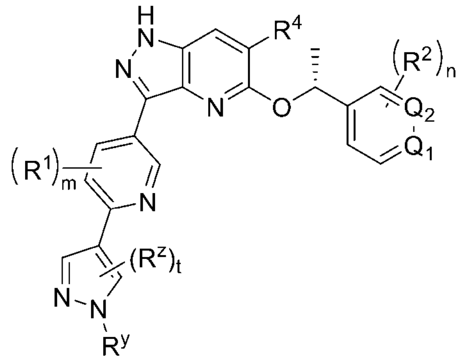
;

Formula V-11 Formula V-12

where $R^1$, $R^2$, $R^4$, Y, $R^y$, m, n, and r are as defined in any one of claims 1 to 4; $R^z$ is as defined for $R^y$ in any one of claims 1 to 4, and preferably, $R^z$ is selected from H, halogen, cyano, hydroxyl, or the following groups which are unsubstituted or optionally substituted with one, two or more $R^{z1}$: amino, a $C_{1-6}$ alkyl, a $C_{1-6}$ alkoxy, and a $C_{3-6}$ cycloalkyl, where each $R^{z1}$ is the same or different, and independently selected from hydrogen, hydroxyl, cyano, halogen, oxo (=O), amino, a $C_{1-6}$ alkyl and a $C_{3-6}$ cycloalkyl; further preferably, $R^z$ is selected from H, halogen, cyano, methyl, ethyl, propyl, or cyclopropyl; W is selected from O, S, $CH_2$, NH,

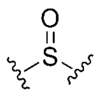
,
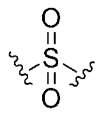
, or
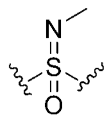
;

$Q_1$ and $Q_2$ are the same or different, and each independently selected from CH or N; V is selected from N or C, and when V is N, $R^5$ is absent; $V_a$ and $V_b$ are the same or different, and each independently selected from CH, $CH_2$, N, or NH; $R^5$ is absent or selected from hydrogen, hydroxyl, cyano, a $C_{1-6}$ alkyl, a $C_{1-6}$ alkoxy, a $C_{1-6}$ haloalkyl, a $C_{1-6}$ haloalkoxy, or a $C_{3-6}$ cycloalkyl; p is selected from 0, 1, 2, 3, 4, or 5; and

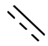

represents a single bond or a double bond;
or the compound of Formula (I) has the following structures:

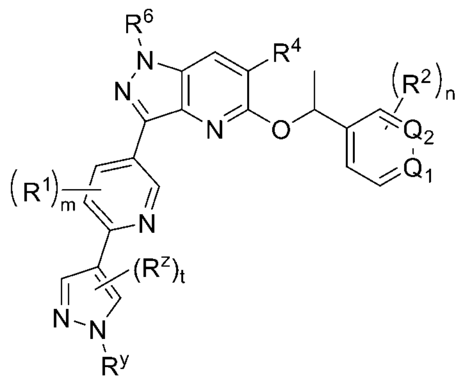
,
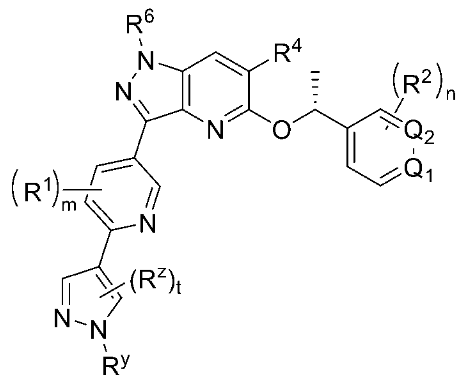
;

Formula V-13 Formula V-14

where $R^1$, $R^2$, $R^4$, $R^6$, $R^y$, $R^z$, $Q_1$, $Q_2$, m, n, and t are as defined in any one of claims 1 to 4, and $R^z$ is as defined for $R^y$ in any one of claims 1 to 4.

**6.** The compound according to any one of claims 1 to 5, wherein the compound is selected from the following structures:

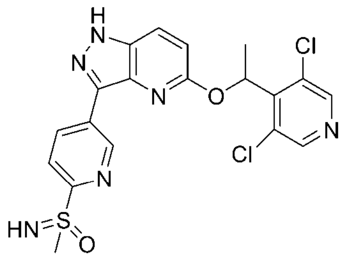
Cpd-01
,
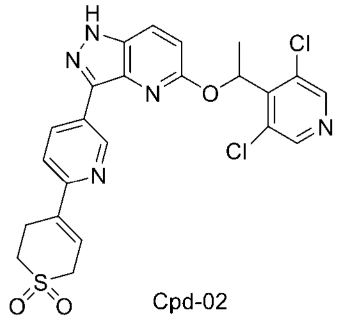
Cpd-02
,
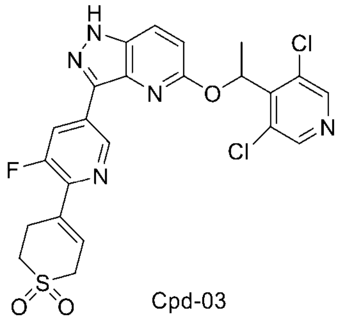
Cpd-03
,

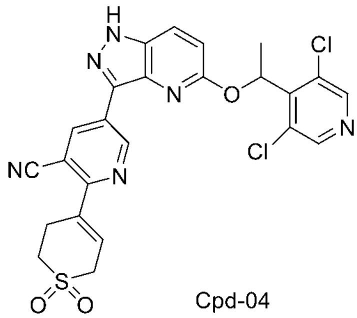
Cpd-04
,
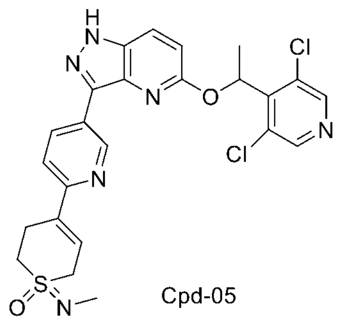
Cpd-05
,
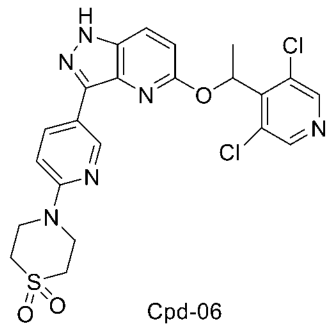
Cpd-06
,

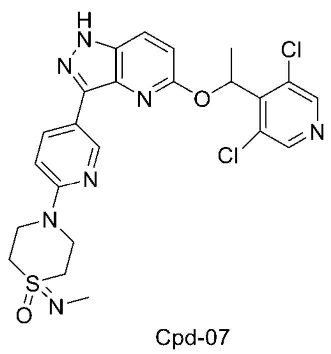
Cpd-07
,
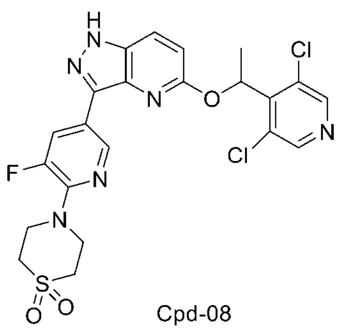
Cpd-08
,
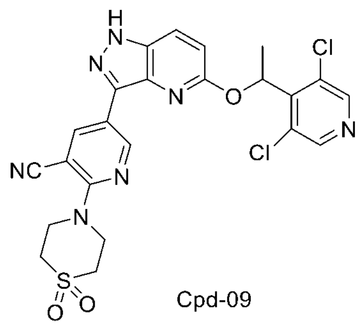
Cpd-09
,

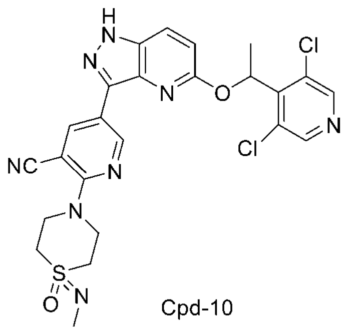
Cpd-10
,
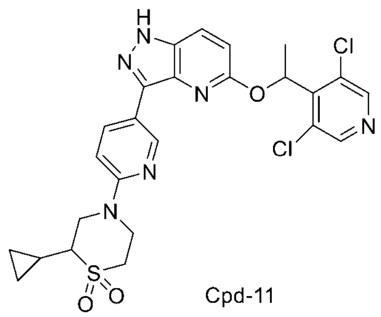
Cpd-11
,
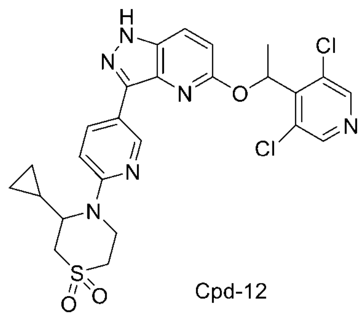
Cpd-12
,

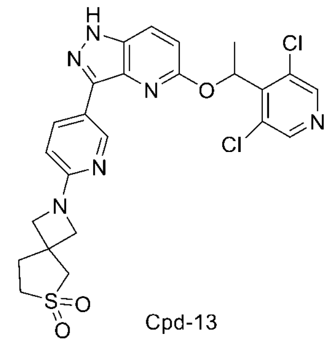
Cpd-13

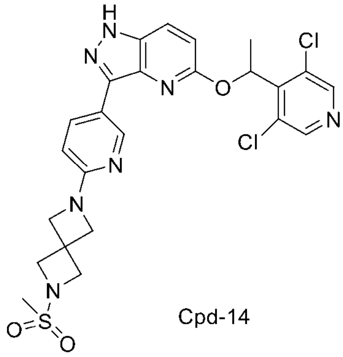
Cpd-14
,

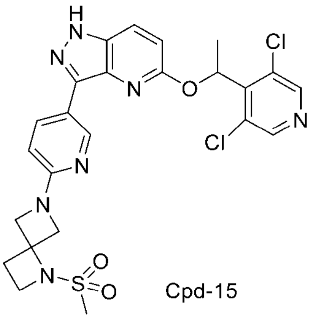
Cpd-15
,

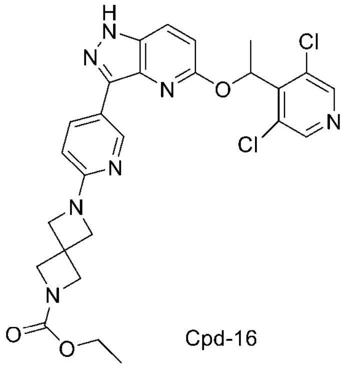
Cpd-16
,

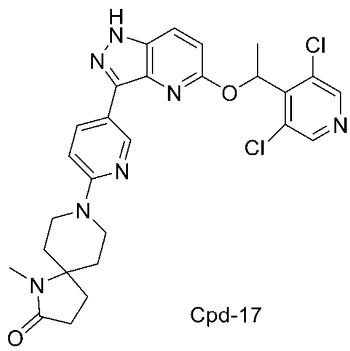
Cpd-17
,

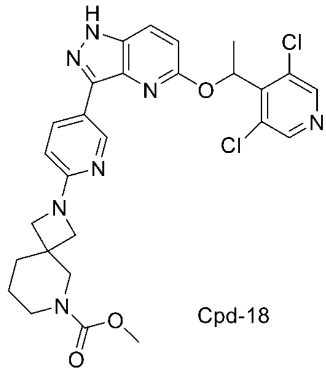
Cpd-18
,

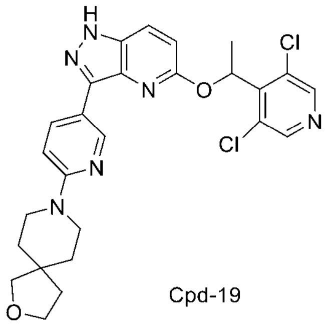
Cpd-19
,

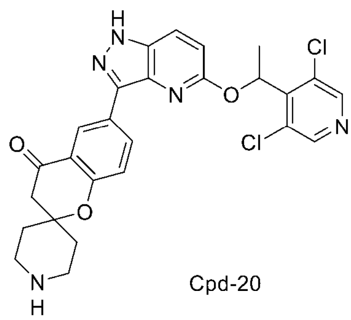
Cpd-20
,

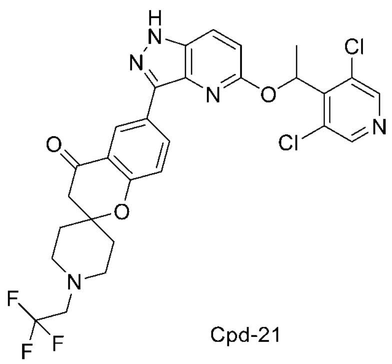
Cpd-21
,

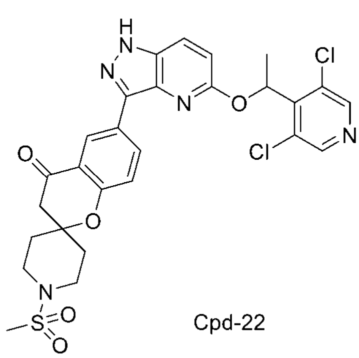
Cpd-22
,

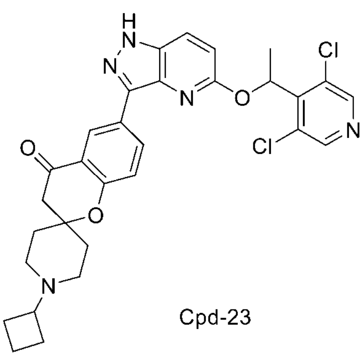
Cpd-23
,

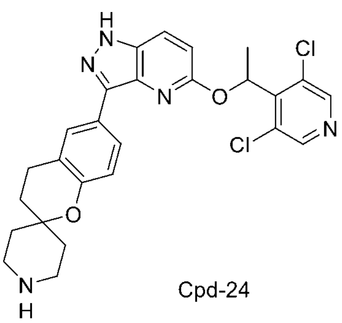
Cpd-24
,

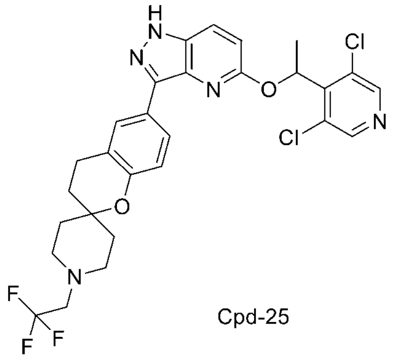
Cpd-25 ,

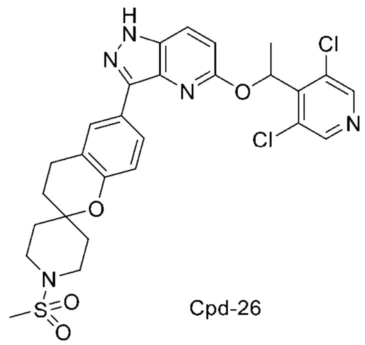
Cpd-26 ,

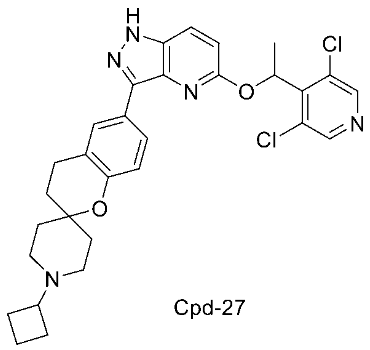
Cpd-27 ,

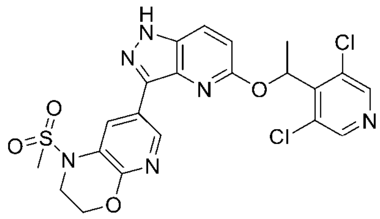
Cpd-28 ,

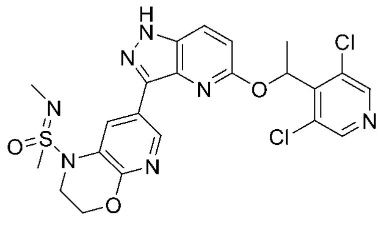
Cpd-29 ,

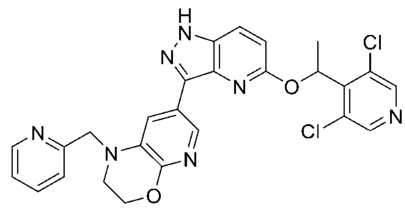
Cpd-30 ,

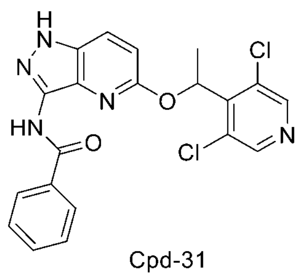
Cpd-31 ,

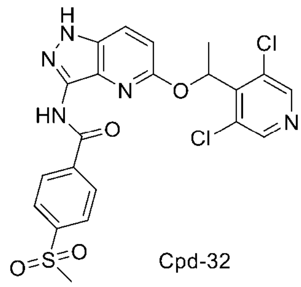
Cpd-32 ,

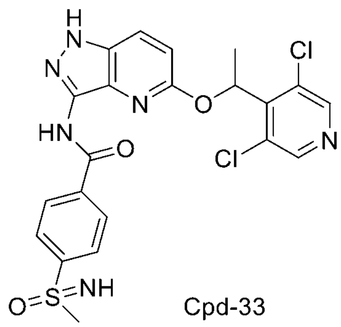
Cpd-33 ,

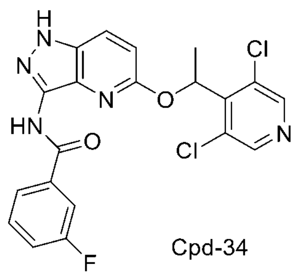
Cpd-34 ,

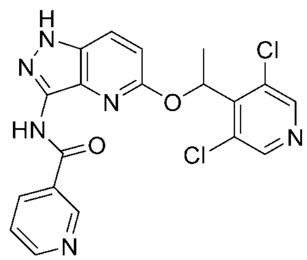
Cpd-35 ,

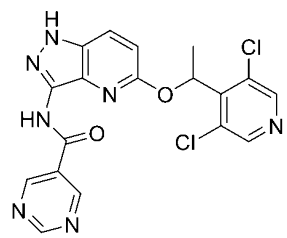
Cpd-36 ,

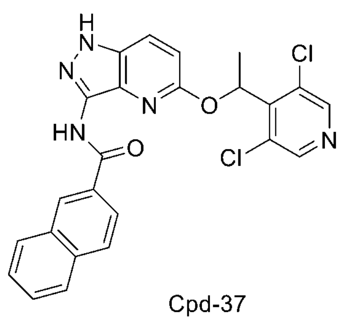
Cpd-37 ,

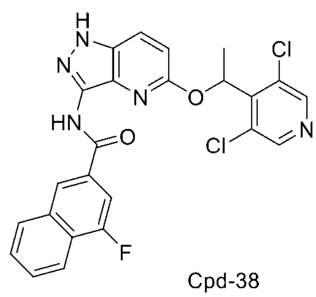
Cpd-38 ,

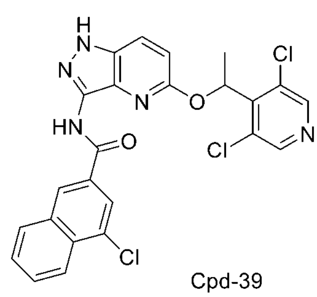
Cpd-39 ,

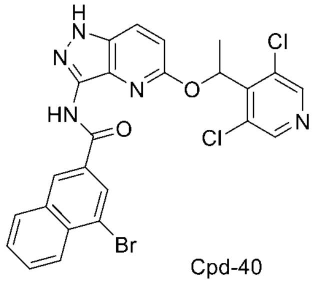
Cpd-40 ,

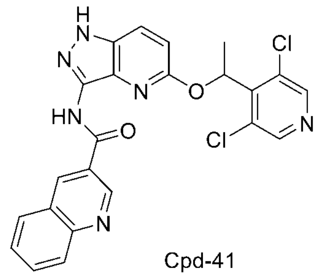
Cpd-41 ,

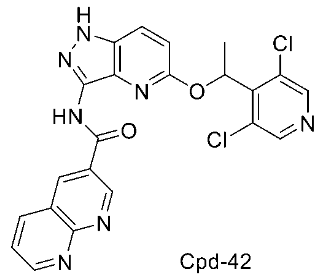
Cpd-42 ,

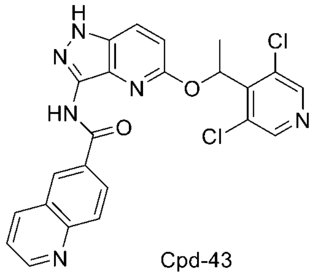
Cpd-43 ,

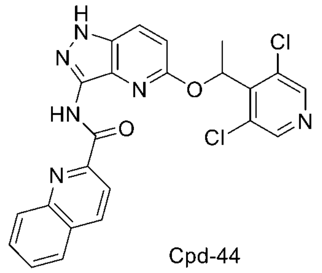
Cpd-44 ,

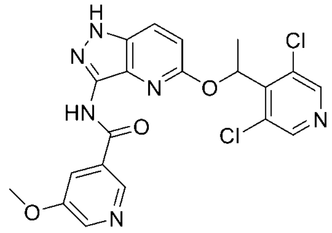
Cpd-45 ,

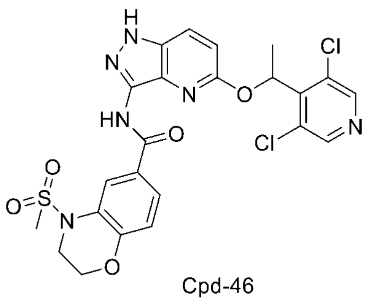
Cpd-46 ,

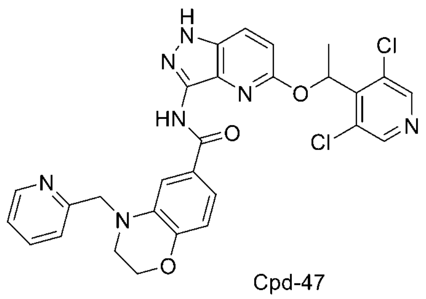
Cpd-47 ,

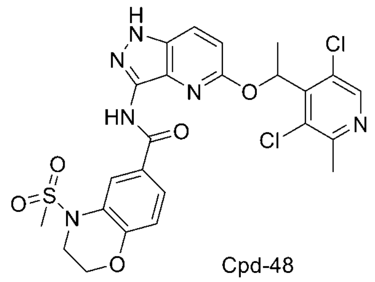
Cpd-48 ,

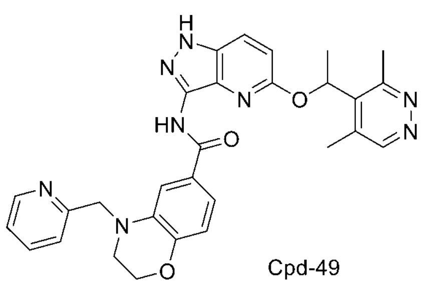
Cpd-49 ,

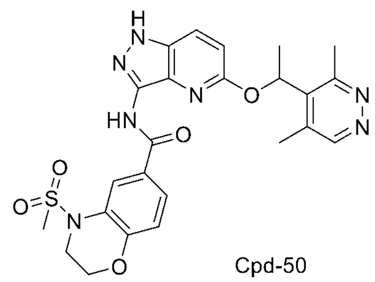
Cpd-50 ,

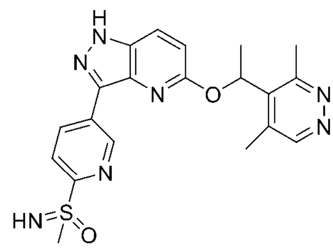
Cpd-51 ,

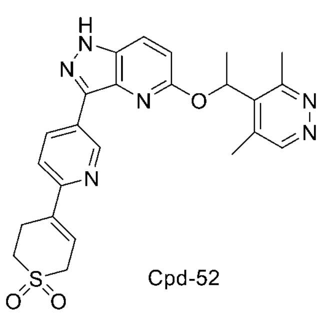
Cpd-52 ,

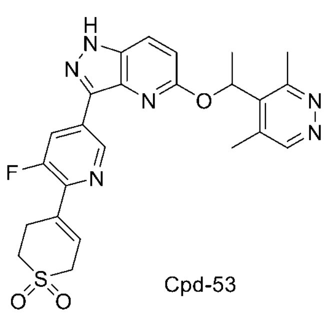
Cpd-53 ,

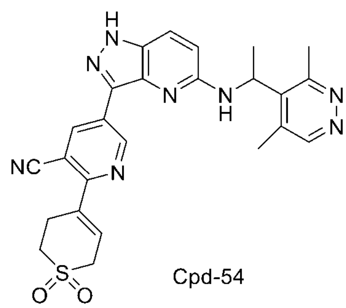
Cpd-54 ,

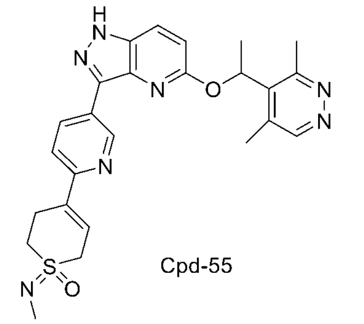
Cpd-55
,
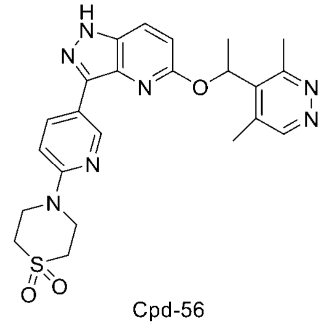
Cpd-56
,
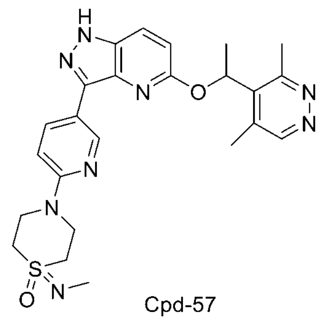
Cpd-57
,

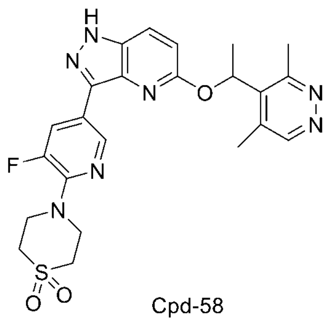
Cpd-58
,
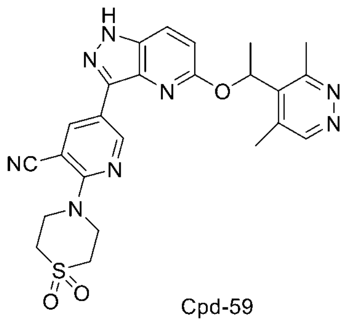
Cpd-59
,
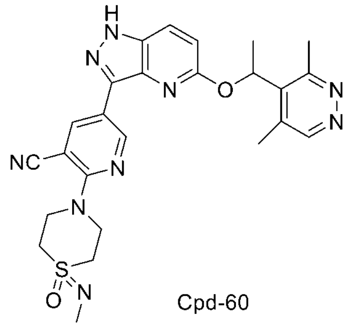
Cpd-60
,

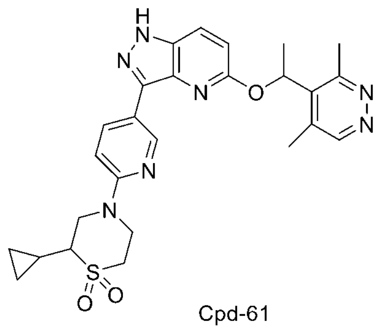
Cpd-61
,
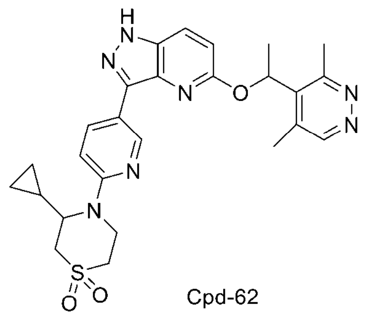
Cpd-62
,
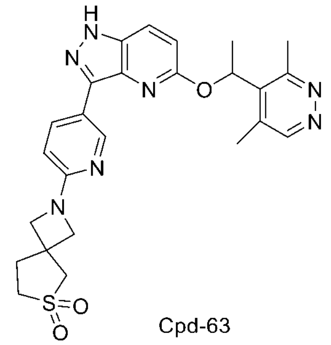
Cpd-63
,

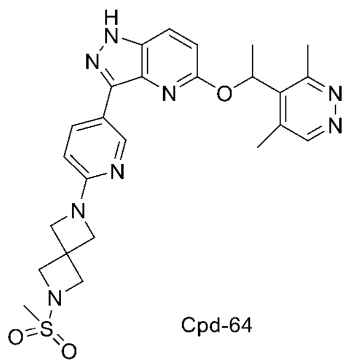
Cpd-64
,
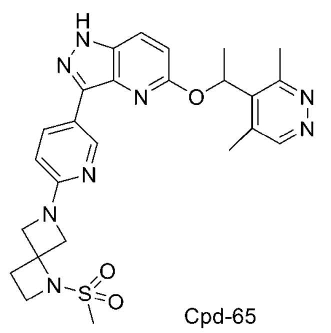
Cpd-65
,
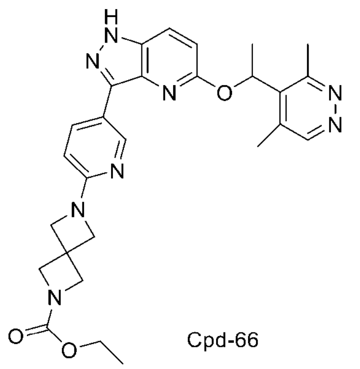
Cpd-66
,

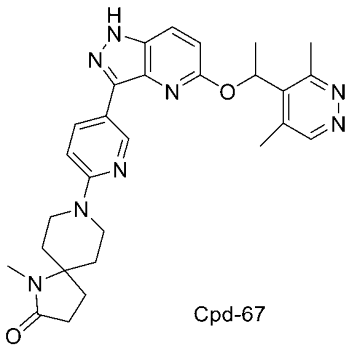
Cpd-67 ,

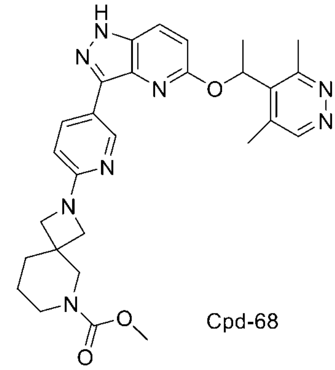
Cpd-68 ,

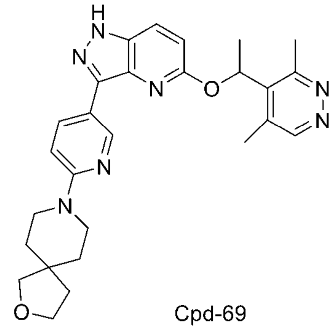
Cpd-69 ,

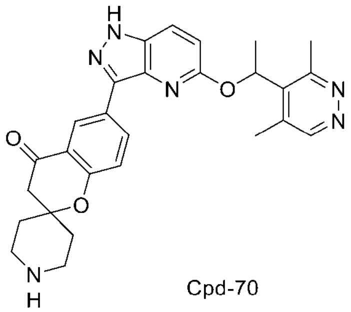
Cpd-70 ,

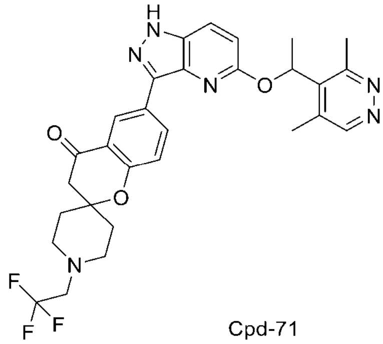
Cpd-71 ,

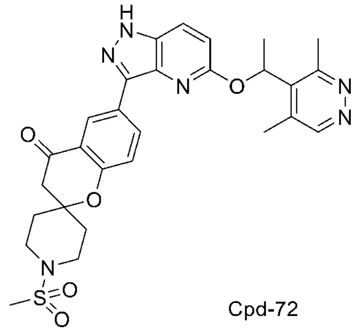
Cpd-72 ,

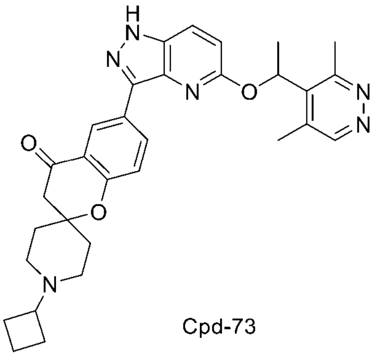
Cpd-73 ,

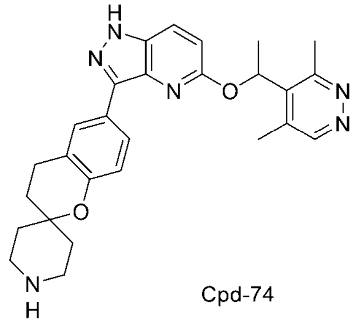
Cpd-74 ,

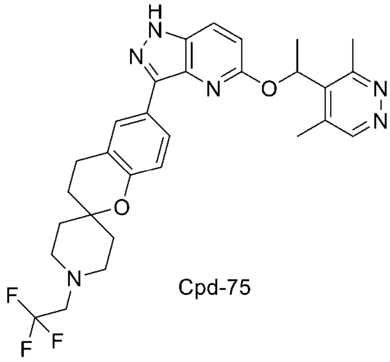
Cpd-75 ,

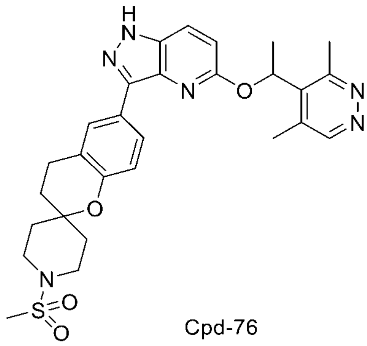
Cpd-76 ,

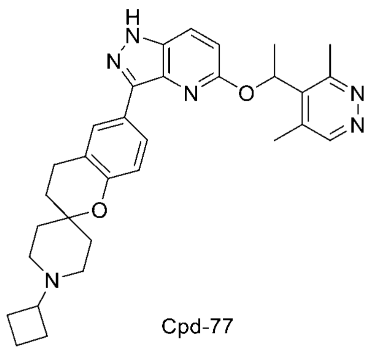
Cpd-77 ,

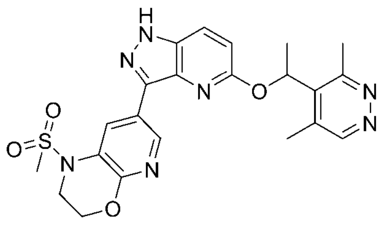
Cpd-78 ,

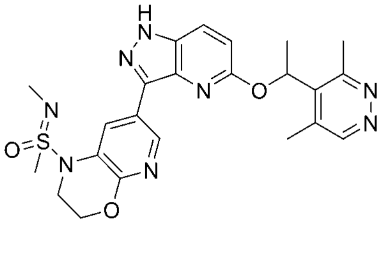
Cpd-79 ,

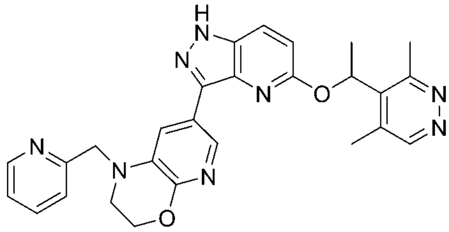
Cpd-80 ,

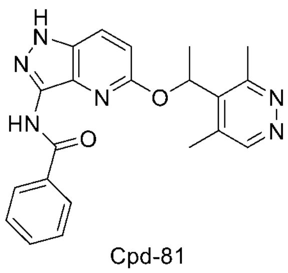
Cpd-81 ,

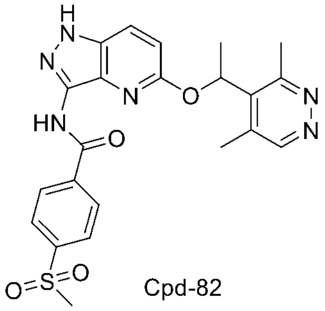
Cpd-82 ,

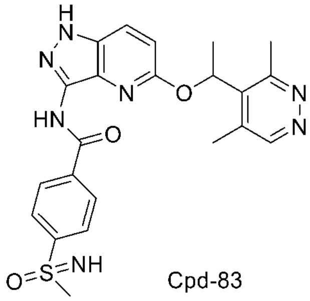
Cpd-83 ,

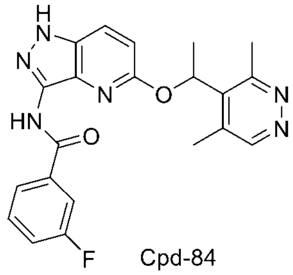
Cpd-84 ,

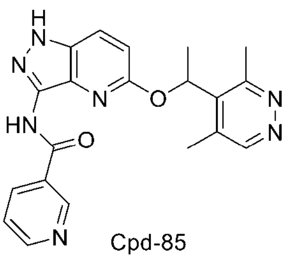
Cpd-85 ,

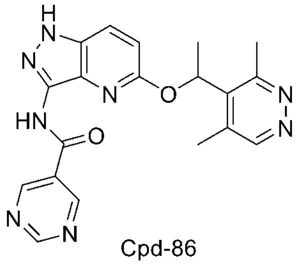
Cpd-86 ,

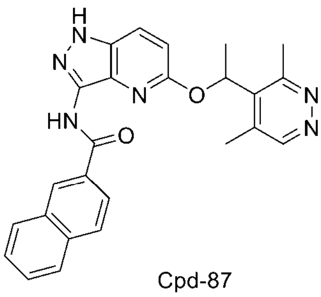
Cpd-87 ,

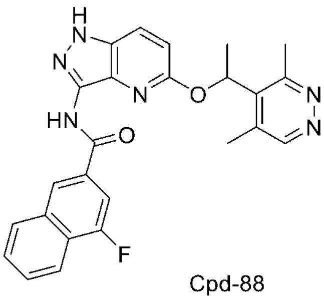
Cpd-88 ,

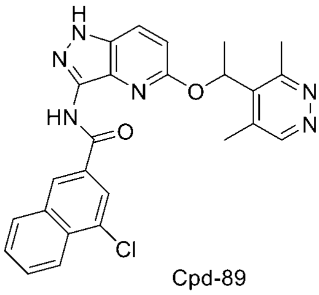
Cpd-89 ,

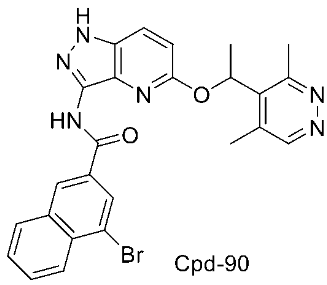
Cpd-90 ,

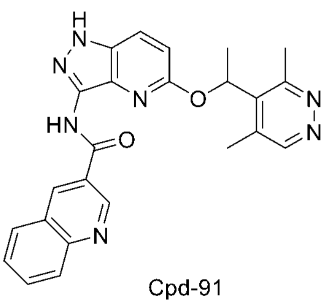
Cpd-91 ,

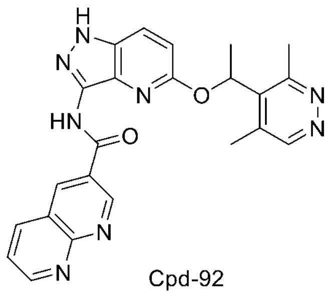
Cpd-92 ,

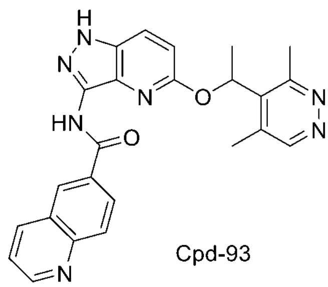
Cpd-93 ,

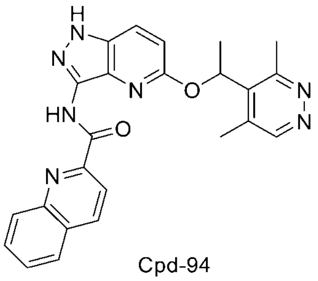

Cpd-94 ,

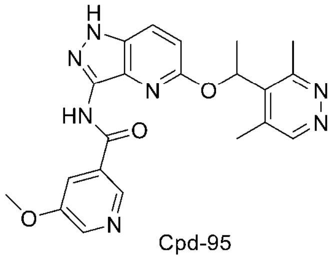

Cpd-95 ,

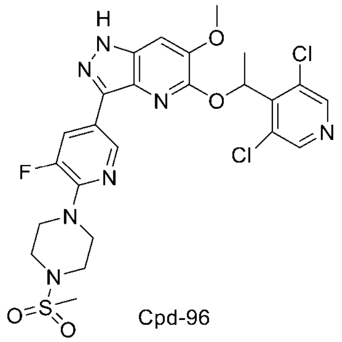

Cpd-96 ,

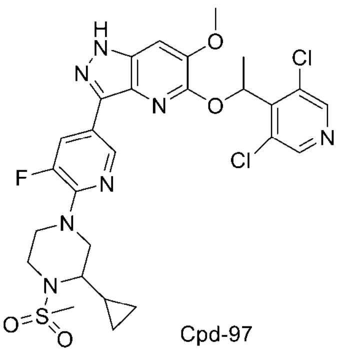

Cpd-97 ,

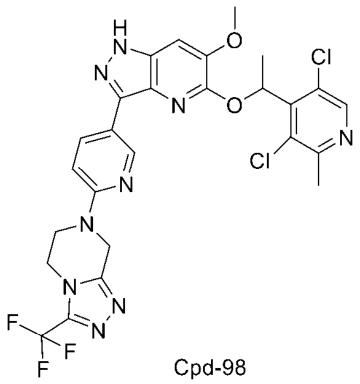

Cpd-98 ,

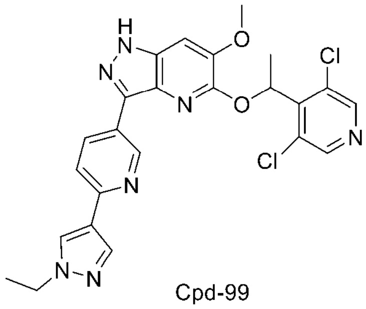

Cpd-99 ,

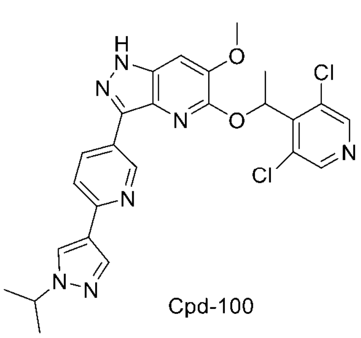

Cpd-100 ,

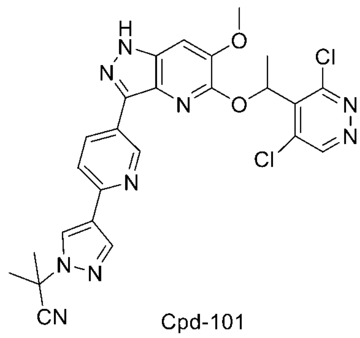

Cpd-101 ,

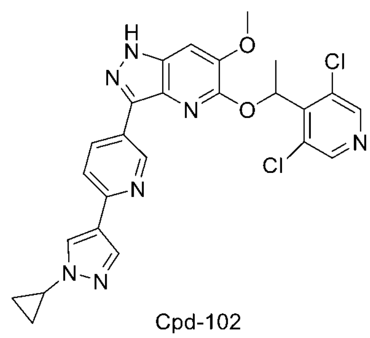

Cpd-102 ,

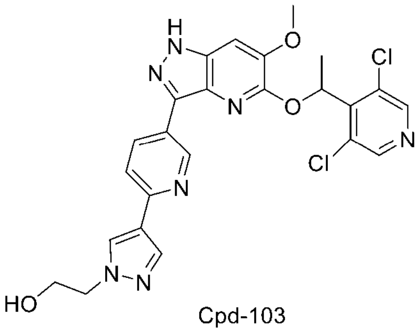

Cpd-103 ,

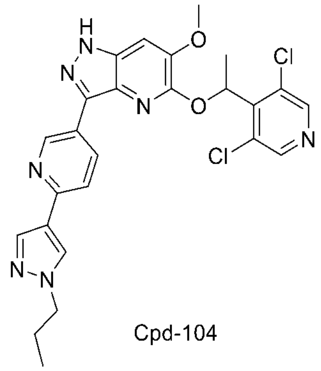

Cpd-104 ,

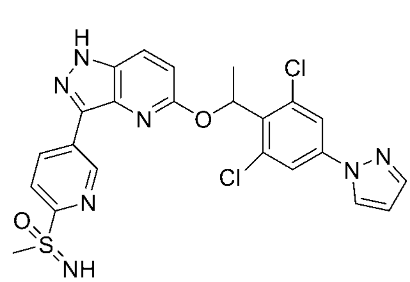

Cpd-105 ,

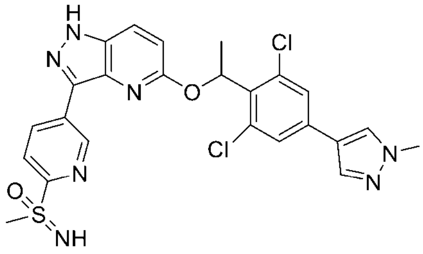

Cpd-106 ,

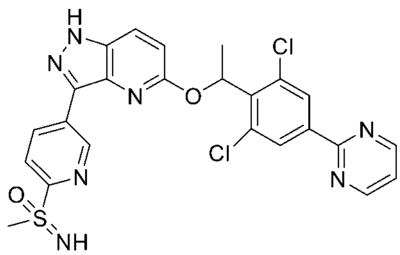

Cpd-107 ,

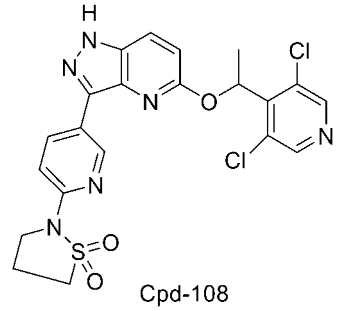

Cpd-108 ,

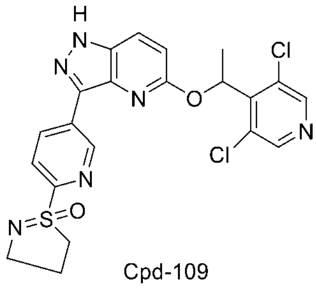

Cpd-109 ,

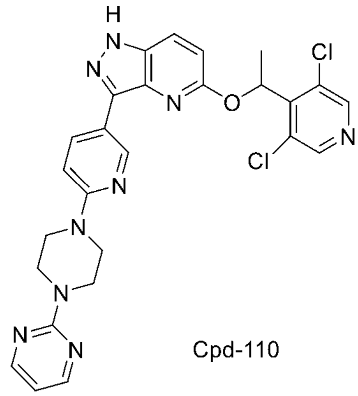

Cpd-110 ,

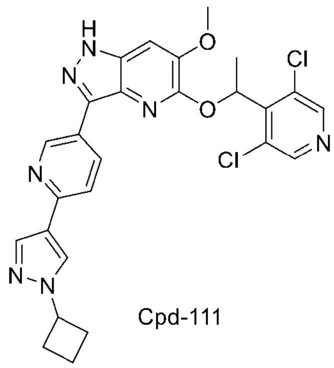

Cpd-111 ,

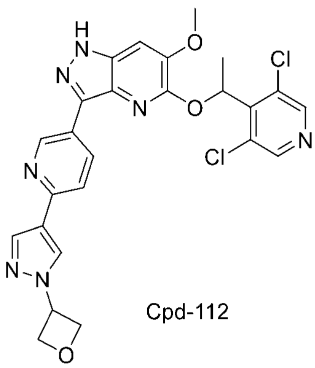

Cpd-112 ,

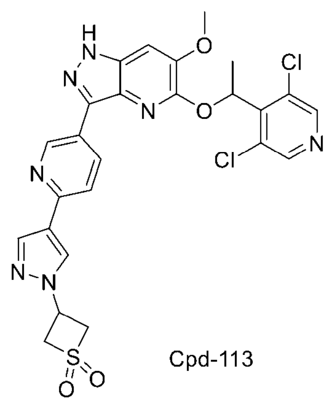

Cpd-113 ,

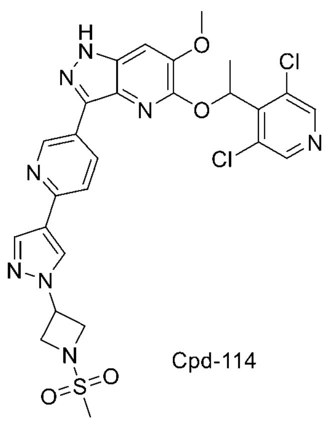

Cpd-114 ,

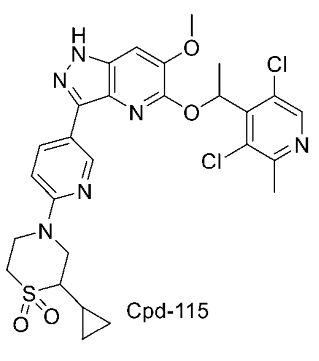

Cpd-115 ,

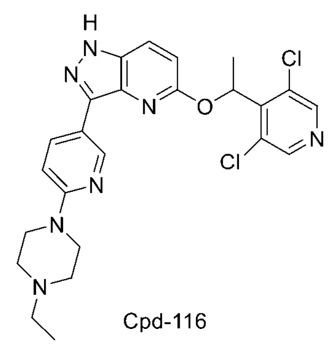

Cpd-116 ,

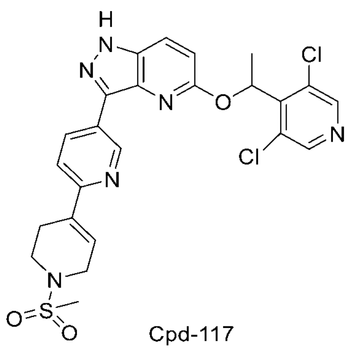

Cpd-117 ,

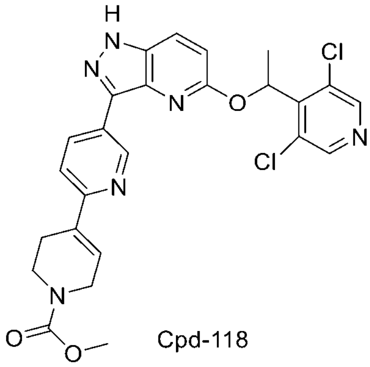
Cpd-118 ,

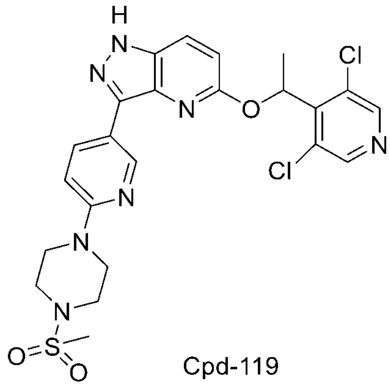
Cpd-119 ,

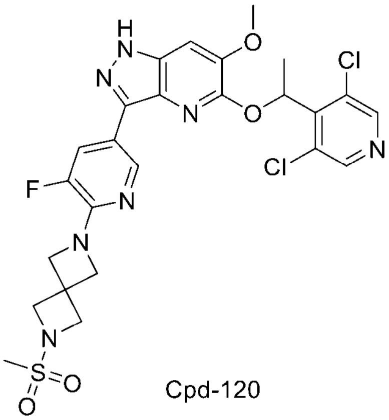
Cpd-120 ,

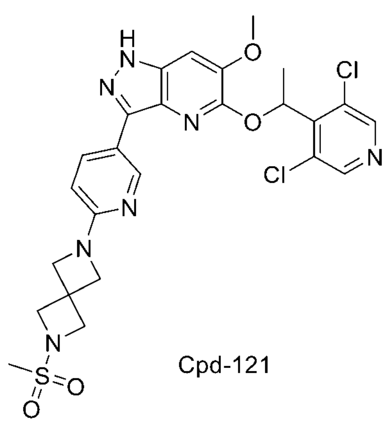
Cpd-121 ,

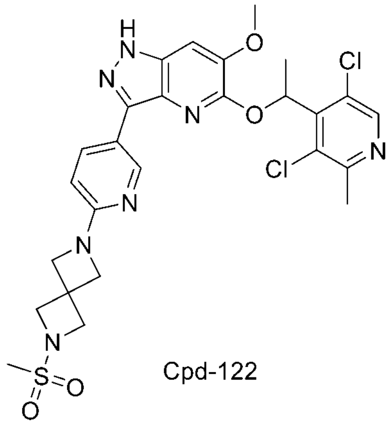
Cpd-122 ,

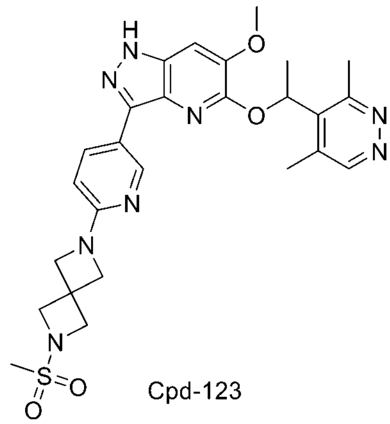
Cpd-123 ,

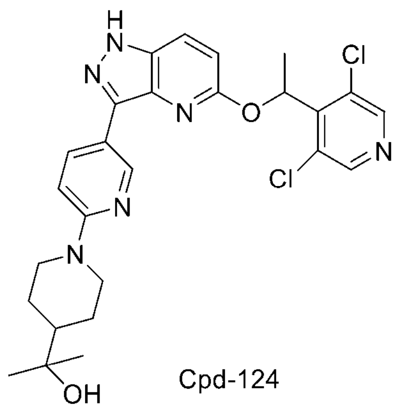
Cpd-124 ,

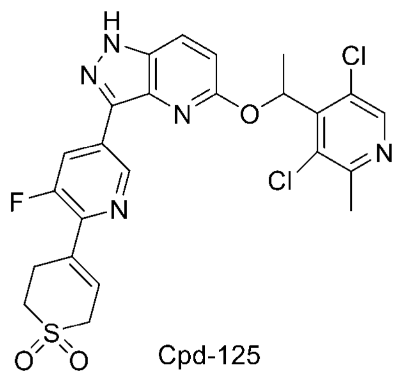
Cpd-125 ,

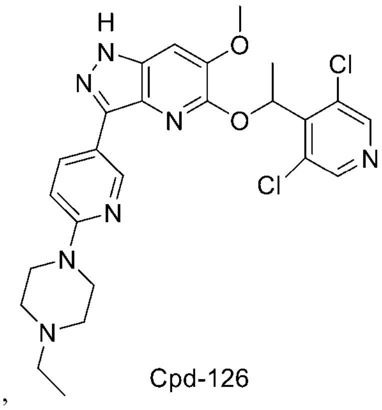
Cpd-126 ,

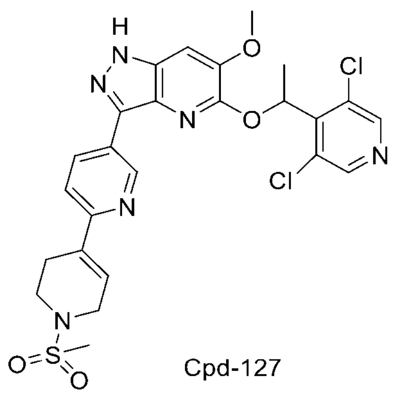
Cpd-127 ,

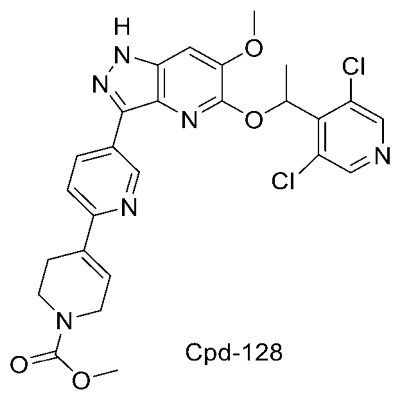
Cpd-128 ,

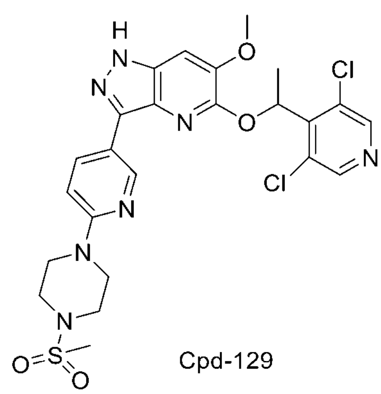
Cpd-129 ,

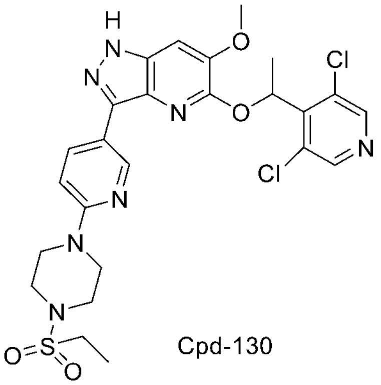
Cpd-130 ,

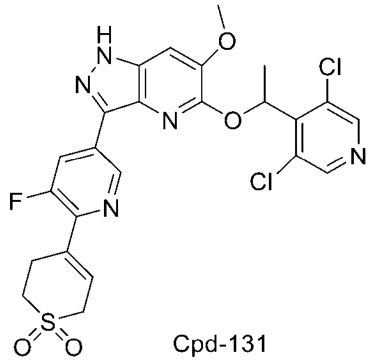
Cpd-131 ,

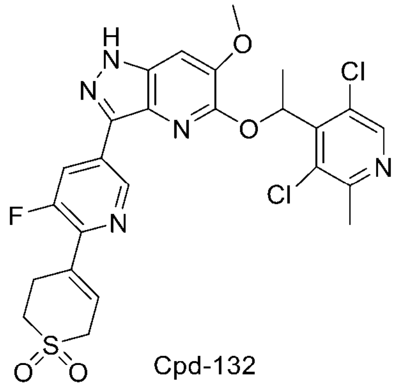
Cpd-132 ,

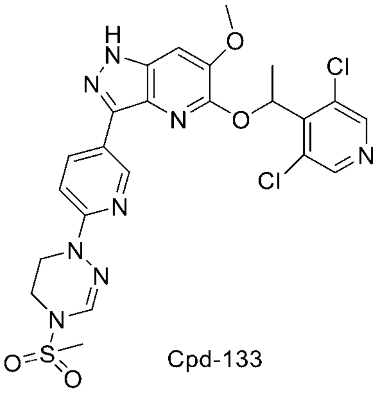
Cpd-133 ,

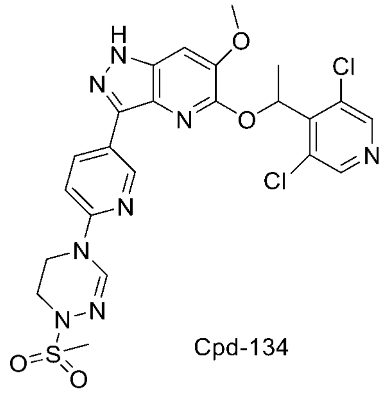
Cpd-134 ,

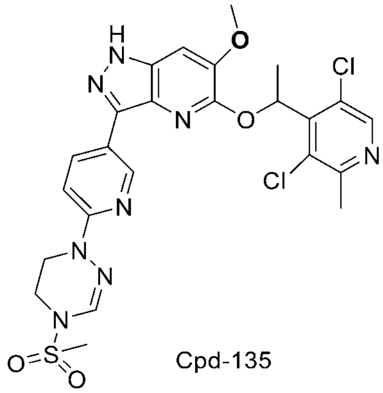
Cpd-135 ,

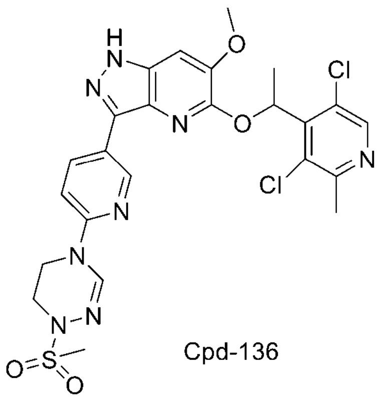
Cpd-136 ,

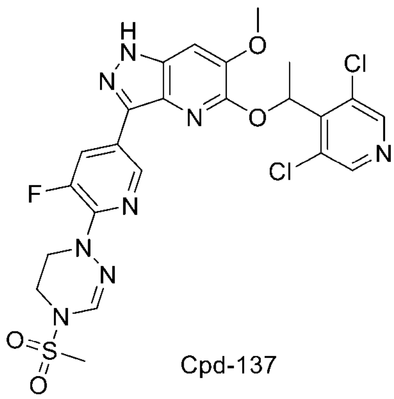
Cpd-137 ,

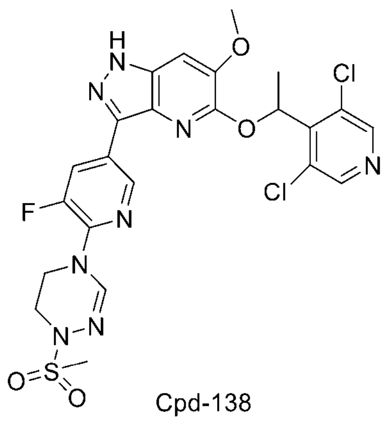
Cpd-138 ,

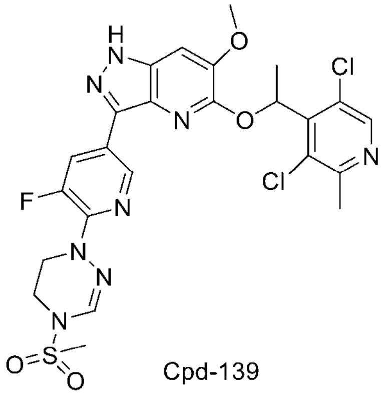
Cpd-139 ,

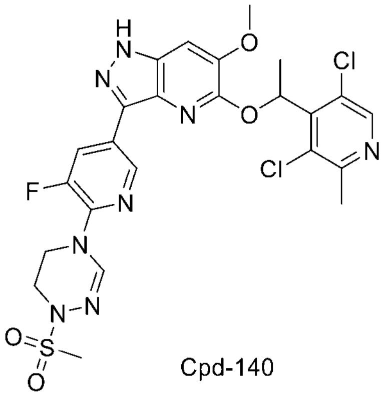
Cpd-140 ,

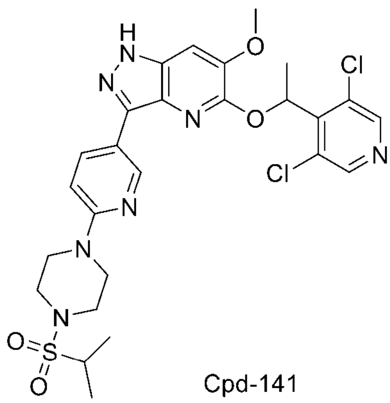
Cpd-141 ,

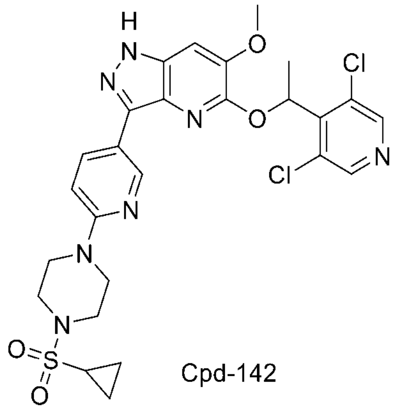

Cpd-142 ,

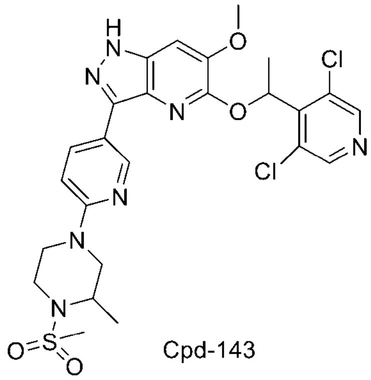

Cpd-143 ,

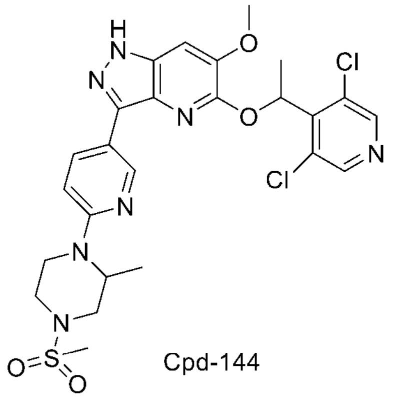

Cpd-144 ,

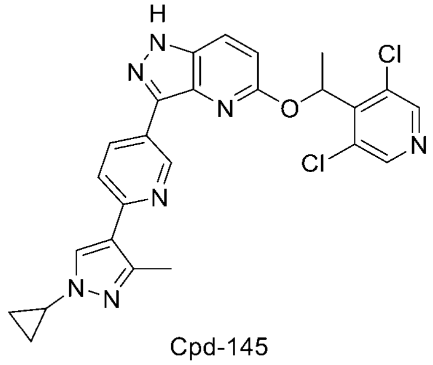

Cpd-145 ,

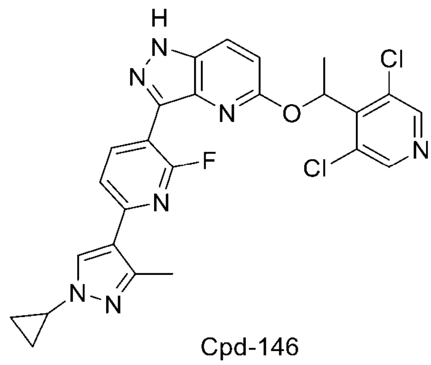

Cpd-146 ,

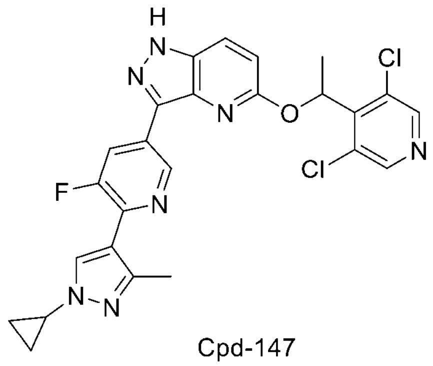

Cpd-147 ,

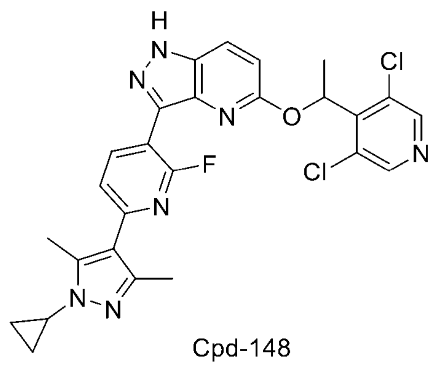

Cpd-148 ,

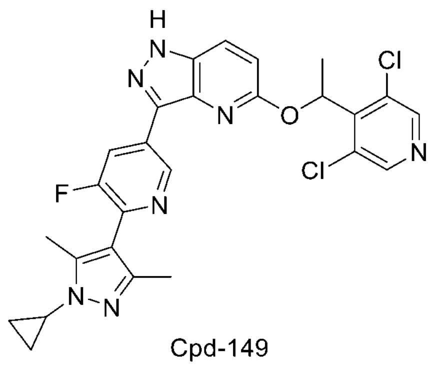

Cpd-149 ,

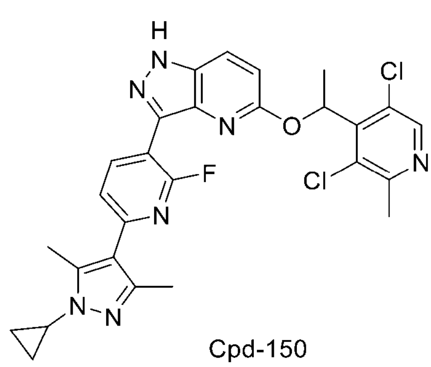

Cpd-150 ,

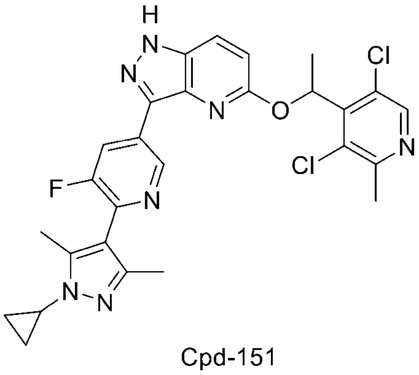

Cpd-151 ,

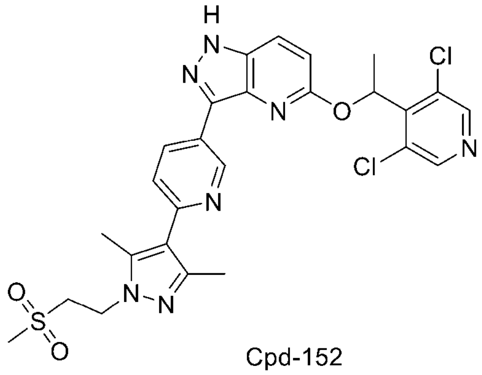

Cpd-152 ,

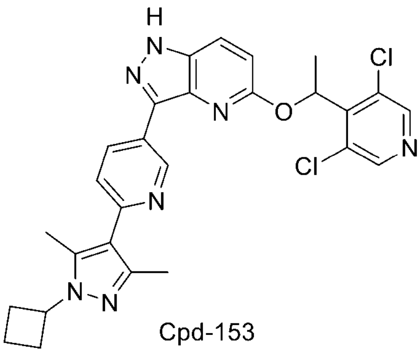

Cpd-153 ,

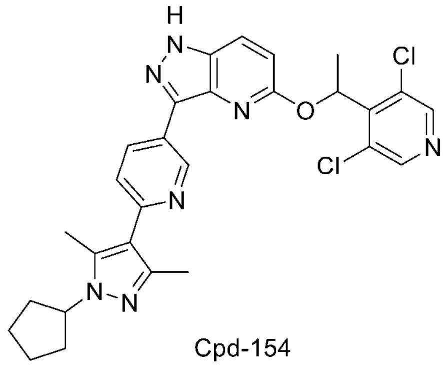

Cpd-154 ,

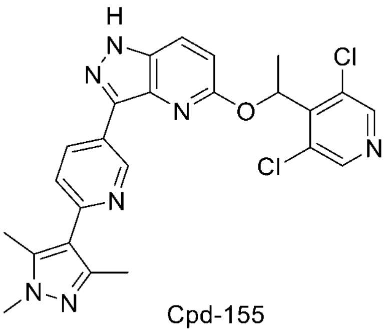

Cpd-155 ,

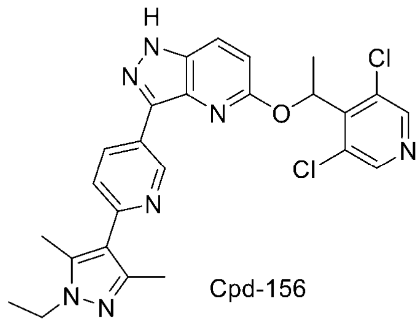

Cpd-156 ,

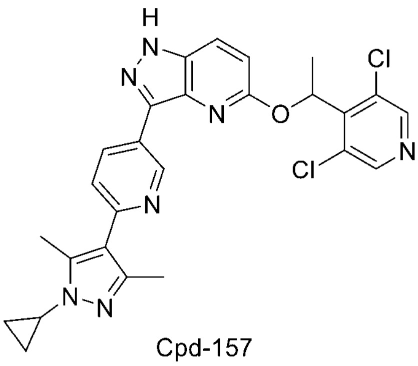

Cpd-157 ,

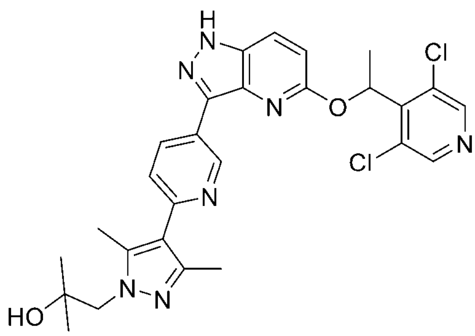

Cpd-158 ,

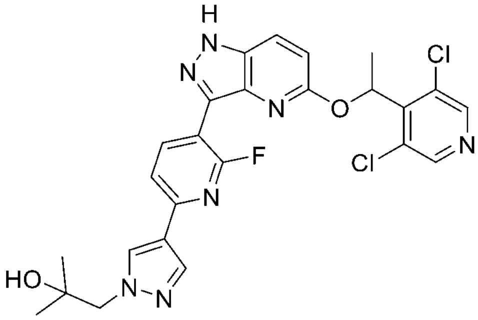

Cpd-159 ,

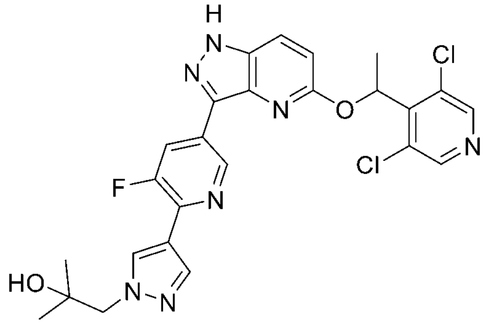

Cpd-160 ,

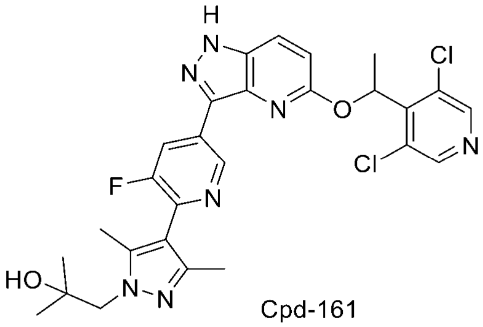

Cpd-161 ,

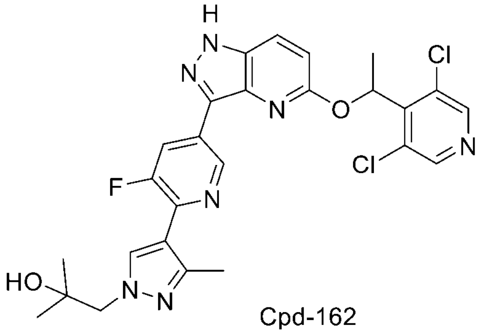

Cpd-162 ,

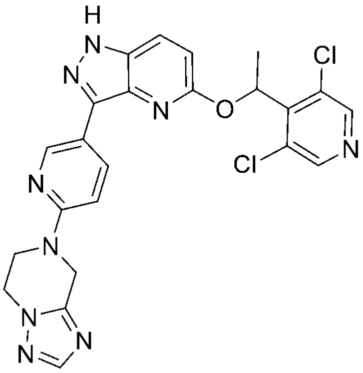

Cpd-163 ,

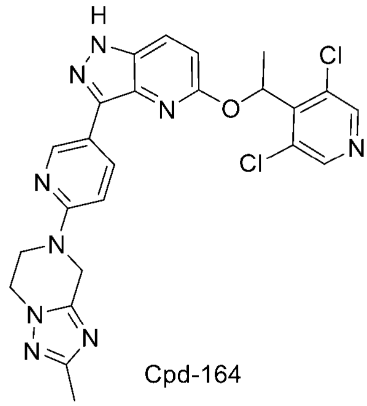

Cpd-164 ,

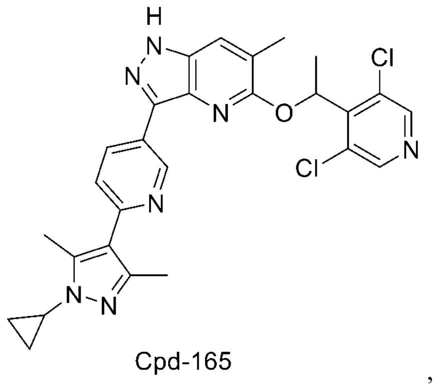

Cpd-165 ,

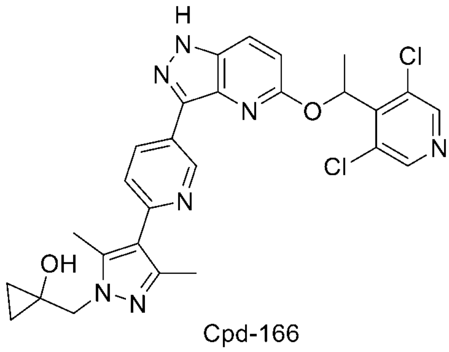

Cpd-166 ,

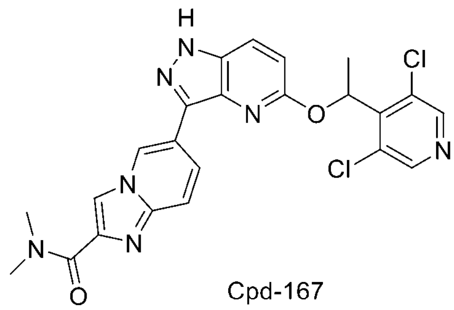

Cpd-167 ,

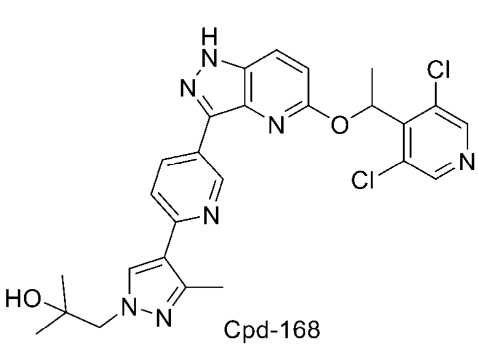

Cpd-168 ,

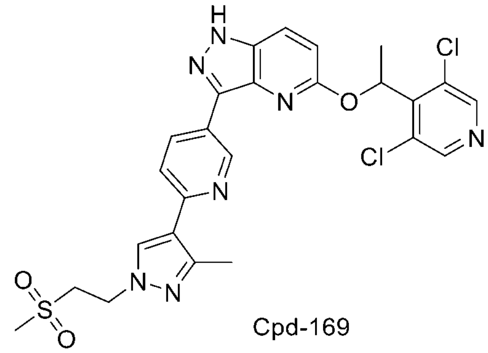

Cpd-169 ,

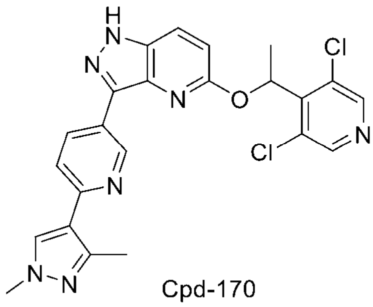

Cpd-170 ,

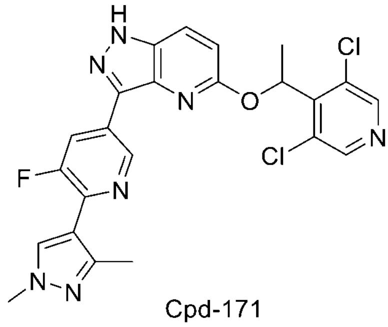

Cpd-171 ,

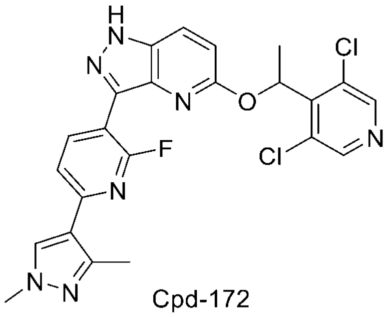

Cpd-172 ,

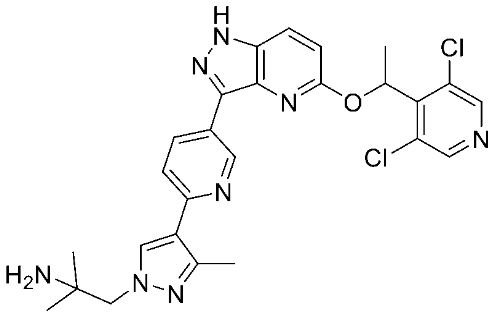

Cpd-173 ,

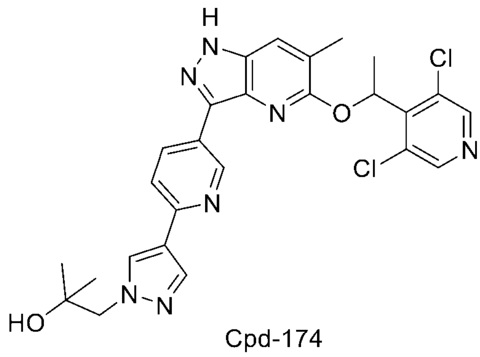

Cpd-174 ,

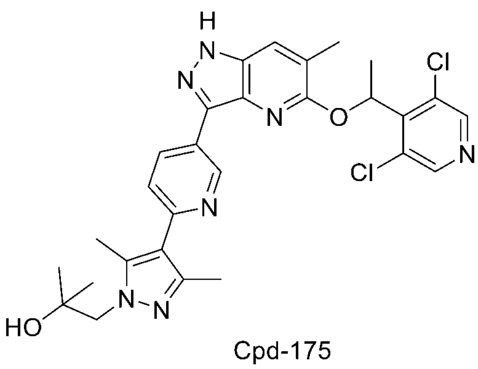

Cpd-175 ,

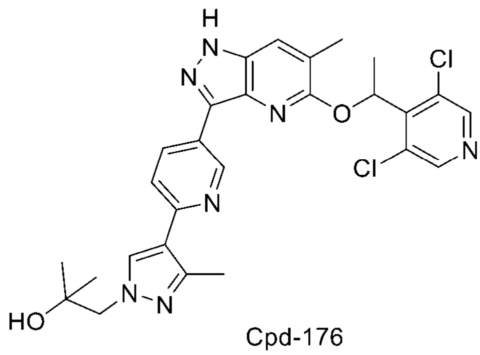

Cpd-176 ,

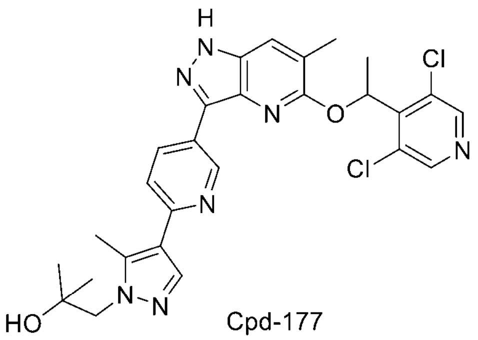

Cpd-177 ,

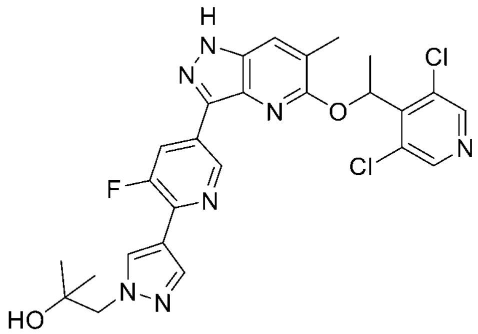

Cpd-178 ,

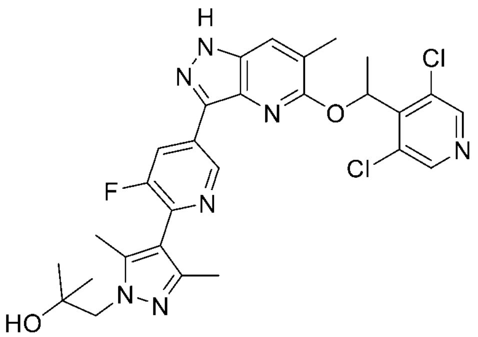

Cpd-179 ,

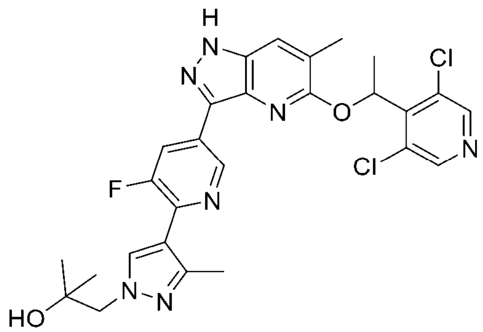

Cpd-180 ,

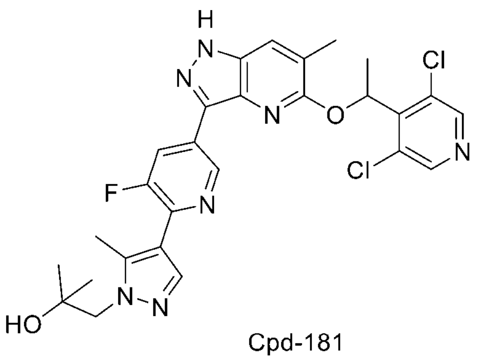

Cpd-181 ,

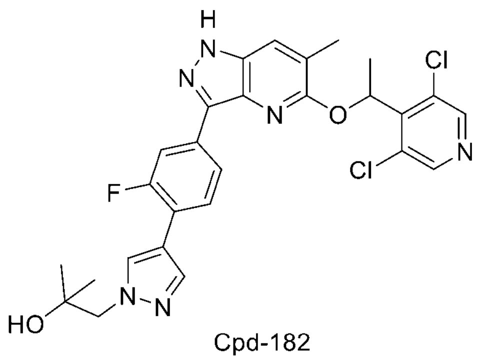

Cpd-182 ,

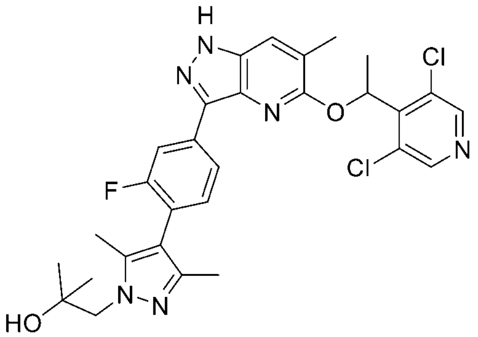

Cpd-183 ,

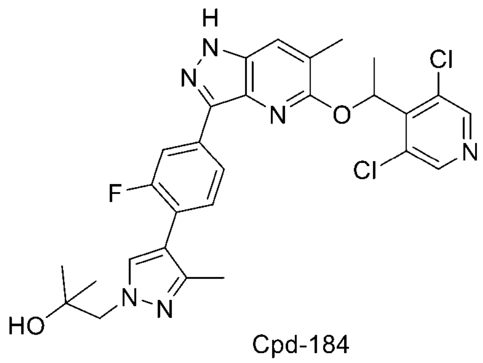

Cpd-184 ,

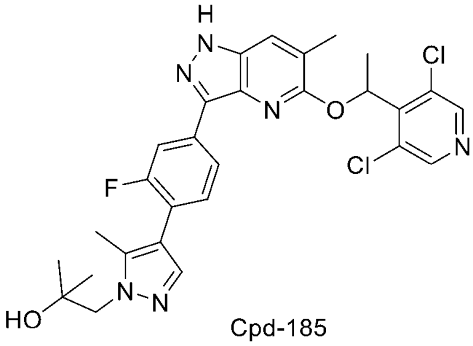

Cpd-185 ,

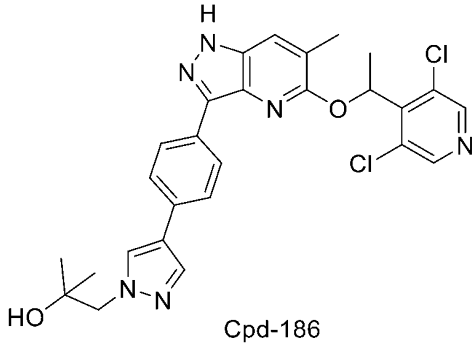

Cpd-186 ,

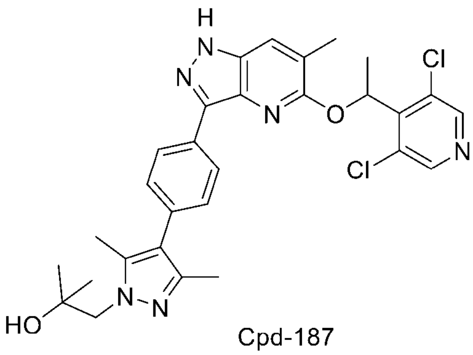

Cpd-187 ,

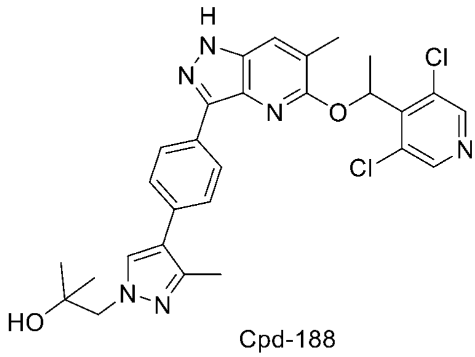

Cpd-188 ,

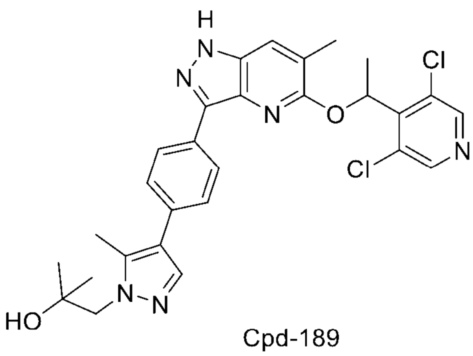

Cpd-189 ,

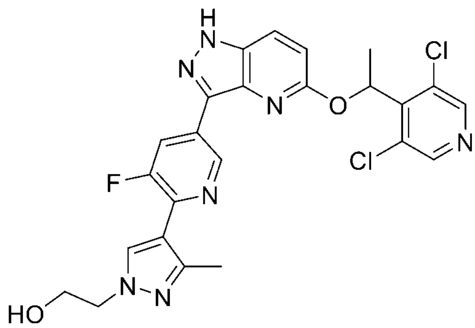

Cpd-190 ,

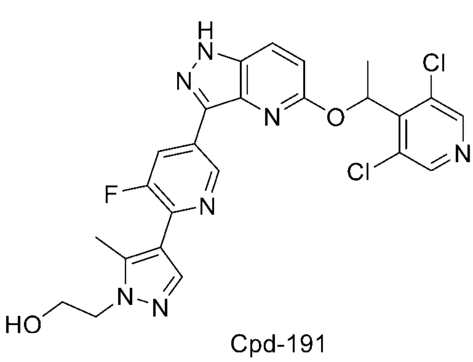

Cpd-191 ,

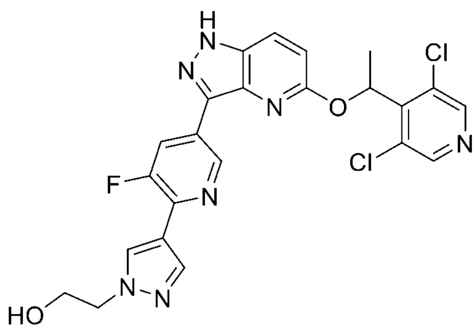

Cpd-192 ,

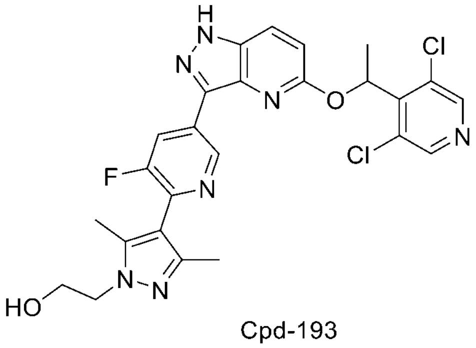

Cpd-193 ,

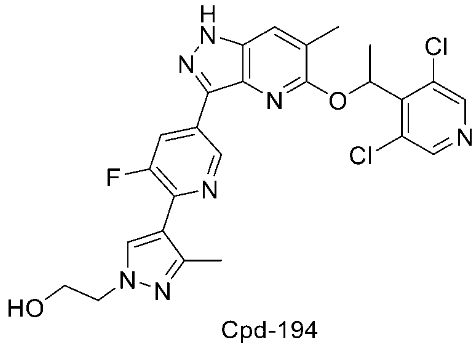

Cpd-194 ,

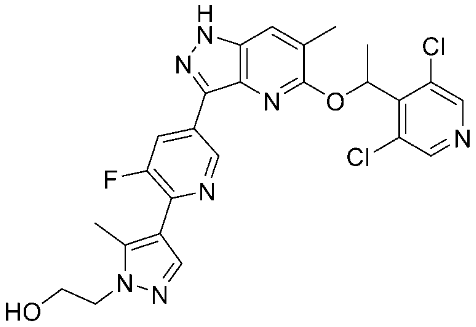

Cpd-195 ,

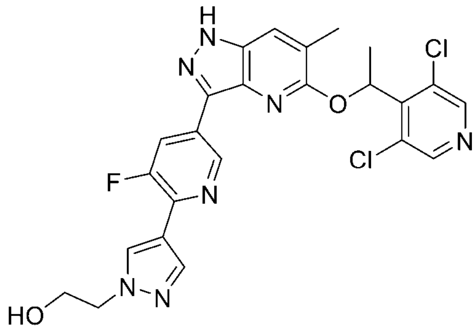

Cpd-196 ,

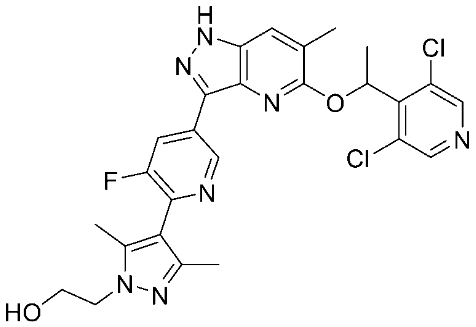

Cpd-197 ,

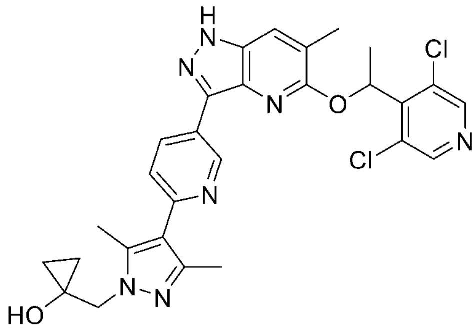

Cpd-198 ,

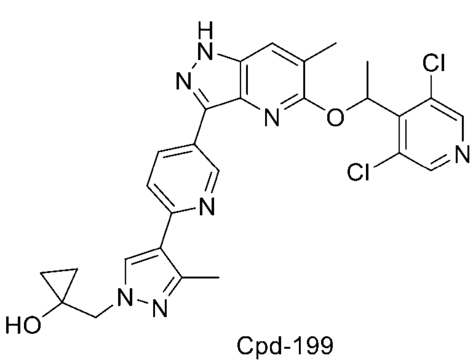

Cpd-199 ,

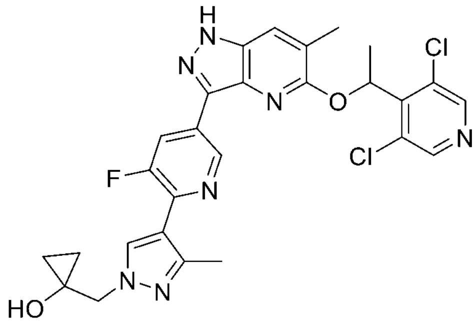

Cpd-200 ,

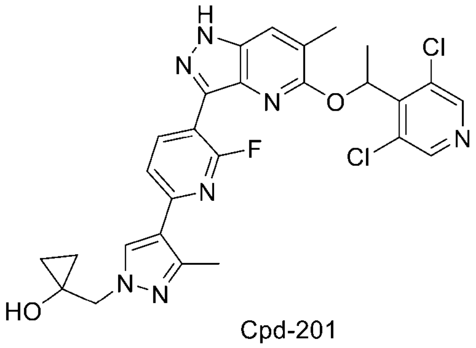

Cpd-201 ,

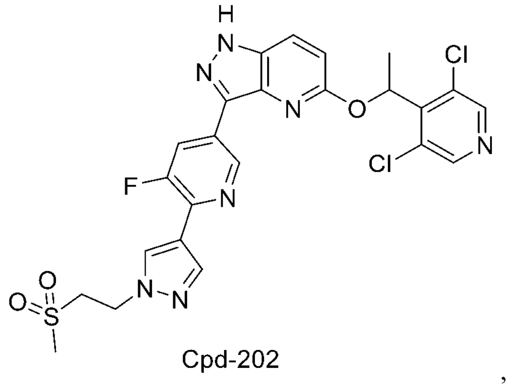

Cpd-202 ,

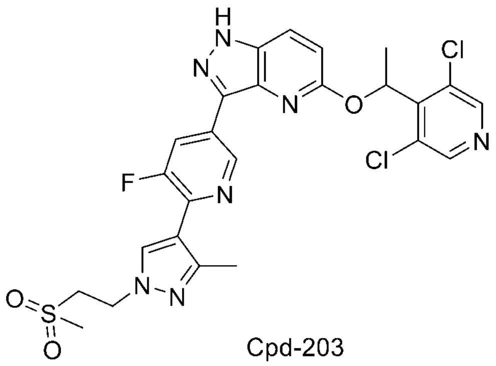

Cpd-203 ,

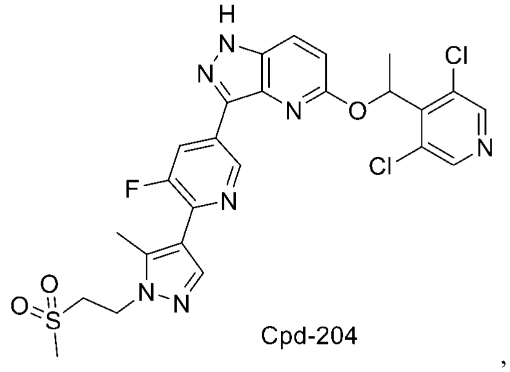

Cpd-204 ,

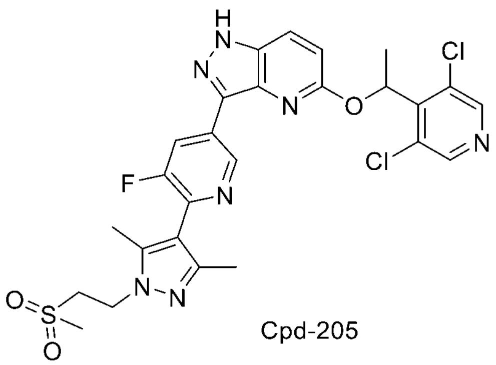

Cpd-205 ,

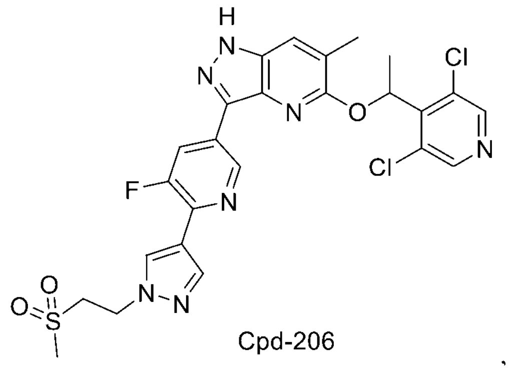

Cpd-206 ,

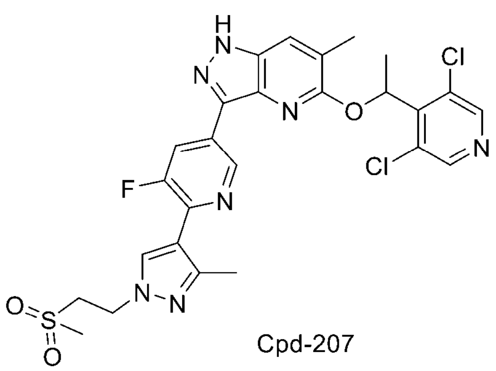

Cpd-207 ,

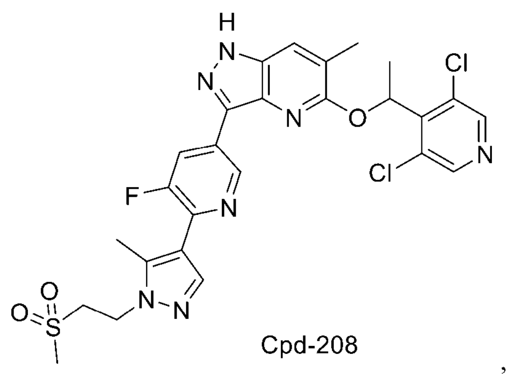

Cpd-208 ,

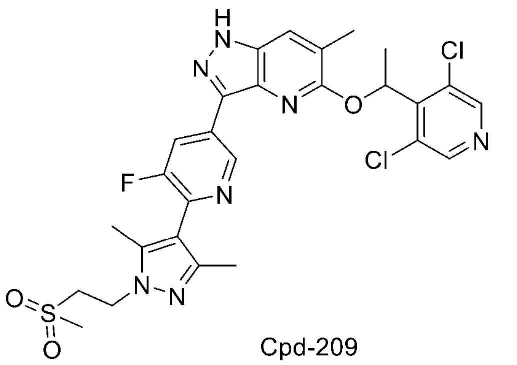

Cpd-209 ,

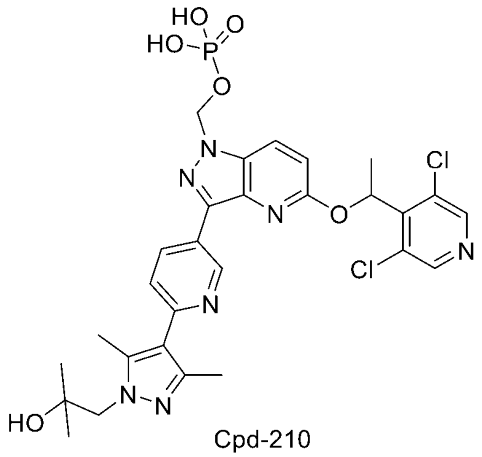

Cpd-210 ,

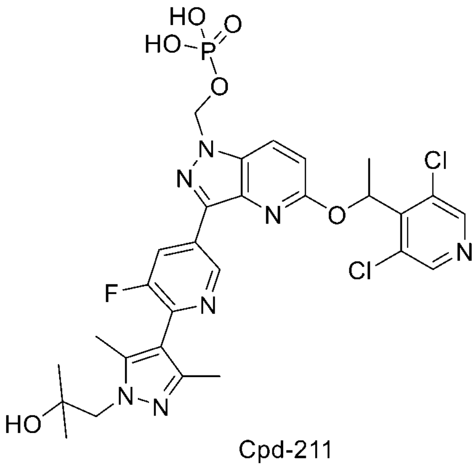

Cpd-211 ,

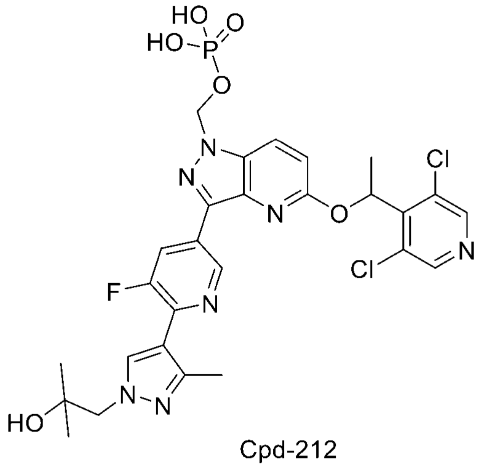

Cpd-212 ,

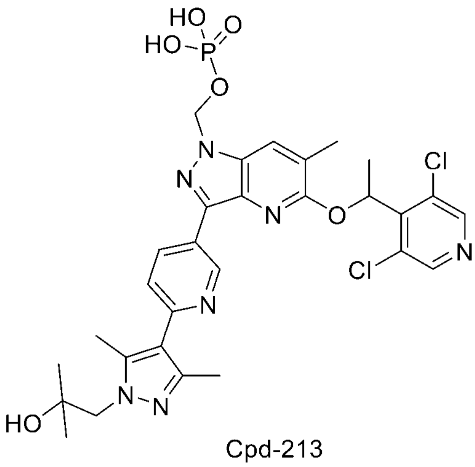

Cpd-213 ,

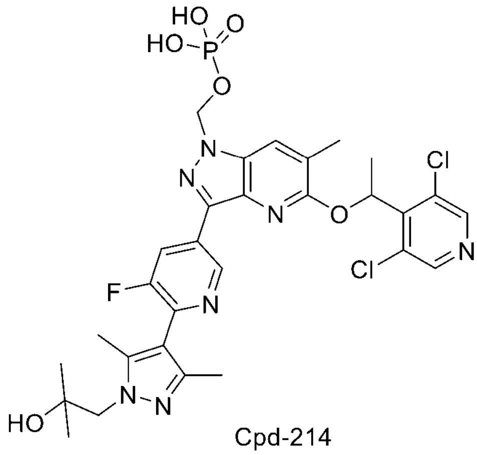

Cpd-214 ,

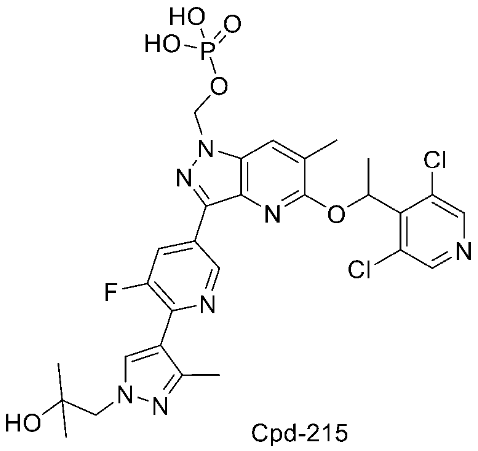
Cpd-215 ,

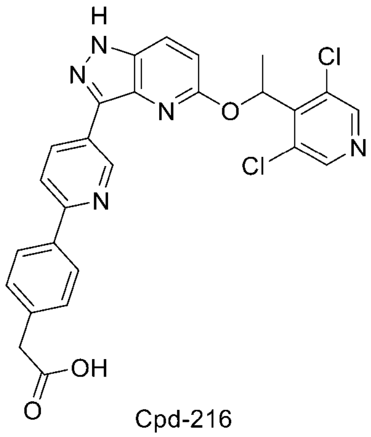
Cpd-216 ,

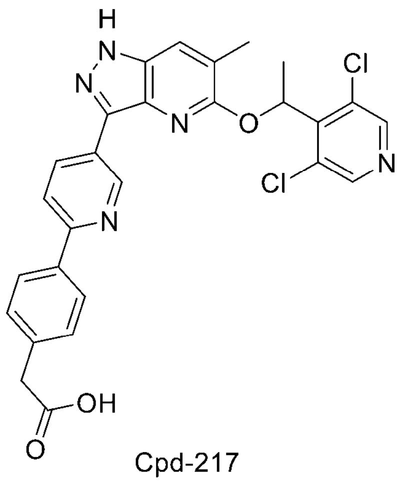
Cpd-217 ,

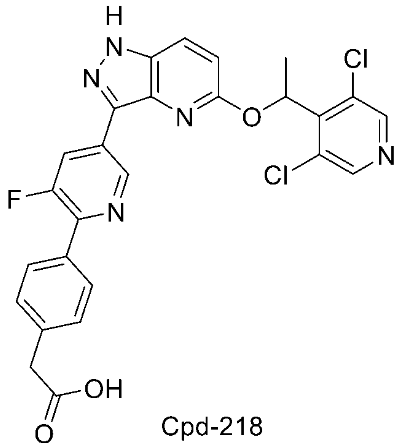
Cpd-218 ,

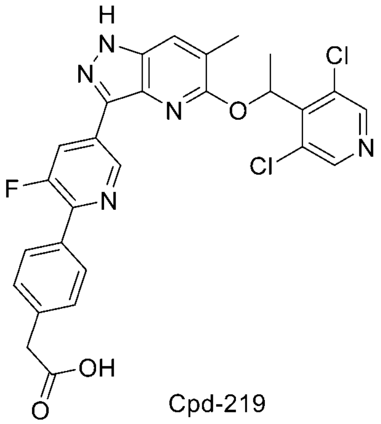
Cpd-219 ,

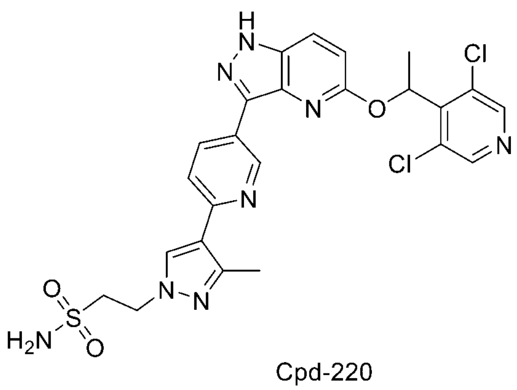
Cpd-220 ,

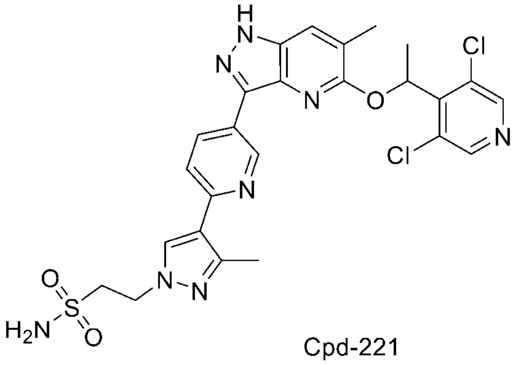

Cpd-221 ,

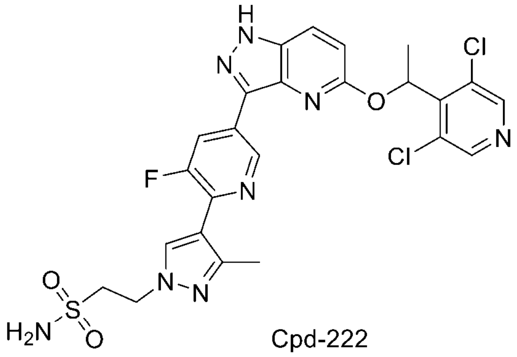

Cpd-222 ,

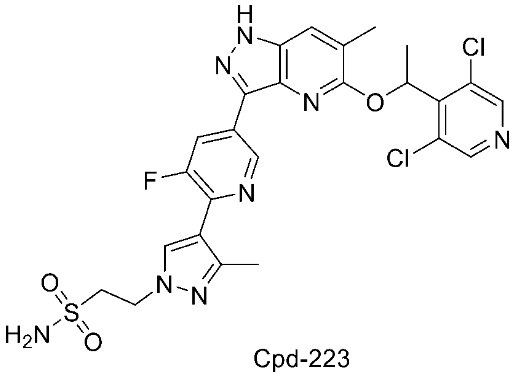

Cpd-223 ,

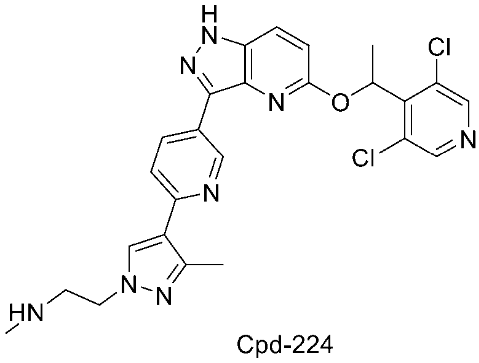

Cpd-224 ,

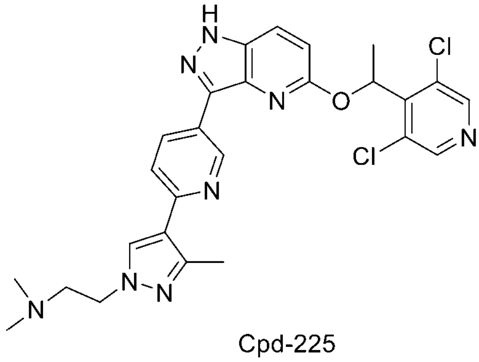

Cpd-225 ,

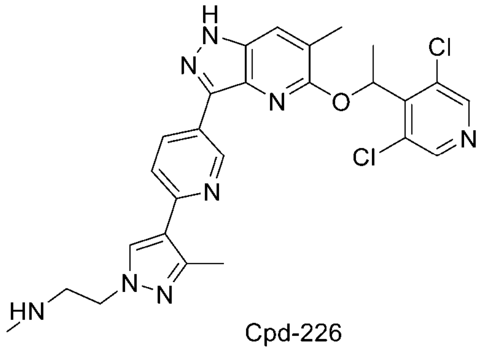

Cpd-226 ,

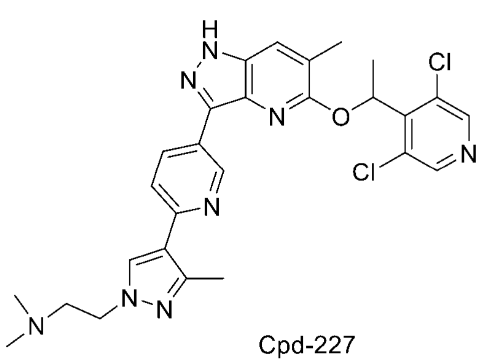

Cpd-227 ,

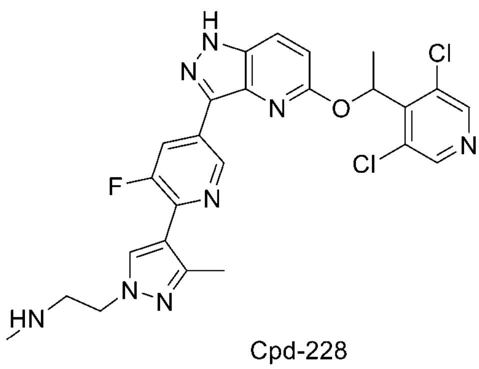

Cpd-228 ,

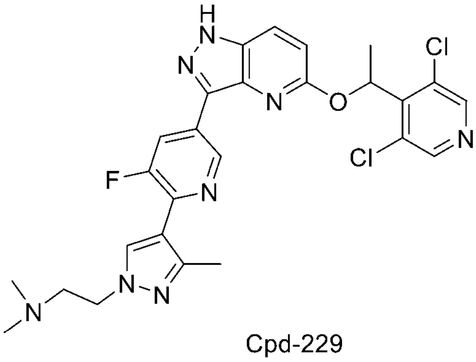

Cpd-229 ,

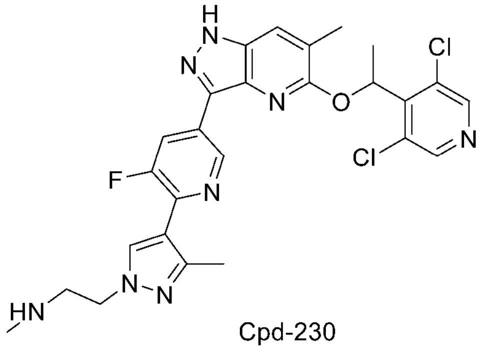

Cpd-230 ,

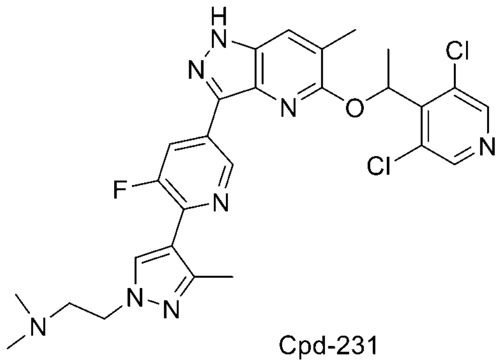

Cpd-231

preferably, the compound of Formula (I) is selected from the following structures:

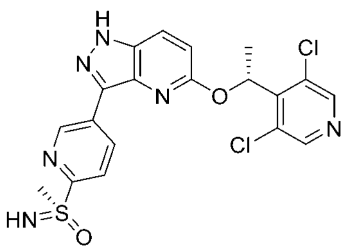

,

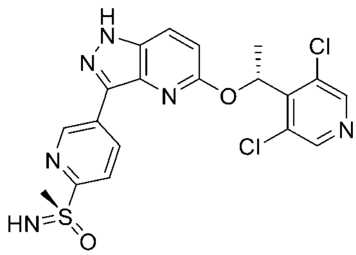

,

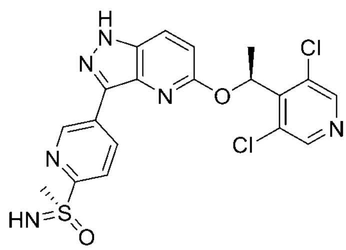

,

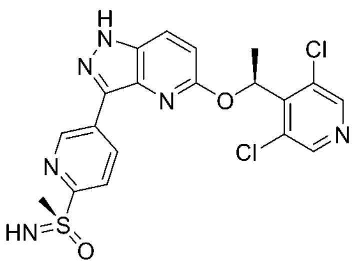

,

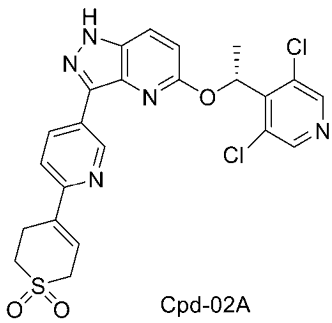

Cpd-02A ,

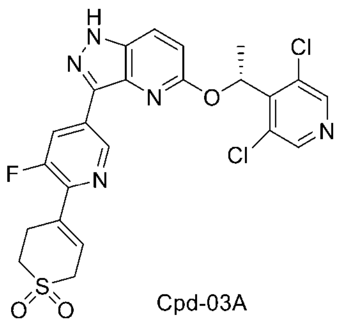

Cpd-03A ,

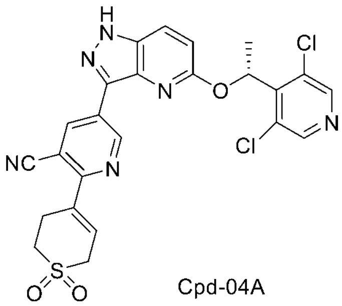

Cpd-04A ,

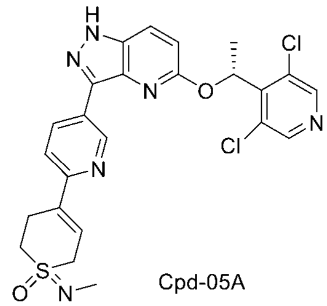

Cpd-05A ,

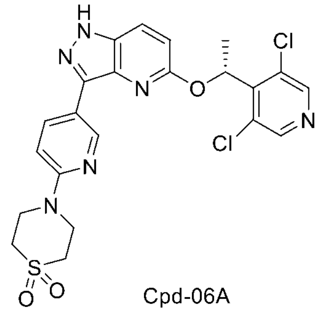

Cpd-06A ,

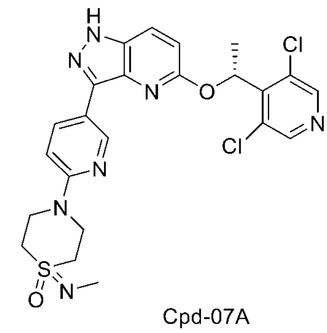

Cpd-07A ,

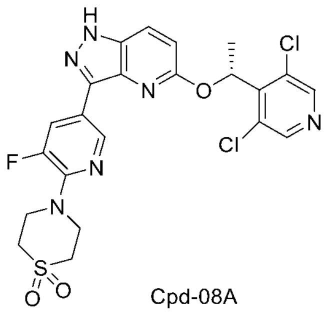

Cpd-08A ,

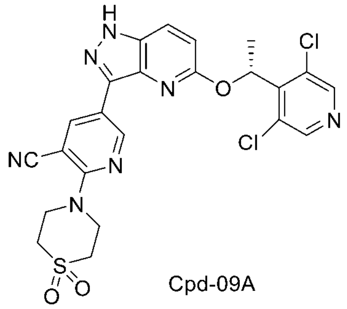

Cpd-09A ,

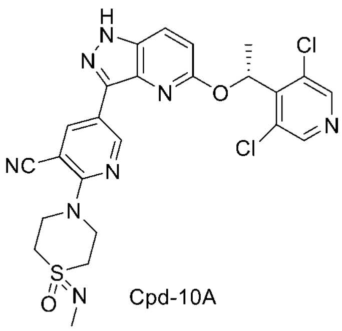

Cpd-10A ,

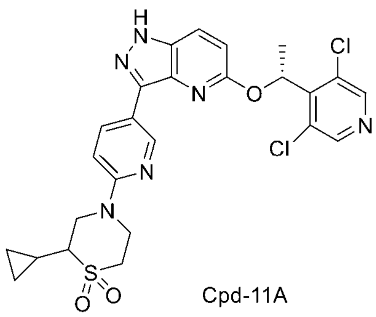

Cpd-11A ,

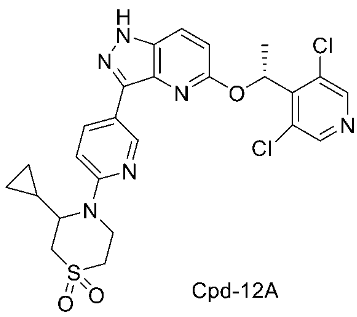

Cpd-12A ,

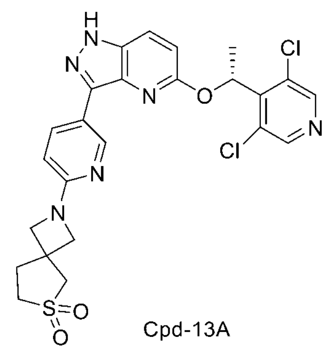

Cpd-13A ,

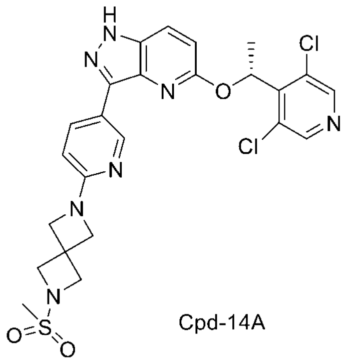

Cpd-14A ,

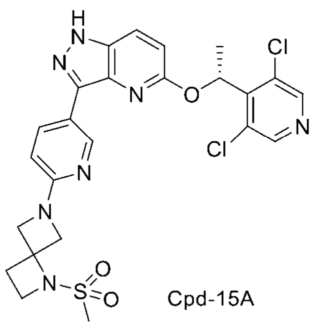

Cpd-15A ,

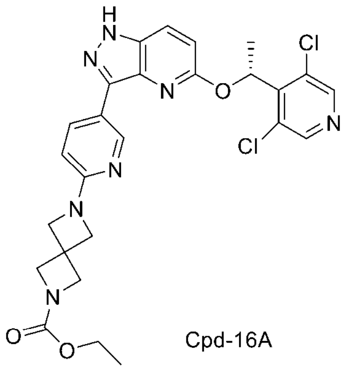

Cpd-16A ,

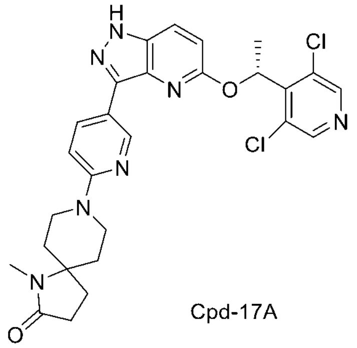

Cpd-17A ,

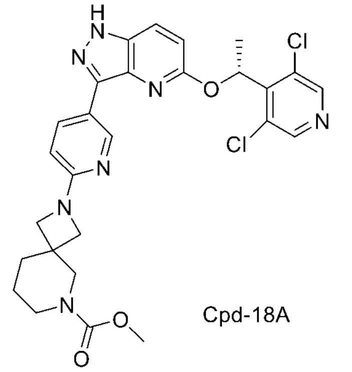

Cpd-18A ,

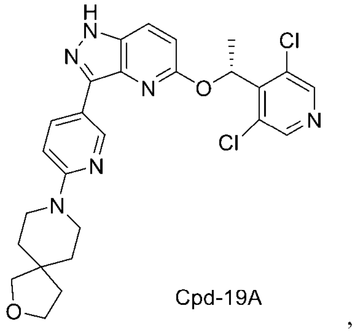

Cpd-19A ,

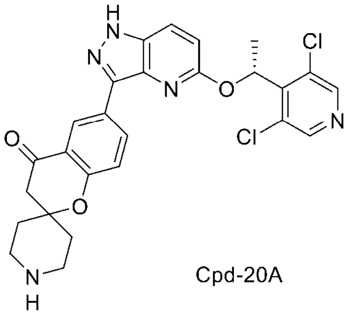

Cpd-20A ,

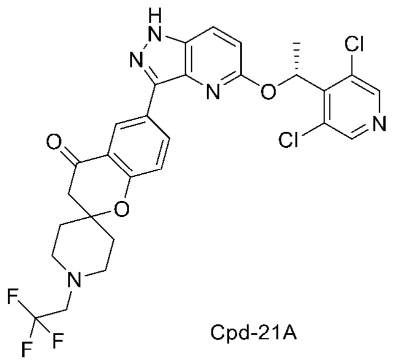

Cpd-21A ,

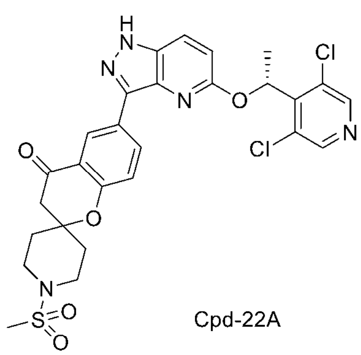

Cpd-22A ,

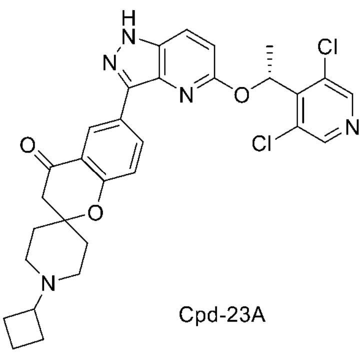

Cpd-23A ,

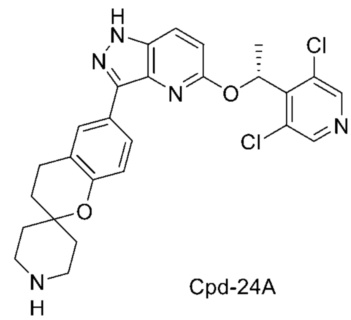

Cpd-24A ,

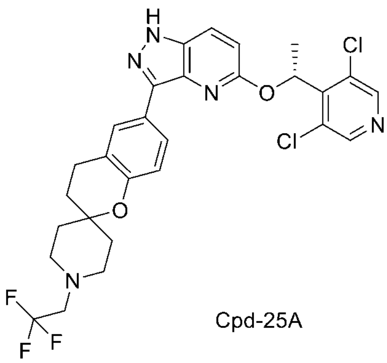

Cpd-25A ,

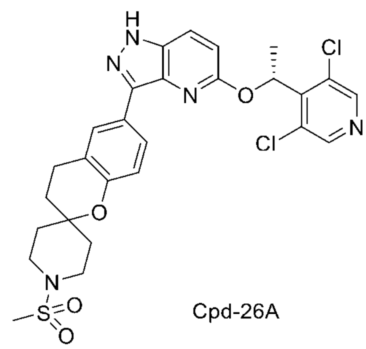

Cpd-26A ,

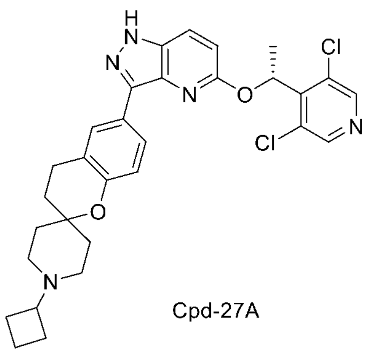

Cpd-27A ,

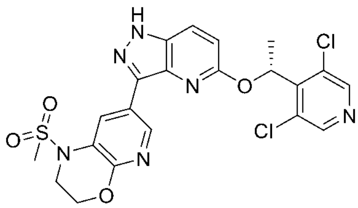
Cpd-28A ,

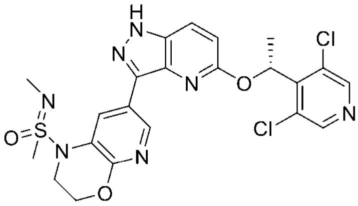
Cpd-29A ,

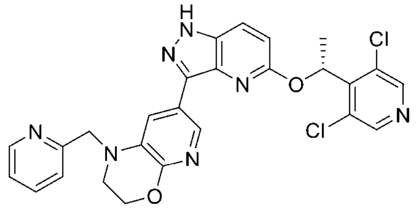
Cpd-30A ,

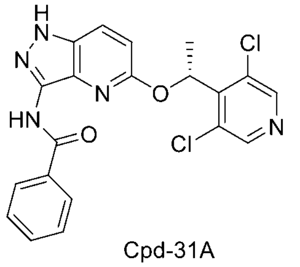
Cpd-31A ,

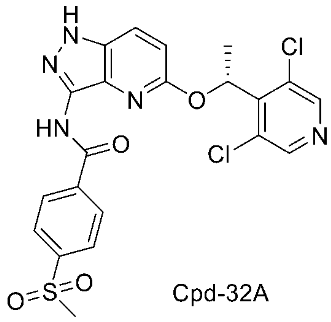
Cpd-32A ,

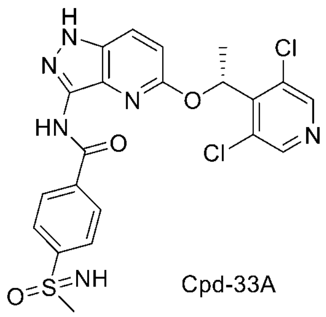
Cpd-33A ,

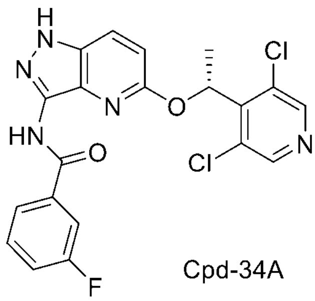
Cpd-34A ,

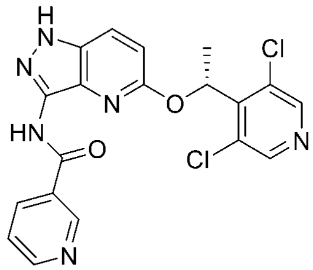
Cpd-35A ,

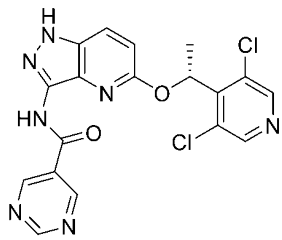
Cpd-36A ,

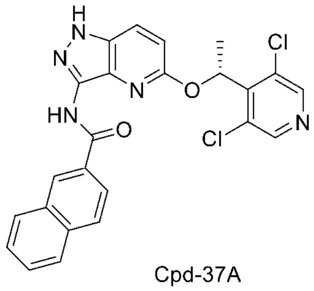
Cpd-37A ,

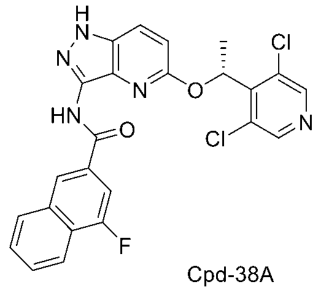
Cpd-38A ,

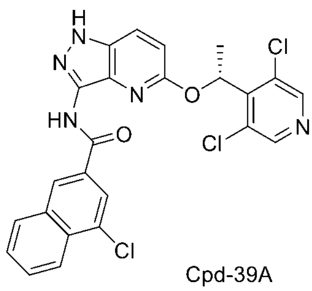
Cpd-39A ,

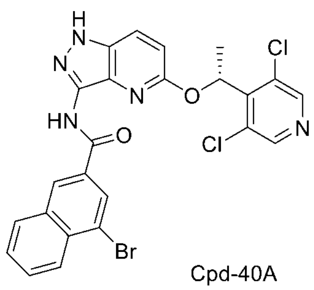
Cpd-40A ,

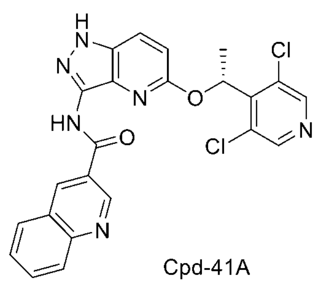
Cpd-41A ,

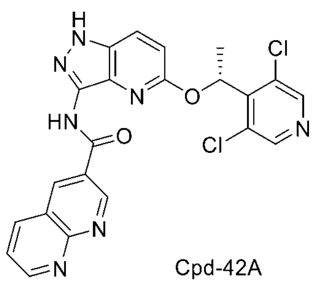
Cpd-42A ,

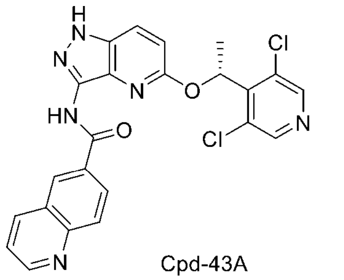

Cpd-43A ,

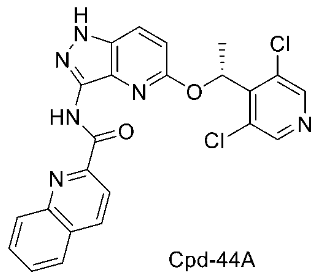

Cpd-44A ,

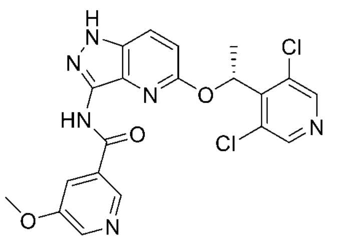

Cpd-45A ,

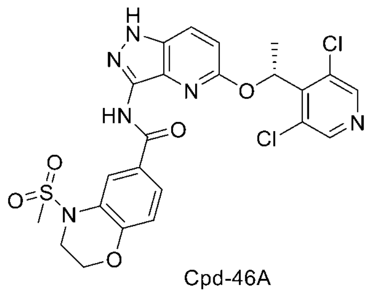

Cpd-46A ,

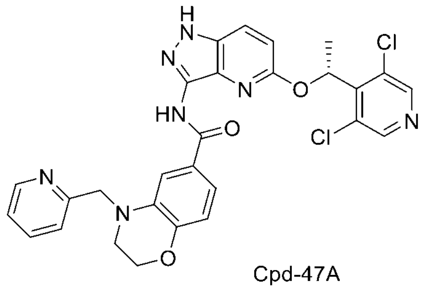

Cpd-47A ,

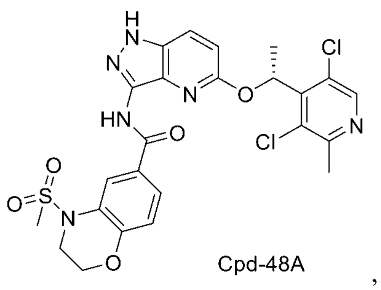

Cpd-48A ,

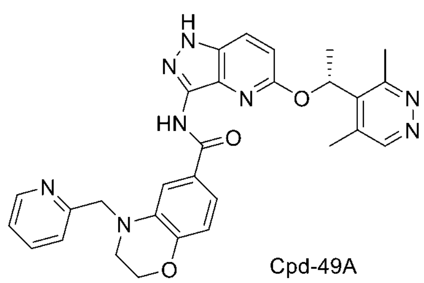

Cpd-49A ,

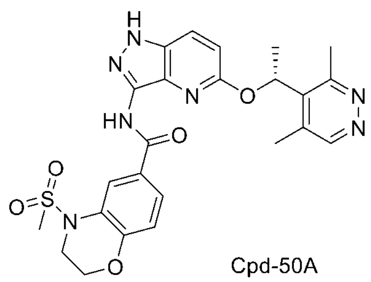

Cpd-50A ,

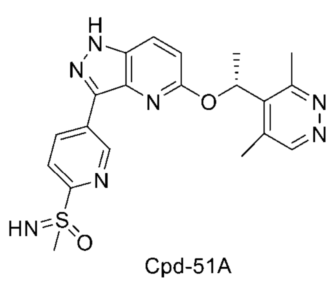

Cpd-51A ,

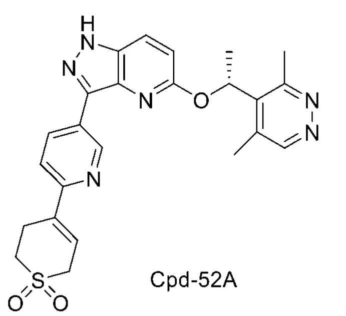

Cpd-52A ,

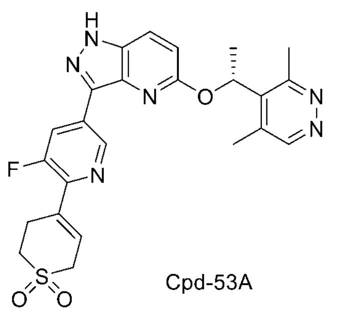

Cpd-53A ,

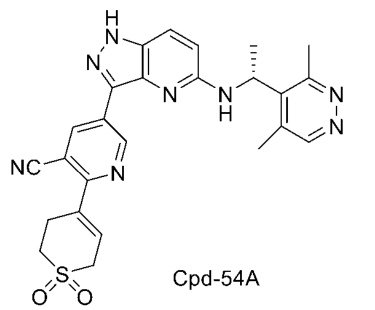

Cpd-54A ,

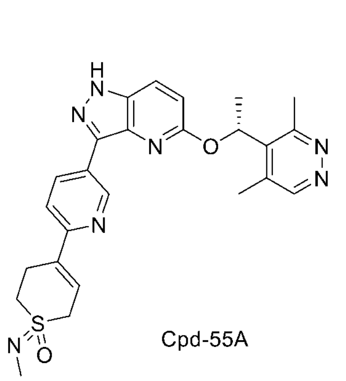

Cpd-55A ,

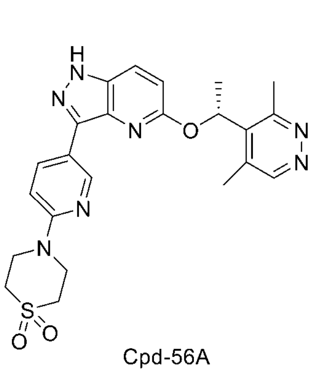

Cpd-56A ,

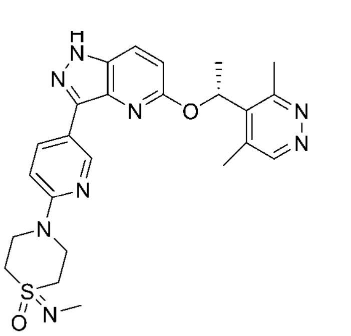

Cpd-57A ,

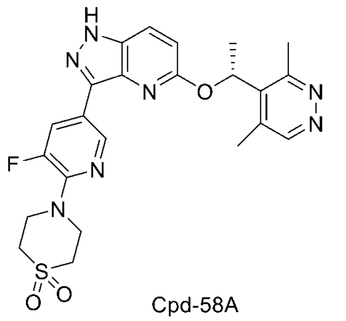
Cpd-58A ,

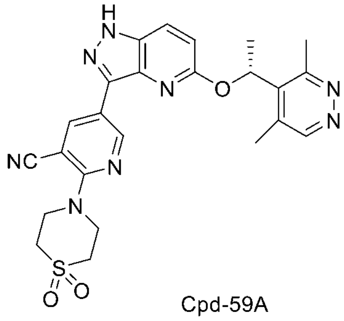
Cpd-59A ,

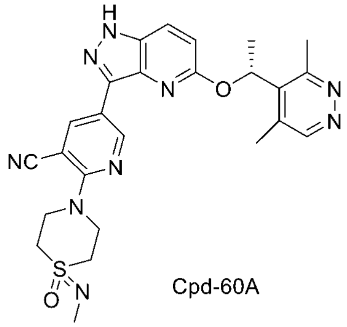
Cpd-60A ,

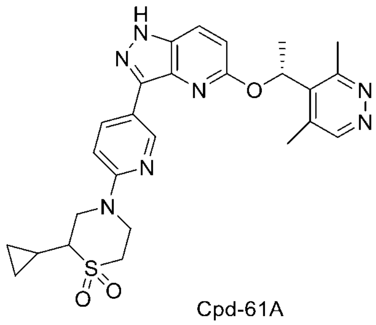
Cpd-61A ,

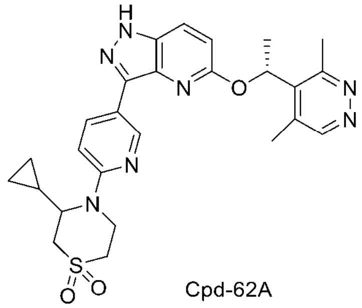
Cpd-62A ,

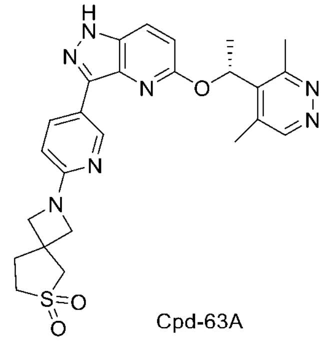
Cpd-63A ,

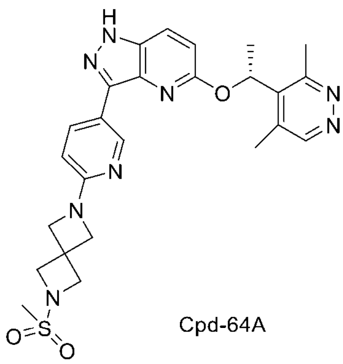
Cpd-64A ,

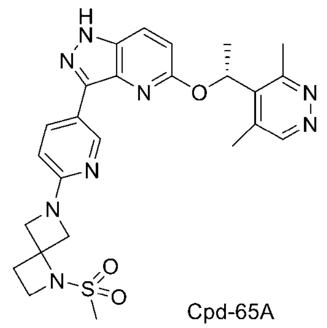
Cpd-65A ,

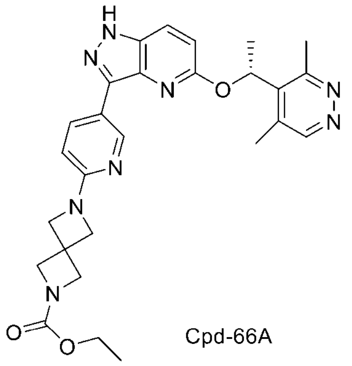
Cpd-66A ,

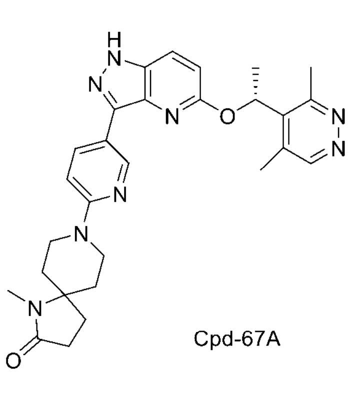
Cpd-67A ,

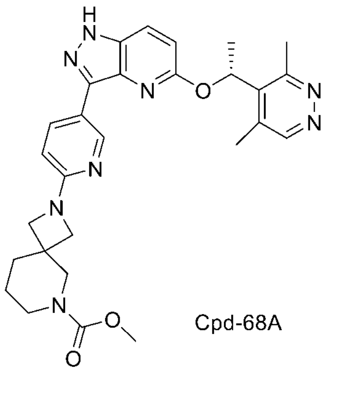
Cpd-68A ,

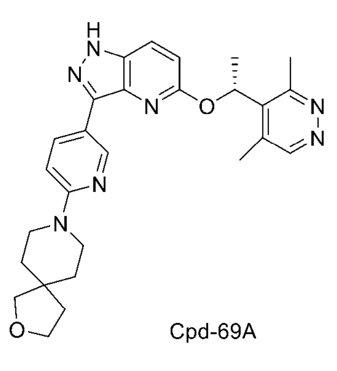
Cpd-69A ,

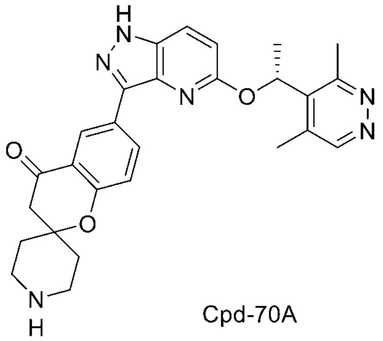

Cpd-70A ,

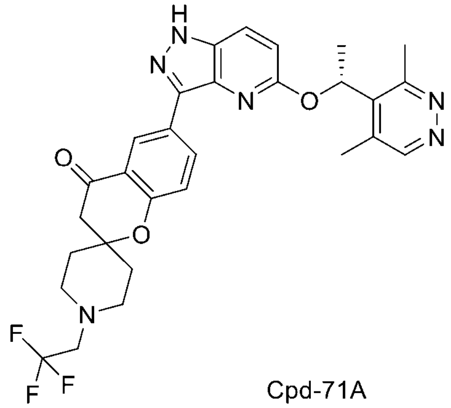

Cpd-71A ,

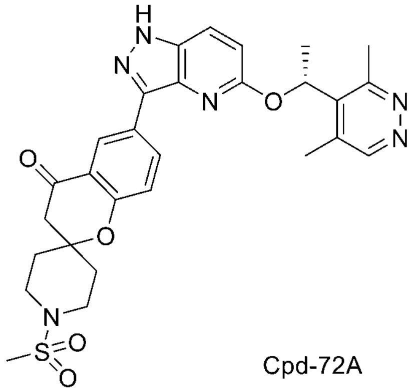

Cpd-72A ,

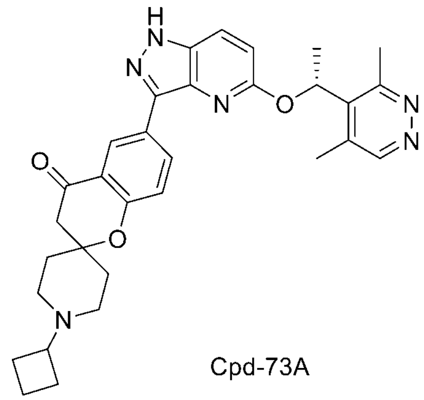

Cpd-73A ,

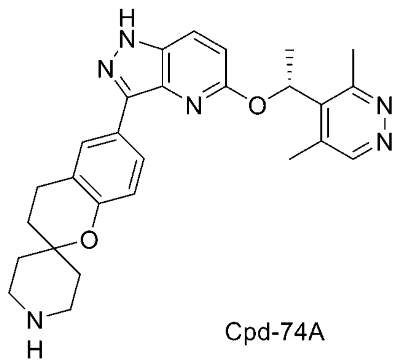

Cpd-74A ,

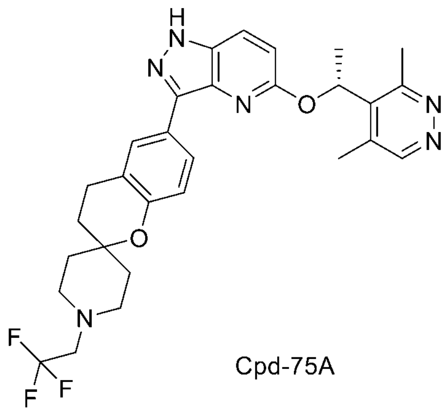

Cpd-75A ,

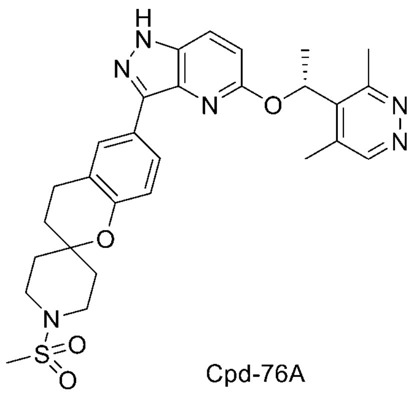

Cpd-76A ,

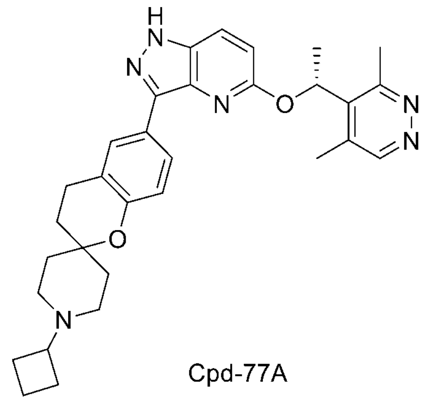

Cpd-77A ,

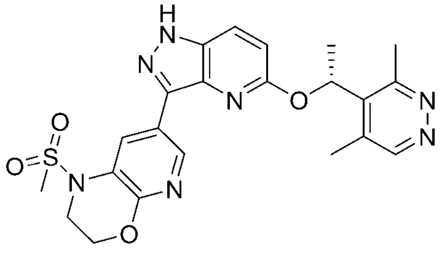
Cpd-78A ,

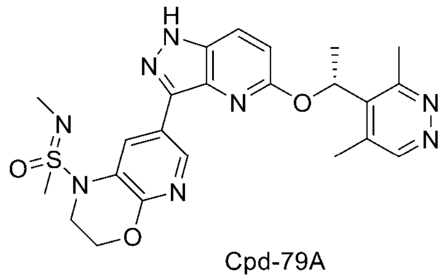
Cpd-79A ,

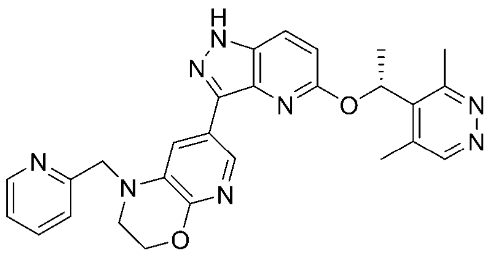
Cpd-80A ,

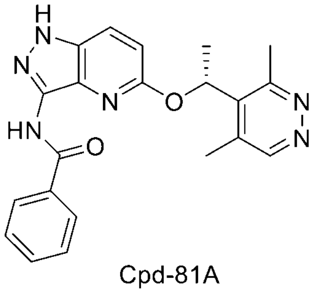
Cpd-81A ,

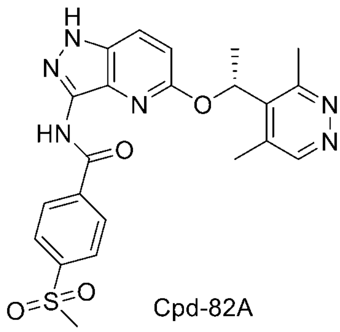
Cpd-82A ,

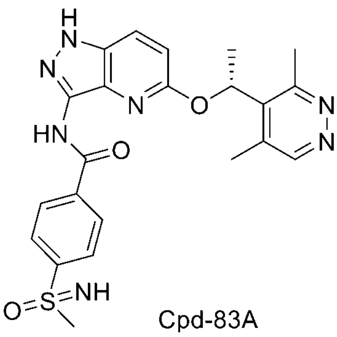
Cpd-83A ,

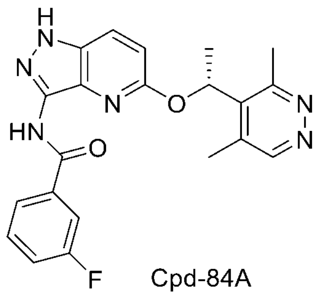
Cpd-84A ,

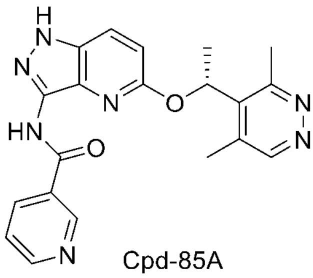
Cpd-85A ,

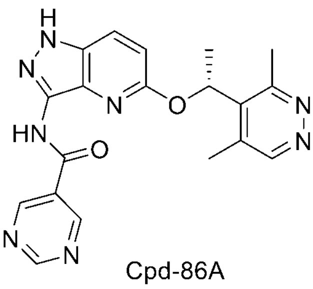
Cpd-86A ,

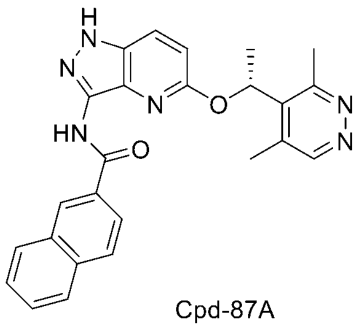
Cpd-87A ,

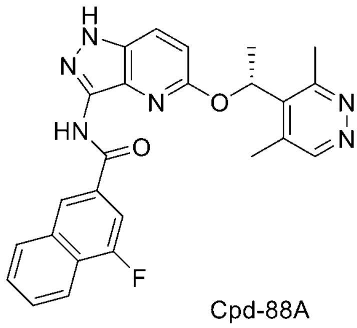
Cpd-88A ,

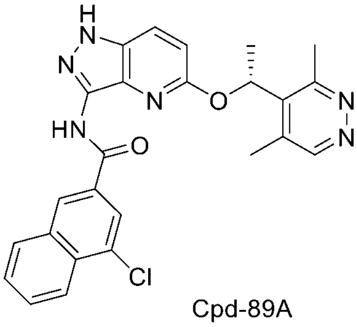

Cpd-89A ,

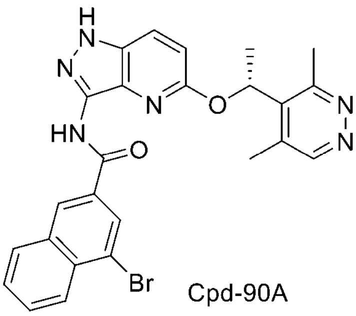

Cpd-90A ,

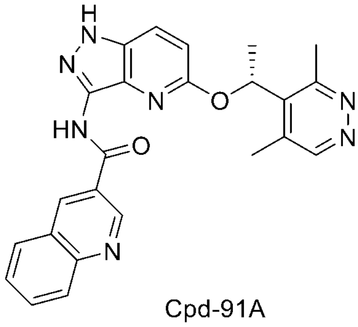

Cpd-91A ,

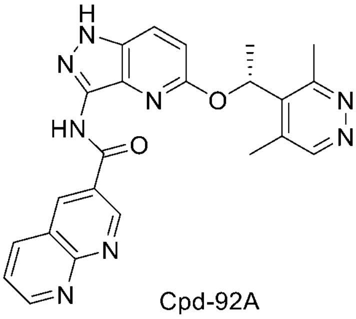

Cpd-92A ,

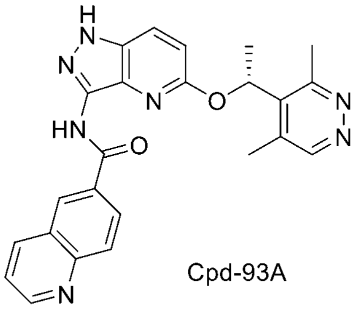

Cpd-93A ,

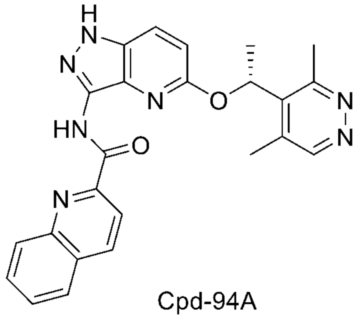

Cpd-94A ,

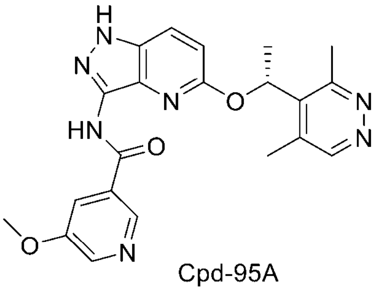

Cpd-95A ,

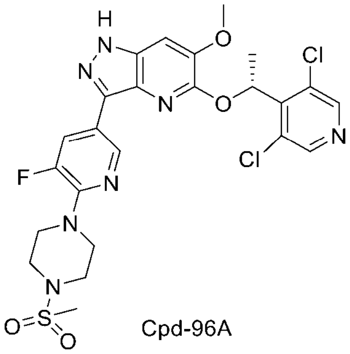

Cpd-96A ,

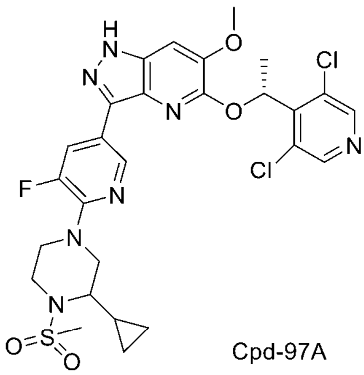

Cpd-97A ,

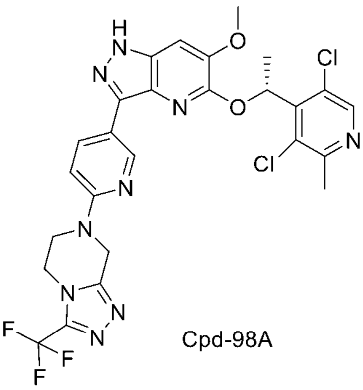

Cpd-98A ,

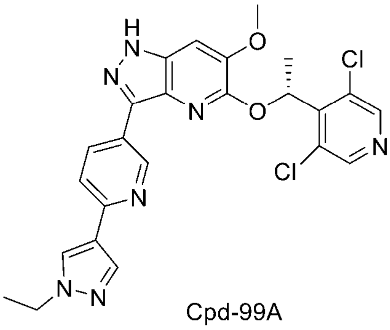

Cpd-99A ,

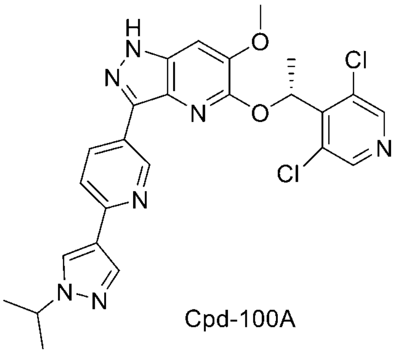

Cpd-100A ,

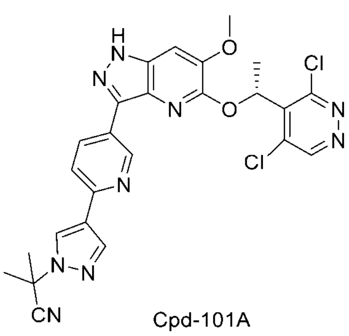
Cpd-101A ,

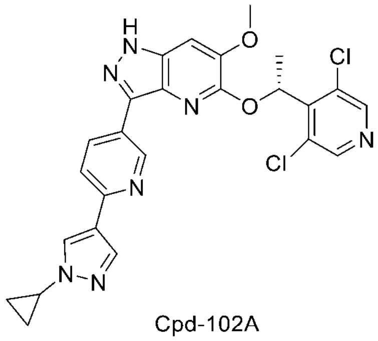
Cpd-102A ,

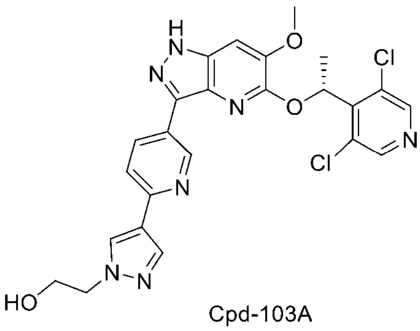
Cpd-103A ,

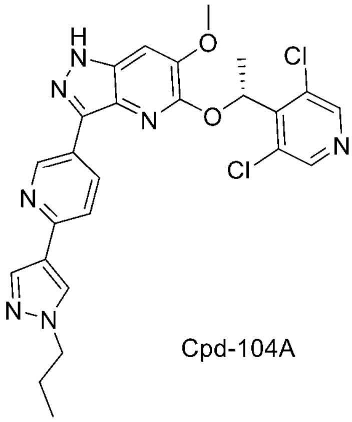
Cpd-104A ,

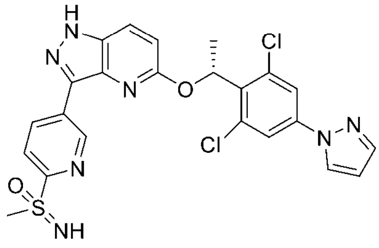
Cpd-105A ,

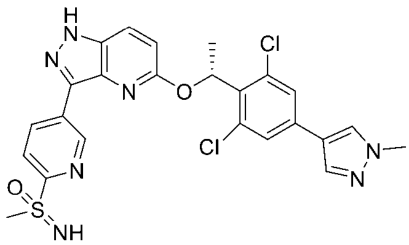
Cpd-106A ,

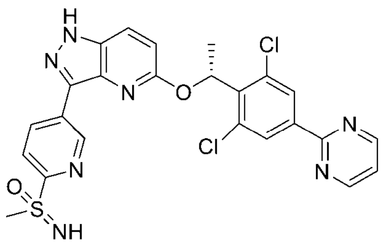
Cpd-107A ,

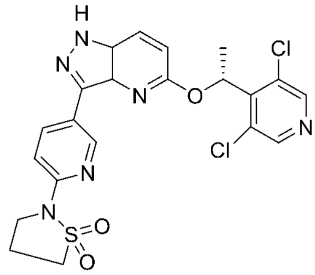
Cpd-108A ,

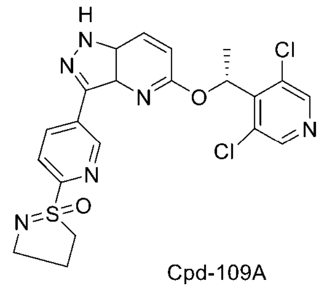
Cpd-109A ,

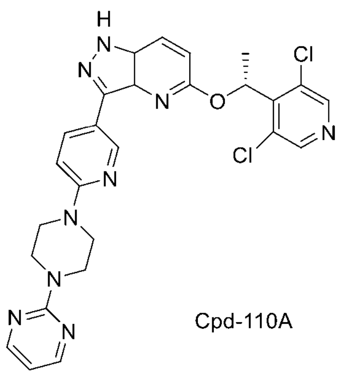
Cpd-110A .

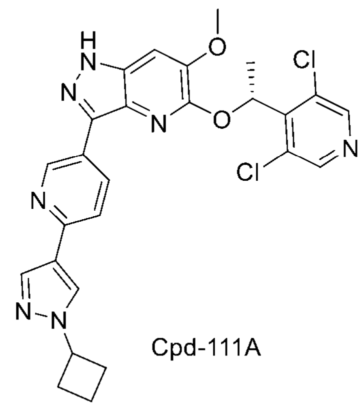
Cpd-111A .

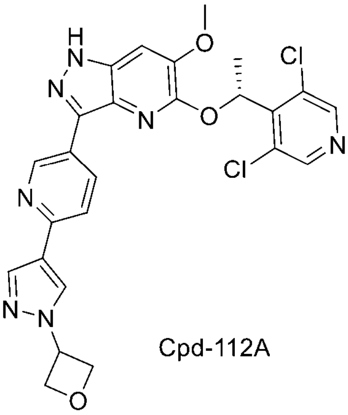
Cpd-112A .

, , ,

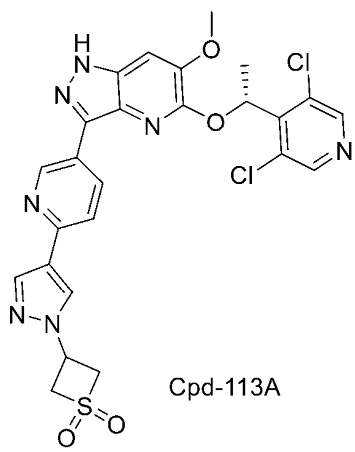

Cpd-113A ,

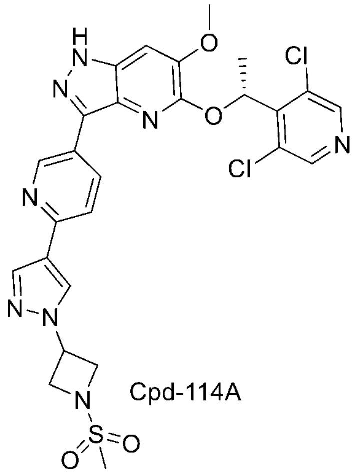

Cpd-114A ,

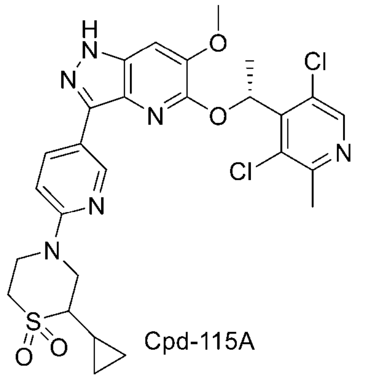

Cpd-115A ,

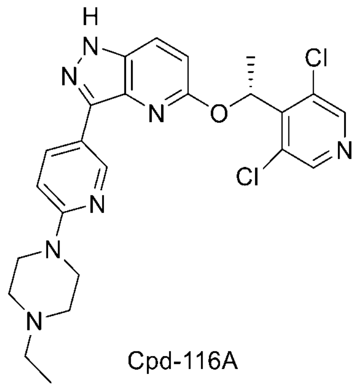

Cpd-116A ,

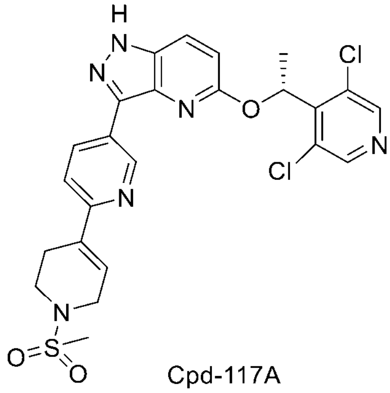

Cpd-117A ,

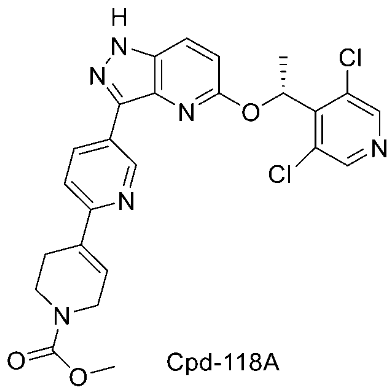

Cpd-118A ,

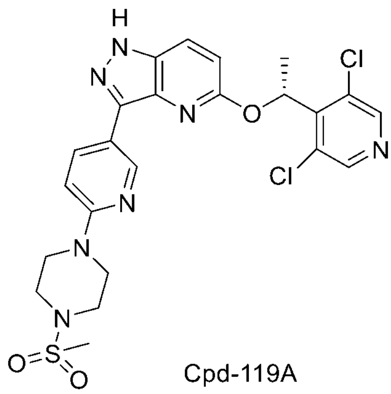

Cpd-119A ,

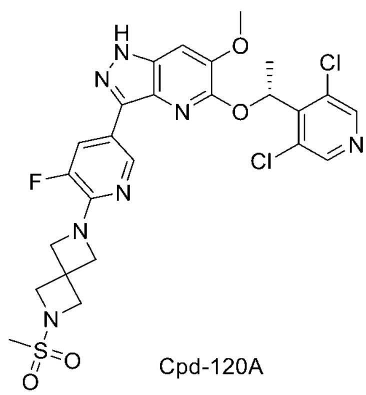

Cpd-120A ,

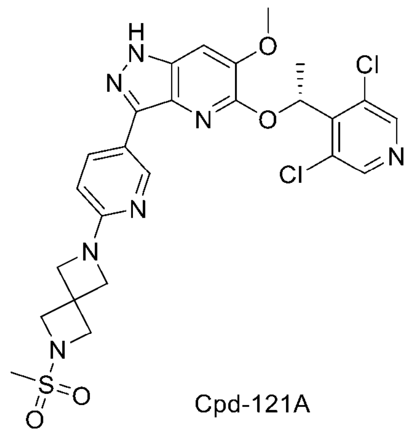

Cpd-121A ,

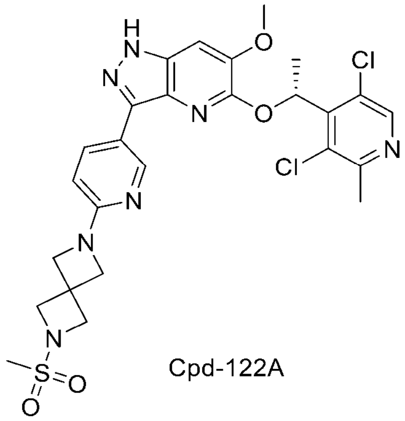

Cpd-122A ,

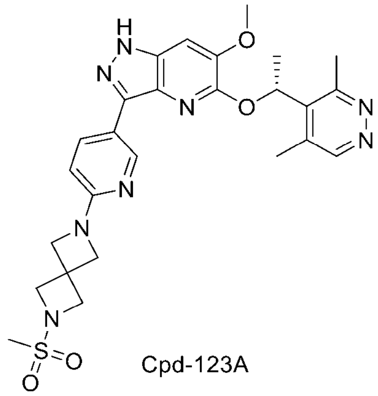

Cpd-123A ,

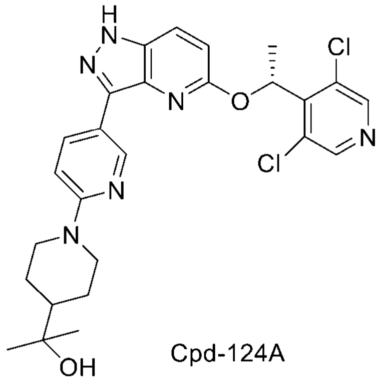

Cpd-124A ,

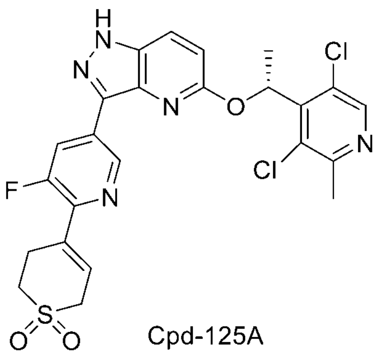

Cpd-125A ,

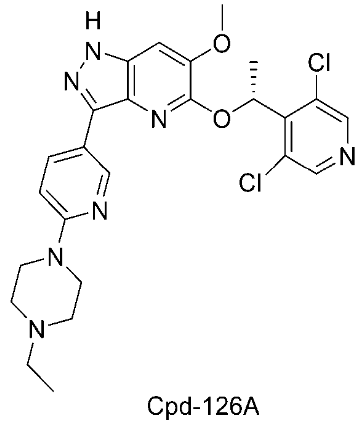

Cpd-126A ,

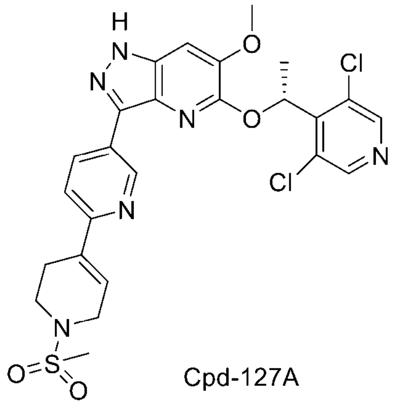

Cpd-127A ,

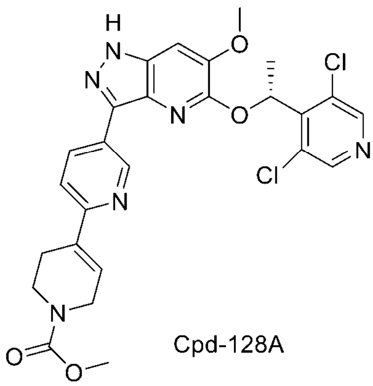

Cpd-128A ,

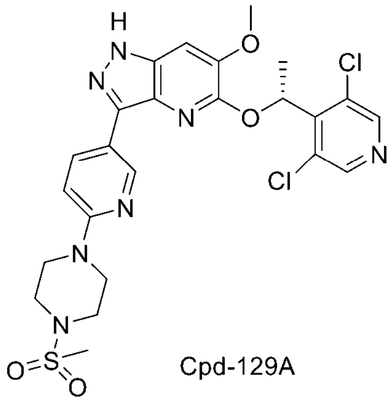

Cpd-129A ,

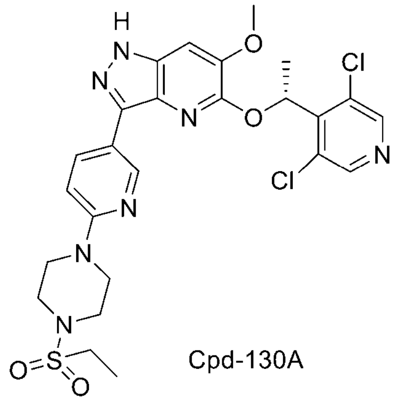

Cpd-130A ,

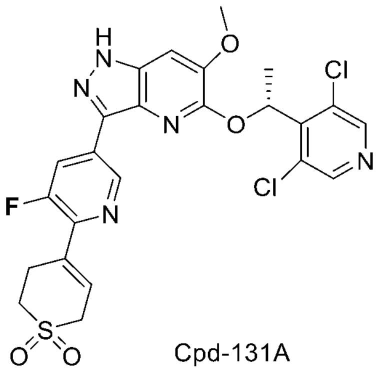
Cpd-131A ,

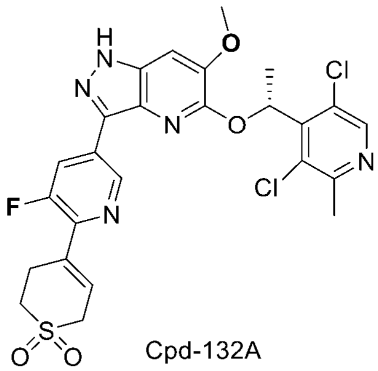
Cpd-132A ,

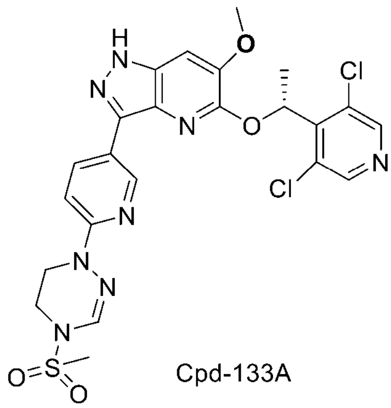
Cpd-133A ,

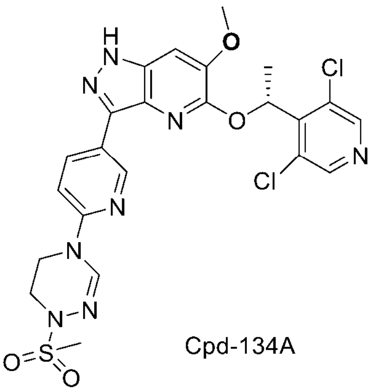
Cpd-134A ,

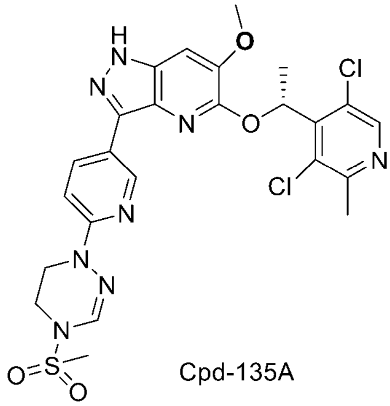
Cpd-135A ,

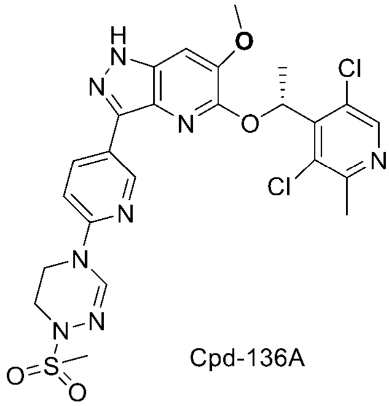
Cpd-136A ,

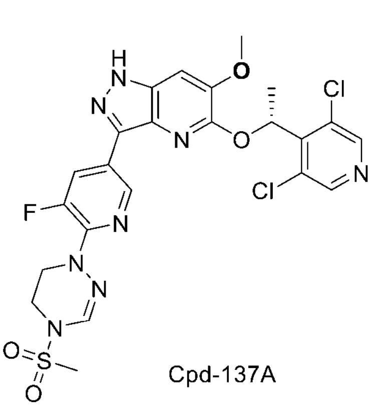
Cpd-137A ,

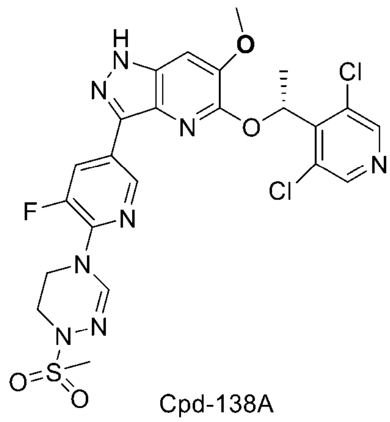
Cpd-138A ,

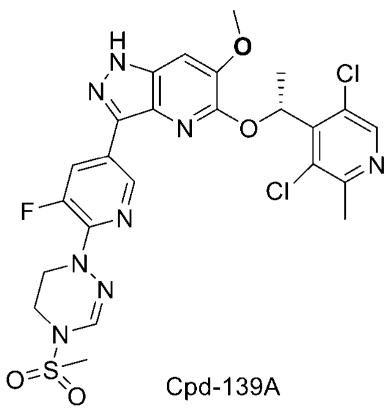
Cpd-139A ,

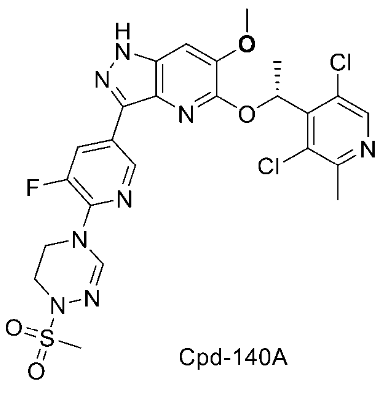
Cpd-140A ,

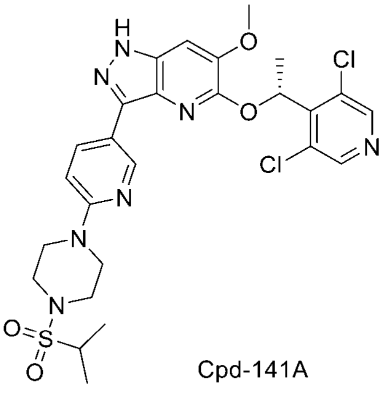
Cpd-141A ,

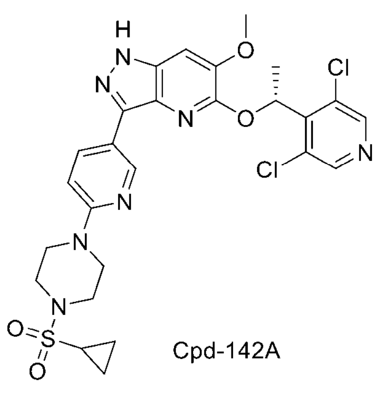
Cpd-142A ,

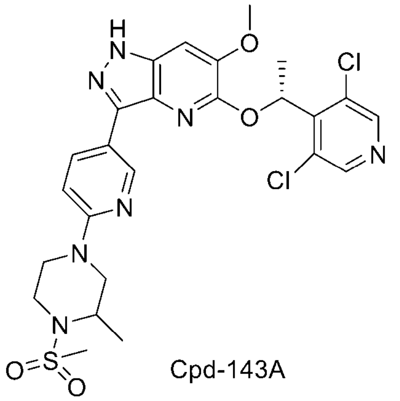

Cpd-143A

,

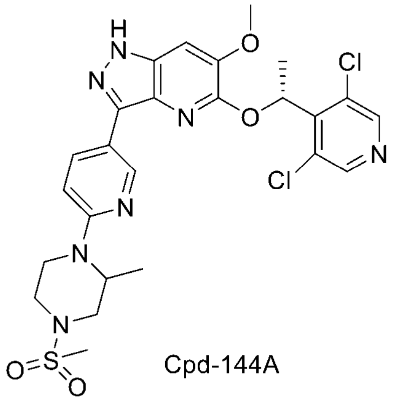

Cpd-144A

,

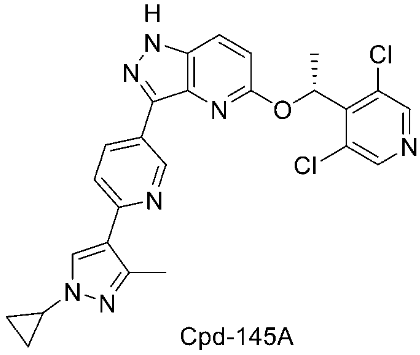

Cpd-145A

,

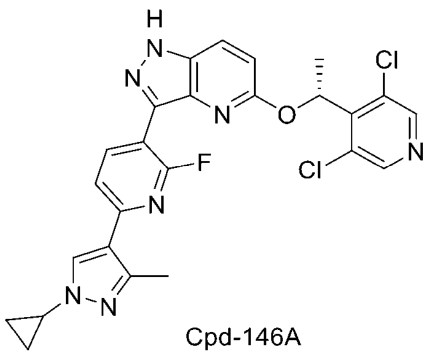

Cpd-146A

,

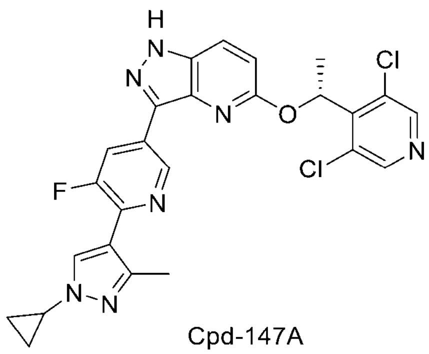

Cpd-147A

,

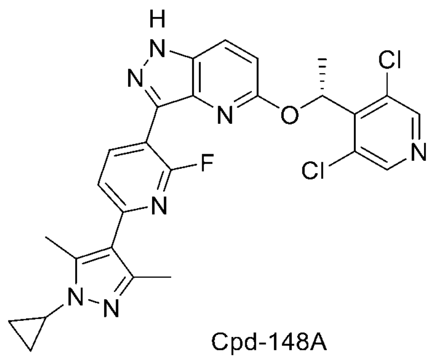

Cpd-148A

,

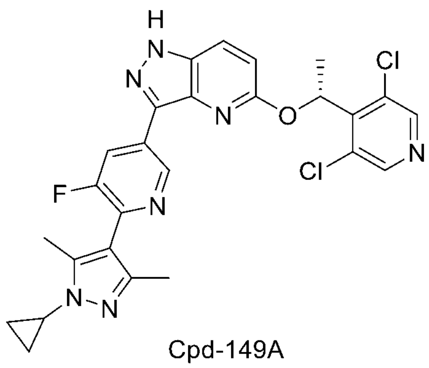

Cpd-149A

,

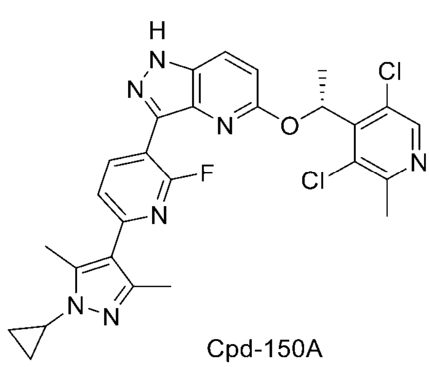

Cpd-150A

,

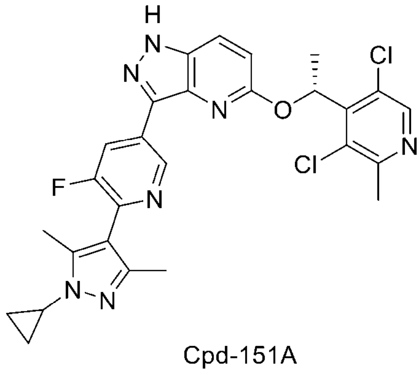

Cpd-151A ,

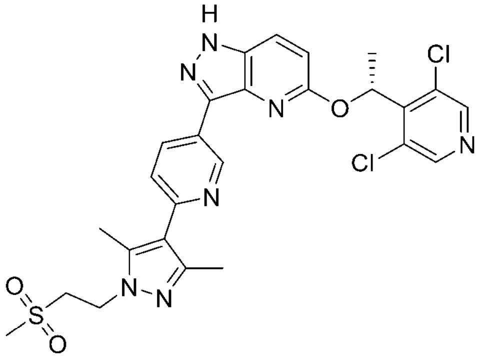

Cpd-152A ,

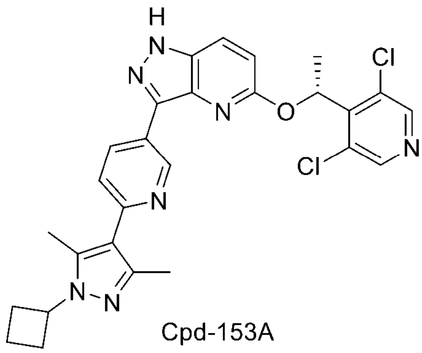

Cpd-153A ,

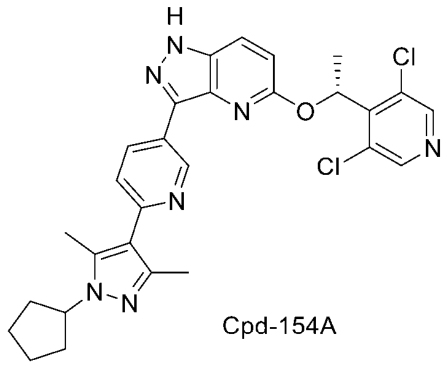

Cpd-154A ,

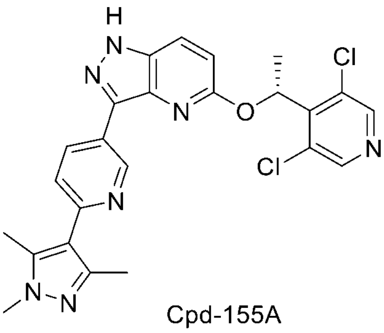

Cpd-155A ,

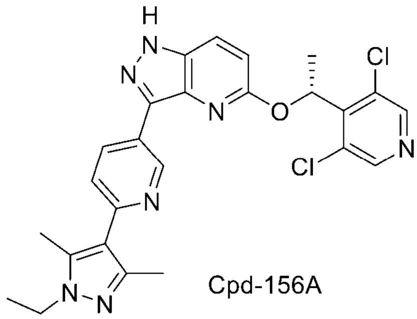

Cpd-156A ,

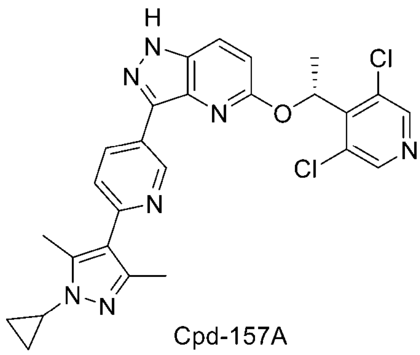

Cpd-157A ,

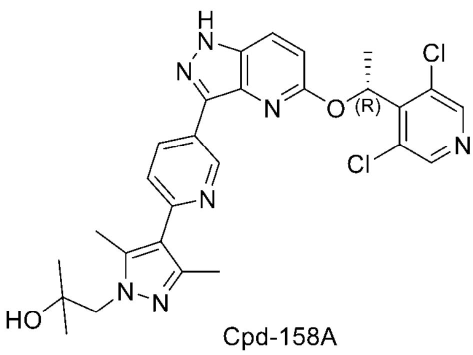

Cpd-158A ,

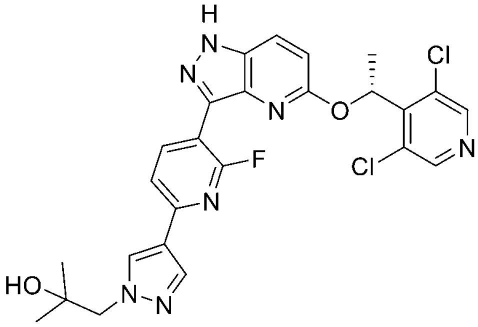

Cpd-159A ,

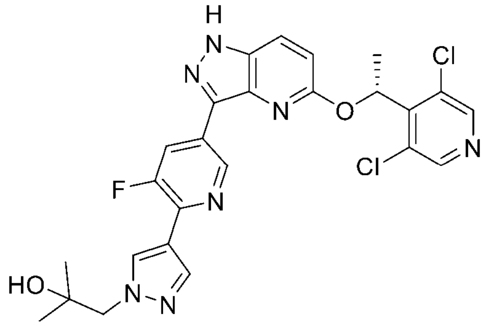

Cpd-160A ,

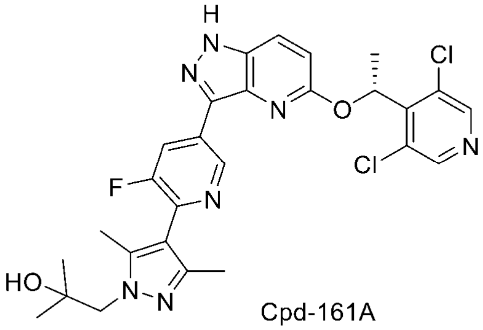

Cpd-161A ,

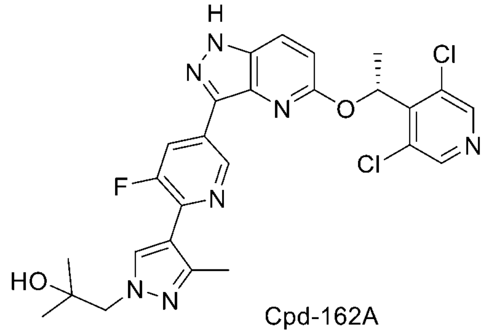

Cpd-162A ,

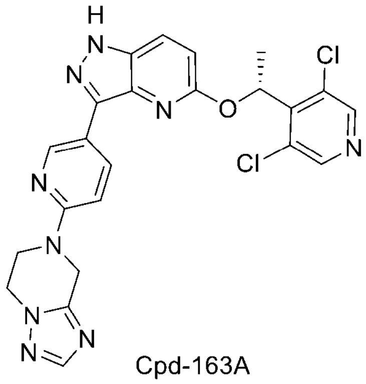

Cpd-163A ,

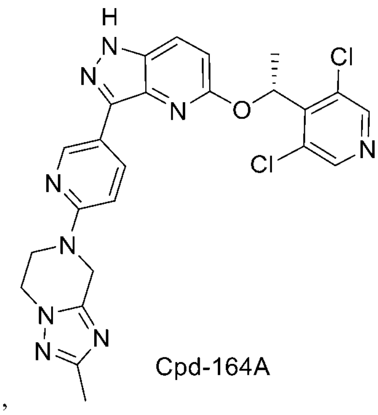

Cpd-164A ,

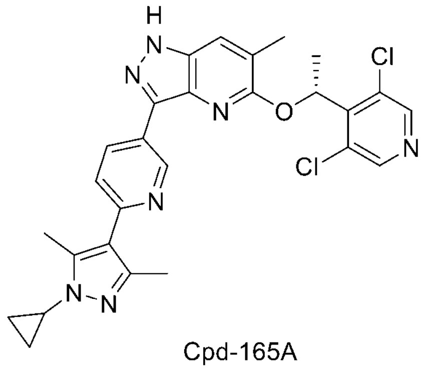

Cpd-165A ,

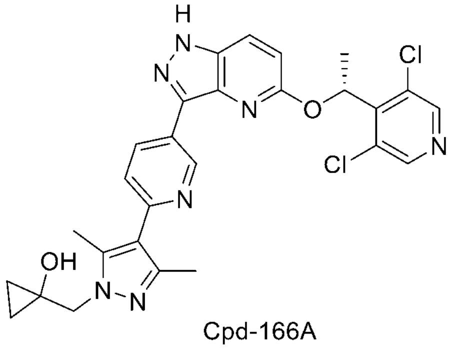

Cpd-166A ,

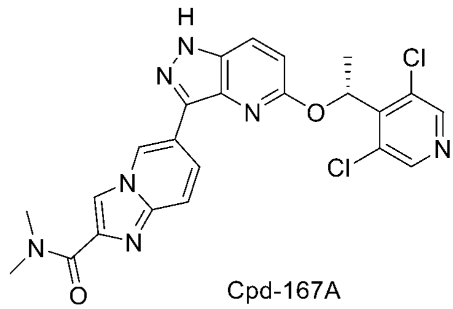

Cpd-167A ,

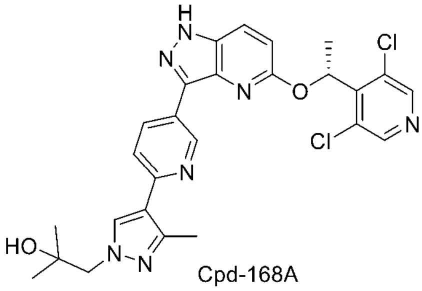
Cpd-168A ,

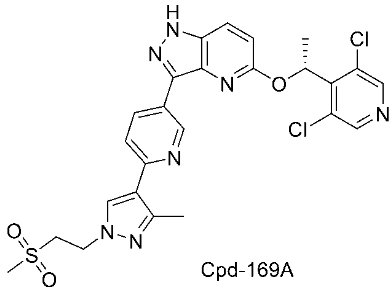
Cpd-169A ,

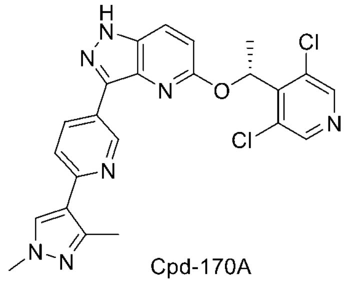
Cpd-170A ,

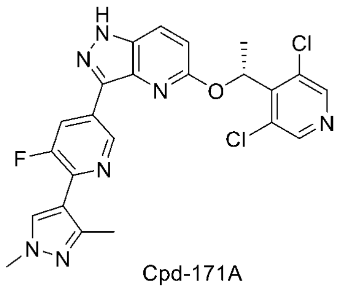
Cpd-171A ,

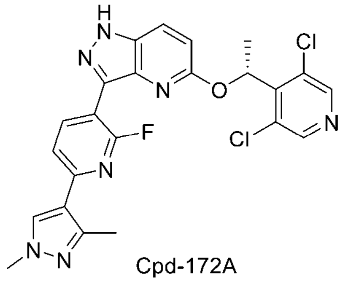
Cpd-172A ,

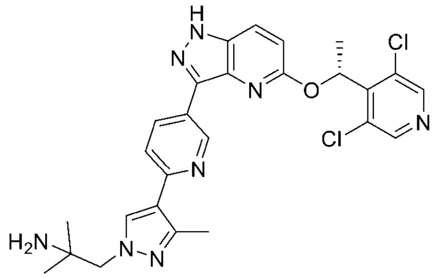
Cpd-173A ,

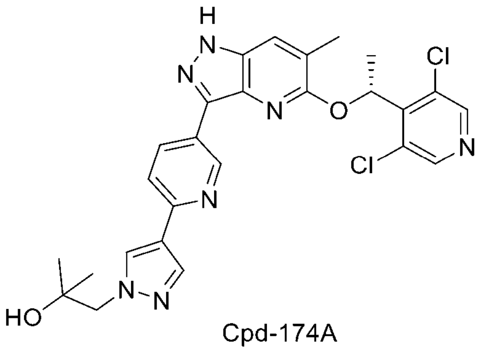
Cpd-174A ,

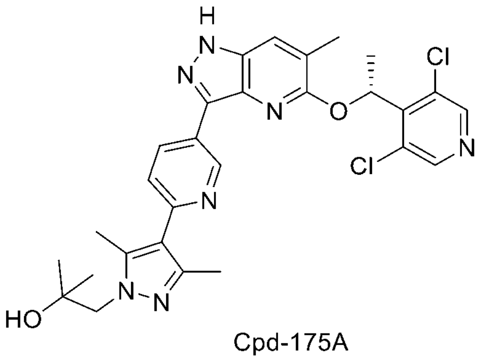
Cpd-175A ,

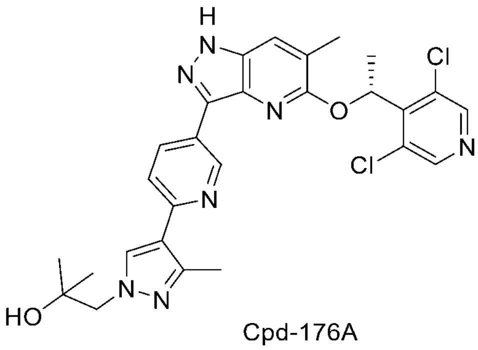
Cpd-176A ,

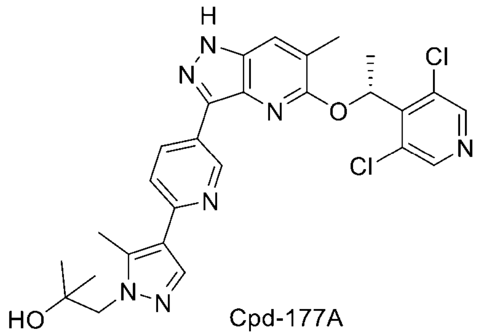
Cpd-177A ,

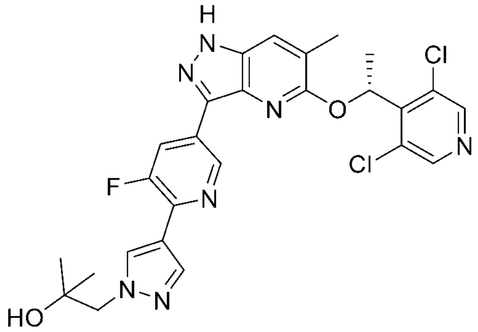

Cpd-178A ,

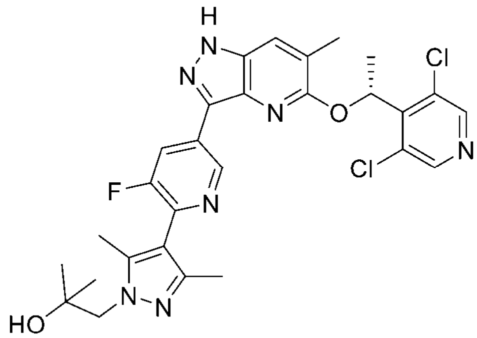

Cpd-179A ,

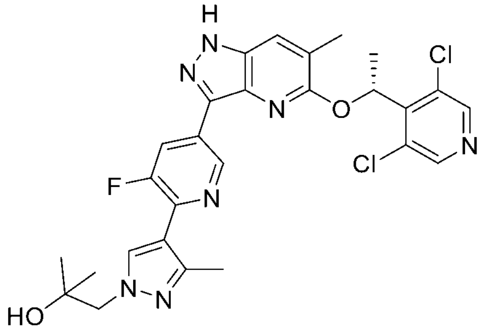

Cpd-180A ,

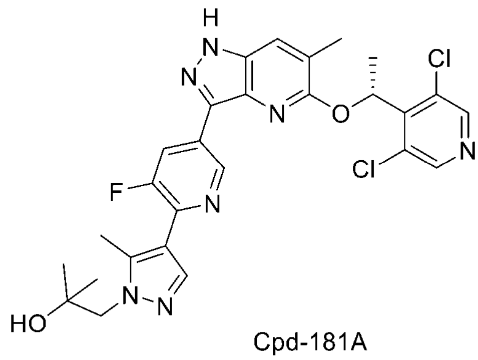

Cpd-181A ,

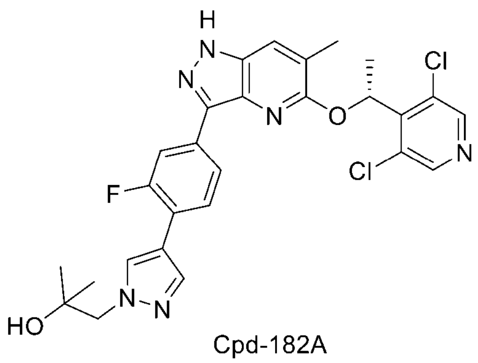

Cpd-182A ,

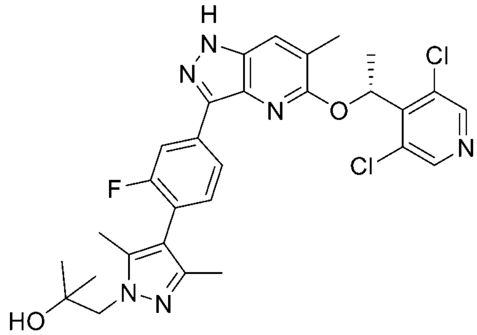

Cpd-183A ,

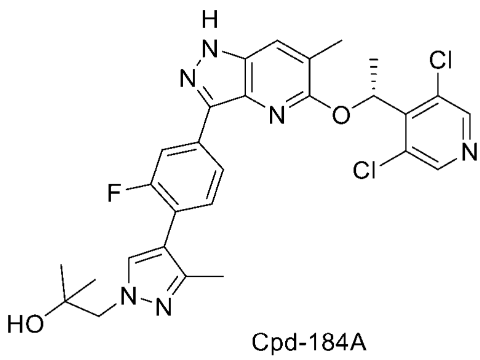

Cpd-184A ,

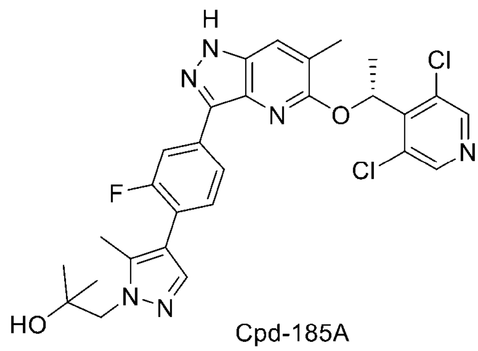

Cpd-185A ,

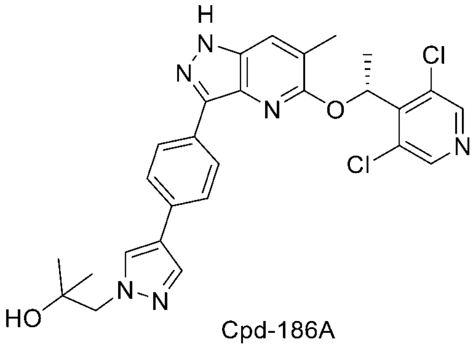
Cpd-186A ,

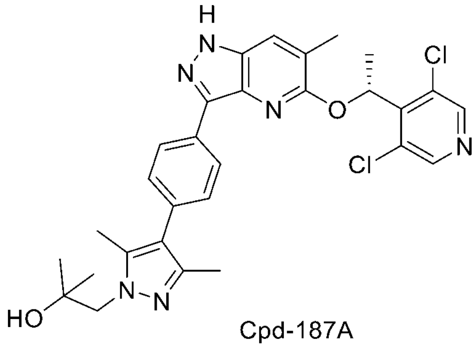
Cpd-187A ,

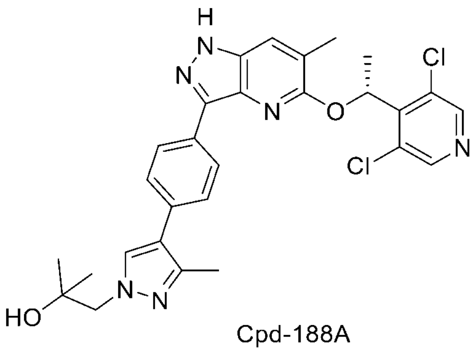
Cpd-188A ,

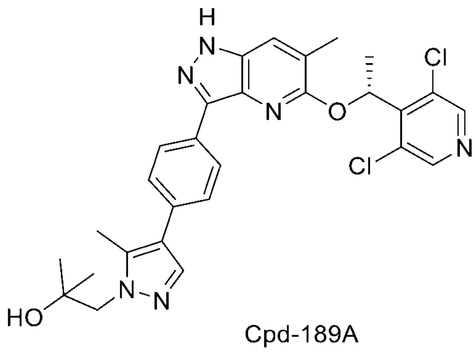
Cpd-189A ,

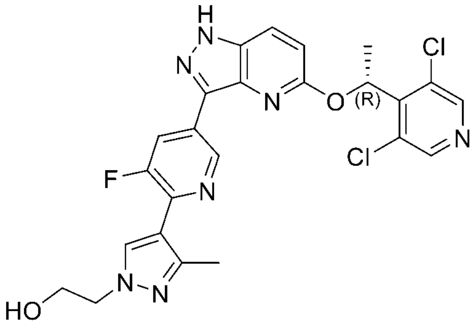
Cpd-190A ,

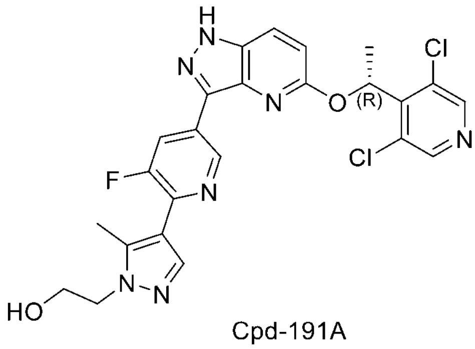
Cpd-191A ,

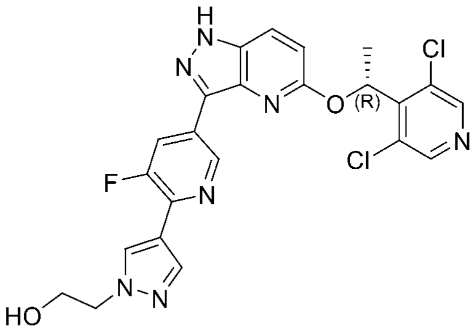
Cpd-192A ,

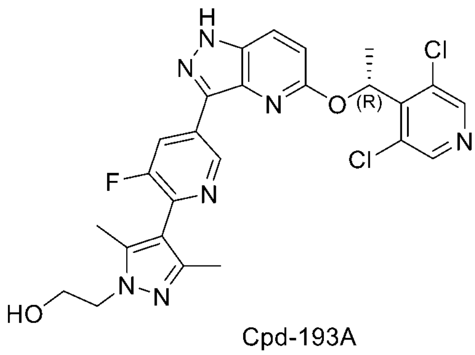
Cpd-193A ,

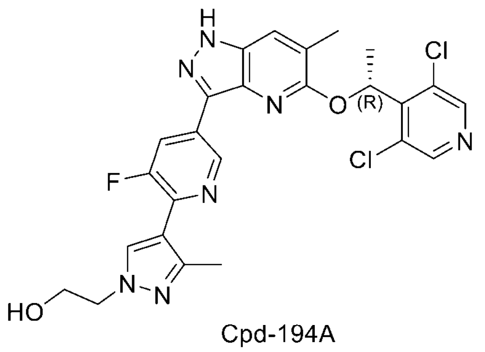

Cpd-194A ,

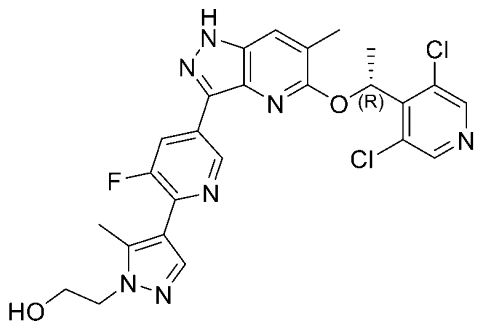

Cpd-195A ,

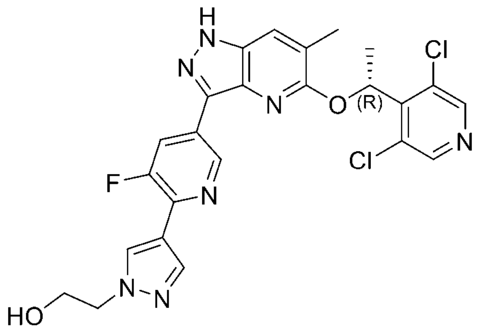

Cpd-196A ,

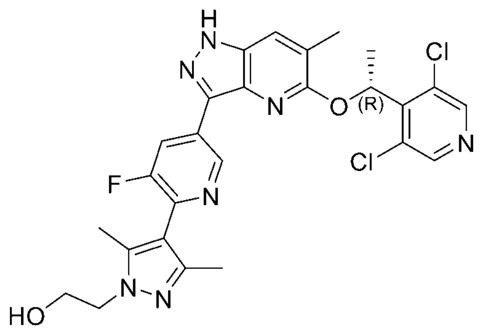

Cpd-197A ,

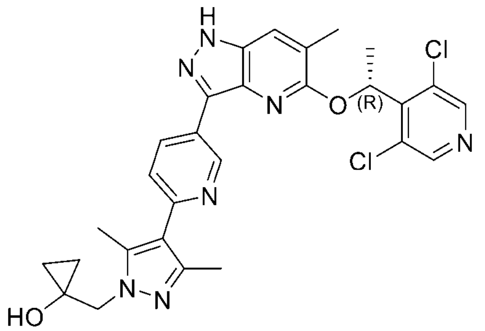

Cpd-198A ,

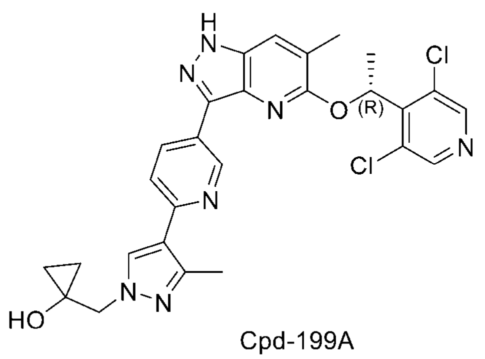

Cpd-199A ,

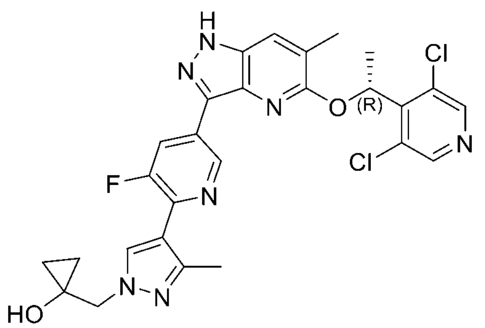

Cpd-200A ,

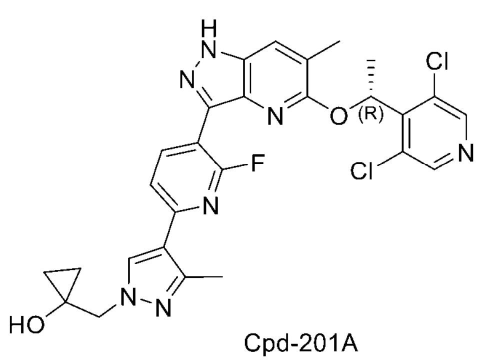

Cpd-201A ,

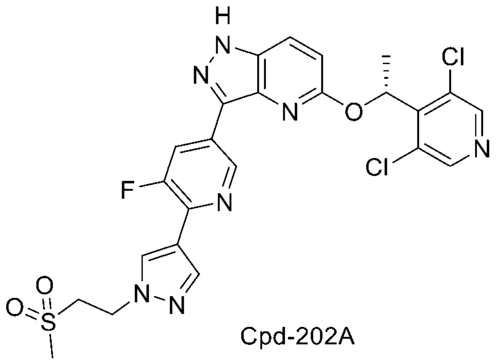

Cpd-202A ,

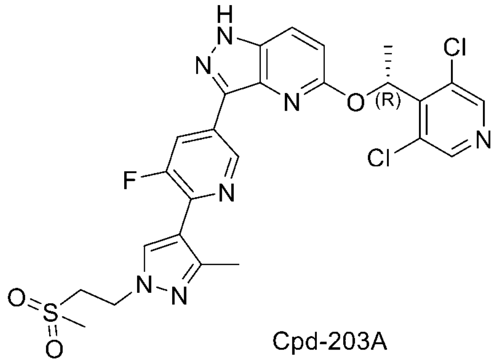

Cpd-203A ,

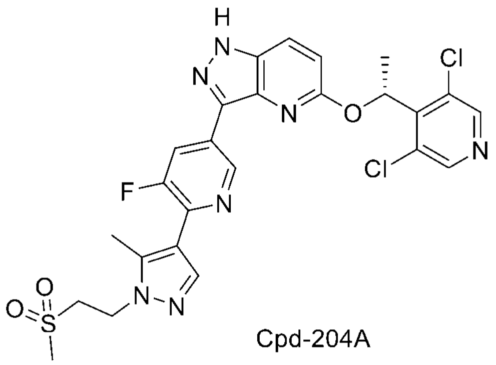

Cpd-204A ,

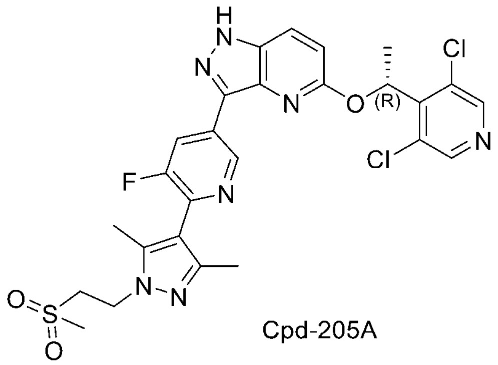

Cpd-205A ,

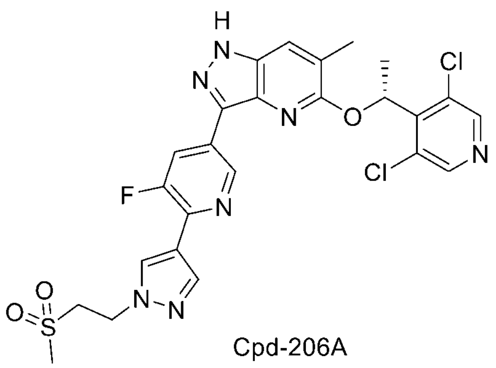

Cpd-206A ,

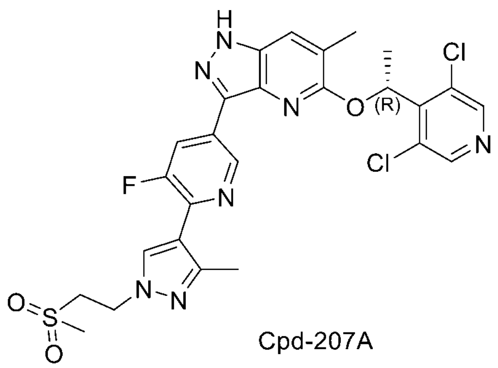

Cpd-207A ,

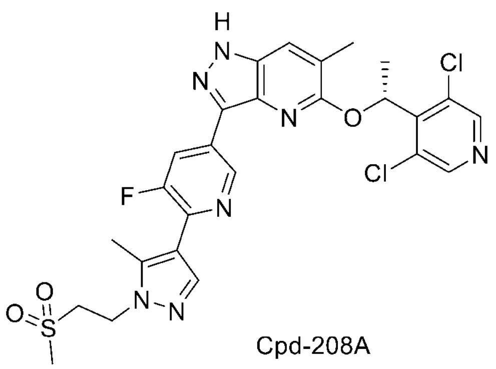

Cpd-208A ,

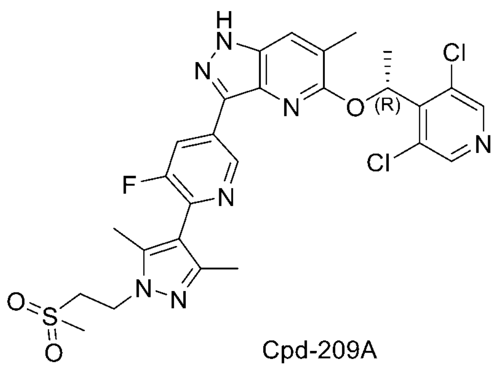

Cpd-209A ,

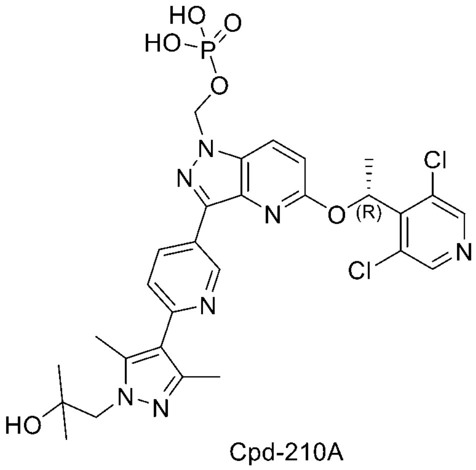

Cpd-210A ,

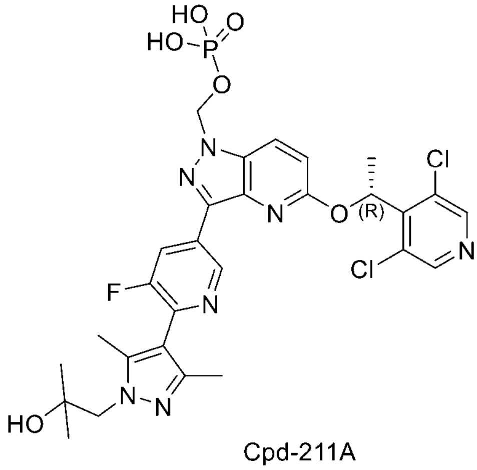

Cpd-211A ,

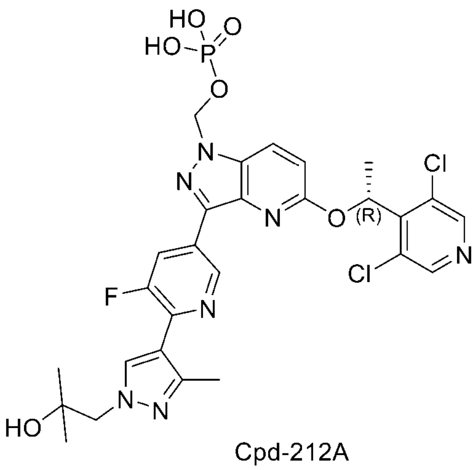

Cpd-212A ,

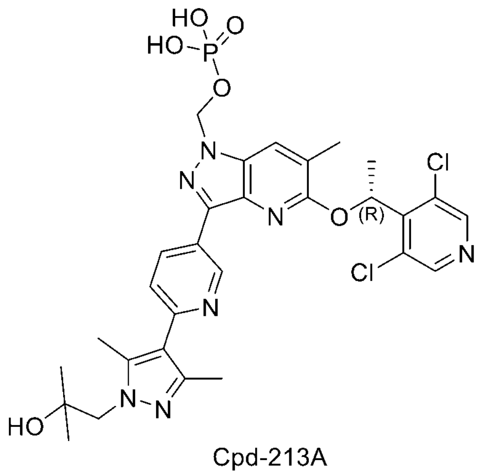

Cpd-213A ,

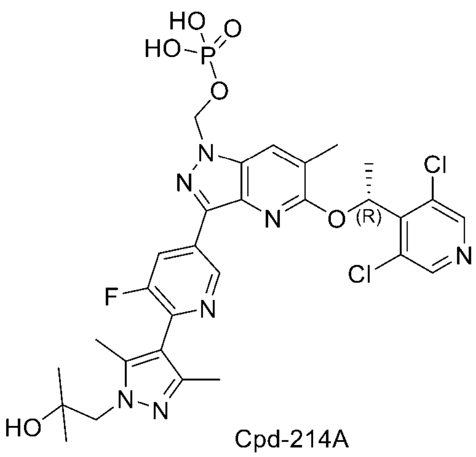

Cpd-214A ,

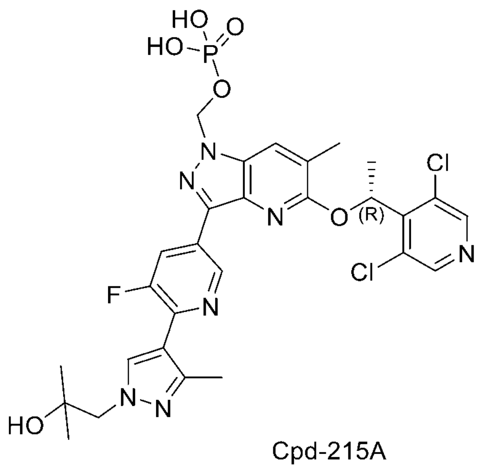

Cpd-215A ,

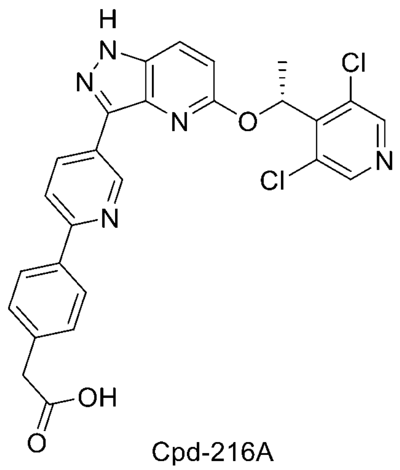
Cpd-216A ,

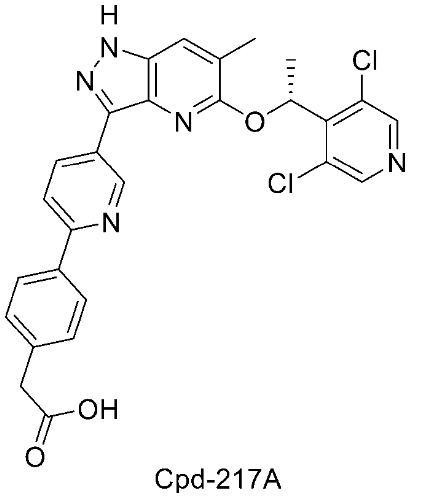
Cpd-217A ,

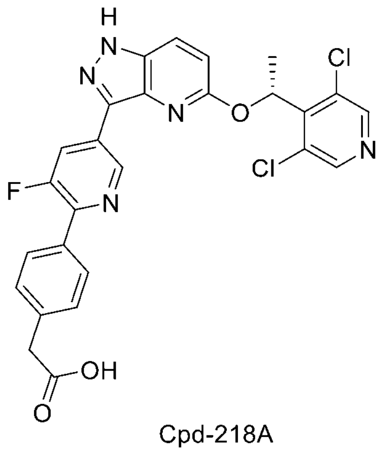
Cpd-218A ,

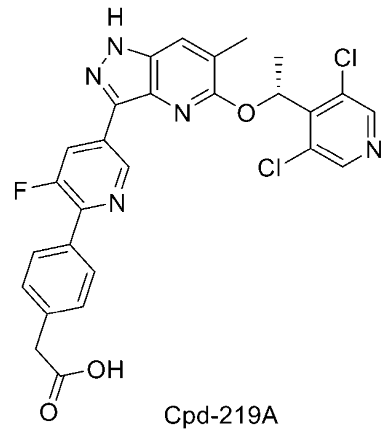
Cpd-219A ,

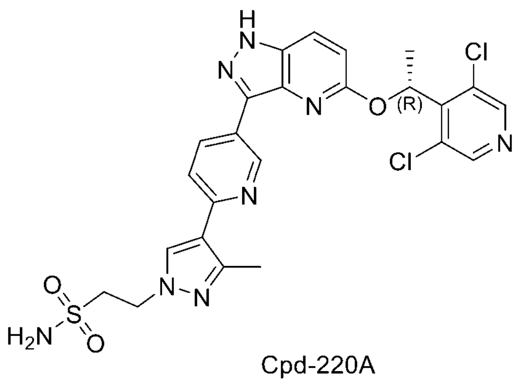
Cpd-220A ,

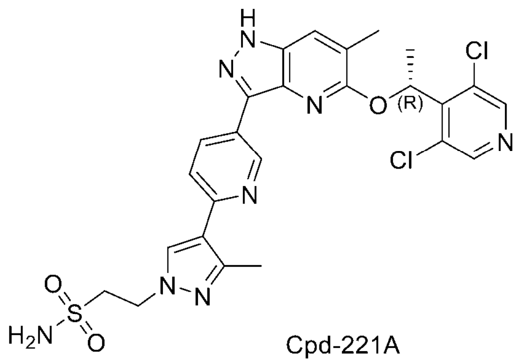
Cpd-221A ,

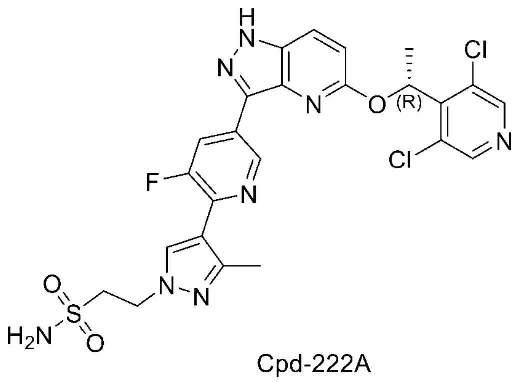

Cpd-222A ,

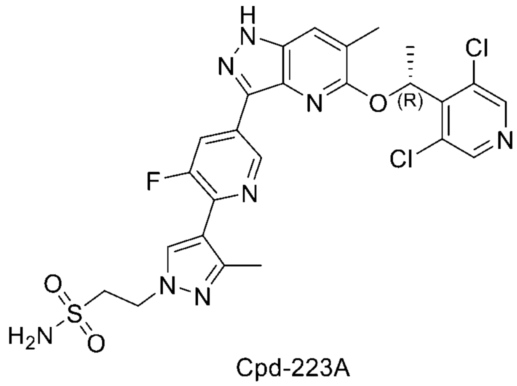

Cpd-223A ,

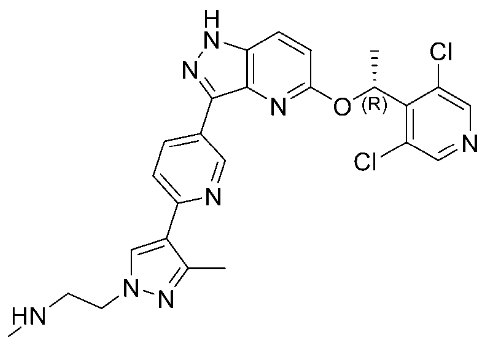

Cpd-224A ,

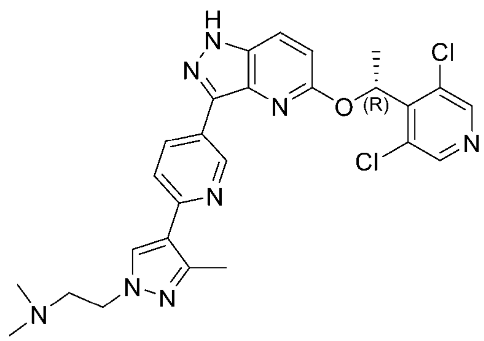

Cpd-225A ,

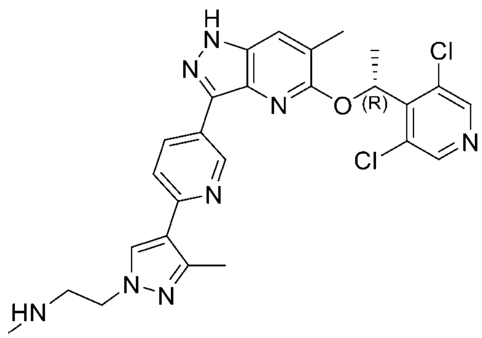

Cpd-226A ,

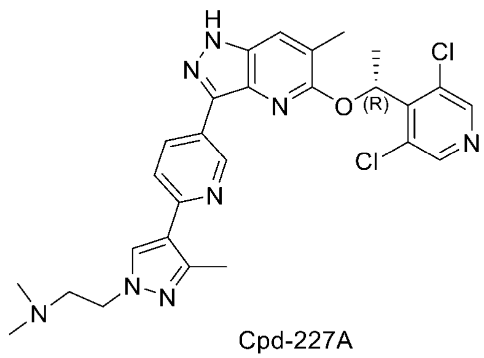

Cpd-227A ,

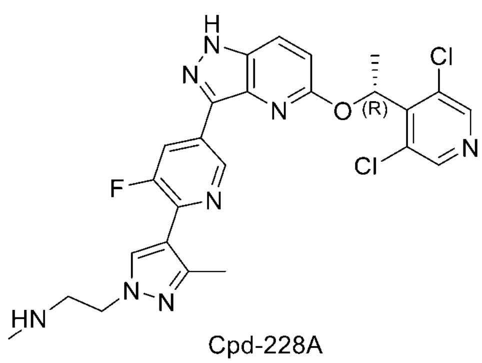

Cpd-228A ,

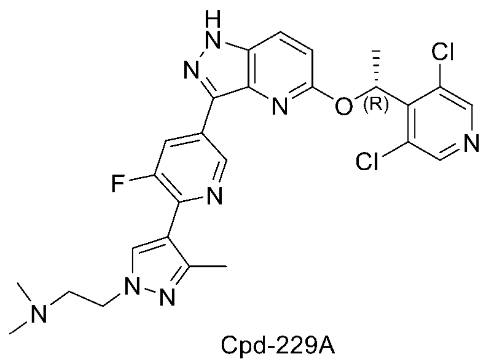

Cpd-229A ,

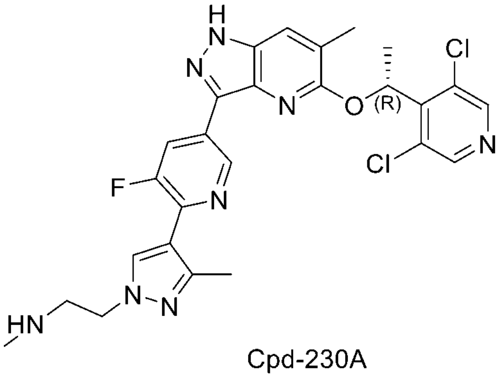

Cpd-230A ,

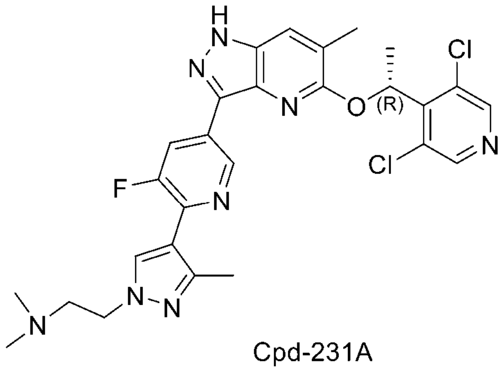

Cpd-231A .

**7.** A pharmaceutical composition, comprising a therapeutically effective amount of at least one of the compound according to any one of claims 1 to 6, and a racemate, a stereoisomer, a tautomer, an isotopically labeled compound, an N-oxide, a solvate, a polymorph, a metabolite, an ester, a prodrug or a pharmaceutically acceptable salt thereof.

**8.** Use of at least one of the compound according to any one of claims 1 to 6, and a racemate, a stereoisomer, a tautomer, an isotopically labeled compound, an N-oxide, a solvate, a polymorph, a metabolite, an ester, a prodrug or a pharmaceutically acceptable salt thereof or the pharmaceutical composition according to claim 7 in the preparation of a drug, wherein

preferably, the drug is a FGFR2 and/or FGFR3 inhibitor; and
preferably, the drug is used for diagnosing, preventing and/or treating FGFR-related diseases or disorders.

**9.** A method for diagnosing, preventing and/or treating FGFR-related diseases or disorders, comprising administering, to a patient in need of such treatment, an effective amount of at least one of the compound according to any one of claims 1 to 6 or the pharmaceutical composition according to claim 7 alone or optionally in combination with at least one therapeutic agent of other type.

**10.** The use according to claim 8 or the method according to claim 9, wherein the FGFR-related diseases or disorders are selected from cancers, chondrodysplasia or achondroplasia, and preferably, the cancers are selected from bladder cancer, brain cancer, breast cancer, cholangiocarcinoma, head and neck cancer, lung cancer, multiple myeloma, rhabdomyosarcoma, urethral carcinoma, or uterine cancer.

**INTERNATIONAL SEARCH REPORT**

International application No.
**PCT/CN2024/094997**

**A. CLASSIFICATION OF SUBJECT MATTER**

C07D471/04(2006.01)i; A61K31/437(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D; A61K; A61P

Documentation searched other than minimum documentation to the extent that such documents are included in the fields searched

Electronic data base consulted during the international search (name of data base and, where practicable, search terms used)

CNTXT, ENTXTC, DWPI, CNKI, CAPLUS(STN), REGISTRY(STN): 吡唑并吡啶, 吡啶, 哒嗪, FGFR抑制剂, 癌, pyrazolopyridine, pyridine, pyridazine, FGFR inhibitor, cancer, tumor, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| X | CN 105906621 A (SICHUAN BAILI PHARMACEUTICAL CO., LTD.) 31 August 2016 (2016-08-31)<br>description, paragraphs [0006]-[0019], and claims 1-10 | 1-10 |
| X | WO 2021088859 A1 (JINAN UNIVERSITY) 14 May 2021 (2021-05-14)<br>description, embodiment 117, and claims 1-30 | 1-5, 7-8 |
| X | WO 2017220431 A1 (ALMIRALL, S.A.) 28 December 2017 (2017-12-28)<br>see description, embodiments 1-83, and claims 1-23 | 1-5, 7-8 |
| X | CN 104684554 A (PIERRE FABRE MÉDICAMENT) 03 June 2015 (2015-06-03)<br>claims 1-16 | 1-5, 7-8 |
| X | WO 2014016434 A1 (PIERRE FABRE MEDICAMENT) 30 January 2014 (2014-01-30)<br>claims 1-17 | 1-5, 7-8 |
| PX | CN 116693519 A (SHANGHAI YINGLI PHARMACEUTICAL CO., LTD.) 05 September 2023 (2023-09-05)<br>description, embodiment 1 | 1-5 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **23 July 2024** | **20 August 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/094997**

**C. DOCUMENTS CONSIDERED TO BE RELEVANT**

| Category* | Citation of document, with indication, where appropriate, of the relevant passages | Relevant to claim No. |
|---|---|---|
| E | WO 2024140990 A1 (JIANGSU YAHONG MEDITECH CO., LTD. et al.) 04 July 2024 (2024-07-04)<br>claims 16 and 20-22 | 1-5, 7-10 |
| A | WO 2023279041 A1 (TYRA BIOSCIENCES, INC.) 05 January 2023 (2023-01-05)<br>entire description | 1-10 |
| A | CN 115835908 A (INCYTE CORPORATION) 21 March 2023 (2023-03-21)<br>entire description | 1-10 |

Form PCT/ISA/210 (second sheet) (July 2022)

INTERNATIONAL SEARCH REPORT

International application No.
**PCT/CN2024/094997**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **9-10**
because they relate to subject matter not required to be searched by this Authority, namely:
Claims 9-10 relate to a method for diagnosing, preventing and/or treating a disease. The search is carried out on the basis of the use of the compound in the preparation of a drug for diagnosing, preventing and/or treating related diseases.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/094997**

| Patent document cited in search report | | | Publication date (day/month/year) | Patent family member(s) | | | Publication date (day/month/year) |
|---|---|---|---|---|---|---|---|
| CN | 105906621 | A | 31 August 2016 | None | | | |
| WO | 2021088859 | A1 | 14 May 2021 | EP | 4056558 | A1 | 14 September 2022 |
| | | | | EP | 4056558 | A4 | 27 December 2023 |
| | | | | US | 2022281821 | A1 | 08 September 2022 |
| WO | 2017220431 | A1 | 28 December 2017 | None | | | |
| CN | 104684554 | A | 03 June 2015 | JP | 2015522650 | A | 06 August 2015 |
| | | | | JP | 6456823 | B2 | 23 January 2019 |
| | | | | ES | 2612349 | T3 | 16 May 2017 |
| | | | | JP | 2019031542 | A | 28 February 2019 |
| | | | | JP | 6732855 | B2 | 29 July 2020 |
| | | | | BR | 112015001502 | A2 | 04 July 2017 |
| | | | | BR | 112015001502 | B1 | 07 March 2023 |
| | | | | ZA | 201501209 | B | 30 March 2016 |
| | | | | HUE | 032919 | T2 | 28 November 2017 |
| | | | | MY | 183398 | A | 18 February 2021 |
| | | | | CY | 1118478 | T1 | 12 July 2017 |
| | | | | UA | 116107 | C2 | 12 February 2018 |
| | | | | DK | 2877177 | T3 | 06 February 2017 |
| | | | | HK | 1207302 | A1 | 29 January 2016 |
| | | | | PL | 2877177 | T3 | 28 April 2017 |
| | | | | LT | 2877177 | T | 25 January 2017 |
| | | | | SI | 2877177 | T1 | 28 February 2017 |
| | | | | PT | 2877177 | T | 06 February 2017 |
| | | | | TN | 2015000034 | A1 | 29 June 2016 |
| | | | | MA | 37866 | A1 | 31 May 2016 |
| | | | | MA | 37866 | B1 | 30 December 2016 |
| | | | | KR | 20150038295 | A | 08 April 2015 |
| | | | | KR | 102120505 | B1 | 08 June 2020 |
| | | | | WO | 2014016433 | A1 | 30 January 2014 |
| | | | | US | 2015190394 | A1 | 09 July 2015 |
| | | | | US | 9381195 | B2 | 05 July 2016 |
| | | | | RS | 55646 | B1 | 30 June 2017 |
| | | | | MX | 2015001009 | A | 04 June 2015 |
| | | | | MX | 363605 | B | 26 March 2019 |
| | | | | HRP | 20170104 | T1 | 24 March 2017 |
| | | | | IL | 236917 | A | 30 November 2016 |
| | | | | RU | 2015106434 | A | 20 September 2016 |
| | | | | RU | 2640046 | C2 | 26 December 2017 |
| | | | | EP | 2689778 | A1 | 29 January 2014 |
| | | | | AU | 2013294920 | A1 | 12 March 2015 |
| | | | | AU | 2013294920 | B2 | 24 August 2017 |
| | | | | NZ | 705253 | A | 26 May 2017 |
| | | | | CA | 2879595 | A1 | 30 January 2014 |
| | | | | CA | 2879595 | C | 28 July 2020 |
| | | | | EP | 2877177 | A1 | 03 June 2015 |
| | | | | EP | 2877177 | B1 | 26 October 2016 |
| WO | 2014016434 | A1 | 30 January 2014 | None | | | |
| CN | 116693519 | A | 05 September 2023 | TW | 202346290 | A | 01 December 2023 |
| | | | | WO | 2023165571 | A1 | 07 September 2023 |

Form PCT/ISA/210 (patent family annex) (July 2022)

**INTERNATIONAL SEARCH REPORT**
**Information on patent family members**

International application No.
**PCT/CN2024/094997**

| Patent document cited in search report | Publication date (day/month/year) | Patent family member(s) | Publication date (day/month/year) |
|---|---|---|---|
| | | US 2023278998 A1 | 07 September 2023 |
| WO 2024140990 A1 | 04 July 2024 | None | |
| WO 2023279041 A1 | 05 January 2023 | None | |
| CN 115835908 A | 21 March 2023 | ECSP 22029777 A | 31 August 2022 |
| | | TW 202128685 A | 01 August 2021 |
| | | CR 20220169 A | 27 October 2022 |
| | | CA 3157361 A1 | 22 April 2021 |
| | | US 2021106588 A1 | 15 April 2021 |
| | | US 11607416 B2 | 21 March 2023 |
| | | IL 291901 A | 01 June 2022 |
| | | JP 2022552324 A | 15 December 2022 |
| | | EP 4045151 A1 | 24 August 2022 |
| | | CL 2022000923 A1 | 28 October 2022 |
| | | PE 20221085 A1 | 05 July 2022 |
| | | MX 2022004513 A | 19 July 2022 |
| | | CO 2022004694 A2 | 10 June 2022 |
| | | JOP 20220083 A1 | 30 January 2023 |
| | | DOP 2022000082 A | 30 September 2022 |
| | | AU 2020366006 A1 | 21 April 2022 |
| | | WO 2021076602 A1 | 22 April 2021 |
| | | US 2023338389 A1 | 26 October 2023 |
| | | BR 112022007163 A2 | 23 August 2022 |
| | | KR 20220100879 A | 18 July 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310593058
- CN 202311019921
- CN 202311275317
- CN 202311767590


### Non-patent literature cited in the description


- **TURNER, N.** ; **GROSE, R.** *Nat. Ref. Cancer*, 2010, vol. 10, 116-129
- **BROOKS, N.S. et al.** *Clin Cancer Res.*, 2012, vol. 18, 18551862
- **DIENSTMANN, R. et al.** *Ann. Oncol.*, 2014, vol. 25, 552-563
- **HELSTEN T** ; **ELKIN S** ; **ARTHUR E** ; **TOMSON BN** ; **CARTER J** ; **KURZROCK R.** The FGFR Landscape in Cancer: Analysis of 4,853 Tumors by Next-Generation Sequencing. *Clin Cancer Res.*, 2016, vol. 22 (1), 259-267
- **MARSEGLIA G** ; **LODOLA A** ; **MOR M**. *Castelli R. Expert Opin Ther Pat.*, December 2019, vol. 29 (12), 965-977
- **KOMMALAPATI A** ; **TELLA SH** ; **BORAD M** ; **JAVLE M** ; **MAHIPAL A.** *Cancers (Basel).*, 13 June 2021, vol. 13 (12), 2968